(19) Europäisches Patentamt

European Patent Office

Office européen des brevets

(11) **EP 4 678 184 A2**

(12) **EUROPEAN PATENT APPLICATION**

(43) Date of publication:
14.01.2026 Bulletin 2026/03

(21) Application number: 25219601.9

(22) Date of filing: 30.04.2020

(51) International Patent Classification (IPC):
*A61K 39/00* (2006.01)

(52) Cooperative Patent Classification (CPC):
(C-Sets available)
A61K 38/19; A61K 9/0019; A61K 31/7088;
A61K 40/17; A61K 40/24; A61K 40/31;
A61K 40/4202; A61K 40/4205; A61K 40/425;
A61K 40/4254; A61P 35/00; C07K 14/7051;
A61K 2239/31; A61K 2239/38; A61K 2239/48;
(Cont.)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR

(30) Priority: 30.04.2019 US 201962841190 P
30.04.2019 US 201962841183 P
23.03.2020 US 202016827381
23.03.2020 US 202016827302

(62) Document number(s) of the earlier application(s) in
accordance with Art. 76 EPC:
20798060.8 / 3 962 497

(71) Applicant: CREATE Medicines, Inc.
Boston, MA 02199 (US)

(72) Inventors:
• GETTS, Daniel
Boston, Massachusetts, 02199 (US)
• WANG, Yuxiao
Boston, Massachusetts, 02199 (US)

(74) Representative: Brand Murray Fuller LLP
50 Eastcastle Street
London W1W 8EA (GB)

Remarks:
•The complete document including Reference
Table(s) and the Sequence Listing(s) can be
downloaded from the EPO website
•This application was filed on 29.11.2025 as a
divisional application to the application mentioned
under INID code 62.

(54) **ENGINEERED CHIMERIC FUSION PROTEIN COMPOSITIONS AND METHODS OF USE THEREOF**

(57) The present disclosure provides compositions and methods for making and using engineered phagocytic cells that express a chimeric antigen receptor having an enhanced phagocytic activity for immunotherapy in cancer or infection.

FIG. 1A

**EP 4 678 184 A2**

(Cont. next page)

(52) Cooperative Patent Classification (CPC): (Cont.)
C07K 16/2896; C07K 16/32; C07K 2317/622;
C07K 2319/03; C07K 2319/033

C-Sets
**A61K 38/19, A61K 2300/00**

**Description**

**CROSS REFERENCE**

This application claims the benefit of U.S. Provisional Application No. 62/841,190, filed on April 30, 2019, U.S. Provisional Application No. 62/841,183, filed on April 30, 2019, U.S. Non-Provisional Application No. 16/827,381, filed on March 23, 2020, and U.S. Non-Provisional Application No. 16/827,302, filed on March 23, 2020, each of which is incorporated herein by reference in its entirety.

**BACKGROUND**

Cellular immunotherapy is a promising new technology for fighting difficult to treat diseases, such as cancer, and persistent infections and also certain diseases that are refractory to other forms of treatment. A major breakthrough has come across with the discovery of CAR-T cell and their potential use in immunotherapy. CAR-T cells are T lymphocytes expressing a chimeric antigen receptor which helps target the T cell to specific diseased cells such as cancer cells, and can induce cytotoxic responses intended to kill the target cancer cell or immunosuppression and/or tolerance depending on the intracellular domain employed and co-expressed immunosuppressive cytokines. However, several limitations along the way has slowed the progress on CAR-T cells and dampened its promise in clinical trials.

Understanding the limitations of CAR-T cells is the key to leveraging the technology and continue innovations towards better immunotherapy models. Specifically, in T cell malignancies, CAR-T cells appear to have faced a major problem. CAR-T cells and malignant T cells share surface antigen in most T cell lymphomas (TCL), therefore, CAR-T cells are subject to cytotoxicity in the same way as cancer cells. In some instances, the CAR-T products may be contaminated by malignant T cells. Additionally, T cell aplasia is a potential problem due to prolonged persistence of the CAR-T cells. Other limitations include the poor ability for CAR-T cells to penetrate into solid tumors and the potent tumor microenvironment which acts to downregulate their anti-tumor potential. CAR-T cell function is also negatively influenced by the immunosuppressive tumor microenvironment (TME) that leads to endogenous T cell inactivation and exhaustion.

Myeloid cells, including macrophages, are cells derived from the myeloid lineage and belong to the innate immune system. They are derived from bone marrow stem cells which egress into the blood and can migrate into tissues. Some of their main functions include phagocytosis, the activation of T cell responses, and clearance of cellular debris and extracellular matrices. They also play an important role in maintaining homeostasis, and initiating and resolving inflammation. Moreover, myeloid cells can differentiate into numerous downstream cells, including macrophages, which can display different responses ranging from pro-inflammatory to anti-inflammatory depending on the type of stimuli they receive from the surrounding microenvironment. Furthermore, tissue macrophages have been shown to play a broad regulatory and activating role on other immune cell types including CDT effector cells, NK cells and T regulatory cells. Macrophages have been shown to be a main immune infiltrate in malignant tumors and have been shown to have a broad immunosuppressive influence on effector immune infiltration and function.

Myeloid cells are a major cellular compartment of the immune system comprising monocytes, dendritic cells, tissue macrophages, and granulocytes. Models of cellular ontogeny, activation, differentiation, and tissue-specific functions of myeloid cells have been revisited during the last years with surprising results. However, their enormous plasticity and heterogeneity, during both homeostasis and disease, are far from understood. Although myeloid cells have many functions, including phagocytosis and their ability to activate T cells, harnessing these functions for therapeutic uses has remained elusive. Newer avenues are therefore sought for using other cell types towards development of improved therapeutics, including but not limited to T cell malignancies.

Engineered myeloid cells can also be short-lived *in vivo,* phenotypically diverse, sensitive, plastic, and are often found to be difficult to manipulate *in vitro.* For example, exogenous gene expression in monocytes has been difficult compared to exogenous gene expression in non-hematopoietic cells. There are significant technical difficulties associated with transfecting myeloid cells (e.g., monocytes/macrophages). As professional phagocytes, myeloid cells, such as monocytes/macrophages, comprise many potent degradative enzymes that can disrupt nucleic acid integrity and make gene transfer into these cells an inefficient process. This is especially true of activated macrophages which undergo a dramatic change in their physiology following exposure to immune or inflammatory stimuli. Viral transduction of these cells has been hampered because macrophages are end-stage cells that generally do not divide; therefore, some of the vectors that depend on integration into a replicative genome have met with limited success. Furthermore, macrophages are quite responsive to "danger signals," and therefore several of the original viral vectors that were used for gene transfer induced potent anti-viral responses in these cells making these vectors inappropriate for gene delivery.

**SUMMARY**

The diverse functionality of myeloid cells makes them an ideal cell therapy candidate that can be engineered to have numerous therapeutic effects. The present disclosure is related to immunotherapy using myeloid cells (e.g., CD14+ cells) of the immune system, particularly phagocytic cells. A number of therapeutic indications could be contemplated using myeloid cells. For example, myeloid cell immunotherapy could be exceedingly important in cancer, autoimmunity, fibrotic diseases and infections. The present disclosure is related to immunotherapy using myeloid cells, including phagocytic cells of the immune system, particularly macrophages. It is an object of the invention disclosed herein to harness one or more of these functions of myeloid cells for therapeutic uses. For example, it is an object of the invention disclosed herein to harness the phagocytic activity of myeloid cells, including engineered myeloid cells, for therapeutic uses. For example, it is an object of the invention disclosed herein to harness the ability of myeloid cells, including engineered myeloid cells, to promote T cell activation. For example, it is an object of the invention disclosed herein to harness the ability of myeloid cells, including engineered myeloid cells, to promote secretion of tumoricidal molecules. For example, it is an object of the invention disclosed herein to harness the ability of myeloid cells, including engineered myeloid cells, to promote recruitment and trafficking of immune cells and molecules. The present disclosure provides innovative methods and compositions that can successfully transfect or transduce a myeloid cell, or otherwise induce a genetic modification in a myeloid cell, with the purpose of augmenting a functional aspect of a myeloid cell, additionally, without compromising the cell's differentiation capability, maturation potential, and/or its plasticity.

The present disclosure involves making and using engineered myeloid cells (e.g., CD14+ cells, such as macrophages or other phagocytic cells, which can attack and kill (ATAK) diseased cells directly and/or indirectly, such as cancer cells and infected cells. Engineered myeloid cells, such as macrophages and other phagocytic cells, can be prepared by incorporating nucleic acid sequences (e.g., mRNA, plasmids, viral constructs) encoding a chimeric fusion protein (CFP), that has an extracellular binding domain specific to disease associated antigens (e.g., cancer antigens), into the cells using, for example, recombinant nucleic acid technology, synthetic nucleic acids, gene editing techniques (e.g., CRISPR), transduction (e.g., using viral constructs), electroporation, or nucleofection. It has been found that myeloid cells can be engineered to have a broad and diverse range of activities. For example, it has been found that myeloid cells can be engineered to express a chimeric fusion protein (CFP) containing an antigen binding domain to have a broad and diverse range of activities. For example, it has been found that myeloid cells can be engineered to have enhanced phagocytic activity such that upon binding of the CFP to an antigen on a target cell, the cell exhibits increased phagocytosis of the target cell. It has also been found that myeloid cells can be engineered to promote T cell activation such that upon binding of the CFP to an antigen on a target cell, the cell promotes activation of T cells, such as T cells in the tumor microenvironment. The engineered myeloid cells can be engineered to promote secretion of tumoricidal molecules such that upon binding of the CFP to an antigen on a target cell, the cell promotes secretion of tumoricidal molecules from nearby cells. The engineered myeloid cells can be engineered to promote recruitment and trafficking of immune cells and molecules such that upon binding of the CFP to an antigen on a target cell, the cell promotes recruitment and trafficking of immune cells and molecules to the target cell or a tumor microenvironment.

The present disclosure is based on the important finding that engineered myeloid cells overcome at least some of the limitations of CAR-T cells, including being readily recruited to solid tumors; having an engineerable duration of survival, therefore lowering the risk of prolonged persistence resulting in aplasia and immunodeficiency; myeloid cells cannot be contaminated with T cells; myeloid cells can avoidance of fratricide, for example because they do not express the same antigens as malignant T cells; and myeloid cells have a plethora of anti-tumor functions that can be deployed. In some respects, engineered myeloid derived cells can be safer immunotherapy tools to target and destroy diseased cells.

Moreover, myeloid cells, such as macrophages, have been ubiquitously found in the tumor environment (TME) and are notably the most abundant cells in some tumor types. As part of their role in the immune system, myeloid cells, such as macrophages, are naturally engaged in clearing diseased cells. The present invention relates too harnessing myeloid cell function and specifically for targeting, killing and directly and/or indirectly clearing diseased cells as well as the delivery payloads such as antigens and cytokines.

Engineered myeloid cells can also be short-lived *in vivo,* phenotypically diverse, sensitive, plastic, and are often found to be difficult to manipulate *in vitro.* For example, exogenous gene expression in monocytes has been difficult compared to exogenous gene expression in non-hematopoietic cells. There are significant technical difficulties associated with transfecting myeloid cells (e.g., monocytes/macrophages). As professional phagocytes, myeloid cells, such as monocytes/macrophages, comprise many potent degradative enzymes that can disrupt nucleic acid integrity and make gene transfer into these cells an inefficient process. This is especially true of activated macrophages which undergo a dramatic change in their physiology following exposure to immune or inflammatory stimuli. Viral transduction of these cells has been hampered because macrophages are end-stage cells that generally do not divide; therefore, some of the vectors that depend on integration into a replicative genome have met with limited success. Furthermore, macrophages are quite responsive to "danger signals," and therefore several of the original viral vectors that were used for gene transfer induced potent anti-viral responses in these cells making these vectors inappropriate for gene delivery. The present disclosure provides innovative methods and compositions that can successfully transfect or transduce a myeloid cell, or otherwise induce a genetic modification in a myeloid cell, with the purpose of augmenting a functional aspect of a myeloid cell, additionally, without compromising the cell's differentiation capability, maturation potential, and/or its plasticity.

## INCORPORATION BY REFERENCE

All publications, patents, and patent applications mentioned in this specification are herein incorporated by reference to the same extent as if each individual publication, patent, or patent application was specifically and individually indicated to be incorporated by reference.

## BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings.

FIG. 1A depicts a diagram showing some of the potentially engineerable functions of myeloid cells.

FIG. 1B depicts a diagram, indicating the presence of various cell types in different types of cancer. Macrophages are the most abundant cells in the depicted cancer types.

FIG. 2A depicts a schematic showing an exemplary chimeric receptor fusion protein (CFP) containing an extracellular binding domain, a transmembrane domain, a first intracellular signaling domain and a second intracellular signaling domain. The signaling domains can be derived from other receptors and be designed to elicit any number of cell functions.

FIG. 2B depicts a schematic showing an exemplary CFP containing an extracellular binding domain, a transmembrane domain, and an intracellular signaling domain (left), and a CFP containing an extracellular binding domain, a transmembrane domain, a first intracellular signaling domain, a second intracellular signaling domain, a third intracellular signaling domain, and one or more additional intracellular signaling domains. The signaling domains can be derived from other receptors and be designed to elicit any number of cell functions.

FIG. 2C depicts a schematic showing an exemplary CFP dimer containing an anti-CD5 extracellular binding domain, a transmembrane domain, and an intracellular signaling domain containing an intracellular domain derived from FcRγ fused to a PI3K recruitment domain.

FIG. 2D depicts a schematic showing an exemplary CFP dimer containing an extracellular antigen binding domain, a transmembrane domain, and an intracellular signaling domain containing a phagocytosis domain a PI3K recruitment domain and a pro-inflammation domain.

FIG. 3 is a schematic depicting an exemplary CFP homodimer in which each subunit contains an extracellular domain fused to an scFv that binds to a single target (left), and an exemplary CFP heterodimer in which a first subunit of the heterodimer contains an extracellular domain fused to an scFv that binds to a first target and in which a second subunit of the heterodimer subunit contains an extracellular domain fused to an scFv that binds to a second target (right).

FIG. 4A is a schematic depicting an exemplary recombinant nucleic acid encoding a CFP containing a signal peptide fused to an antigen-specific scFv that is fused to an extracellular domain (ECD), transmembrane domain (TM) and intracellular domain of a scavenger receptor.

FIG. 4B is a schematic depicting the CFP of FIG. 4A incorporated within a cell membrane of a myeloid cell. The depicted CFP contains an scFv bound to a cancer antigen of a cancer cell. The extracellular domain, transmembrane domain and intracellular domain can be derived from one or more scavenger receptors.

FIG. 4C is an exemplary graph depicting expected results of relative phagocytosis in human primary myeloid cells transduced with empty vector (control) or a vector encoding a CFP co-cultured with dye loaded tumor cells. Phagocytosis is quantified using flow cytometry.

FIG. 4D is an exemplary graph depicting expected results of percent specific lysis of tumor cells when incubated in the presence of human primary myeloid cells (effector cells) transduced with empty vector (control) or a vector encoding a CFP co-cultured with tumor cells (target cells) expressing luciferase at the indicated effector cell:target cell ratios (E:T ratio).

FIG. 4E is an exemplary graph depicting expected results of percent survival in a mouse xenograft tumor model after treatment with cells transduced with empty vector (control) or a vector encoding a CFP.

FIG. 5A is a schematic depicting an exemplary recombinant nucleic acid encoding a CFP (M1-CAR) containing a signal peptide fused to an antigen-specific scFv that is fused to a CD8 hinge domain, a CD8 transmembrane domain and intracellular domain containing a phagocytosis activation domain of and pro-inflammation domain.

FIG. 5B is a schematic depicting the CFP (M1-CAR) of FIG. 5A incorporated within a cell membrane of a myeloid cell. The depicted CFP contains an scFv bound to a cancer antigen of a cancer cell.

FIG. 5C is an exemplary graph depicting expected results of relative phagocytosis in human primary myeloid cells transduced with empty vector (control) or a vector encoding a CFP (M1-CAR) co-cultured with dye loaded tumor cells. Phagocytosis is quantified using flow cytometry.

**FIG. 5D** is an exemplary graph depicting expected results of fold increase in production of the depicted cytokines in myeloid cells transduced with a vector control or a vector encoding a CFP (M1-CAR).

**FIG. 5E** is an exemplary graph depicting expected results of fold increase in production of the depicted M1 markers in human primary myeloid cells transduced with a vector control or a vector encoding a CFP (M1-CAR).

**FIG. 5F** is an exemplary graph depicting expected results of percent specific lysis of tumor cells when incubated in the presence of human primary myeloid cells (effector cells) transduced with empty vector (control) or a vector encoding a CFP (M1-CAR) co-cultured with tumor cells (target cells) expressing luciferase at the indicated effector cell:target cell ratios (E:T ratio). Specific lysis is quantified using a luciferase assay.

**FIG. 5G** is an exemplary graph depicting expected results of percent survival in a mouse xenograft tumor model after treatment with human primary myeloid cells transduced with empty vector (control) or a vector encoding a CFP (M1-CAR).

**FIG. 6A** is a schematic depicting an exemplary recombinant nucleic acid encoding a CFP (Integrin-CAR) containing a signal peptide fused to an antigen-specific scFv that is fused to a CD8 hinge domain, a CD8 transmembrane domain and intracellular phagocytosis activation domain and an intracellular integration activation domain.

**FIG. 6B** is a schematic depicting the CFP (Integrin-CAR) of **FIG. 6A** incorporated within a cell membrane of a myeloid cell. The depicted CFP contains an scFv bound to a cancer antigen of a cancer cell.

**FIG. 6C** is an exemplary graph depicting expected results of relative phagocytosis in human primary myeloid cells transduced with empty vector (control) or a vector encoding a CFP (Integrin-CAR) co-cultured with dye loaded tumor cells. Phagocytosis is quantified using flow cytometry.

**FIG. 6D** is an exemplary graph depicting expected results of percent specific lysis of tumor cells when incubated in the presence of human primary myeloid cells (effector cells) transduced with empty vector (control) or a vector encoding a CFP (Integrin-CAR) co-cultured with tumor cells (target cells) expressing luciferase at the indicated effector cell:target cell ratios (E:T ratio).

**FIG. 6E** is an exemplary graph depicting expected results of relative infiltration of human primary myeloid cells transduced with empty vector (control) or a vector encoding a CFP (Integrin-CAR).

**FIG. 6F** is an exemplary graph depicting expected results of percent survival in a mouse xenograft tumor model after treatment with human primary myeloid cells transduced with empty vector (control) or a vector encoding a CFP (Integrin-CAR).

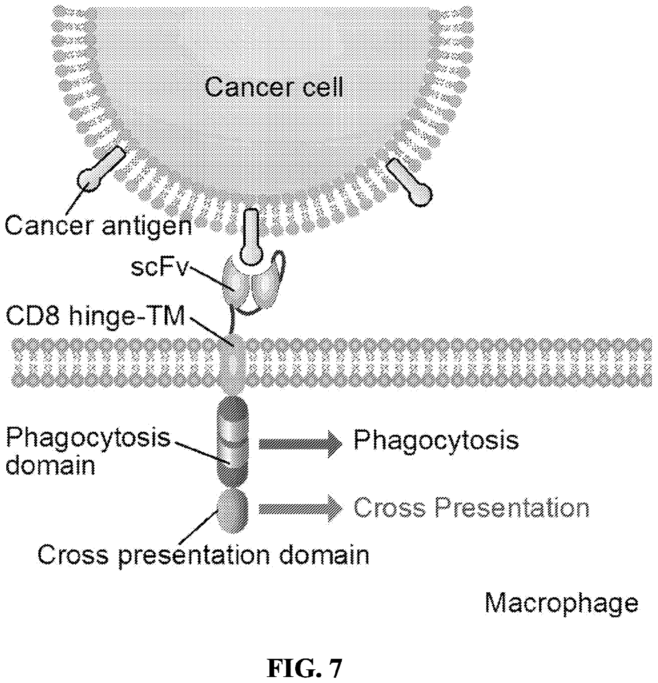

**FIG. 7** is a schematic depicting the CFP (cross presentation-CAR) incorporated within a cell membrane of a myeloid cell. The depicted cross presentation-CAR contains an scFv bound to a cancer antigen of a cancer cell that is fused to a CD8 hinge domain, a CD8 transmembrane domain, an intracellular phagocytosis activation domain and an intracellular cross presentation activation domain. Cross presentation-CARs may direct antigens to a cross presentation pathway.

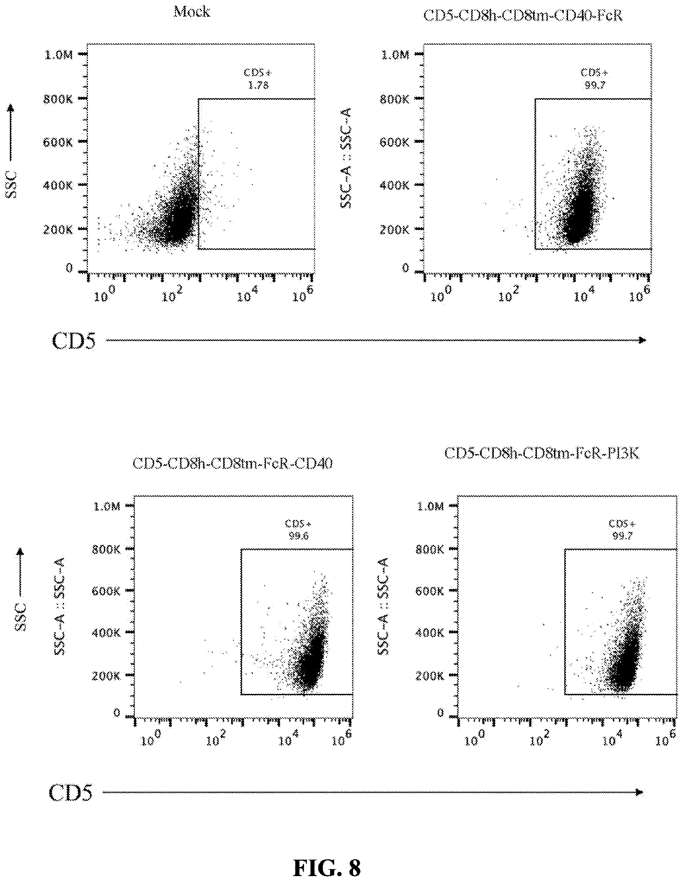

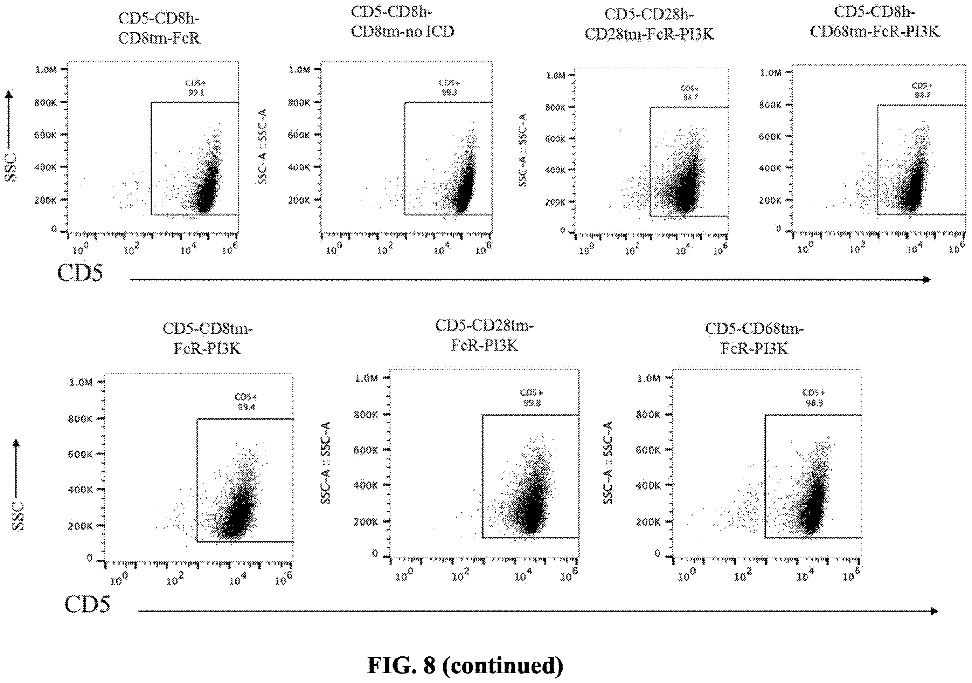

**FIG. 8** depicts exemplary flow cytometry data (side scatter (SSC) vs CD5+) after mock expression or expression of various constructs having an extracellular domain (ECD) with an anti-CD5 scFv in myeloid cells. The depicted constructs include an ECD containing an anti-CD5 scFv fused to a CD8 hinge domain fused to a CD8 transmembrane domain fused to a CD40 intracellular domain, fused to an FcRγ intracellular domain (CD5-CD8h-CD8tm-CD40-FcR); an ECD containing an anti-CD5 scFv fused to a CD8 hinge domain fused to a CD8 transmembrane domain fused to an FcRγ intracellular domain, fused to a CD40 intracellular domain (CD5-CD8h-CD8tm-FcR-CD40); an ECD containing an anti-CD5 scFv fused to a CD8 hinge domain fused to a CD8 transmembrane domain fused to an FcRγ intracellular domain, fused to a PI3K recruitment domain (CD5-CD8h-CD8tm-FcR-PI3K); an ECD containing an anti-CD5 scFv fused to a CD8 hinge domain fused to a CD8 transmembrane domain fused to an FcRγ intracellular domain (CD5-CD8h-CD8tm-FcR); an ECD containing an anti-CD5 scFv fused to a CD8 hinge domain fused to a CD8 transmembrane domain (CD5-CD8h-CD8tm-no ICD); an ECD containing an anti-CD5 scFv fused to a CD28 hinge domain fused to a CD28 transmembrane domain fused to an FcRγ intracellular domain fused to a PI3K recruitment domain (CD5-CD28h-CD28tm-FcR-PI3K); an ECD containing an anti-CD5 scFv fused to a CD8 hinge domain fused to a CD68 transmembrane domain fused to an FcRγ intracellular domain fused to a PI3K recruitment domain (CD5-CD8h-CD68tm-FcR-PI3K); an ECD containing an anti-CD5 scFv fused to a CD8 transmembrane domain fused to an FcRγ intracellular domain fused to a PI3K recruitment domain (CD5-CD8tm-FcR-PI3K); an ECD containing an anti-CD5 scFv fused to a CD28 transmembrane domain fused to an FcRγ intracellular domain fused to a PI3K recruitment domain (CD5-CD28tm-FcR-PI3K); and an ECD containing an anti-CD5 scFv fused to a CD68 transmembrane domain fused to an FcRγ intracellular domain fused to a PI3K recruitment domain (CD5-CD68tm-FcR-PI3K).

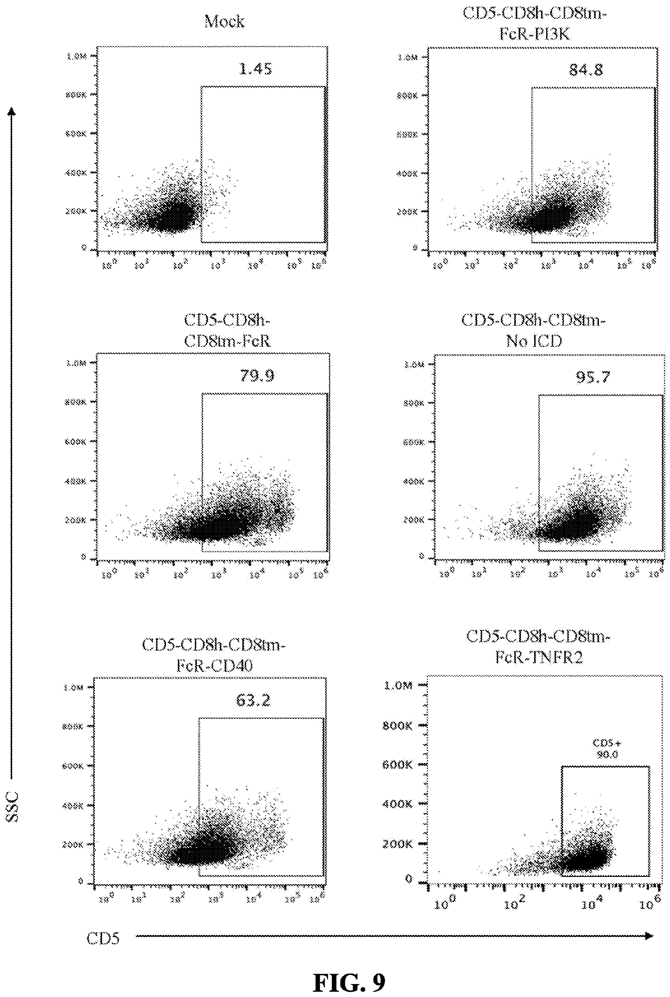

**FIG. 9** depicts exemplary flow cytometry data (side scatter (SSC) vs anti-CD5 CFP+) after mock expression or expression of various constructs having an extracellular domain (ECD) with an anti-CD5 scFv in myeloid cells. The depicted constructs include an ECD containing an anti-CD5 scFv fused to a CD8 hinge domain fused to a CD8 transmembrane domain fused to an FcRγ intracellular domain, fused to a PI3K recruitment domain (CD5-CD8h-CD8tm-FcR-PI3K); an ECD containing an anti-CD5 scFv fused to a CD8 hinge domain fused to a CD8 transmembrane domain fused to an FcRγ intracellular domain (CD5-CD8h-CD8tm-FcR); an ECD containing an anti-CD5 scFv fused to a CD8 hinge domain fused to a CD8 transmembrane domain (CD5-CD8h-CD8tm-no ICD); an ECD containing an anti-CD5 scFv fused to a CD8 hinge domain fused to a CD8 transmembrane domain fused to an FcRγ intracellular domain fused to a CD40 intracellular domain (CD5-CD8h-CD8tm-FcR-CD40); and an ECD containing an anti-CD5 scFv fused to a CD8 hinge domain fused to a CD8 transmembrane domain fused to an FcRγ intracellular domain, fused to a TNFR2 intracellular domain (CD5-CD8h-CD8tm-FcR-TNFR2).

**FIG. 10A** depicts a schematic showing an exemplary experimental flow diagram of a phagocytic assay using FITC-labeled beads coated in antigen targeted by FarRed fluorophore-labeled CFPs expressed in THP-1 cells.

**FIG. 10B** depicts exemplary flow cytometry data (side scatter (SSC) vs CSFE-FarRed) after mock expression or expression of anti-CD5 CFPs using the experimental design of **FIG. 10A.**

**FIG. 10C** depicts an exemplary graph showing relative phagocytosis in human primary myeloid cells transduced with empty vector (mock) or a vector encoding the depicted CFPs co-cultured with FITC-labeled beads coated with BSA or CD5 using the experimental design of **FIG. 10A.**

**FIG. 10D** depicts exemplary bar graphs of the concentration (pg/mL) of the indicated proteins after mock expression or expression of the indicated anti-CD5 CFPs using the experimental design of **FIG. 10A.** Each of the CFPs contained an ECD containing an anti-CD5 scFv fused to a CD8 hinge domain fused to a CD8 transmembrane domain fused to the indicated intracellular domains.

**FIG. 10E** depicts an exemplary graph measuring expression of M1 associated markers (CD16 and MHC class I) in primary human monocyte cells expressing an anti-CD5 CFP that were incubated in the presence of IL-10, IL-4 and TGFβ for 24 hours and then incubated with H9 T cell lymphoma cells. The primary human monocyte cells expressing the anti-CD5 CFP demonstrated potent activity in an M2 environment.

**FIG. 10F** depicts an exemplary bar graph of the concentration (pg/mL) of TNF-α after incubating primary human monocyte cells expressing an anti-CD5 chimeric antigen receptor in the presence of IL-10, IL-4 and TGFβ for 24 hours and then H9 T cell lymphoma cells overnight. The primary human monocyte cells expressing the anti-CD5 CFP were able to function in tumor microenvironment (TME) like conditions to produce inflammatory mediators.

**FIG. 10G** depicts exemplary bar graphs of the concentration (pg/mL) of the indicated chemoattractants (CCL3, CCL4, CXCL10 and CXCL12) after incubating primary human monocyte cells expressing an anti-CD5 CFP in the presence of IL-10, IL-4 and TGFβ for 24 hours and then H9 T cell lymphoma cells overnight. The primary human monocyte cells expressing the anti-CD5 CFP were able to function to secrete a broad range of chemokines, including T cell chemoattractants and NK cell chemoattractants in tumor microenvironment (TME) like conditions.

**FIG. 10H** depicts exemplary bar graphs of the concentration (pg/mL) of the indicated chemoattractants (CCL8, CXCL1, eotaxin and CCL5) after incubating primary human monocyte cells expressing an anti-CD5 CFP in the presence of IL-10, IL-4 and TGFβ for 24 hours and then H9 T cell lymphoma cells overnight. The primary human monocyte cells expressing the anti-CD5 CFP were able to function to secrete a broad range of chemokines, including chemokines that activate polymorphonuclear neutrophis (PMN) and eosinophil and leukocyte chemoattractants.

**FIG. 11A** depicts a schematic showing an exemplary experimental flow diagram of a phagocytic assay using CFSE-labeled target cells targeted by FarRed fluorophore-labeled CFPs expressed in THP-1 cells.

**FIG. 11B** depicts exemplary flow cytometry data (side scatter (SSC) vs forward scatter (FSC); CSFE vs FarRed; and cell counts vs CSFE) after mock expression or expression of anti-CD5 CFPs in THP-1 cells using the experimental design of **FIG. 11A**. A myeloid cell line was electroporated with an anti-CD5 CFP and labelled with the intracellular FarRed dye. These cells were incubated with H9 T cell cancer cells that were pre-labelled with CFSE at a 1:3 myeloid cell:tumor cell ratio. After 4 hours, phagocytosis was measured by flow cytometry.

**FIG. 11C** depicts an exemplary graph showing relative phagocytosis in a myeloid cell line electroporated with empty vector (mock) or a vector encoding the depicted CFP and labelled with the intracellular FarRed dye using the experimental design of **FIG. 11A.** These cells were incubated with H9 T cell cancer cells that were pre-labelled with CFSE at a 1:3 myeloid cell:tumor cell ratio. After 4 hours, phagocytosis was measured by flow cytometry.

**FIG. 12A** depicts a schematic showing an exemplary experimental flow diagram of a phagocytic assay using pHRodo-labeled target cells targeted by FarRed fluorophore-labeled CFPs expressed in primary human monocyte cells.

**FIG. 12B** depicts exemplary flow cytometry data (pHRodo vs FarRed) after mock expression or expression of anti-CD5 CFPs in primary human monocyte cells using the experimental design of **FIG. 12A.** Primary human monocyte cells were electroporated with an anti-CD5 CFP and labelled with the intracellular FarRed dye. These cells were incubated with H9 T cell cancer cells that were pre-labelled with pHRodo. After incubation, phagocytosis was measured by flow cytometry.

**FIG. 12C** depicts an exemplary graph quantifying the results of **FIG. 12B** showing relative phagocytosis after mock expression or expression of the depicted anti-CD5 CFPs in primary human monocyte cells using the experimental design of **FIG. 12A.**

**FIG. 12D** depicts exemplary bar graphs of the concentration (pg/mL) of the indicated proteins after mock expression or expression of the indicated anti-CD5 CFPs in monocyte cells after performing a bead-based phagocytic assay.

**FIG. 13** depicts an exemplary graph of relative fluorescence units (RFUs) over time after incubation of no cells or THP-1 cells expressing an anti-CD5 CFP with CCL2 at the indicated concentrations. Fold increase over control depicts a ratio of CCL2-induced chemotaxis as compared to cells treated with assay buffer alone. Each bar on the graph represents a mean $\pm$ SD of two replicate wells.

**FIG. 14** depicts an exemplary graph of relative fluorescence units (RFUs) over time after incubation of no cells or primary human monocyte cells expressing an anti-CD5 CFP with CCL2 at the indicated concentrations. Fold increase over control depicts a ratio of CCL2-induced chemotaxis as compared to cells treated with assay buffer alone. Each bar on the graph represents a mean $\pm$ SD of two replicate wells.

**FIG. 15A** depicts a schematic showing an exemplary experimental flow diagram of a peripheral T cell lymphoma animal model experiment. Treatment with the indicated amounts of human primary monocytes expressing an anti-CD5 CFP was initiated at day 11 post tumor seeding. IVIS imaging was used to measure tumor mass.

**FIG. 15B** depicts exemplary flow cytometry data (side scatter (SSC) vs anti-CD5 CFP+) after expression of an anti-CD5 CFPs in human primary monocyte cells according to the experiment shown in **FIG. 15A.**

**FIG. 15C** depicts exemplary results of a mouse xenograft model treated with vehicle or human primary monocytes expressing an anti-CD5 CFP according to the experiment shown in **FIG. 15A.** On day 0, NSG mice were injected with CD5+ tumor cells expressing luciferase. Mice were then either untreated or injected with the indicated regimens of human primary monocytes electroporated with an anti-CD5 CFP.

**FIG. 15D** depicts a graph of relative tumor size over time from the results of **FIG. 15C.** IVIS imaging of luciferase fluorescence was used to measure tumor mass.

**FIG. 16A** depicts a schematic showing an exemplary experimental flow diagram of a peripheral T cell lymphoma animal model experiment. Treatment with the indicated amounts of human primary monocytes expressing an anti-CD5 CFP was initiated at day 11 post tumor seeding.

**FIG. 16B** depicts exemplary flow cytometry data (side scatter (SSC) vs anti-CD5 CFP+) after expression of an anti-CD5 CFPs in human primary monocyte cells according to the experiment shown in **FIG. 16A.** The data shows achievement of 95% transfection efficiency.

**FIG. 16C** depicts a graph of relative tumor size over time according to the experiment shown in **FIG. 16A.** IVIS imaging of luciferase fluorescence was used to measure tumor mass.

**FIG. 16D** depicts a graph of relative tumor size over time according to the experiment shown in **FIG. 16A.** Caliper measure was used to measure tumor mass. The data demonstrates that treatment was associated with delayed tumor progression, and a statistically significant reduction in tumor mass in an immune compromised mouse model. Statistical significance was determined using the Bonferroni-Dunn method, with alpha = 0.5. Each row was analyzed individually, without assuming a consistent SD. Number of t tests: 8 or 4.

**FIG. 17A** depicts a schematic showing an exemplary CFP containing an extracellular binding domain, a transmembrane domain, a first intracellular signaling domain derived from FcRy, and a second intracellular signaling domain derived from MDA5.

**FIG. 17B** depicts exemplary flow cytometry data (side scatter (SSC) vs anti-CD5 CFP+) showing expression in untransfected primary monocytes (top) and primary monocytes transfected with in vitro transcribed mRNA encoding a CFP containing an extracellular CD5 binding domain, a transmembrane domain, a first intracellular signaling domain derived from FcRy, and a second intracellular signaling domain derived from MDA5.

**FIG. 17C** depicts exemplary bar graphs of the concentration (pg/mL) of the indicated cytokines that are secreted in untransfected primary monocytes and primary monocytes transfected with in vitro transcribed mRNA encoding a CFP containing an extracellular CD5 binding domain, a transmembrane domain, a first intracellular signaling domain derived from FcRy, and a second intracellular signaling domain derived from MDA5.

**FIG. 18A** depicts a schematic showing an exemplary chimeric receptor fusion protein (CFP) containing an extracellular binding domain, a transmembrane domain, a first intracellular signaling domain derived from FcRy, and a second intracellular signaling domain derived from TNFR1 or TNFR2.

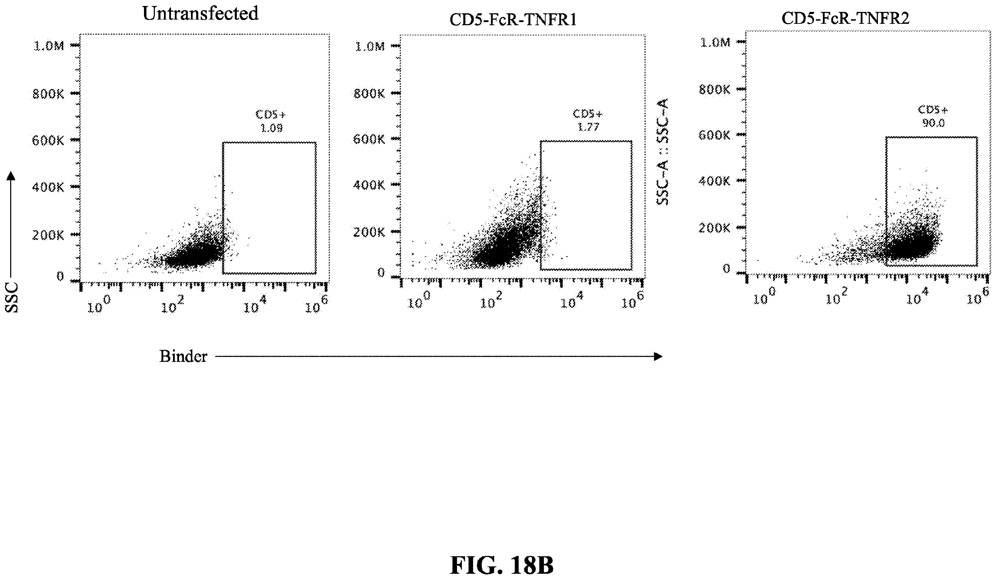

**FIG. 18B** depicts exemplary flow cytometry data (side scatter (SSC) vs anti-CD5 CFP+) showing expression in untransfected primary monocytes (left); primary monocytes transfected with in vitro transcribed mRNA encoding a CFP containing an extracellular CD5 binding domain, a transmembrane domain, a first intracellular signaling domain derived from FcRy, and a second intracellular signaling domain derived from TNFR1 (middle); and primary monocytes transfected with in vitro transcribed mRNA encoding a CFP containing an extracellular CD5 binding domain, a transmembrane domain, a first intracellular signaling domain derived from FcRy, and a second intracellular signaling domain derived from TNFR2 (right).

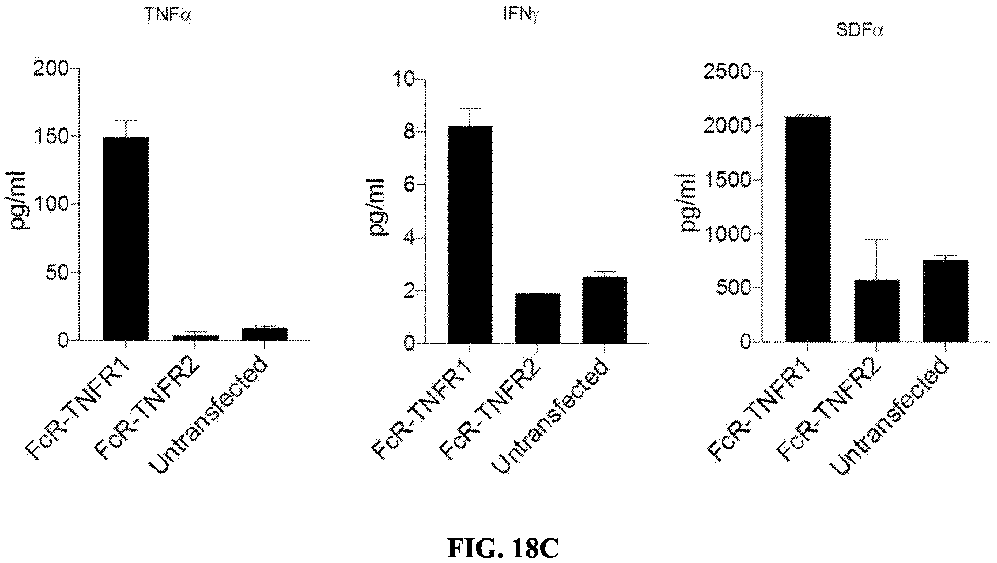

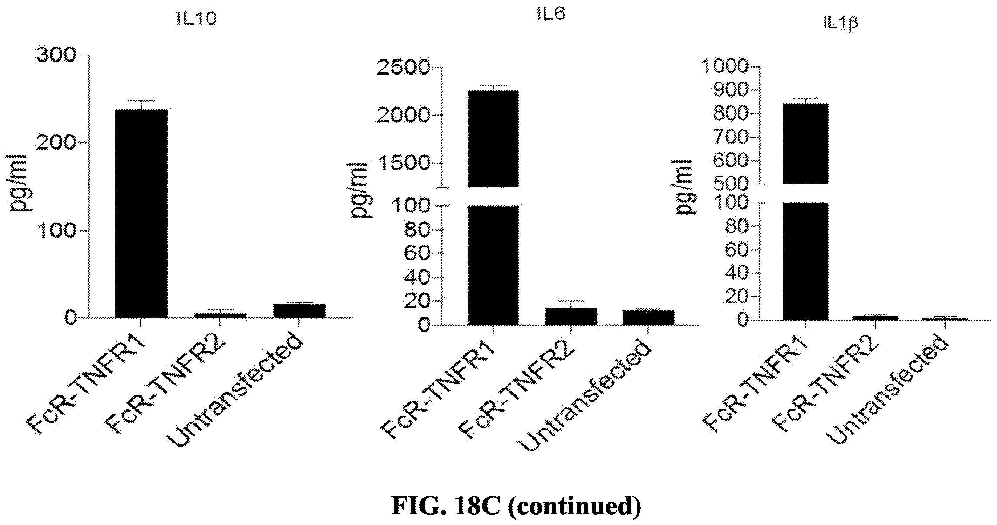

**FIG. 18C** depicts exemplary bar graphs of the concentration (pg/mL) of the indicated cytokines/chemokines that are secreted in untransfected primary monocytes; primary monocytes transfected with in vitro transcribed mRNA encoding a CFP containing an extracellular CD5 binding domain, a transmembrane domain, a first intracellular signaling domain derived from FcRy, and a second intracellular signaling domain CFP from TNFR1; and primary monocytes transfected with in vitro transcribed mRNA encoding a CFP containing an extracellular CD5 binding domain, a transmembrane domain, a first intracellular signaling domain derived from FcRy, and a second intracellular signaling domain derived from TNFR2.

**FIG. 19A** depicts a schematic showing an CFP containing an extracellular binding domain, a transmembrane domain, a first intracellular signaling domain derived from FcRy, and a second intracellular signaling domain derived from CD40, a PI3K recruitment domain or TNFR2.

**FIG. 19B** depicts a schematic showing an exemplary experimental flow diagram of an M2 stimulation assay. Primary monocytes expressing different CFP constructs were cultured in M2 conditions (IL4, IL10, TGFβ) for 24 hrs before being added to culture plates without coating or coated with recombinant CD5 antigen. Cells were incubated on the plate for 24 hrs and the amount of various cytokines secreted into the medium was measured.

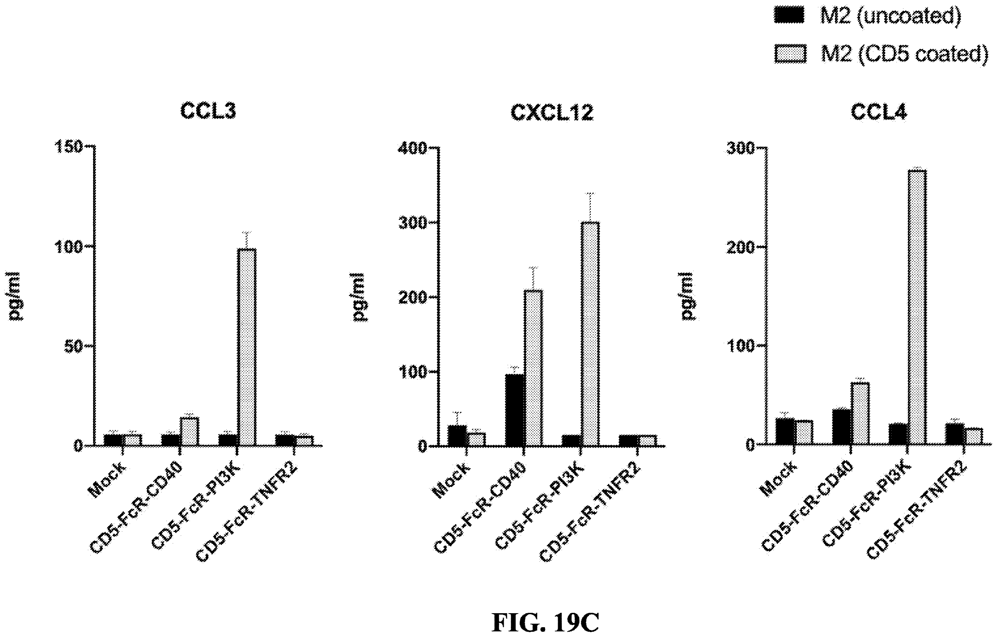

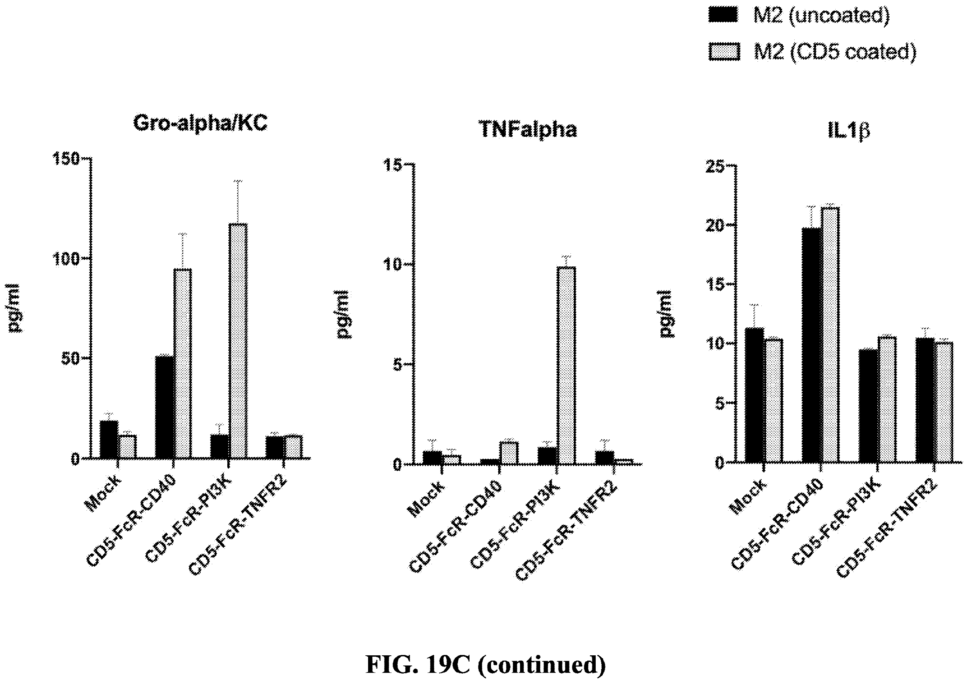

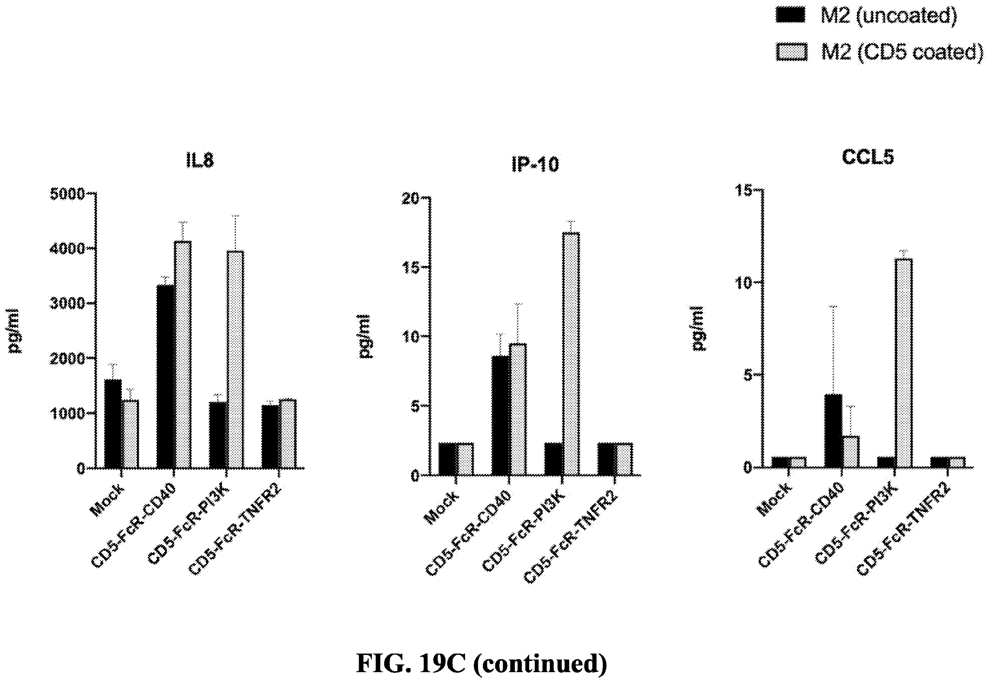

**FIG. 19C** depicts exemplary bar graphs of the concentration (pg/mL) of the indicated cytokines/chemokines (TNFα, IL8, IL1β, IP-10, Gro-alpha/KC, CCL3, CCL4, CCL5 and CXCL12) that are secreted in untransfected primary monocytes; primary monocytes transfected with in vitro transcribed mRNA encoding a CFP containing an extracellular CD5 binding domain, a transmembrane domain, a first intracellular signaling domain derived from FcRy, and a second intracellular signaling domain derived from TNFR1; and primary monocytes transfected with in vitro transcribed mRNA encoding a CFP containing an extracellular binding domain, a transmembrane domain, a first intracellular signaling domain derived from FcRy, and a second intracellular signaling domain derived from TNFR2. CD5 ligation induced upregulation of several proinflammatory cytokines including and chemokines including.

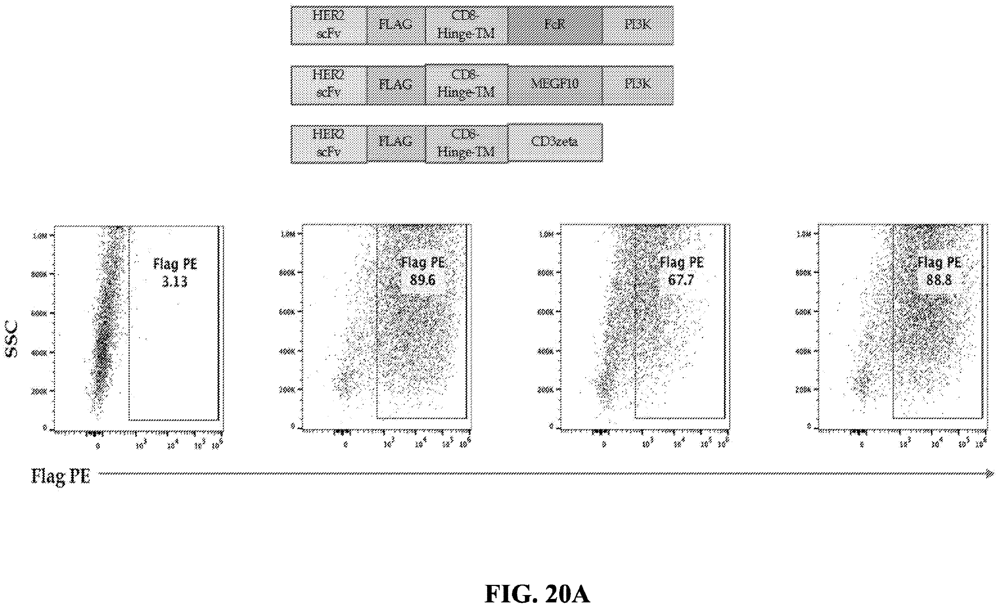

**FIG. 20A** depicts schematics of exemplary lentiviral constructs encoding CFPs containing an extracellular HER2 binding domain (scFv), an extracellular Flag tag, an extracellular hinge domain derived from CD8, a CD8 transmembrane domain, and either (a) a first intracellular signaling domain derived from FcRγ and a second intracellular signaling domain containing a PI3K recruitment domain, (b) a first intracellular signaling domain derived from MEGF10 and a second intracellular signaling domain containing a PI3K recruitment domain or (c) an intracellular signaling domain derived from CD3ζ in THP-1 cells. Also depicted is exemplary flow cytometry data (side scatter (SSC) vs Flag-PE) showing expression in untransduced primary monocytes or primary monocytes transduced with the depicted CFP constructs.

**FIG. 20B** depicts a schematic showing an exemplary experimental flow diagram of a phagocytosis assay.

**FIG. 20C** depicts an exemplary bar graph of the percentage of phagocytosis of THP-1 cells transduced with the lentiviral constructs depicted in **FIG. 20A** using the phagocytosis assay depicted in **FIG. 20B.** Transduced THP-1 cells, activated with or without phorbol-12-myristate-13-acetate (PMA), were incubated overnight with FarRed labelled SKOV3 tumor cells. Also depicted are exemplary fluorescent microscopic images of the cells showing phagocytosis.

**FIG. 20D** depicts exemplary flow cytometry data (FarRed vs PE) showing phagocytosis after performing the phagocytosis assay depicted in **FIG. 20B.** Transduced THP-1 cells, activated with or without PMA, were incubated overnight with FarRed labelled SKOV3 tumor cells.

**FIG. 20E** depicts exemplary flow cytometry data (SSC vs FSC and FarRed vs PE) after performing the phagocytosis assay depicted in **FIG. 20B.** Transduced THP-1 cells, activated with or without PMA, were incubated overnight with FarRed labelled SKOV3 tumor cells. Also depicted is an exemplary bar graph showing percent cell death of target cells as calculated by the following formula
$$\frac{(\#SKOV3\ alone - \#SKOV3\ with\ Effectors)}{\#SKOV3\ alone} \times 100\%.$$

**FIG. 21A** depicts a schematic showing an exemplary experimental flow diagram of a phagocytosis assay using CD14+ cells isolated from a healthy donor Leukopak and transduced with a lentiviral vector encoding a CFP containing an extracellular HER2 binding domain (scFv), an extracellular Flag tag, an extracellular hinge domain derived from CD8, a CD8 transmembrane domain, and a first intracellular signaling domain derived from FcRγ and a second intracellular signaling domain containing a PI3K recruitment domain.

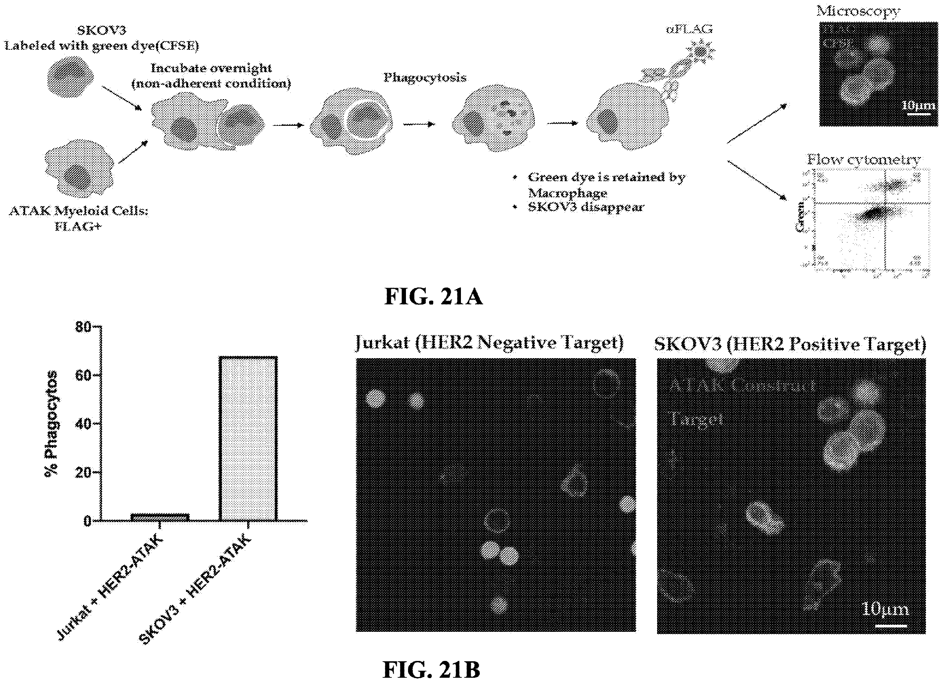

**FIG. 21B** depicts an exemplary bar graph of the percentage of phagocytosis of CD14+ cells isolated from a healthy donor Leukopak and transduced with a lentiviral vector encoding a CFP containing an extracellular HER2 binding domain (scFv), an extracellular Flag-tag, an extracellular hinge domain derived from CD8, a CD8 transmembrane domain, and a first intracellular signaling domain derived from FcRγ and a second intracellular signaling domain containing a PI3K recruitment domain using the phagocytosis assay depicted in **FIG. 21A.** Transduced cells were incubated overnight with target cells (Jurkat (HER2-) or SKOV3 (HER2+)). Also depicted are exemplary fluorescent microscopic images of the cells showing phagocytosis of SKOV3 cells, but not Jurkat cells.

**FIG. 21C** depicts exemplary flow cytometry data (CSFE vs PE) showing phagocytosis after performing the phagocytosis assay depicted in **FIG. 21A.** Transduced CD14+ cells isolated from a healthy donor Leukopak, were incubated overnight with CFSE labelled HER2+ SKOV3 ovarian tumor cells and CFSE labelled HER2- Jurkat cells. Also depicted is an exemplary bar graph showing percent cell death of target cells in the experiment depicted in **FIG. 21A.**

**FIG. 22A** depicts a schematic showing an exemplary experimental flow diagram of a MSTO mesothelioma animal model experiment to investigate the ability of CFP expressing cells to penetrate tumor sites and to assess activation of the CFP expressing cells after penetration.

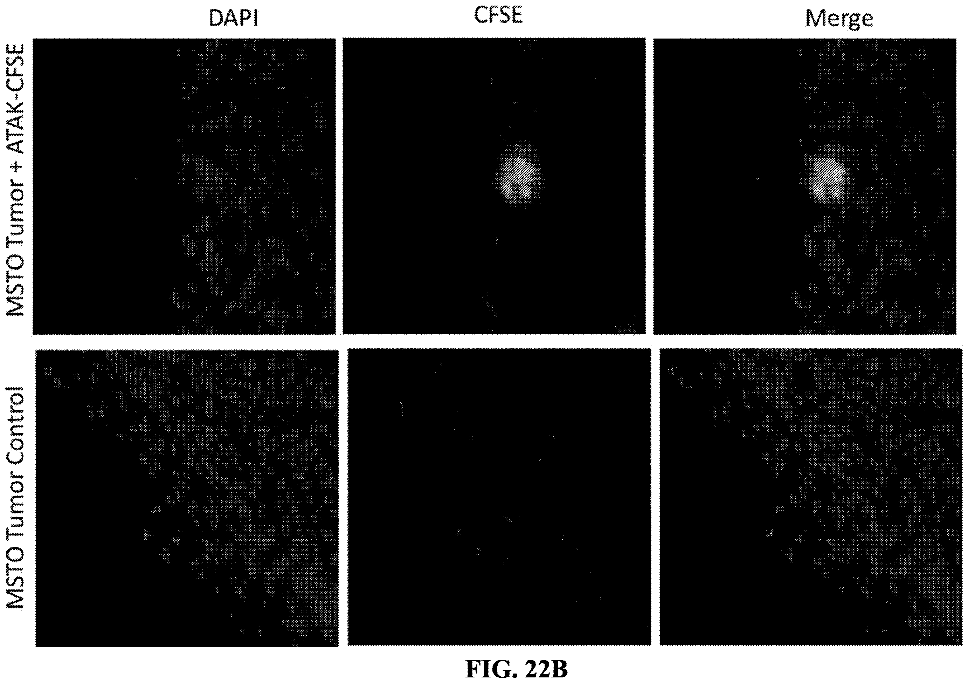

**FIG. 22B** depicts fluorescent microscopic images showing bioimaging of tumor samples that were removed 24 hours after CFSE labelled CD14+ cells isolated from a healthy donor Leukopak and transduced with a lentiviral vector encoding a CFP containing an extracellular HER2 binding domain (scFv), an extracellular Flag tag, an extracellular hinge domain derived from CD8, a CD8 transmembrane domain, and a first intracellular signaling domain derived from FcRγ and a second intracellular signaling domain containing a PI3K recruitment domain were administered to MSTO tumor bearing NSG mice. The transduced cells were observed to migrate into the tumor and accumulate around tumor cells.

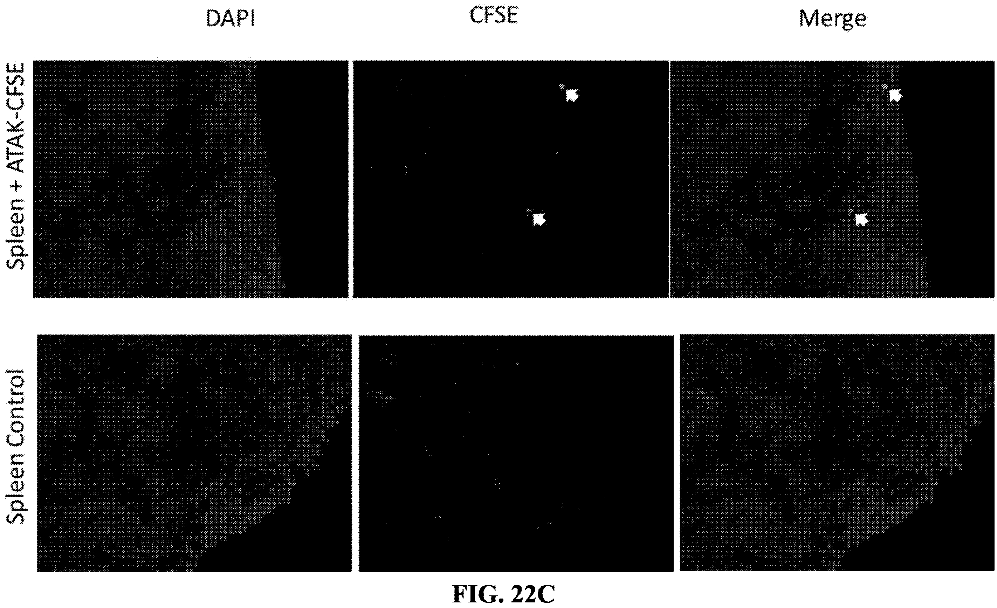

**FIG. 22C** depicts fluorescent microscopic images showing bioimaging of spleen samples that were removed 24 hours after CFSE labelled CD14+ cells isolated from a healthy donor Leukopak and transduced with a lentiviral vector encoding a CFP containing an extracellular HER2 binding domain (scFv), an extracellular Flag tag, an extracellular hinge domain derived from CD8, a CD8 transmembrane domain, and a first intracellular signaling domain derived from FcRγ and a second intracellular signaling domain containing a PI3K recruitment domain were administered to MSTO tumor bearing NSG mice. The transduced cells were observed to migrate into the spleen. CFSE labelled cells isolated from the spleen 24 hours after cell infusion were also examined by flow cytometry. CFSE labeled cells in the spleen maintained expression of HLA, CD14 and CD303. Interestingly, CCR2 expression was observed to decrease with a concurrent increased in CD370 (CLEC9A), potentially suggesting the cells migrate into the spleen and develop into a professional APC capable of stimulating T cells responses. Interestingly, CD206 (Mannose) expression was observed to decrease as did CD45. The reduction of mannose receptor expression may be associated with differentiation into M1 phenotype.

**FIG. 23** depicts a schematic showing an exemplary experimental flow diagram of a MSTO mesothelioma animal model experiment. Treatment with the indicated amounts of human primary monocytes expressing an anti-HER2 CFP was initiated at day 21 post tumor seeding. IVIS imaging was used to measure tumor mass.

**FIG. 24** depicts a graph of tumor size over time according to the experiment shown in **FIG. 23.** Infusion of human primary monocytes expressing an anti-HER2 CFP was associated with a delay in tumor progression compared to control treated animals.

**FIG. 25** depicts a diagram, indicating inhibition of a phagocytic receptor by target cell CD47 receptor SIRP-alpha (SIRPα) mediated signaling.

**FIG. 26A** depicts a graphical representation of the design of a recombinant dominant-negative CFP construct (upper panel), and a graphical representation showing inhibition of endogenous SIRPα by the recombinant CFP protein which is expressed in a macrophage. The CFP has an extracellular SIRPα domain capable of binding CD47 in the target cell, a SIRPα TM domain but lacks an intracellular signaling domain.

**FIG. 26B** shows an example expected result of relative phagocytosis by control and dominant-negative CFP transduced cells.

**FIG. 26C** shows an example expected result of target cell lysis by control and dominant-negative CFP transduced cells (E:T, effector : target).

**FIG. 26D** shows an example expected result mouse survival in a tumor model, after treatment with dominant negative CFP transduced macrophages.

**FIG. 27A** depicts an graphical representation of the design of a recombinant CFP, SIRPα-PI3K, (upper panel) comprising a SIRPα extracellular domain capable of binding CD47 in the target cell, a SIRPα TM domain but lacks an intracellular SIRPα signaling domain. The CFP is fused at intracellular end with an intracellular signaling domain having a PI3-kinase (PI3K) binding domain. BD: binding domain. The lower panel shows a graphical representation showing inhibition of endogenous SIRPα by the recombinant CFP protein which is expressed in a macrophage.

**FIG. 27B** shows an example of expected result of relative phagocytosis by control and SIRPα-PI3K CFP transduced cells.

**FIG. 27C** shows an example of expected result of relative Akt phosphorylation by control and SIRPα-PI3K CFP transduced cells.

**FIG. 27D** shows expected results of increased lysis of tumor cells by cells expressing a CFP (integrin-CAR) compared to control (empty vector transduced) macrophages.

**FIG. 27E** shows expected survival curve in mouse xenograft model of a tumor after treatment with SIRPα-PI3K CFP transduced macrophages, or no treatment controls.

**FIG. 28A** upper panel depicts a graphical representation of the design of a recombinant CFP, (SIRPα-M1) (upper panel) comprising a SIRPα extracellular domain capable of binding CD47, a SIRPα TM domain, but lacking an intracellular SIRPα signaling domain. The CFP contains an intracellular signaling domain having a pro-inflammation domain. The lower panel shows a graphical representation showing inhibition of endogenous SIRPα by the recombinant CFP protein when expressed in a myeloid cell (e.g., a macrophage). The pro-inflammation domain can induce M1 polarization.

**FIG. 28B** shows an example of expected result of flow cytometry assay showing an increase of M1 state marker expression when myeloid cells (e.g., a macrophages) are transduced with SIRPα-M1 compared with vector control.

**FIG. 28C** shows an example of expected result of flow cytometry assay showing an increase of pro-inflammatory markers when myeloid cells (e.g., a macrophages) are transduced with SIRPα-M1 compared with vector control.

**FIG. 28D** shows expected results of increased lysis of tumor cells by cells expressing SIRPα-M1 compared to control (empty vector transduced) myeloid cells (e.g., a macrophages).

**FIG. 28E** shows expected survival curve in mouse xenograft model of a tumor after treatment with SIRPα-M1 transduced myeloid cells (e.g., a macrophages), or no treatment controls.

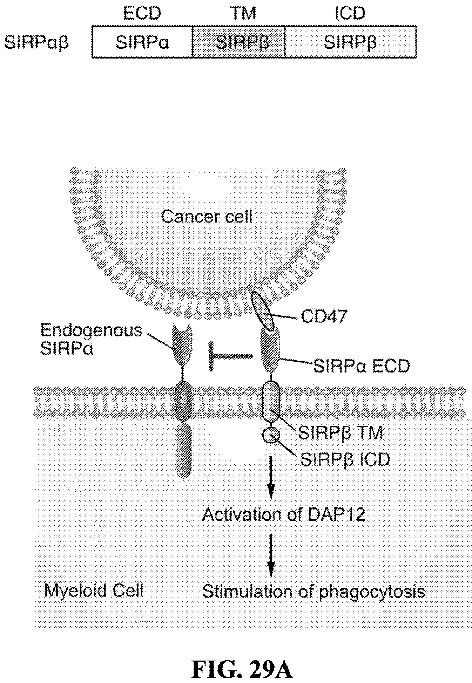

**FIG. 29A** upper panel depicts an illustrative schematic diagram of receptor based CFP, SIRPαβ, comprising an extracellular scFv specific for a cancer antigen, fused with an SIRPαβ chain. The extracellular portion of the CD47 receptor SIRPα is fused to a scFv specific to a cancer antigen. The ECD of SIRPα is fused with the transmembrane domain of SIRPβ The intracellular domain of the CFP comprises an intracellular domain derived from SIRPβ. Activation of CFP by binding of the scFv with the target ligand activates the SIRPβ intracellular domain, which triggers phagocytosis of the target cell through activation of DAP12. The lower panel shows a graphical representation of a recombinant SIRPαβ protein expressed in myeloid cells (e.g., a macrophage).

**FIG. 29B** depicts a graphical representation of a phagocytic receptor fusion protein SIRPα☐ compared to vector control.

**FIG. 29C** shows expected results of increased lysis of target cells by SIRPα☐ transduced macrophages compared to control (empty vector transduced) macrophages.

**FIG. 29D** shows expected results depicting survival curve in mouse xenograft model of a tumor after treatment with SIRPαβ transduced macrophages, or no treatment controls.

**FIG. 30A** depicts an illustrative schematic diagram of nucleic acid construct, comprising a regulatory element sequence, a sequence encoding a CFP, a sequence encoding a T2A and a sequence encoding a sialidase, The T2A sequence allows for cleavage of the sialidase from the CFP upon translation.

**FIG. 30B** depicts a graphical representation of enhanced phagocytic engulfment of a target cell in the presence of secreted sialidase.

**FIG. 30C** depicts expected results showing enhanced lysis of a target cell by an engineered myeloid cell expressing a CFP in the presence of sialidase.

**FIG. 30D** depicts an illustrative schematic diagram of nucleic acid construct encoding sialidase, with regulatory elements for expression in activated monocytes (e.g., macrophages).

**FIG. 30E** depicts a graphical representation of enhanced phagocytic engulfment of a target cell as a result of NF-kappa B (NF-κB) activation in the phagocytic cell. Activation of NF-kappa B activates the expression of a nucleic acid construct encoding sialidase.

**FIG. 30F** depicts an illustrative schematic diagram of nucleic acid construct encoding sialidase, with regulatory elements at the 3'UTR. The ARE domain has binding sequence motifs for RNA binding proteins that can be used for targeted expression of the construct as well as increase or decrease the duration of the mRNA half-life.

**FIG. 30G** is a graphical representation of enhanced phagocytic engulfment of a target cell as a result of expressing the sialidase construct shown in **FIG. 6F.**

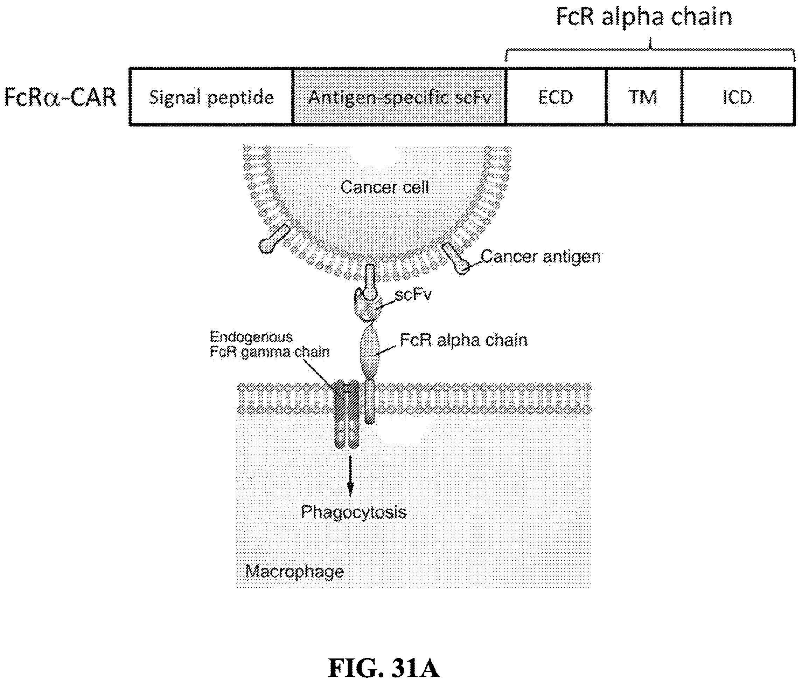

**FIG. 31A** depicts an illustrative schematic diagram of FcRα based CFP, comprising an extracellular scFv specific for a cancer antigen, fused with an FcRα chain (upper panel). The FcRα chain lacks an intracellular domain. The transmembrane domain trimerizes with endogenous Fcγ receptor transmembrane domains for expression in macrophages. Activation of the CFP by binding of the scFv with the target antigen activates the FcRα-Fcγ receptors, which triggers phagocytosis of the target cell. The lower panel shows a graphical representation of the recombinant FcRα-CFP which is expressed in a myeloid cell (e.g., a macrophage).

**FIG. 31B** depicts a graphical representation of relative phagocytosis activity of a cell expressing a CFP (FcRα-CAR) compared to vector control.

**FIG. 31C** shows expected results of increased lysis of target cells by CFP (FcRα-CAR) transduced myeloid cells (e.g., a macrophages) compared to control (empty vector transduced) myeloid cells (e.g., a macrophages).

**FIG. 31D** shows expected results depicting a survival curve in a mouse xenograft model of a tumor after treatment with CFP (FcRα-CAR) transduced myeloid cells (e.g., a macrophages), or no treatment controls.

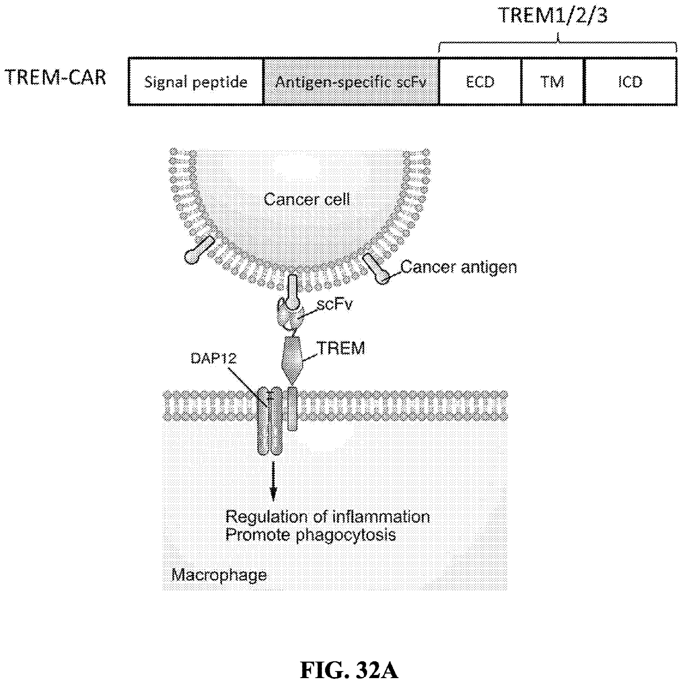

**FIG. 32A** depicts an illustrative schematic diagram of a CFP (TREM-CAR), comprising an extracellular scFv specific for a cancer antigen, fused with an ECD of TREM 1/2/3 (upper panel). The CFP comprises the TM and ICD of TREM 1/2/3. The transmembrane domain of TREM trimerizes with endogenous DAP12 transmembrane domains, which promote phagocytosis and regulate inflammation. Activation of the CFP by binding of the scFv to the target antigen activates TREM-mediated endogenous DAP12 signaling, which triggers phagocytosis of the target cell. The lower panel shows a graphical representation of the recombinant CFP (TREM-CAR) which is expressed in myeloid cells (e.g., a macrophage).

**FIG. 32B** depicts a graphical representation of relative phagocytosis activity of a cell expressing a CFP (TREM-CAR) compared to vector control.

**FIG. 32C** shows expected results of increased lysis of target cells by CFP (TREM-CAR) transduced myeloid cells (e.g., a macrophages) compared to control (empty vector transduced) myeloid cells (e.g., a macrophages).

**FIG. 32D** shows expected results depicting survival curve in mouse xenograft model of a tumor after treatment with CFP (TREM-CAR) transduced myeloid cells (e.g., a macrophages), or no treatment controls.

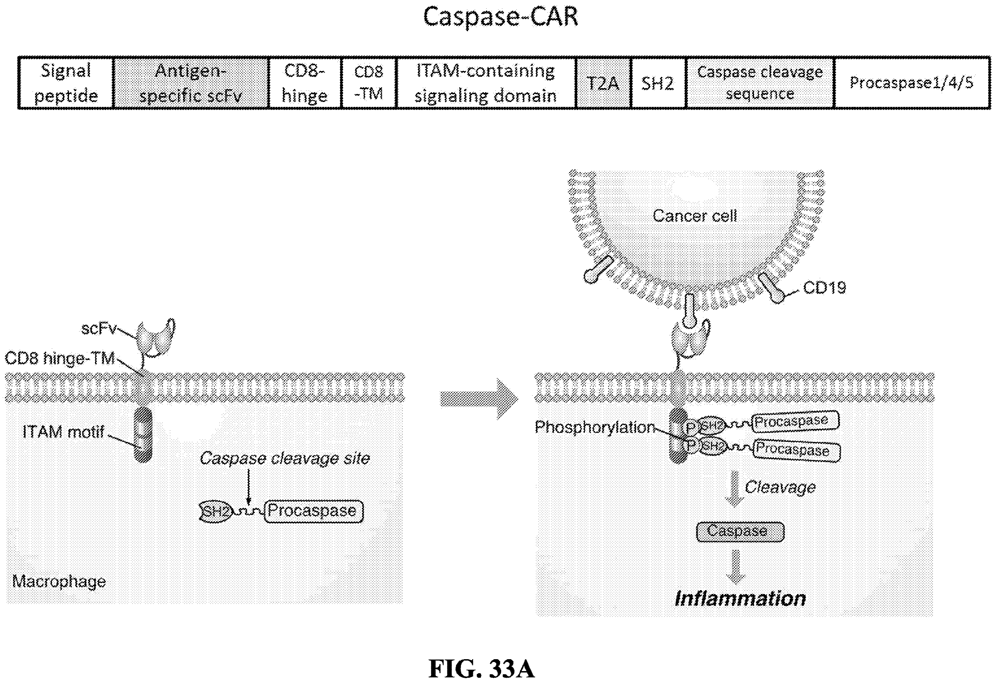

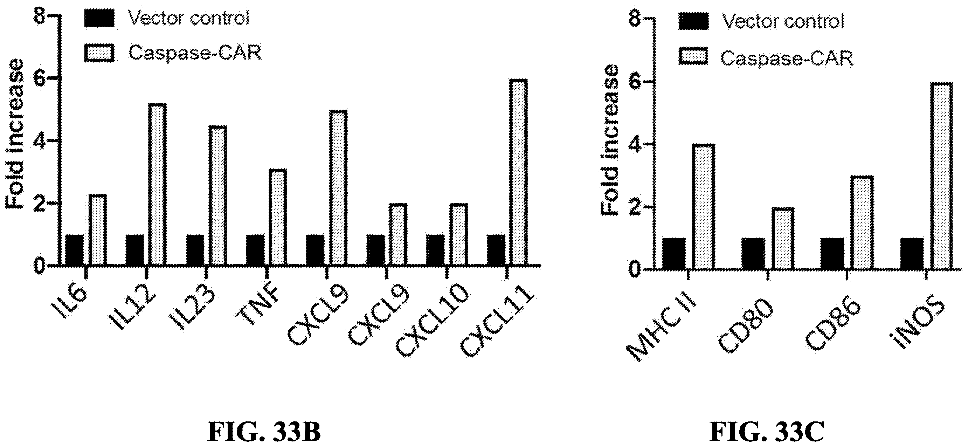

**FIG. 33A** shows an illustrative schematic of a caspase-recruiting CFP (caspase-CAR). The construct is composed of from N-terminus to C-terminus a signal peptide, an antigen-specific scFv, a hinge region (e.g., from CD8), a TM (e.g., from CD8) region, an ITAM containing phagocytosis signaling domain (e.g., FcRγ), a T2A sequence for bicistronic expression, an SH2 domain, a caspase cleavage sequence and procaspase (upper panel). When transduced into macrophages, this construct will co-express the CFP and an SH2-Procaspase. The procaspase is autoinhibited in the resting state. Binding of tumor surface antigen to the CAR receptor cause phosphorylation of ITAM tyrosine motifs, leading to recruitment of SH2 fused procaspase. Clustering of procaspase causes autocleavage and activation. The linker between SH2 and procaspase will also be cleaved at the recognition site. Activated caspase 1/4/5 drives strong inflammation (lower panel).

**FIG. 33B** shows expected results depicting increased inflammatory gene expression in cells expressing a caspase-recruiting CFP (caspase-CAR) compared to empty vector, when human primary myeloid cells (e.g., a macrophages) are co-cultured with target tumor cells. Cytokine profiling with ELISA shows increased secretion of pro-inflammatory cytokines and chemokines compared to vector control.

**FIG. 33C** shows expected flow cytometry results depicting increased pro-inflammatory cell surface marker expression in cells expressing a caspase-recruiting CFP (caspase-CAR) compared to empty vector when human primary myeloid cells (e.g., a macrophages) are co-cultured with target tumor cells.

**FIG. 33D** shows expected results of increased lysis of target tumor cells by caspase-recruiting CFP (caspase-CAR) transduced myeloid cells (e.g., macrophages) compared to control (empty vector transduced) myeloid cells (e.g., a macrophages).

**FIG. 33E** shows expected results depicting survival curve in mouse xenograft model of a tumor after treatment with caspase-recruiting CFP (caspase-CAR) transduced macrophages, or no treatment controls.

**FIG. 34A** depicts a graphical illustration of exemplary modular designs of a CFP construct.

**FIG. 34B** depicts a graphical illustration of exemplary modular designs of a CFP construct.

**FIG. 34C** depicts a graphical illustration of exemplary modular designs of a CFP construct.

## DETAILED DESCRIPTION

All terms are intended to be understood as they would be understood by a person skilled in the art. Unless defined otherwise, all technical and scientific terms used herein have the same meaning as commonly understood by one of ordinary skill in the art to which the disclosure pertains.

The section headings used herein are for organizational purposes only and are not to be construed as limiting the subject matter described.

Although various features of the present disclosure can be described in the context of a single embodiment, the features can also be provided separately or in any suitable combination. Conversely, although the present disclosure can be described herein in the context of separate embodiments for clarity, the disclosure can also be implemented in a single embodiment.

Reference in the specification to "some embodiments," "an embodiment," "one embodiment" or "other embodiments" means that a feature, structure, or characteristic described in connection with the embodiments is included in at least some embodiments, but not necessarily all embodiments, of the present disclosure.

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps. It is contemplated that any embodiment discussed in this specification can be implemented with respect to any method or composition of the disclosure, and vice versa. Furthermore, compositions of the disclosure can be used to achieve methods of the disclosure.

The term "about" or "approximately" as used herein when referring to a measurable value such as a parameter, an amount, a temporal duration, and the like, is meant to encompass variations of +/-30% or less, +/-20% or less, +/-10% or less, +/-5% or less, or +/-1% or less of and from the specified value, insofar such variations are appropriate to perform in the present disclosure. It is to be understood that the value to which the modifier "about" or "approximately" refers is itself also specifically disclosed.

Provided herein are engineered myeloid cells (including, but not limited to, neutrophils, monocytes, myeloid dendritic cells (mDCs), mast cells and macrophages), designed to specifically bind a target cell. The engineered myeloid cells can attack and kill target cells directly (e.g., by phagocytosis) and/or indirectly (e.g., by activating T cells). In some embodiments, the target cell is a cancer cell.

While cancer is one exemplary embodiment described in detail in the instant disclosure, the methods and technologies described herein are contemplated to be useful in targeting an infected or otherwise diseased cell inside the body. Similarly, therapeutic and vaccine compositions using the engineered cells are described herein.

Provided herein are compositions and methods for treating diseases or conditions, such as cancer. The compositions and methods provided herein utilize human myeloid cells, including, but not limited to, neutrophils, monocytes, myeloid dendritic cells (mDCs), mast cells and macrophages, to target diseased cells, such as cancer cells. The compositions and methods provided herein can be used to eliminate diseased cells, such as cancer cells and or diseased tissue, by a variety of mechanisms, including T cell activation and recruitment, effector immune cell activation (e.g., CD8 T cell and NK cell activation), antigen cross presentation, enhanced inflammatory responses, reduction of regulatory T cells and phagocytosis. For example, the myeloid cells can be used to sustain immunological responses against cancer cells.

Provided herein are compositions comprising a recombinant nucleic acid encoding a chimeric fusion protein (CFP), such as a phagocytic receptor (PR) fusion protein (PFP), a scavenger receptor (SR) fusion protein (SFP), an integrin receptor (IR) fusion protein (IFP) or a caspase-recruiting receptor (caspase-CAR) fusion protein. A CFP encoded by the recombinant nucleic acid can comprise an extracellular domain (ECD) comprising an antigen binding domain that binds to an antigen of a target cell. The extracellular domain can be fused to a hinge domain or an extracellular domain derived from a receptor, such as CD2, CD8, CD28, CD68, a phagocytic receptor, a scavenger receptor or an integrin receptor. The CFP encoded by the recombinant nucleic acid can further comprise a transmembrane domain, such as a transmembrane domain derived from CD2, CD8, CD28, CD68, a phagocytic receptor, a scavenger receptor or an integrin receptor. In some embodiments, a CFP encoded by the recombinant nucleic acid further comprises an intracellular domain comprising an intracellular signaling domain, such as an intracellular signaling domain derived from a phagocytic receptor, a scavenger receptor or an integrin receptor. For example, the intracellular domain can comprise one or more intracellular signaling domains derived from a phagocytic receptor, a scavenger receptor or an integrin receptor. For example, the intracellular domain can comprise one or more intracellular signaling domains that promote phagocytic activity, inflammatory response, nitric oxide production, integrin activation, enhanced effector cell migration (e.g., via chemokine receptor expression), antigen presentation, and/or enhanced cross presentation. In some embodiments, the CFP is a phagocytic receptor fusion protein (PFP). In some embodiments, the CFP is a phagocytic scavenger receptor fusion protein (PFP). In some embodiments, the CFP is an integrin receptor fusion protein (IFP). In some embodiments, the CFP is an inflammatory receptor fusion protein. In some embodiments, a CFP encoded by the recombinant nucleic acid further comprises an intracellular domain comprising a recruitment domain. For example, the intracellular domain can comprise one or more PI3K recruitment domains, caspase recruitment domains or caspase activation and recruitment domains (CARDs).

Provided herein is a composition comprising a recombinant nucleic acid encoding a CFP comprising a phagocytic or tethering receptor (PR) subunit (e.g., a phagocytic receptor fusion protein (PFP)) comprising: (i) a transmembrane domain, and (ii) an intracellular domain comprising a phagocytic receptor intracellular signaling domain; and an extracellular antigen binding domain specific to an antigen, e.g., an antigen of or presented on a target cell; wherein the transmembrane domain and the extracellular antigen binding domain are operatively linked such that antigen binding to the target by the extracellular antigen binding domain of the fused receptor activated in the intracellular signaling domain of the phagocytic receptor.

Provided herein is a composition comprising a recombinant nucleic acid sequence encoding a CFP comprising a phagocytic or tethering receptor (PR) subunit (e.g., a phagocytic receptor fusion protein (PFP)) comprising: a PR subunit comprising: a transmembrane domain, and an intracellular domain comprising an intracellular signaling domain; and an extracellular domain comprising an antigen binding domain specific to an antigen of a target cell; wherein the transmembrane domain and the extracellular domain are operatively linked; and wherein upon binding of the CFP to the antigen of the target cell, the killing or phagocytosis activity of a myeloid cell, such as a neutrophil, monocyte, myeloid dendritic cell (mDC), mast cell or macrophage expressing the CFP is increased by at least greater than 5%, 6%, 7%, 8%, 9%, 10%, 11%, 12%, 13%, 14%, 15%, 16%, 17%, 18%, 19%, 20%, 25%, 30%, 35%, 40%, 45%, 50%, 55%, 60%, 65%, 70%, 75%, 80%, 85%, 90%, 100%, 150%, 200%, 250%, 300%, 350%, 400%, 450%, 500%, 550%, 600%, 650%, 700%, 750%, 800%, 850%, 900%, 950%, or 1000% compared to a cell not expressing the CFP.

Provided herein is a composition comprising a recombinant nucleic acid sequence encoding a CFP comprising a phagocytic or tethering receptor (PR) subunit (e.g., a phagocytic receptor fusion protein (PFP)) comprising: a PR subunit comprising: a transmembrane domain, and an intracellular domain comprising an intracellular signaling domain; and an extracellular domain comprising an antigen binding domain specific to an antigen of a target cell; wherein the transmembrane domain and the extracellular domain are operatively linked; and wherein upon binding of the CFP to the antigen of the target cell, the killing or phagocytosis activity of a myeloid cell, such as a neutrophil, monocyte, myeloid dendritic cell (mDC), mast cell or macrophage expressing the CFP is increased by at least 1.1-fold, 1.5-fold, 2-fold, 2.5-fold, 3-fold, 3.5-fold, 4-fold, 4.5-fold, 5-fold, 5.5-fold, 6-fold, 6.5-fold, 7-fold, 7.5-fold, 8-fold, 8.5-fold, 9-fold, 9.5-fold, 10-fold, 11-fold, 12-fold, 13-fold, 14-fold, 15-fold, 16-fold,-fold, 17-fold, 18-fold, 19-fold, 20-fold, 25-fold, 30-fold, 40-fold, 50-fold, 75-fold, or 100-fold compared to a cell not expressing the CFP.

In one aspect, provided herein is a pharmaceutical composition comprising: (a) a myeloid cell, such as a neutrophil, monocyte, myeloid dendritic cell (mDC), mast cell or macrophage cell comprising a recombinant polynucleic acid, wherein the recombinant polynucleic acid comprises a sequence encoding a chimeric fusion protein (CFP), the CFP comprising: (i) an extracellular domain comprising an anti-CD5 binding domain, and (ii) a transmembrane domain operatively linked to the extracellular domain; and (b) a pharmaceutically acceptable carrier; wherein the myeloid cell expresses the CFP and exhibits at least a 1.1-fold increase in phagocytosis of a target cell expressing CD5 compared to a myeloid cell not expressing the CFP. In some embodiments, the CD5 binding domain is a CD5 binding protein that comprises an antigen binding fragment of an antibody, an Fab fragment, an scFv domain or an sdAb domain. In some embodiments, the CD5 binding domain comprises an scFv comprising: (i) a variable heavy chain ($V_H$) sequence of SEQ ID NO: 1 or with at least 90% sequence identity to SEQ ID NO: 1; and (ii) a variable light chain ($V_L$) sequence of SEQ ID NO: 2 or with at least 70%, 75%, 80%, 85%, 90%, 91%, 92%, 93%, 94%, 95%, 96%, 97%, 98% or 99% sequence identity to SEQ ID NO: 2. In some embodiments, the CD5 binding domain comprises an scFv comprising SEQ ID NO: 33 or with at least 70%, 75%, 80%, 85%, 90%, 91%, 92%, 93%, 94%, 95%, 96%, 97%, 98% or 99% sequence identity to SEQ ID NO: 33. In some embodiments, the HER2 binding domain comprises an scFv comprising: (i) a variable heavy chain ($V_H$) sequence of SEQ ID NO: 8 or with at least 70%, 75%, 80%, 85%, 90%, 91%, 92%, 93%, 94%, 95%, 96%, 97%, 98% or 99% sequence identity to SEQ ID NO: 8; and (ii) a variable light chain ($V_L$) sequence of SEQ ID NO: 9 or with at least 70%, 75%, 80%, 85%, 90%, 91%, 92%, 93%, 94%, 95%, 96%, 97%, 98% or 99% sequence identity to SEQ ID NO: 9. In some embodiments, the CD5 binding domain comprises an scFv comprising SEQ ID NO: 32 or with at least 70%, 75%, 80%, 85%, 90%, 91%, 92%, 93%, 94%, 95%, 96%, 97%, 98% or 99% sequence identity to SEQ ID NO: 32. In some embodiments, the CFP further comprises an intracellular domain, wherein the intracellular domain comprises one or more intracellular signaling domains, and wherein a wild-type protein comprising the intracellular domain does not comprise the extracellular domain.

In some embodiments, the extracellular domain further comprises a hinge domain derived from CD8, wherein the hinge domain is operatively linked to the transmembrane domain and the anti-CD5 binding domain. In some embodiments, the extracellular hinge domain comprises a sequence of SEQ ID NO: 7 or with at least 70%, 75%, 80%, 85%, 90%, 91%, 92%, 93%, 94%, 95%, 96%, 97%, 98% or 99% sequence identity to SEQ ID NO: 7.

In some embodiments, the CFP comprises an extracellular domain fused to a transmembrane domain of SEQ ID NO: 30 or with at least 70%, 75%, 80%, 85%, 90%, 91%, 92%, 93%, 94%, 95%, 96%, 97%, 98% or 99% sequence identity to SEQ ID NO: 30. In some embodiments, the CFP comprises an extracellular domain fused to a transmembrane domain of SEQ ID NO: 31 or with at least 70%, 75%, 80%, 85%, 90%, 91%, 92%, 93%, 94%, 95%, 96%, 97%, 98% or 99% sequence identity to SEQ ID NO: 31.

In some embodiments, the transmembrane domain comprises a CD8 transmembrane domain. In some embodiments, the transmembrane domain comprises a sequence of SEQ ID NO: 6 or 29 or with at least 70%, 75%, 80%, 85%, 90%, 91%, 92%, 93%, 94%, 95%, 96%, 97%, 98% or 99% sequence identity to SEQ ID NO: 6 or 29. In some embodiments, the transmembrane domain comprises a sequence of SEQ ID NO: 18 or with at least 70%, 75%, 80%, 85%, 90%, 91%, 92%, 93%, 94%, 95%, 96%, 97%, 98% or 99% sequence identity to SEQ ID NO: 18. In some embodiments, the transmembrane domain comprises a sequence of SEQ ID NO: 34 or with at least 70%, 75%, 80%, 85%, 90%, 91%, 92%, 93%, 94%, 95%, 96%, 97%, 98% or 99% sequence identity to SEQ ID NO: 34. In some embodiments, the transmembrane domain comprises a sequence of SEQ ID NO: 19 or with at least 70%, 75%, 80%, 85%, 90%, 91%, 92%, 93%, 94%, 95%, 96%, 97%, 98% or 99% sequence identity to SEQ ID NO: 19.

In some embodiments, the CFP comprises one or more intracellular signaling domains that comprise a phagocytic signaling domain. In some embodiments, the phagocytosis signaling domain comprises an intracellular signaling domain derived from a receptor other than Megf10, MerTk, FcRα, and Bail. In some embodiments, the phagocytosis signaling domain comprises an intracellular signaling domain derived from a receptor other than Megf10, MerTk, an FcR, and Bai1. In some embodiments, the phagocytosis signaling domain comprises an intracellular signaling domain derived from a receptor other than CD3ζ. In some embodiments, the phagocytosis signaling domain comprises an intracellular signaling domain derived from FcRy, FcRα or FcRε. In some embodiments, the phagocytosis signaling domain comprises an intracellular signaling domain derived from CD3ζ. In some embodiments, the CFP comprises an intracellular signaling domain of any one of SEQ ID NOs: 3, 20, 27 and 28 or with at least 70%, 75%, 80%, 85%, 90%, 91%, 92%, 93%, 94%, 95%, 96%, 97%, 98% or 99% sequence identity to any one of SEQ ID NOs: 3, 20, 27 and 28. In some embodiments, the one or more intracellular signaling domains further comprises a proinflammatory signaling domain. In some embodiments, the proinflammatory signaling domain comprises a PI3-kinase (PI3K) recruitment domain. In some embodiments, the proinflammatory signaling domain comprises a sequence of SEQ ID NO: 4 or with at least 70%, 75%, 80%, 85%, 90%, 91%, 92%, 93%, 94%, 95%, 96%, 97%, 98% or 99% sequence identity to SEQ ID NO: 4. In some embodiments, the proinflammatory signaling domain is derived from an intracellular signaling domain of CD40. In some embodiments, the proinflammatory signaling domain comprises a sequence of SEQ ID NO: 5 or with at least 70%, 75%, 80%, 85%, 90%, 91%, 92%, 93%, 94%, 95%, 96%, 97%, 98% or 99% sequence identity to SEQ ID NO: 5. In some embodiments, the CFP comprises an intracellular signaling domain of SEQ ID NO: 21 or with at least 70%, 75%, 80%, 85%, 90%, 91%, 92%, 93%, 94%, 95%, 96%, 97%, 98% or 99% sequence identity to SEQ ID NO: 21. In some embodiments, the CFP comprises an intracellular signaling domain of SEQ ID NO: 23 or with at least 70%, 75%, 80%, 85%, 90%, 91%, 92%, 93%, 94%, 95%, 96%, 97%, 98% or 99% sequence identity to SEQ ID NO: 23.

In some embodiments, the CFP comprises a sequence of SEQ ID NO: 14 or with at least 70%, 75%, 80%, 85%, 90%, 91%, 92%, 93%, 94%, 95%, 96%, 97%, 98% or 99% sequence identity to SEQ ID NO: 14. In some embodiments, the CFP comprises a sequence of SEQ ID NO: 15 or with at least 70%, 75%, 80%, 85%, 90%, 91%, 92%, 93%, 94%, 95%, 96%, 97%, 98% or 99% sequence identity to SEQ ID NO: 15. In some embodiments, the CFP comprises a sequence of SEQ ID NO: 16 or with at least 70%, 75%, 80%, 85%, 90%, 91%, 92%, 93%, 94%, 95%, 96%, 97%, 98% or 99% sequence identity to SEQ ID NO: 16. In some embodiments, the CFP comprises a sequence of SEQ ID NO: 24 or with at least 70%, 75%, 80%, 85%, 90%, 91%, 92%, 93%, 94%, 95%, 96%, 97%, 98% or 99% sequence identity to SEQ ID NO: 24. In some embodiments, the CFP comprises a sequence of SEQ ID NO:25 or with at least 70%, 75%, 80%, 85%, 70%, 75%, 80%, 85%, 90%, 91%, 92%, 93%, 94%, 95%, 96%, 97%, 98% or 99%, 91%, 92%, 93%, 94%, 95%, 96%, 97%, 98% or 99% sequence identity to SEQ ID NO: 25.

In some embodiments, the CFP comprises: (a) an extracellular domain comprising: (i) a scFv that specifically binds CD5, and (ii) a hinge domain derived from CD8; a hinge domain derived from CD28 or at least a portion of an extracellular domain from CD68; (b) a CD8 transmembrane domain, a CD28 transmembrane domain, a CD2 transmembrane domain or a CD68 transmembrane domain; and (c) an intracellular domain comprising at least two intracellular signaling domains, wherein the at least two intracellular signaling domains comprise: (i) a first intracellular signaling domain derived from FcRα, FcRγ or FcRε, and (ii) a second intracellular signaling domain: (A) comprising a PI3K recruitment domain, or (B) derived from CD40. In some embodiments, the CFP comprises as an alternative (c) to the above: an intracellular domain comprising at least two intracellular signaling domains, wherein the at least two intracellular signaling domains comprise: (i) a first intracellular signaling domain derived from a phagocytic receptor intracellular domain, and (ii) a second intracellular signaling domain derived from a scavenger receptor phagocytic receptor intracellular domain comprising: (A) comprising a PI3K recruitment domain, or (B) derived from CD40. Exemplary scavenger receptors from which an intracellular signaling domain may be derived may be found in Table 2. In some embodiments, the CFP comprises and intracellular signaling domain derived from an intracellular signaling domain of an innate immune receptor.

In some embodiments, the recombinant polynucleic acid is an mRNA. In some embodiments, the recombinant polynucleic acid is a circRNA. In some embodiments, the recombinant polynucleic acid is a viral vector. In some embodiments, the recombinant polynucleic acid is delivered via a viral vector.

In some embodiments, the myeloid cell is a CD14+ cell, a CD14+/CD16- cell, a CD14+/CD16+ cell, a CD14-/CD16+ cell, CD14-/CD16- cell, a dendritic cell, an M0 macrophage, an M2 macrophage, an M1 macrophage or a mosaic myeloid cell/macrophage/dendritic cell.

In one aspect, provided herein is a method of treating cancer in a human subject in need thereof comprising administering a pharmaceutical composition to the human subject, the pharmaceutical composition comprising: (a) a myeloid cell comprising a recombinant polynucleic acid sequence, wherein the polynucleic acid sequence comprises a sequence encoding a chimeric fusion protein (CFP), the CFP comprising: (i) an extracellular domain comprising an anti-CD5 binding domain, and (ii) a transmembrane domain operatively linked to the extracellular domain; and (b) a pharmaceutically acceptable carrier; wherein the myeloid cell expresses the CFP.

In some embodiments, upon binding of the CFP to CD5 expressed by a target cancer cell of the subject killing or phagocytosis activity of the myeloid cell is increased by greater than 20% compared to a myeloid cell not expressing the CFP. In some embodiments, growth of a tumor is inhibited in the human subject.

In some embodiments, the cancer is a CD5+ cancer. In some embodiments, the cancer is leukemia, T cell lymphoma, or B cell lymphoma.

In some embodiments, the anti-CD5 binding domain is a CD5 binding protein that comprises an antigen binding fragment of an antibody, an scFv domain, an Fab fragment, or an sdAb domain. In some embodiments, the anti-CD5 binding domain is a protein or fragment thereof that binds to CD5, such as a ligand of CD5 (e.g., a natural ligand of CD5).

In some embodiments, the CFP further comprises an intracellular domain, wherein the intracellular domain comprises one or more intracellular signaling domains, wherein the one or more intracellular signaling domains comprises a phagocytosis signaling domain and wherein a wild-type protein comprising the intracellular domain does not comprise the extracellular domain.

In some embodiments, the phagocytosis signaling domain comprises an intracellular signaling domain derived from a receptor other than Megf10, MerTk, FcRα and Bai1. In some embodiments, the phagocytosis signaling domain comprises an intracellular signaling domain derived from FcRy, FcRα or FcRε.

In some embodiments, the one or more intracellular signaling domains further comprises a proinflammatory signaling domain. In some embodiments, the proinflammatory signaling domain comprises a PI3-kinase (PI3K) recruitment domain. In some embodiments, the transmembrane domain comprises a CD8 transmembrane domain. In some embodiments, the extracellular domain comprises a hinge domain derived from CD8, a hinge domain derived from CD28 or at least a portion of an extracellular domain from CD68.

In some embodiments, the CFP comprises: (a) an extracellular domain comprising: (i) a scFv that specifically binds CD5, and (ii) a hinge domain derived from CD8, a hinge domain derived from CD28 or at least a portion of an extracellular domain from CD68; (b) a CD8 transmembrane domain, a CD28 transmembrane domain, a CD2 transmembrane domain or a CD68 transmembrane domain; and (c) an intracellular domain comprising at least two intracellular signaling domains, wherein the at least two intracellular signaling domains comprise: (i) a first intracellular signaling domain derived from FcRγ or FcRε, and (ii) a second intracellular signaling domain that: (A) comprises a PI3K recruitment domain, or (B) is derived from CD40. In some embodiments, the recombinant nucleic acid is mRNA or circRNA. In some embodiments, the myeloid cell is a CD14+ cell, a CD14+/CD16- cell, a CD14+/CD16+ cell, a CD14-/CD16+ cell, CD14-/CD16- cell, a dendritic cell, an M0 macrophage, an M2 macrophage, an M1 macrophage or a mosaic myeloid cell/macrophage/dendritic cell.

In some embodiments, the method further comprises administering an additional therapeutic agent selected from the group consisting of a CD47 agonist, an agent that inhibits Rac, an agent that inhibits Cdc42, an agent that inhibits a GTPase, an agent that promotes F-actin disassembly, an agent that promotes PI3K recruitment to the PFP, an agent that promotes PI3K activity, an agent that promotes production of phosphatidylinositol 3,4,5-trisphosphate, an agent that promotes ARHGAP12 activity, an agent that promotes ARHGAP25 activity, an agent that promotes SH3BP1 activity, an agent that promotes sequestration of lymphocytes in primary and/or secondary lymphoid organs, an agent that increases concentration of naive T cells and central memory T cells in secondary lymphoid organs, and any combination thereof.

In some embodiments, the myeloid cell further comprises: (a) an endogenous peptide or protein that dimerizes with the CFP, (b) a non-endogenous peptide or protein that dimerizes with the CFP; and/or (c) a second recombinant polynucleic acid sequence, wherein the second recombinant polynucleic acid sequence comprises a sequence encoding a peptide or protein that interacts with the CFP; wherein the dimerization or the interaction potentiates phagocytosis by the myeloid cell expressing the CFP as compared to a myeloid cell that does not express the CFP.

In some embodiments, the myeloid cell exhibits (i) an increase in effector activity, cross-presentation, respiratory burst, ROS production, iNOS production, inflammatory mediators, extra-cellular vesicle production, phosphatidylinositol 3,4,5-trisphosphate production, trogocytosis with the target cell expressing the antigen, resistance to CD47 mediated inhibition of phagocytosis, resistance to LILRB1 mediated inhibition of phagocytosis, or any combination thereof; and/or (ii) an increase in expression of a IL-1, IL3, IL-6, IL-10, IL-12, IL-13, IL-23, TNFα, a TNF family of cytokines, CCL2, CXCL9, CXCL10, CXCL11, IL-18, IL-23, IL-27, CSF, MCSF, GMCSF, IL-17, IP-10, RANTES, an interferon, MHC class I protein, MHC class II protein, CD40, CD48, CD58, CD80, CD86, CD112, CD155, a TRAIL/TNF Family death receptor, TGFβ, B7-DC, B7-H2, LIGHT, HVEM, TL1A, 41BBL, OX40L, GITRL, CD30L, TIM1, TIM4, SLAM, PDL1, an MMP (e.g., MMP2, MMP7 and MMP9) or any combination thereof.

In some embodiments, the intracellular signaling domain is derived from a phagocytic or tethering receptor or wherein the intracellular signaling domain comprises a phagocytosis activation domain. In some embodiments, the intracellular signaling domain is derived from a receptor other than a phagocytic receptor selected from Megf10, MerTk, FcR-alpha, or Bai1. In some embodiments, the intracellular signaling domain is derived from a protein, such as receptor (e.g., a phagocytic receptor), selected from the group consisting of TNFR1, MDA5, CD40, lectin, dectin 1, CD206, scavenger receptor A1 (SRA1), MARCO, CD36, CD163, MSR1, SCARA3, COLEC12, SCARA5, SCARB1, SCARB2, CD68, OLR1, SCARF1, SCARF2, CXCL16, STAB1, STAB2, SRCRB4D, SSC5D, CD205, CD207, CD209, RAGE, CD14, CD64, F4/80, CCR2, CX3CR1, CSF1R, Tie2, HuCRIg(L), CD64, CD32a, CD16a, CD89, Fcα receptor I, CR1, CD35, CD3ζ, a complement receptor, CR3, CR4, Tim-1, Tim-4 and CD169. In some embodiments, the intracellular signaling domain comprises a pro-inflammatory signaling domain. In some embodiments, the intracellular signaling domain comprises a pro-inflammatory signaling domain that is not a PI3K recruitment domain.

In some embodiments, the intracellular signaling domain is derived from an ITAM domain containing receptor.

Provided herein is a composition comprising a recombinant nucleic acid encoding a CFP, such as a phagocytic or tethering receptor (PR) fusion protein (PFP), comprising: a PR subunit comprising: a transmembrane domain, and an intracellular domain comprising an intracellular signaling domain; and an extracellular domain comprising an antigen binding domain specific to an antigen of a target cell; wherein the transmembrane domain and the extracellular domain are operatively linked; and wherein the intracellular signaling domain is derived from a phagocytic receptor other than a phagocytic receptor selected from Megf10, MerTk, FcRα, or Bai1.

In some embodiments, upon binding of the CFP to the antigen of the target cell, the killing activity of a cell expressing the CFP is increased by at least greater than 5%, 6%, 7%, 8%, 9%, 10%, 11%, 12%, 13%, 14%, 15%, 16%, 17%, 18%, 19%, 20%, 25%, 30%, 35%, 40%, 45%, 50%, 55%, 60%, 65%, 70%, 75%, 80%, 85%, 90%, 100%, 150%, 200%, 250%, 300%, 350%, 400%, 450%, 500%, 550%, 600%, 650%, 700%, 750%, 800%, 850%, 900%, 950%, or 1000% compared to a cell not expressing the CFP. In some embodiments, the CFP functionally incorporates into a cell membrane of a cell when the CFP is expressed in the cell. In some embodiments, upon binding of the CFP to the antigen of the target cell, the killing activity of a cell expressing the CFP is increased by at least 1.1-fold, 1.5-fold, 2-fold, 2.5-fold, 3-fold, 3.5-fold, 4-fold, 4.5-fold, 5-fold, 5.5-fold, 6-fold, 6.5-fold, 7-fold, 7.5-fold, 8-fold, 8.5-fold, 9-fold, 9.5-fold, 10-fold, 11-fold, 12-fold, 13-fold, 14-fold, 15-fold, 16-fold,-fold, 17-fold, 18-fold, 19-fold, 20-fold, 25-fold, 30-fold, 40-fold, 50-fold, 75-fold, or 100-fold compared to a cell not expressing the CFP.

In some embodiments, the intracellular signaling domain is derived from a receptor, such as a phagocytic receptor, selected from the group consisting of TNFR1, MDA5, CD40, lectin, dectin 1, CD206, scavenger receptor A1 (SRA1), MARCO, CD36, CD163, MSR1, SCARA3, COLEC12, SCARA5, SCARB1, SCARB2, CD68, OLR1, SCARF1, SCARF2, CXCL16, STAB1, STAB2, SRCRB4D, SSC5D, CD205, CD207, CD209, RAGE, CD14, CD64, F4/80, CCR2, CX3CR1, CSF1R, Tie2, HuCRIg(L), CD64, CD32a, CD16a, CD89, Fcα receptor I, CR1, CD35, CD3ζ, CR3, CR4, Tim-1, Tim-4 and CD169. In some embodiments, the intracellular signaling domain comprises a pro-inflammatory signaling domain.

Provided herein is a composition comprising a recombinant nucleic acid encoding a CFP, such as a phagocytic or tethering receptor (PR) fusion protein (PFP), comprising: a PR subunit comprising: a transmembrane domain, and an intracellular domain comprising an intracellular signaling domain; and an extracellular domain comprising an antigen binding domain specific to an antigen of a target cell; wherein the transmembrane domain and the extracellular domain are operatively linked; and wherein the intracellular signaling domain is derived from a receptor, such as a phagocytic receptor, selected from the group consisting of TNFR1, MDA5, CD40, lectin, dectin 1, CD206, scavenger receptor A1 (SRA1), MARCO, CD36, CD163, MSR1, SCARA3, COLEC12, SCARA5, SCARB1, SCARB2, CD68, OLR1, SCARF1, SCARF2, CXCL16, STAB1, STAB2, SRCRB4D, SSC5D, CD205, CD207, CD209, RAGE, CD14, CD64, F4/80, CCR2, CX3CR1, CSF1R, Tie2, HuCRIg(L), CD64, CD32a, CD16a, CD89, Fcα receptor I, CR1, CD35, CD3ζ, CR3, CR4, Tim-1, Tim-4 and CD169.

In some embodiments, upon binding of the CFP to the antigen of the target cell, the killing activity of a cell expressing the CFP is increased by at least greater than 5%, 6%, 7%, 8%, 9%, 10%, 11%, 12%, 13%, 14%, 15%, 16%, 17%, 18%, 19%, 20%, 25%, 30%, 35%, 40%, 45%, 50%, 55%, 60%, 65%, 70%, 75%, 80%, 85%, 90%, 100%, 150%, 200%, 250%, 300%, 350%, 400%, 450%, 500%, 550%, 600%, 650%, 700%, 750%, 800%, 850%, 900%, 950%, or 1000% compared to a cell not expressing the CFP. In some embodiments, the intracellular signaling domain is derived from a phagocytic receptor other than a phagocytic receptor selected from Megf10, MerTk, FcRα, or Bai1. In some embodiments, the intracellular signaling domain comprises a pro-inflammatory signaling domain. In some embodiments, the intracellular signaling domain comprises a PI3K recruitment domain, such as a PI3K recruitment domain derived from CD19. In some embodiments, the intracellular signaling domain comprises a pro-inflammatory signaling domain that is not a PI3K recruitment domain.

Provided herein is a composition comprising a recombinant nucleic acid encoding a CFP, such as a phagocytic or tethering receptor (PR) fusion protein (PFP), comprising: a PR subunit comprising: a transmembrane domain, and an intracellular domain comprising an intracellular signaling domain; and an extracellular domain comprising an antigen binding domain specific to an antigen of a target cell; wherein the transmembrane domain and the extracellular domain are operatively linked; and wherein the intracellular signaling domain comprises a pro-inflammatory signaling domain that is not a PI3K recruitment domain.

Provided herein is a composition of an engineered CFP, such as a phagocytic receptor fusion protein, that may be expressed in a cell, such as a myeloid cell, such as to generate an engineered myeloid cell that can target a target cell, such as a diseased cell.

A target cell is, for example, a cancer cell. In some embodiments, the engineered myeloid cell, after engulfment of a cancer cell may present a cancer antigen on its cell surface to activate a T cell. An "antigen" is a molecule capable of stimulating an immune response. Antigens recognized by T cells, whether helper T lymphocytes (T helper (TH) cells) or cytotoxic T lymphocytes (CTLs), are not recognized as intact proteins, but rather as small peptides that associate with MHC proteins (such as class I or class II MHC proteins) on the surface of cells. During the course of a naturally occurring immune response, antigens that are recognized in association with class II MHC molecules on antigen presenting cells (APCs) are acquired from outside the cell, internalized, and processed into small peptides that associate with the class II MHC molecules.

In some embodiments, upon binding of the CFP to the antigen of the target cell, the killing activity of a cell expressing the CFP is increased by at least greater than 5%, 6%, 7%, 8%, 9%, 10%, 11%, 12%, 13%, 14%, 15%, 16%, 17%, 18%, 19%, 20%, 25%, 30%, 35%, 40%, 45%, 50%, 55%, 60%, 65%, 70%, 75%, 80%, 85%, 90%, 100%, 150%, 200%, 250%, 300%, 350%, 400%, 450%, 500%, 550%, 600%, 650%, 700%, 750%, 800%, 850%, 900%, 950%, or 1000% compared to a cell not expressing the CFP. In some embodiments, the CFP functionally incorporates into a cell membrane of a cell when the CFP is expressed in the cell. In some embodiments, upon binding of the CFP to the antigen of the target cell, the killing activity of a cell expressing the CFP is increased by at least 1.1-fold, 1.5-fold, 2-fold, 2.5-fold, 3-fold, 3.5-fold, 4-fold, 4.5-fold, 5-fold, 5.5-fold, 6-fold, 6.5-fold, 7-fold, 7.5-fold, 8-fold, 8.5-fold, 9-fold, 9.5-fold, 10-fold, 11-fold, 12-fold, 13-fold, 14-fold, 15-fold, 16-fold,-fold, 17-fold, 18-fold, 19-fold, 20-fold, 25-fold, 30-fold, 40-fold, 50-fold, 75-fold, or 100-fold compared to a cell not expressing the CFP.

In some embodiments, the target cell expressing the antigen is a cancer cell. In some embodiments, the target cell expressing the antigen is at least 0.8 microns in diameter.

In some embodiments, a cell expressing the CFP exhibits an increase in phagocytosis of a target cell expressing the antigen compared to a cell not expressing the CFP. In some embodiments, a cell expressing the CFP exhibits at least a 1.1-fold increase in phagocytosis of a target cell expressing the antigen compared to a cell not expressing the CFP. In some embodiments, a cell expressing the CFP exhibits at least a 2-fold, 3-fold, 4-fold, 5-fold, 6-fold, 7-fold, 8-fold, 9-fold, 10-fold, 20-fold, 30-fold or 50-fold increase in phagocytosis of a target cell expressing the antigen compared to a cell not expressing the CFP. In some embodiments, a cell expressing the CFP exhibits an increase in production of a cytokine compared to a cell not expressing the CFP. In some embodiments, the cytokine is selected from the group consisting of IL-1, IL3, IL-6, IL-12, IL-13, IL-23, TNF, CCL2, CXCL9, CXCL10, CXCL11, IL-18, IL-23, IL-27, CSF, MCSF, GMCSF, IL17, IP-10, RANTES, an interferon and combinations thereof. In some embodiments, a cell expressing the CFP exhibits an increase in effector activity compared to a cell not expressing the CFP. In some embodiments, a cell expressing the CFP exhibits an increase in cross-presentation compared to a cell not expressing the CFP. In some embodiments, a cell expressing the CFP exhibits an increase in expression of an MHC class II protein compared to a cell not expressing the CFP. In some embodiments, a cell expressing the CFP exhibits an increase in expression of CD80 compared to a cell not expressing the CFP. In some embodiments, a cell expressing the CFP exhibits an increase in expression of CD86 compared to a cell not expressing the CFP. In some embodiments, a cell expressing the CFP exhibits an increase in expression of MHC class I protein compared to a cell not expressing the CFP. In some embodiments, a cell expressing the CFP exhibits an increase in expression of TRAIL/TNF Family death receptors compared to a cell not expressing the CFP. In some embodiments, a cell expressing the CFP exhibits an increase in expression of B7-H2 compared to a cell not expressing the CFP. In some embodiments, a cell expressing the CFP exhibits an increase in expression of LIGHT compared to a cell not expressing the CFP. In some embodiments, a cell expressing the CFP exhibits an increase in expression of HVEM compared to a cell not expressing the CFP. In some embodiments, a cell expressing the CFP exhibits an increase in expression of CD40 compared to a cell not expressing the CFP. In some embodiments, a cell expressing the CFP exhibits an increase in expression of TL1A compared to a cell not expressing the CFP. In some embodiments, a cell expressing the CFP exhibits an increase in expression of 41BBL compared to a cell not expressing the CFP. In some embodiments, a cell expressing the CFP exhibits an increase in expression of OX40L compared to a cell not expressing the CFP. In some embodiments, a cell expressing the CFP exhibits an increase in expression of GITRL death receptors compared to a cell not expressing the CFP. In some embodiments, a cell expressing the CFP exhibits an increase in expression of CD30L compared to a cell not expressing the CFP. In some embodiments, a cell expressing the CFP exhibits an increase in expression of TIM4 compared to a cell not expressing the CFP. In some embodiments, a cell expressing the CFP exhibits an increase in expression of TIM1 ligand compared to a cell not expressing the CFP. In some embodiments, a cell expressing the CFP exhibits an increase in expression of SLAM compared to a cell not expressing the CFP. In some embodiments, a cell expressing the CFP exhibits an increase in expression of CD48 compared to a cell not expressing the CFP. In some embodiments, a cell expressing the CFP exhibits an increase in expression of CD58 compared to a cell not expressing the CFP. In some embodiments, a cell expressing the CFP exhibits an increase in expression of CD155 compared to a cell not expressing the CFP. In some embodiments, a cell expressing the CFP exhibits an increase in expression of CD112 compared to a cell not expressing the CFP. In some embodiments, a cell expressing the CFP exhibits an increase in expression of PDL1 compared to a cell not expressing the CFP. In some embodiments, a cell expressing the CFP exhibits an increase in expression of B7-DC compared to a cell not expressing the CFP. In some embodiments, a cell expressing the CFP exhibits an increase in respiratory burst compared to a cell not expressing the CFP. In some embodiments, a cell expressing the CFP exhibits an increase in ROS production compared to a cell not expressing the CFP. In some embodiments, a cell expressing the CFP exhibits an increase in iNOS production compared to a cell not expressing the CFP. In some embodiments, a cell expressing the CFP exhibits an increase in iNOS production compared to a cell not expressing the CFP. In some embodiments, a cell expressing the CFP exhibits an increase in extra-cellular vesicle production compared to a cell not expressing the CFP. In some embodiments, a cell expressing the CFP exhibits an increase in trogocytosis with a target cell expressing the antigen compared to a cell not expressing the CFP. In some embodiments, a cell expressing the CFP exhibits an increase in resistance to CD47 mediated inhibition of phagocytosis compared to a cell not expressing the CFP. In some embodiments, a cell expressing the CFP exhibits an increase in resistance to LILRB1 mediated inhibition of phagocytosis compared to a cell not expressing the CFP. In some embodiments, a cell expressing the CFP exhibits an increase in phosphatidylinositol 3,4,5-trisphosphate production.

In some embodiments, the extracellular domain of a CFP comprises an Ig binding domain. In some embodiments, the extracellular domain comprises an IgA, IgD, IgE, IgG, IgM, FcRγI, FcγIIA, FcRyIIB, FcRγIIC, FcRyIIIA, FcRyIIIB, FcRn, TRIM21, FcRL5 binding domain. In some embodiments, the extracellular domain of a CFP comprises an FcR extracellular domain. In some embodiments, the extracellular domain of a CFP comprises an FcRα, FcRβ, FcRε or FcRγ extracellular domain. In some embodiments, the extracellular domain comprises an FcRα (FCAR) extracellular domain. In some embodiments, the extracellular domain comprises an FcRβ extracellular domain. In some embodiments, the extracellular domain comprises an FCER1A extracellular domain. In some embodiments, the extracellular domain comprises an FDGR1A, FCGR2A, FCGR2B, FCGR2C, FCGR3A, or FCGR3B extracellular domain. In some embodiments, the extracellular domain comprises an integrin domain or an integrin receptor domain. In some embodiments, the extracellular domain comprises one or more integrin $\alpha 1$, $\alpha 2$, $\alpha IIb$, $\alpha 3$, $\alpha 4$, $\alpha 5$, $\alpha 6$, $\alpha 7$, $\alpha 8$, $\alpha 9$, $\alpha 10$, $\alpha 11$, $\alpha D$, $aE$, $\alpha L$, $\alpha M$, $\alpha V$, $\alpha X$, $\beta 1$, $\beta 2$, $\beta 3$, $\beta 4$, $\beta 5$, $\beta 6$, $\beta 7$, or $\beta 8$ domains.

In some embodiments, the CFP further comprises an extracellular domain operatively linked to the transmembrane domain and the extracellular antigen binding domain. In some embodiments, the extracellular domain further comprises an extracellular domain of a receptor, a hinge, a spacer and/or a linker. In some embodiments, the extracellular domain comprises an extracellular portion of a phagocytic receptor. In some embodiments, the extracellular portion of the CFP is derived from the same receptor as the receptor from which the intracellular signaling domain is derived. In some embodiments, the extracellular domain comprises an extracellular domain of a scavenger receptor. In some embodiments, the extracellular domain comprises an immunoglobulin domain. In some embodiments, the immunoglobulin domain comprises an extracellular domain of an immunoglobulin or an immunoglobulin hinge region. In some embodiments, the extracellular domain comprises a phagocytic engulfment domain. In some embodiments, the extracellular domain comprises a structure capable of multimeric assembly. In some embodiments, the extracellular domain comprises a scaffold for multimerization. In some embodiments, the extracellular domain is at least 10, 20, 30, 40, 50, 60, 70, 80, 90, 100, 150, 200, 300, 300, 400, or 500 amino acids in length. In some embodiments, the extracellular domain is at most 500, 400, 300, 200, or 100 amino acids in length. In some embodiments, the extracellular antigen binding domain specifically binds to the antigen of a target cell. In some embodiments, the extracellular antigen binding domain comprises an antibody domain. In some embodiments, the extracellular antigen binding domain comprises a receptor domain, antibody domain, wherein the antibody domain comprises a functional antibody fragment, a single chain variable fragment (scFv), an Fab, a single-domain antibody (sdAb), a nanobody, a $V_H$ domain, a $V_L$ domain, a VNAR domain, a $V_{HH}$ domain, a bispecific antibody, a diabody, or a functional fragment or a combination thereof. In some embodiments, the extracellular antigen binding domain comprises a ligand, an extracellular domain of a receptor or an adaptor. In some embodiments, the extracellular antigen binding domain comprises a single extracellular antigen binding domain that is specific for a single antigen. In some embodiments, the extracellular antigen binding domain comprises at least two extracellular antigen binding domains, wherein each of the at least two extracellular antigen binding domains is specific for a different antigen.

In some embodiments, the antigen is a cancer associated antigen, a lineage associated antigen, a pathogenic antigen or an autoimmune antigen. In some embodiments, the antigen comprises a viral antigen. In some embodiments, the antigen is a T lymphocyte antigen. In some embodiments, the antigen is an extracellular antigen. In some embodiments, the antigen is an intracellular antigen. In some embodiments, the antigen is selected from the group consisting of an antigen from Thymidine Kinase (TK1), Hypoxanthine-Guanine Phosphoribosyltransferase (HPRT), Receptor Tyrosine Kinase-Like Orphan Receptor 1 (ROR1), Mucin-1, Mucin-16 (MUC16), MUC1, Epidermal Growth Factor Receptor vIII (EGFRvIII), Mesothelin, Human Epidermal Growth Factor Receptor 2 (HER2), EBNA-1, LEMD1, Phosphatidyl Serine, Carcinoembryonic Antigen (CEA), B-Cell Maturation Antigen (BCMA), Glypican 3 (GPC3), Follicular Stimulating Hormone receptor, Fibroblast Activation Protein (FAP), Erythropoietin-Producing Hepatocellular Carcinoma A2 (EphA2), EphB2, a Natural Killer Group 2D (NKG2D) ligand, Disialoganglioside 2 (GD2), CD2, CD3, CD4, CD5, CD7, CD8, CD19, CD20, CD22, CD24, CD30, CD33, CD38, CD44v6, CD45, CD56CD79b, CD97, CD117, CD123, CD133, CD138, CD171, CD179a, CD213A2, CD248, CD276, PSCA, CS-1, CLECL1, GD3, PSMA, FLT3, TAG72, EPCAM, IL-1, an integrin receptor, PRSS21, VEGFR2, PDGFR$\beta$, SSEA-4, EGFR, NCAM, prostase, PAP, ELF2M, GM3, TEM7R, CLDN6, TSHR, GPRC5D, ALK, Dsg1, Dsg3, IGLL1 and combinations thereof. In some embodiments, the antigen is an antigen of a protein selected from the group consisting of CD2, CD3, CD4, CD5, CD7, CCR4, CD8, CD30, CD45, and CD56. In some embodiments, the antigen is an ovarian cancer antigen or a T lymphoma antigen. In some embodiments, the antigen is an antigen of an integrin receptor. In some embodiments, the antigen is an antigen of an integrin receptor or integrin selected from the group consisting of $\alpha 1$, $\alpha 2$, $\alpha IIb$, $\alpha 3$, $\alpha 4$, $\alpha 5$, $\alpha 6$, $\alpha 7$, $\alpha 8$, $\alpha 9$, $\alpha 10$, $\alpha 11$, $\alpha D$, $\alpha E$, $\alpha L$, $\alpha M$, $\alpha V$, $\alpha X$, $\beta 1$, $\beta 2$, $\beta 3$, $\beta 4$, $\beta 5$, $\beta 6$, $\beta 7$, and $\beta 8$. In some embodiment, the antigen is an antigen of an integrin receptor ligand. In some embodiments, the antigen is an antigen of fibronectin, vitronectin, collagen, or laminin. In some embodiments, the antigen binding domain can bind to two or more different antigens.

In some embodiments, the antigen binding domain comprises an autoantigen or fragment thereof, such as Dsg1 or Dsg3. In some embodiments, the extracellular antigen binding domain comprises a receptor domain or an antibody domain wherein the antibody domain binds to an auto antigen, such as Dsg1 or Dsg3.

In some embodiments, the transmembrane domain and the extracellular antigen binding domain are operatively linked through a linker. In some embodiments, the transmembrane domain and the extracellular antigen binding domain are operatively linked through a linker such as a hinge region of CD8$\alpha$, IgG1 or IgG4.

In some embodiments, the extracellular domain comprises a multimerization scaffold.

In some embodiments, the transmembrane domain comprises a CD8 transmembrane domain. In some embodiments, the transmembrane domain comprises a CD28 transmembrane domain. In some embodiments, the transmembrane domain comprises a CD68 transmembrane domain. In some embodiments, the transmembrane domain comprises a CD2 transmembrane domain. In some embodiments, the transmembrane domain comprises an FcR transmembrane domain. In some embodiments, the transmembrane domain comprises an FcR$\gamma$ transmembrane domain.

In some embodiments, the transmembrane domain comprises an FcRα transmembrane domain. In some embodiments, the transmembrane domain comprises an FcRβ transmembrane domain. In some embodiments, the transmembrane domain comprises an FcRε transmembrane domain. In some embodiments, the transmembrane domain comprises a transmembrane domain from a syntaxin, such as syntaxin 3 or syntaxin 4 or syntaxin 5. In some embodiments, the transmembrane domain oligomerizes with a transmembrane domain of an endogenous receptor when the CFP is expressed in a cell. In some embodiments, the transmembrane domain oligomerizes with a transmembrane domain of an exogenous receptor when the CFP is expressed in a cell. In some embodiments, the transmembrane domain dimerizes with a transmembrane domain of an endogenous receptor when the CFP is expressed in a cell. In some embodiments, the transmembrane domain dimerizes with a transmembrane domain of an exogenous receptor when the CFP is expressed in a cell. In some embodiments, the transmembrane domain is derived from a protein that is different than the protein from which the intracellular signaling domain is derived. In some embodiments, the transmembrane domain is derived from a protein that is different than the protein from which the extracellular domain is derived. In some embodiments, the transmembrane domain comprises a transmembrane domain of a phagocytic receptor. In some embodiments, the transmembrane domain and the extracellular domain are derived from the same protein. In some embodiments, the transmembrane domain is derived from the same protein as the intracellular signaling domain. In some embodiments, the recombinant nucleic acid encodes a DAP12 recruitment domain. In some embodiments, the transmembrane domain comprises a transmembrane domain that oligomerizes with DAP12.

In some embodiments, the transmembrane domain is at least 12, 13, 14, 15, 16, 17, 18, 19, 20, 21, 22, 23, 24, 25, 26, 27, 28, 29, 30, 31 or 32 amino acids in length. In some embodiments, the transmembrane domain is at most 12, 13, 14, 15, 16, 17, 18, 19, 20, 21, 22, 23, 24, 25, 26, 27, 28, 29, 30, 31 or 32 amino acids in length.

In some embodiments, the intracellular signaling domain comprises an intracellular signaling domain derived from a phagocytic receptor. In some embodiments, the intracellular signaling domain comprises an intracellular signaling domain derived from a phagocytic receptor other than a phagocytic receptor selected from Megf10, MerTk, FcRα, or Bai1. In some embodiments, the intracellular signaling domain comprises an intracellular signaling domain derived from a phagocytic receptor selected from the group consisting of TNFR1, MDA5, CD40, lectin, dectin 1, CD206, scavenger receptor A1 (SRA1), MARCO, CD36, CD163, MSR1, SCARA3, COLEC12, SCARA5, SCARB1, SCARB2, CD68, OLR1, SCARF1, SCARF2, CXCL16, STAB1, STAB2, SRCRB4D, SSC5D, CD205, CD207, CD209, RAGE, CD14, CD64, F4/80, CCR2, CX3CR1, CSF1R, Tie2, HuCRIg(L), CD64, CD32a, CD16a, CD89, Fc-alpha receptor I, CR1, CD35, CD3ζ, CR3, CR4, Tim-1, Tim-4 and CD169. In some embodiments, the intracellular signaling domain comprises a PI3K recruitment domain. In some embodiments, the intracellular signaling domain comprises an intracellular signaling domain derived from a scavenger receptor. In some embodiments, the intracellular domain comprises a CD47 inhibition domain. In some embodiments, the intracellular domain comprises a Rac inhibition domain, a Cdc42 inhibition domain or a GTPase inhibition domain. In some embodiments, the Rac inhibition domain, the Cdc42 inhibition domain or the GTPase inhibition domain inhibits Rac, Cdc42 or GTPase at a phagocytic cup of a cell expressing the PFP. In some embodiments, the intracellular domain comprises an F-actin disassembly activation domain, a ARHGAP12 activation domain, a ARHGAP25 activation domain or a SH3BP1 activation domain. In some embodiments, the intracellular domain comprises a phosphatase inhibition domain. In some embodiments, the intracellular domain comprises an ARP2/3 inhibition domain. In some embodiments, the intracellular domain comprises at least one ITAM domain. In some embodiments, the intracellular domain comprises at least 1, 2, 3, 4, 5, 6, 7, 8, 9, 10, or more ITAM domains. In some embodiments, the intracellular domain comprises at least one ITAM domain select from an ITAM domain of CD3 zeta, CD3 epsilon, CD3 gamma, CD3 delta, Fc epsilon receptor 1 chain, Fc epsilon receptor 2 chain, Fc gamma receptor 1 chain, Fc gamma receptor 2a chain, Fc gamma receptor 2b 1 chain, Fc gamma receptor 2b2 chain, Fc gamma receptor 3a chain, Fc gamma receptor 3b chain, Fc beta receptor 1 chain, TYROBP (DAP12), CD5, CD16a, CD16b, CD22, CD23, CD32, CD64, CD79a, CD79b, CD89, CD278, CD66d, functional fragments thereof, and amino acid sequences thereof having at least one but not more than 20 modifications thereto. In some embodiments, the at least one ITAM domain comprises a Src-family kinase phosphorylation site. In some embodiments, the at least one ITAM domain comprises a Syk recruitment domain. In some embodiments, the intracellular domain comprises an F-actin depolymerization activation domain. In some embodiments, the intracellular domain lacks enzymatic activity.

In some embodiments, the intracellular domain does not comprise a domain derived from a CD3 zeta intracellular domain. In some embodiments, the intracellular domain does not comprise a domain derived from a MerTK intracellular domain. In some embodiments, the intracellular domain does not comprise a domain derived from a TLR4 intracellular domain. In some embodiments, the intracellular domain comprises a CD47 inhibition domain. In some embodiments, the intracellular signaling domain comprises a domain that activates integrin, such as the intracellular region of PSGL-1. In some embodiments, the intracellular signaling domain comprises a domain that activates Rap1 GTPase, such as that from EPAC and C3G. In some embodiments, the intracellular signaling domain is derived from paxillin. In some embodiments, the intracellular signaling domain activates focal adhesion kinase. In some embodiments, the intracellular signaling domain is derived from a single phagocytic receptor. In some embodiments, the intracellular signaling domain is derived from a single scavenger receptor. In some embodiments, the intracellular domain comprises a phagocytosis enhancing domain.

In some embodiments, the intracellular domain comprises a pro-inflammatory signaling domain. In some embodiments, the pro-inflammatory signaling domain comprises a kinase activation domain or a kinase binding domain. In some embodiments, the pro-inflammatory signaling domain comprises an IL-1 signaling cascade activation domain. In some embodiments, the pro-inflammatory signaling domain comprises an intracellular signaling domain derived from TLR3, TLR4, TLR7, TLR 9, TRIF, RIG-1, MYD88, MAL, IRAK1, MDA-5, an IFN-receptor, STING, an NLRP family member, NLRP1-14, NOD1, NOD2, Pyrin, AIM2, NLRC4, FCGR3A, FCERIG, CD40, Tank1-binding kinase (TBK), a caspase domain, a procaspase binding domain or any combination thereof.

In some embodiments, the intracellular domain comprises a signaling domain, such as an intracellular signaling domain, derived from a connexin (Cx) protein. For example, the intracellular domain can comprise a signaling domain, such as an intracellular signaling domain, derived from Cx43, Cx46, Cx37, Cx40, Cx33, Cx50, Cx59, Cx62, Cx32, Cx26, Cx31, Cx30.3, Cx31.1, Cx30, Cx25, Cx45, Cx47, Cx31.3, Cx36, Cx31.9, Cx39, Cx40.1 or Cx23. For example, the intracellular domain can comprise a signaling domain, such as an intracellular signaling domain, derived from Cx43.

In some embodiments, the intracellular domain comprises a signaling domain, such as an intracellular signaling domain, derived from a SIGLEC protein. For example, the intracellular domain can comprise a signaling domain, such as an intracellular signaling domain, derived from Siglec-1 (Sialoadhesin), Siglec-2 (CD22), Siglec-3 (CD33), Siglec-4 (MAG), Siglec-5, Siglec-6, Siglec-7, Siglec-8, Siglec-9, Siglec-10, Siglec-11, Siglec-12, Siglec-13, Siglec-14, Siglec-15, Siglec-16 or Siglec-17.

In some embodiments, the intracellular domain comprises a signaling domain, such as an intracellular signaling domain, derived from a C-type lectin protein. For example, the intracellular domain can comprise a signaling domain, such as an intracellular signaling domain, derived from a mannose receptor protein. For example, the intracellular domain can comprise a signaling domain, such as an intracellular signaling domain, derived from an asialoglycoprotein receptor protein. For example, the intracellular domain can comprise a signaling domain, such as an intracellular signaling domain, derived from macrophage galactose-type lectin (MGL), DC-SIGN (CLEC4L), Langerin (CLEC4K), Myeloid DAP12 associating lectin (MDL)-1 (CLEC5A), a DC associated C type lectin 1 (Dectinl) subfamily protein, dectin 1/CLEC7A, DNGR1/CLEC9A, Myeloid C type lectin like receptor (MICL) (CLEC12A), CLEC2 (CLEC1B), CLEC12B, a DC immunoreceptor (DCIR) subfamily protein, DCIR/CLEC4A, Dectin 2/CLEC6A, Blood DC antigen 2 (BDCA2) ( CLEC4C), Mincle (macrophage inducible C type lectin) (CLEC4E), a NOD-like receptor protein, NOD-like receptor MHC Class II transactivator (CIITA), IPAF, BIRC1, a RIG-I-like receptor (RLR) protein, RIG-I, MDA5, LGP2, NAIP5/Birc1e, an NLRP protein, NLRP1, NLRP2, NLRP3, NLRP4, NLRP5, NLRP6, NLRP7, NLRP89, NLRP9, NLRP10, NLRP11, NLRP12, NLRP13, NLRP14, an NLR protein, NOD1 or NOD2, or any combination thereof.

In some embodiments, the intracellular domain comprises a signaling domain, such as an intracellular signaling domain, derived from a cell adhesion molecule. For example, the intracellular domain can comprise a signaling domain, such as an intracellular signaling domain, derived from an IgCAMs, a cadherin, an integrin, a C-type of lectin-like domains protein (CTLD) and/or a proteoglycan molecule. For example, the intracellular domain can comprise a signaling domain, such as an intracellular signaling domain, derived from an E-cadherin, a P-cadherin, an N-cadherin, an R-cadherin, a B-cadherin, a T-cadherin, or an M-cadherin. For example, the intracellular domain can comprise a signaling domain, such as an intracellular signaling domain, derived from a selectin, such as an E-selectin, an L-selectin or a P-selectin.

In some embodiments, the CFP does not comprise a full length intracellular signaling domain. In some embodiments, the intracellular domain is at least 5, 10, 20, 30, 40, 50, 60, 70, 80, 90, 100, 150, 200, 300, 300, 400, or 500 amino acids in length. In some embodiments, the intracellular domain is at most 10, 20, 30, 40, 50, 60, 70, 80, 90, 100, 150, 200, 300, 300, 400, or 500 amino acids in length.

In some embodiments, the recombinant nucleic acid encodes an FcRα chain extracellular domain, an FcRα chain transmembrane domain and/or an FcRα chain intracellular domain. In some embodiments, the recombinant nucleic acid encodes an FcRβ chain extracellular domain, an FcRβ chain transmembrane domain and/or an FcRβ chain intracellular domain. In some embodiments, the FcRα chain or the FcRβ chain forms a complex with FcRγ when expressed in a cell. In some embodiments, the FcRα chain or FcRβ chain forms a complex with endogenous FcRγ when expressed in a cell. In some embodiments, the FcRα chain or the FcRβ chain does not incorporate into a cell membrane of a cell that does not express FcRγ. In some embodiments, the CFP does not comprise an FcRα chain intracellular signaling domain. In some embodiments, the CFP does not comprise an FcRβ chain intracellular signaling domain. In some embodiments, the recombinant nucleic acid encodes a TREM extracellular domain, a TREM transmembrane domain and/or a TREM intracellular domain. In some embodiments, the TREM is TREM1, TREM 2 or TREM 3.

In some embodiments, the recombinant nucleic acid comprises a sequence encoding a pro-inflammatory polypeptide. In some embodiments, the composition further comprises a proinflammatory nucleotide or a nucleotide in the recombinant nucleic acid, for example, an ATP, ADP, UTP, UDP, and/or UDP-glucose.

In some embodiments, the composition further comprises a pro-inflammatory polypeptide. In some embodiments, the pro-inflammatory polypeptide is a chemokine, cytokine. In some embodiments, the chemokine is selected from the group consisting of IL-1, IL3, IL5, IL-6, i18, IL-12, IL-13, IL-23, TNF, CCL2, CXCL9, CXCL10, CXCL11, IL-18, IL-23, IL-27, CSF, MCSF, GMCSF, IL17, IP-10, RANTES, and interferon. In some embodiments, the cytokine is selected from the group consisting of IL-1, IL3, IL5, IL-6, IL-12, IL-13, IL-23, TNF, CCL2, CXCL9, CXCL10, CXCL11, IL-18, IL-23, IL-27, CSF, MCSF, GMCSF, IL17, IP-10, RANTES, and interferon.

In some embodiments, the myeloid cells are specifically targeted for delivery. Myeloid cells can be targeted using specialized biodegradable polymers, such as PLGA (poly(lactic-co-glycolic) acid and/or polyvinyl alcohol (PVA). In some embodiments, one or more compounds can be selectively incorporated in such polymeric structures to affect the myeloid cell function. In some embodiments, the targeting structures are multilayered, e.g., of one or more PLGA and one or more PVA layers. In some embodiments, the targeting structures are assembled in an order for a layered activity. In some embodiments, the targeted polymeric structures are organized in specific shaped components, such as labile structures that can adhere to a myeloid cell surface and deliver one or more components such as growth factors and cytokines, such as to maintain the myeloid cell in a microenvironment that endows a specific polarization. In some embodiments, the polymeric structures are such that they are not phagocytosed by the myeloid cell, but they can remain adhered on the surface. In some embodiments the one or more growth factors may be M1 polarization factors, such as a cytokine. In some embodiments the one or more growth factors may be an M2 polarization factor, such as a cytokine. In some embodiments, the one or more growth factors may be a macrophage activating cytokine, such as IFNγ. In some embodiments the polymeric structures are capable of sustained release of the one or more growth factors in an in vivo environment, such as in a solid tumor.

In some embodiments, the recombinant nucleic acid comprises a sequence encoding a homeostatic regulator of inflammation. In some embodiments, the homeostatic regulator of inflammation is a sequence in an untranslated region (UTR) of an mRNA. In some embodiments, the sequence in the UTR is a sequence that binds to an RNA binding protein. In some embodiments, translation is inhibited or prevented upon binding of the RNA binding protein to the sequence in an untranslated region (UTR). In some embodiments, the sequence in the UTR comprises a consensus sequence of WWWU(AUUUA)UUUW (SEQ ID NO: 35), wherein W is A or U. In some embodiments, the recombinant nucleic acid is expressed on a bicistronic vector.

In some embodiments, the target cell is a mammalian cell. In some embodiments, the target cell is a human cell. In some embodiments, the target cell comprises a cell infected with a pathogen. In some embodiments, the target cell is a cancer cell. In some embodiments, the target cell is a cancer cell that is a lymphocyte. In some embodiments, the target cell is a cancer cell that is an ovarian cancer cell. In some embodiments, the target cell is a cancer cell that is a breast cell. In some embodiments, the target cell is a cancer cell that is a pancreatic cell. In some embodiments, the target cell is a cancer cell that is a glioblastoma cell.

In some embodiments, the recombinant nucleic acid is DNA. In some embodiments, the recombinant nucleic acid is RNA. In some embodiments, the recombinant nucleic acid is mRNA. In some embodiments, the recombinant nucleic acid is an unmodified mRNA. In some embodiments, the recombinant nucleic acid is a modified mRNA. In some embodiments, the recombinant nucleic acid is a circRNA. In some embodiments, the recombinant nucleic acid is a tRNA. In some embodiments, the recombinant nucleic acid is a microRNA.

Also provided herein is a vector comprising a recombinant nucleic acid sequence encoding a CFP described herein. In some embodiments, the vector is viral vector. In some embodiments, the viral vector is retroviral vector or a lentiviral vector. In some embodiments, the vector further comprises a promoter operably linked to at least one nucleic acid sequence encoding one or more polypeptides. In some embodiments, the vector is polycistronic. In some embodiments, each of the at least one nucleic acid sequence is operably linked to a separate promoter. In some embodiments, the vector further comprises one or more internal ribosome entry sites (IRESs). In some embodiments, the vector further comprises a 5'UTR and/or a 3'UTR flanking the at least one nucleic acid sequence encoding one or more polypeptides. In some embodiments, the vector further comprises one or more regulatory regions.

Also provided herein is a polypeptide encoded by the recombinant nucleic acid of a composition described herein.

Also provided herein is a cell comprising a composition described herein, a vector described herein or a polypeptide described herein. In some embodiments, the cell is a phagocytic cell. In some embodiments, the cell is a stem cell derived cell, a myeloid cell, a macrophage, a dendritic cell, a lymphocyte, a mast cell, a monocyte, a neutrophil, a microglia, or an astrocyte. In some embodiments, the cell is an autologous cell. In some embodiments, the cell is an allogeneic cell. In some embodiments, the cell is an M1 cell. In some embodiments, the cell is an M2 cell. In some embodiments, the cell is an M1 macrophage cell. In some embodiments, the cell is an M2 macrophage cell. In some embodiments, the cell is an M1 myeloid cell. In some embodiments, the cell is an M2 myeloid cell.

Also provided herein is a pharmaceutical composition comprising a composition described herein, such as a recombinant nucleic acid described herein, a vector described herein, a polypeptide described herein or a cell described herein; and a pharmaceutically acceptable excipient.

In some embodiments, the pharmaceutical composition further comprises an additional therapeutic agent. In some embodiments, the additional therapeutic agent is selected from the group consisting of a CD47 agonist, an agent that inhibits Rac, an agent that inhibits Cdc42, an agent that inhibits a GTPase, an agent that promotes F-actin disassembly, an agent that promotes PI3K recruitment to the PFP, an agent that promotes PI3K activity, an agent that promotes production of phosphatidylinositol 3,4,5-trisphosphate, an agent that promotes ARHGAP12 activity, an agent that promotes ARHGAP25 activity, an agent that promotes SH3BP1 activity and any combination thereof. In some embodiments, the pharmaceutically acceptable excipient comprises serum free media, a lipid, or a nanoparticle.

Also provided herein is a method of treating a disease in a subject in need thereof comprising administering to the subject a pharmaceutical composition described herein. In some embodiments, the disease is cancer. In some embodiments, the cancer is a solid cancer. In some embodiments, the solid cancer is selected from the group consisting of ovarian cancer, suitable cancers include ovarian cancer, renal cancer, breast cancer, prostate cancer, liver cancer, brain cancer, lymphoma, leukemia, skin cancer, pancreatic cancer, colorectal cancer, lung cancer. In some embodiments, the cancer is a liquid cancer. In some embodiments, the liquid cancer is leukemia or a lymphoma. In some embodiments, the liquid cancer is a T cell lymphoma. In some embodiments, the disease is a T cell malignancy.

In some embodiments, the method further comprises administering an additional therapeutic agent to the subject. In some embodiments, the additional therapeutic agent is selected from the group consisting of a CD47 agonist, an agent that inhibits Rac, an agent that inhibits Cdc42, an agent that inhibits a GTPase, an agent that promotes F-actin disassembly, an agent that promotes PI3K recruitment to the PFP, an agent that promotes PI3K activity, an agent that promotes production of phosphatidylinositol 3,4,5-trisphosphate, an agent that promotes ARHGAP12 activity, an agent that promotes ARHGAP25 activity, an agent that promotes SH3BP1 activity and any combination thereof.

In some embodiments, administering comprises infusing or injecting. In some embodiments, administering comprises administering directly to the solid cancer. In some embodiments, administering comprises a circRNA-based delivery procedure, anon-particle encapsulated mRNA-based delivery proceudre, an mRNA-based delivery procedure, viral-based delivery procedure, particle-based delivery procedure, liposome-based delivery procedure, or an exosome-based delivery procedure. In some embodiments, a CD4+ T cell response or a CD8+ T cell response is elicited in the subject.

Also provided herein is a method of preparing a cell, the method comprising contacting a cell with a composition described herein, a vector described herein or a polypeptide described herein. In some embodiments, contacting comprises transducing. In some embodiments, contacting comprises chemical transfection, electroporation, nucleofection, or viral infection or transduction.

Also provided herein is a method of preparing a pharmaceutical composition comprising contacting a lipid to a composition described herein or a vector described herein. In some embodiments, contacting comprises forming a lipid nanoparticle.

Also provided herein is a method of preparing a pharmaceutical composition comprising contacting an antibody to a composition described herein or the vector described herein. In some embodiments, contacting comprises forming a lipid nanoparticle.

## Definitions

An "agent" can refer to any cell, small molecule chemical compound, antibody or fragment thereof, nucleic acid molecule, or polypeptide.

An "alteration" or "change" can refer to an increase or decrease. For example, an alteration can be an increase or decrease of 1%, 2%, 3%, 4%, 5%, 10%, 20%, 30%, or by 40%, 50%, 60%, or even by as much as 70%, 75%, 80%, 90%, or 100%. For example, an alteration can be an increase or decrease of 1-fold, 2-fold, 3-fold, 4-fold, 5-fold, 10-fold, 20-fold, 30-fold, or by 40-fold, 50-fold, 60-fold, or even by as much as 70-fold, 75-fold, 80-fold, 90-fold, or 100-fold.

An "antigen presenting cell" or "APC" as used herein includes professional antigen presenting cells (e.g., B lymphocytes, macrophages, monocytes, dendritic cells, Langerhans cells), as well as other antigen presenting cells (e.g., keratinocytes, endothelial cells, astrocytes, fibroblasts, oligodendrocytes, thymic epithelial cells, thyroid epithelial cells, glial cells (brain), pancreatic beta cells, and vascular endothelial cells). An APC can express Major Histocompatibility complex (MHC) molecules and can display antigens complexed with MHC on its surface which can be recognized by T cells and trigger T cell activation and an immune response. Professional antigen-presenting cells, notably dendritic cells, play a key role in stimulating naive T cells. Nonprofessional antigen-presenting cells, such as fibroblasts, may also contribute to this process. APCs can also cross-present peptide antigens by processing exogenous antigens and presenting the processed antigens on class I MHC molecules. Antigens that give rise to proteins that are recognized in association with class I MHC molecules are generally proteins that are produced within the cells, and these antigens are processed and associate with class I MHC molecules.

A "biological sample" can refer to any tissue, cell, fluid, or other material derived from an organism.

The term "epitope" can refer to any protein determinant, such as a sequence or structure or amino acid residues, capable of binding to an antibody or binding fragment thereof, a T cell receptor, and/or an antibody-like molecule. Epitopic determinants typically consist of chemically active surface groups of molecules such as amino acids or sugar side chains and generally have specific three dimensional structural characteristics as well as specific charge characteristics. A "T cell epitope" can refer to peptide or peptide-MHC complex recognized by a T cell receptor.

An engineered cell, such as an engineered myeloid cell, can refer to a cell that has at least one exogenous nucleic acid sequence in the cell, even if transiently expressed. Expressing an exogenous nucleic acid may be performed by various methods described elsewhere, and encompasses methods known in the art. The present disclosure relates to preparing and using engineered cells, for example, engineered myeloid cells, such as engineered phagocytic cells. The present disclosure relates to, *inter alia,* an engineered cell comprising an exogenous nucleic acid encoding, for example, a chimeric fusion protein (CFP).

The term "immune response" includes, but is not limited to, T cell mediated, NK cell mediated and/or B cell mediated immune responses. These responses may be influenced by modulation of T cell costimulation and NK cell costimulation. Exemplary immune responses include T cell responses, e.g., cytokine production, and cellular cytotoxicity. In addition, immune responses include immune responses that are indirectly affected by NK cell activation, B cell activation and/or T cell activation, e.g., antibody production (humoral responses) and activation of cytokine responsive cells, e.g., macrophages. Immune responses include adaptive immune responses. The adaptive immune system can react to foreign molecular structures, such as antigens of an intruding organism. Unlike the innate immune system, the adaptive immune system is highly specific to a pathogen. Adaptive immunity can also provide long-lasting protection. Adaptive immune reactions include humoral immune reactions and cell-mediated immune reactions. In humoral immune reactions, antibodies secreted by B cells into bodily fluids bind to pathogen-derived antigens leading to elimination of the pathogen through a variety of mechanisms, e.g. complement-mediated lysis. In cell-mediated immune reactions, T cells capable of destroying other cells are activated. For example, if proteins associated with a disease are present in a cell, they can be fragmented proteolytically to peptides within the cell. Specific cell proteins can then attach themselves to the antigen or a peptide formed in this manner, and transport them to the surface of the cell, where they can be presented to molecular defense mechanisms, such as T cells. Cytotoxic T cells can recognize these antigens and kill cells that harbor these antigens.

A "ligand" can refer to a molecule which is capable of binding or forming a complex with another molecule, such as a receptor. A ligand can include, but is not limited to, a protein, a glycoprotein, a carbohydrate, a lipoprotein, a hormone, a fatty acid, a phospholipid, or any component that binds to a receptor. In some embodiments, a receptor has a specific ligand. In some embodiments, a receptor may have promiscuous binding to a ligand, in which case it can bind to several ligands that share at least a similarity in structural configuration, charge distribution or any other physicochemical characteristic. A ligand may be a biomolecule. A ligand may be an abiotic material. For example, a ligand may be a negative charged particle that is a ligand for scavenger receptor MARCO. For example, a ligand may be $TiO_2$, which is a ligand for the scavenger receptor SRA1.

The term "major histocompatibility complex (MHC)", "MHC molecule", or "MHC protein" refers to a protein capable of binding an antigenic peptide and present the antigenic peptide to T lymphocytes. Such antigenic peptides can represent T cell epitopes. The human MHC is also called the HLA complex. Thus, the terms "human leukocyte antigen (HLA)", "HLA molecule" or "HLA protein" are used interchangeably with the terms "major histocompatibility complex (MHC)", "MHC molecule", and "MHC protein". HLA proteins can be classified as HLA class I or HLA class II. The structures of the proteins of the two HLA classes are very similar; however, they have very different functions. Class I HLA proteins are present on the surface of almost all cells of the body, including most tumor cells. Class I HLA proteins are loaded with antigens that usually originate from endogenous proteins or from pathogens present inside cells, and are then presented to naive or cytotoxic T-lymphocytes (CTLs). HLA class II proteins are present on antigen presenting cells (APCs), including but not limited to dendritic cells, B cells, and macrophages. They mainly present peptides which are processed from external antigen sources, e.g. outside of cells, to helper T cells.

In the HLA class II system, phagocytes such as macrophages and immature dendritic cells can take up entities by phagocytosis into phagosomes - though B cells exhibit the more general endocytosis into endosomes - which fuse with lysosomes whose acidic enzymes cleave the uptaken protein into many different peptides. Authophagy is another source of HLA class II peptides. The most studied subclass II HLA genes are: HLA-DPA1, HLA-DPB1, HLA-DQA1, HLA-DQB1, HLA-DRA, and HLA-DRB1.

Presentation of peptides by HLA class II molecules to CD4+ helper T cells can lead to immune responses to foreign antigens. Once activated, CD4+ T cells can promote B cell differentiation and antibody production, as well as CD8+ T cell (CTL) responses. CD4+ T cells can also secrete cytokines and chemokines that activate and induce differentiation of other immune cells. HLA class II molecules are typically heterodimers of α-and β-chains that interact to form a peptide-binding groove that is more open than class I peptide-binding grooves.

HLA alleles are typically expressed in codominant fashion. For example, each person carries 2 alleles of each of the 3 class I genes, (HLA-A, HLA-B and HLA-C) and so can express six different types of class II HLA. In the class II HLA locus, each person inherits a pair of HLA-DP genes (DPA1 and DPB1, which encode α and β chains), HLA-DQ (DQA1 and DQB1, for α and β chains), one gene HLA-DRα (DRA1), and one or more genes HLA-DRβ (DRB1 and DRB3, -4 or-5). HLA-DRB1, for example, has more than nearly 400 known alleles. That means that one heterozygous individual can inherit six or eight functioning class II HLA alleles: three or more from each parent. Thus, the HLA genes are highly polymorphic;

many different alleles exist in the different individuals inside a population. Genes encoding HLA proteins have many possible variations, allowing each person's immune system to react to a wide range of foreign invaders. Some HLA genes have hundreds of identified versions (alleles), each of which is given a particular number. In some embodiments, the class I HLA alleles are HLA-A*02:01, HLA-B*14:02, HLA-A*23:01, HLA-E*01:01 (non-classical). In some embodiments, class II HLA alleles are HLA-DRB*01:01, HLA-DRB*01:02, HLA-DRB*11:01, HLA-DRB*15:01, and HLA-DRB*07:01.

A "myeloid cell" can refer broadly to cells of the myeloid lineage of the hematopoietic cell system, and can exclude, for example, the lymphocytic lineage. Myeloid cells comprise, for example, cells of the granulocyte lineage and monocyte lineages. Myeloid cells are differentiated from common progenitors derived from the hematopoietic stem cells in the bone marrow. Commitment to myeloid cell lineages may be governed by activation of distinct transcription factors, and accordingly myeloid cells may be characterized as cells having a level of plasticity, which may be described as the ability to further differentiate into terminal cell types based on extracellular and intracellular stimuli. Myeloid cells can be rapidly recruited into local tissues via various chemokine receptors on their surface. Myeloid cells are responsive to various cytokines and chemokines.

A myeloid cell, for example, may be a cell that originates in the bone marrow from a hematopoietic stem cell under the influence of one or more cytokines and chemokines, such as G-CSF, GM-CSF, Flt3L, CCL2, VEGF and S100A8/9. In some embodiments, the myeloid cell is a precursor cell. In some embodiments, the myeloid cell may be a cell having characteristics of a common myeloid progenitor, or a granulocyte progenitor, a myeloblast cell, or a monocyte-dendritic cell progenitor or a combination thereof. A myeloid can include a granulocyte or a monocyte or a precursor cell thereof. A myeloid can include an immature granulocyte, an immature monocyte, an immature macrophage, an immature neutrophil, and an immature dendritic cell. A myeloid can include a monocyte or a pre-monocytic cell or a monocyte precursor. In some cases, a myeloid cell as used herein may refer to a monocyte having an M0 phenotype, an M1 phenotype or an M2 phenotype. A myeloid can include a dendritic cell (DC), a mature DC, a monocyte derived DC, a plasmacytoid DC, a pre-dendritic cell, or a precursor of a DC. A myeloid can include a neutrophil, which may be a mature neutrophil, a neutrophil precursor, or a polymorphonucleocyte (PMN). A myeloid can include a macrophage, a monocyte-derived macrophage, a tissue macrophage, a macrophage of an M0, an M1 or an M2 phenotype. A myeloid can include a tumor infiltrating monocyte (TIM). A myeloid can include a tumor associated monocyte (TAM). A myeloid can include a myeloid derived suppressor cell (MDSC). A myeloid can include a tissue resident macrophage. A myeloid can include a tumor associated DC (TADC). Accordingly, a myeloid cell may express one or more cell surface markers, for example, CD11b, CD14, CD15, CD16, CD38, CCR5, CD66, Lox-1, CD11c, CD64, CD68, CD163, CCR2, CCR5, HLA-DR, CD1c, CD83, CD141, CD209, MHC-II, CD123, CD303, CD304, a SIGLEC family protein and a CLEC family protein. In some cases, a myeloid cell may be characterized by a high or a low expression of one or more of cell surface markers, for example, CD11b, CD14, CD15, CD16, CD66, Lox-1, CD11c, CD64, CD68, CD163, CCR2, CCR5, HLA-DR, CD1c, CD83, CD141, CD209, MHC-II, CD123, CD303, CD304 or a combination thereof.

"Phagocytosis" is used interchangeably with "engulfment" and can refer to a process by which a cell engulfs a particle, such as a cancer cell or an infected cell. This process can give rise to an internal compartment (phagosome) containing the particle. This process can be used to ingest and or remove a particle, such as a cancer cell or an infected cell from the body. A phagocytic receptor may be involved in the process of phagocytosis. The process of phagocytosis can be closely coupled with an immune response and antigen presentation. The processing of exogenous antigens follows their uptake into professional antigen presenting cells by some type of endocytic event. Phagocytosis can also facilitate antigen presentation. For example, antigens from phagocytosed cells or pathogens, including cancer antigens, can be processed and presented on the cell surface of APCs.

A "polypeptide" can refer to a molecule containing amino acids linked together via a peptide bond, such as a glycoprotein, a lipoprotein, a cellular protein or a membrane protein. A polypeptide may comprise one or more subunits of a protein. A polypeptide may be encoded by a recombinant nucleic acid. In some embodiments, polypeptide may comprise more than one peptide sequence in a single amino acid chain, which may be separated by a spacer, a linker or peptide cleavage sequence. A polypeptide may be a fused polypeptide. A polypeptide may comprise one or more domains, modules or moieties.

A "receptor" can refer to a chemical structure composed of a polypeptide, which transduces a signal, such as a polypeptide that transduces an extracellular signal to a cell. A receptor can serve to transmit information in a cell, a cell formation or an organism. A receptor comprises at least one receptor unit and can contain two or more receptor units, where each receptor unit comprises a protein molecule, e.g., a glycoprotein molecule. A receptor can contain a structure that binds to a ligand and can form a complex with the ligand. Signaling information can be transmitted by a conformational change of the receptor following binding with the ligand on the surface of a cell.

The term "antibody" refers to a class of proteins that are generally known as immunoglobulins, including, but not limited to IgG1, IgG2, IgG3, and IgG4), IgA (including IgAl and IgA2), IgD, IgE, IgM, and IgY, The term "antibody" includes, but is not limited to, full length antibodies, single-chain antibodies, single domain antibodies (sdAb) and antigen-binding fragments thereof. Antigen-binding antibody fragments include, but are not limited to, Fab, Fab' and F(ab')2, Fd (consisting of $V_H$ and $C_H1$), single-chain variable fragment (scFv), single-chain antibodies, disulfide-linked variable fragment (dsFv) and fragments comprising a $V_L$ and/or a $V_H$ domain. Antibodies can be from any animal origin. Antigen-binding antibody fragments, including single-chain antibodies, can comprise variable region(s) alone or in combination with tone or more of a hinge region, a CH1 domain, a CH2 domain, and a CH3 domain. Also included are any combinations of variable region(s) and hinge region, CH1, CH2, and CH3 domains. Antibodies can be monoclonal, polyclonal, chimeric, humanized, and human monoclonal and polyclonal antibodies which, e.g., specifically bind an HLA-associated polypeptide or an HLA-peptide complex.

The term "recombinant nucleic acid" refers a nucleic acid prepared, expressed, created or isolated by recombinant means. A recombinant nucleic acid can contain a nucleotide sequence that is not naturally occurring. A recombinant nucleic acid may be synthesized in the laboratory. A recombinant nucleic acid may be prepared by using recombinant DNA technology, for example, enzymatic modification of DNA, such as enzymatic restriction digestion, ligation, and DNA cloning. A recombinant nucleic acid can be DNA, RNA, analogues thereof, or a combination thereof. A recombinant DNA may be transcribed ex vivo or in vitro, such as to to generate a messenger RNA (mRNA). A recombinant mRNA may be isolated, purified and used to transfect a cell. A recombinant nucleic acid may encode a protein or a polypeptide.

The process of introducing or incorporating a nucleic acid into a cell can be via transformation, transfection or transduction. Transformation is the process of uptake of foreign nucleic acid by a bacterial cell. This process is adapted for propagation of plasmid DNA, protein production, and other applications. Transformation introduces recombinant plasmid DNA into competent bacterial cells that take up extracellular DNA from the environment. Some bacterial species are naturally competent under certain environmental conditions, but competence is artificially induced in a laboratory setting. Transfection is the introduction of small molecules such as DNA, RNA, or antibodies into eukaryotic cells. Transfection may also refer to the introduction of bacteriophage into bacterial cells. 'Transduction' is mostly used to describe the introduction of recombinant viral vector particles into target cells, while 'infection' refers to natural infections of humans or animals with wild-type viruses.

The term "vector", can refer to a nucleic acid molecule capable of autonomous replication in a host cell, and which allow for cloning of nucleic acid molecules. As known to those skilled in the art, a vector includes, but is not limited to, a plasmid, cosmid, phagemid, viral vectors, phage vectors, yeast vectors, mammalian vectors and the like. For example, a vector for exogenous gene transformation may be a plasmid. In certain embodiments, a vector comprises a nucleic acid sequence containing an origin of replication and other elements necessary for replication and/or maintenance of the nucleic acid sequence in a host cell. In some embodiments, a vector or a plasmid provided herein is an expression vector. Expression vectors are capable of directing the expression of genes and/or nucleic acid sequence to which they are operatively linked. In some embodiments, an expression vector or plasmid is in the form of circular double stranded DNA molecules. A vector or plasmid may or may not be integrated into the genome of a host cell. In some embodiments, nucleic acid sequences of a plasmid are not integrated in a genome or chromosome of the host cell after introduction. For example, the plasmid may comprise elements for transient expression or stable expression of the nucleic acid sequences, e.g. genes or open reading frames harbored by the plasmid, in a host cell. In some embodiments, a vector is a transient expression vector. In some embodiments, a vector is a stably expressed vector that replicates autonomously in a host cell. In some embodiments, nucleic acid sequences of a plasmid are integrated into a genome or chromosome of a host cell upon introduction into the host cell. Expression vectors that can be used in the methods as disclosed herein include, but are not limited to, plasmids, episomes, bacterial artificial chromosomes, yeast artificial chromosomes, bacteriophages or viral vectors. A vector can be a DNA or RNA vector. In some embodiments, a vector provide herein is a RNA vector that is capable of integrating into a host cell's genome upon introduction into the host cell (e.g., via reverse transcription), for example, a retroviral vector or a lentiviral vector. Other forms of expression vectors known by those skilled in the art which serve the equivalent functions can also be used, for example, self-replicating extrachromosomal vectors or vectors capable of integrating into a host genome. Exemplary vectors are those capable of autonomous replication and/or expression of nucleic acids to which they are linked.

The terms "spacer" or "linker" as used in reference to a fusion protein refers to a peptide sequence that joins two other peptide sequences of the fusion protein. In some embodiments, a linker or spacer has no specific biological activity other than to join or to preserve some minimum distance or other spatial relationship between the proteins or RNA sequences. In some embodiments, the constituent amino acids of a spacer can be selected to influence some property of the molecule such as the folding, flexibility, net charge, or hydrophobicity of the molecule. Suitable linkers for use in an embodiment of the present disclosure are well known to those of skill in the art and include, but are not limited to, straight or branched-chain carbon linkers, heterocyclic carbon linkers, or peptide linkers. In some embodiments, a linker is used to separate two or more polypeptides, e.g. two antigenic peptides by a distance sufficient to ensure that each antigenic peptide properly folds. Exemplary peptide linker sequences adopt a flexible extended conformation and do not exhibit a propensity for developing an ordered secondary structure. Amino acids in flexible linker protein region may include Gly, Asn and Ser, or any permutation of amino acid sequences containing Gly, Asn and Ser. Other near neutral amino acids, such as Thr and Ala, also can be used in the linker sequence.

The terms "treat," "treated," "treating," "treatment," and the like are meant to refer to reducing, preventing, or ameliorating a disorder and/or symptoms associated therewith (e.g., a neoplasia or tumor or infectious agent or an autoimmune disease). "Treating" can refer to administration of the therapy to a subject after the onset, or suspected onset, of a disease (e.g., cancer or infection by an infectious agent or an autoimmune disease). "Treating" includes the concepts of "alleviating", which can refer to lessening the frequency of occurrence or recurrence, or the severity, of any symptoms or other ill effects related to the disease and/or the side effects associated with therapy. The term "treating" also encompasses the concept of "managing" which refers to reducing the severity of a disease or disorder in a patient, e.g., extending the life or prolonging the survivability of a patient with the disease, or delaying its recurrence, e.g., lengthening the period of remission in a patient who had suffered from the disease. It is appreciated that, although not precluded, treating a disorder or condition does not require that the disorder, condition, or symptoms associated therewith be completely eliminated. The term "prevent", "preventing", "prevention" and their grammatical equivalents as used herein, can refer to avoiding or delaying the onset of symptoms associated with a disease or condition in a subject that has not developed such symptoms at the time the administering of an agent or compound commences. In certain embodiments, treating a subject or a patient as described herein comprises administering a therapeutic composition, such as a drug, a metabolite, a preventive component, a nucleic acid, a peptide, or a protein that encodes or otherwise forms a drug, a metabolite or a preventive component. In some embodiments, treating comprises administering a cell or a population of cells to a subject in need thereof. In some embodiments, treating comprises administering to the subject one or more of engineered cells described herein, e.g. one or more engineered myeloid cells, such as phagocytic cells. Treating comprises treating a disease or a condition or a syndrome, which may be a pathological disease, condition or syndrome, or a latent disease, condition or syndrome. In some cases, treating, as used herein may comprise administering a therapeutic vaccine. In some embodiments, the engineered phagocytic cell is administered to a patient or a subject. In some embodiments, a cell administered to a human subject results in reduced immunogenicity. For example, an engineered phagocytic cell may lead to no or reduced graft versus host disease (GVHD) or fratricide effect. In some embodiments, an engineered cell administered to a human subject is immunocompatible to the subject (i.e. having a matching HLA subtype that is naturally expressed in the subject). Subject specific HLA alleles or HLA genotype of a subject can be determined by any method known in the art. In exemplary embodiments, the methods include determining polymorphic gene types that can comprise generating an alignment of reads extracted from a sequencing data set to a gene reference set comprising allele variants of the polymorphic gene, determining a first posterior probability or a posterior probability derived score for each allele variant in the alignment, identifying the allele variant with a maximum first posterior probability or posterior probability derived score as a first allele variant, identifying one or more overlapping reads that aligned with the first allele variant and one or more other allele variants, determining a second posterior probability or posterior probability derived score for the one or more other allele variants using a weighting factor, identifying a second allele variant by selecting the allele variant with a maximum second posterior probability or posterior probability derived score, the first and second allele variant defining the gene type for the polymorphic gene, and providing an output of the first and second allele variant.

A "fragment" can refer to a portion of a protein or nucleic acid. In some embodiments, a fragment retains at least 50%, 75%, or 80%, or 90%, 95%, or even 99% of the biological activity of a reference protein or nucleic acid.

The terms "isolated," "purified", "biologically pure" and their grammatical equivalents refer to material that is free to varying degrees from components which normally accompany it as found in its native state. "Isolate" denotes a degree of separation from original source or surroundings. "Purify" denotes a degree of separation that is higher than isolation. A "purified" or "biologically pure" protein is sufficiently free of other materials such that any impurities do not materially affect the biological properties of the protein or cause other adverse consequences. That is, a nucleic acid or peptide of the present disclosure is purified if it is substantially free of cellular material, viral material, or culture medium when produced by recombinant DNA techniques, or chemical precursors or other chemicals when chemically synthesized. Purity and homogeneity are typically determined using analytical chemistry techniques, for example, polyacrylamide gel electrophoresis or high performance liquid chromatography. The term "purified" can denote that a nucleic acid or protein gives rise to essentially one band in an electrophoretic gel. For a protein that can be subjected to modifications, for example, phosphorylation or glycosylation, different modifications can give rise to different isolated proteins, which can be separately purified.

The terms "neoplasia" or "cancer" refers to any disease that is caused by or results in inappropriately high levels of cell division, inappropriately low levels of apoptosis, or both. Glioblastoma is one non-limiting example of a neoplasia or cancer. The terms "cancer" or "tumor" or "hyperproliferative disorder" refer to the presence of cells possessing characteristics typical of cancer-causing cells, such as uncontrolled proliferation, immortality, metastatic potential, rapid growth and proliferation rate, and certain characteristic morphological features. Cancer cells are often in the form of a tumor, but such cells can exist alone within an animal, or can be a non-tumorigenic cancer cell, such as a leukemia cell.

The term "vaccine" is to be understood as meaning a composition for generating immunity for the prophylaxis and/or treatment of diseases (e.g., neoplasia/tumor/infectious agents/autoimmune diseases). Accordingly, vaccines as used herein are medicaments which comprise recombinant nucleic acids, or cells comprising and expressing a recombinant nucleic acid and are intended to be used in humans or animals for generating specific defense and protective substance by vaccination. A "vaccine composition" can include a pharmaceutically acceptable excipient, carrier or diluent. Aspects of the present disclosure relate to use of the technology in preparing a phagocytic cell-based vaccine.

The term "pharmaceutically acceptable" refers to approved or approvable by a regulatory agency of the Federal or a state government or listed in the U.S. Pharmacopeia or other generally recognized pharmacopeia for use in animals, including humans. A "pharmaceutically acceptable excipient, carrier or diluent" refers to an excipient, carrier or diluent that can be administered to a subject, together with an agent, and which does not destroy the pharmacological activity thereof and is nontoxic when administered in doses sufficient to deliver a therapeutic amount of the agent.

Nucleic acid molecules useful in the methods of the disclosure include, but are not limited to, any nucleic acid molecule with activity or that encodes a polypeptide. Polynucleotides having substantial identity to an endogenous sequence are typically capable of hybridizing with at least one strand of a double-stranded nucleic acid molecule. "Hybridize" refers to when nucleic acid molecules pair to form a double-stranded molecule between complementary polynucleotide sequences, or portions thereof, under various conditions of stringency. (See, e.g., Wahl, G. M. and S. L. Berger (1987) Methods Enzymol. 152:399; Kimmel, A. R. (1987) Methods Enzymol. 152:507). For example, stringent salt concentration can ordinarily be less than about 750 mM NaCl and 75 mM trisodium citrate, less than about 500 mM NaCl and 50 mM trisodium citrate, or less than about 250 mM NaCl and 25 mM trisodium citrate. Low stringency hybridization can be obtained in the absence of organic solvent, e.g., formamide, while high stringency hybridization can be obtained in the presence of at least about 35% formamide, or at least about 50% formamide. Stringent temperature conditions can ordinarily include temperatures of at least about 30° C, at least about 37°C, or at least about 42°C. Varying additional parameters, such as hybridization time, the concentration of detergent, e.g., sodium dodecyl sulfate (SDS), and the inclusion or exclusion of carrier DNA, are well known to those skilled in the art. Various levels of stringency are accomplished by combining these various conditions as needed. In an exemplary embodiment, hybridization can occur at 30° C in 750 mM NaCl, 75 mM trisodium citrate, and 1% SDS. In another exemplary embodiment, hybridization can occur at 37° C in 500 mM NaCl, 50 mM trisodium citrate, 1% SDS, 35% formamide, and 100 $\mu$g/ml denatured salmon sperm DNA (ssDNA). In another exemplary embodiment, hybridization can occur at 42° C in 250 mM NaCl, 25 mM trisodium citrate, 1% SDS, 50% formamide, and 200 $\mu$g/ml ssDNA. Useful variations on these conditions will be readily apparent to those skilled in the art. For most applications, washing steps that follow hybridization can also vary in stringency. Wash stringency conditions can be defined by salt concentration and by temperature. As above, wash stringency can be increased by decreasing salt concentration or by increasing temperature. For example, stringent salt concentration for the wash steps can be less than about 30 mM NaCl and 3 mM trisodium citrate, or less than about 15 mM NaCl and 1.5 mM trisodium citrate. Stringent temperature conditions for the wash steps can include a temperature of at least about 25°C, of at least about 42°C, or at least about 68°C. In exemplary embodiments, wash steps can occur at 25° C in 30 mM NaCl, 3 mM trisodium citrate, and 0.1% SDS. In other exemplary embodiments, wash steps can occur at 42° C in 15 mM NaCl, 1.5 mM trisodium citrate, and 0.1% SDS. In another exemplary embodiment, wash steps can occur at 68° C in 15 mM NaC1, 1.5 mM trisodium citrate, and 0.1% SDS. Additional variations on these conditions will be readily apparent to those skilled in the art. Hybridization techniques are well known to those skilled in the art and are described, for example, in Benton and Davis (Science 196:180, 1977); Grunstein and Hogness (Proc. Natl. Acad. Sci., USA 72:3961, 1975); Ausubel et al. (Current Protocols in Molecular Biology, Wiley Interscience, New York, 2001); Berger and Kimmel (Guide to Molecular Cloning Techniques, 1987, Academic Press, New York); and Sambrook et al., Molecular Cloning: A Laboratory Manual, Cold Spring Harbor Laboratory Press, New York.

"Substantially identical" refers to a polypeptide or nucleic acid molecule exhibiting at least 50% identity to a reference amino acid sequence (for example, any one of the amino acid sequences described herein) or nucleic acid sequence (for example, any one of the nucleic acid sequences described herein). Such a sequence can be at least 60%, 80% or 85%, 90%, 95%, 96%, 97%, 98%, or even 99% or more identical at the amino acid level or nucleic acid to the sequence used for comparison. Sequence identity is typically measured using sequence analysis software (for example, Sequence Analysis Software Package of the Genetics Computer Group, University of Wisconsin Biotechnology Center, 1710 University Avenue, Madison, Wis. 53705, BLAST, BESTFIT, GAP, or PILEUP/PRETTYBOX programs). Such software matches identical or similar sequences by assigning degrees of homology to various substitutions, deletions, and/or other modifications. Conservative substitutions typically include substitutions within the following groups: glycine, alanine; valine, isoleucine, leucine; aspartic acid, glutamic acid, asparagine, glutamine; serine, threonine; lysine, arginine; and phenylalanine, tyrosine. In an exemplary approach to determining the degree of identity, a BLAST program can be used, with a probability score between e-3 and e-m° indicating a closely related sequence. A "reference" is a standard of comparison. It will be understood that the numbering of the specific positions or residues in the respective sequences depends on the particular protein and numbering scheme used. Numbering might be different, e.g., in precursors of a mature protein and the mature protein itself, and differences in sequences from species to species may affect numbering. One of skill in the art will be able to identify the respective residue in any homologous protein and in the respective encoding nucleic acid by methods well known in the art, e.g., by sequence alignment to a reference sequence and determination of homologous residues.

The term "subject" or "patient" refers to an organism, such as an animal (e.g., a human) which is the object of treatment, observation, or experiment. By way of example only, a subject includes, but is not limited to, a mammal, including, but not limited to, a human or a non-human mammal, such as a non-human primate, murine, bovine, equine, canine, ovine, or feline.

The term "therapeutic effect" refers to some extent of relief of one or more of the symptoms of a disorder (e.g., a neoplasia, tumor, or infection by an infectious agent or an autoimmune disease) or its associated pathology. "Therapeutically effective amount" as used herein refers to an amount of an agent which is effective, upon single or multiple dose administration to the cell or subject, in prolonging the survivability of the patient with such a disorder, reducing one or more signs or symptoms of the disorder, preventing or delaying, and the like beyond that expected in the absence of such treatment. "Therapeutically effective amount" is intended to qualify the amount required to achieve a therapeutic effect. A physician or veterinarian having ordinary skill in the art can readily determine and prescribe the "therapeutically effective amount" (e.g., ED50) of the pharmaceutical composition required..

## ENGINEERED MYELOD CELLS "TARGETED" TO ATTACK DISEASED CELLS

The present disclosure involves compositions and methods for preparing targeted killer myeloid cells; by leveraging the innate functional role in immune defense, ranging from properties related to detecting foreign bodies, particles, diseased cells, cellular debris, inflammatory signal, chemoattract; activating endogenous DAMP and PAMP signaling pathways; trigger myelopoiesis, extravasation; chemotaxis; phagocytes; pinocytosis; recruitment; engulfment; scavenging; activating intracellular oxidative burst and lysis or killing of pathogens, detecting, engulfing and killing diseased or damaged cells; removing unwanted cellular, tissue or acellular debris *in vivo*; antigen presentation and role in activating innate immunity; activating and modulating an immune response cascade; activating T cell repertoire; autophagy; inflammatory and non-inflammatory apoptosis; pyroptosis, immune editing to response to stress and restoration of tissue homeostasis. In one aspect, provided herein are methods and compositions to augment one or more functions of a myeloid cell for use in a therapeutic application, the one or more functions may be one or more of: detecting foreign bodies, particles, diseased cells, cellular debris, inflammatory signal, chemoattract; activating endogenous DAMP and PAMP signaling pathways; trigger myelopoiesis, extravasation; chemotaxis; phagocytosis; pinocytosis; recruitment; trogocytosis; engulfment; scavenging; activating intracellular oxidative burst and intracellular lysis or killing of pathogens, detecting, engulfing and killing diseased or damaged cells; removing unwanted cellular, tissue or acellular debris *in vivo*; antigen presentation and role in activating innate immunity; activating and modulating an immune response cascade; activating T cell repertoire; autophagy; inflammatory and non-inflammatory apoptosis; pyroptosis, immune editing to response to stress and restoration of tissue homeostasis. In one embodiment, the compositions and methods are also directed to augmenting the targeting, and killing function of certain myeloid cells, by genetic modification of these cells. The compositions and methods described herein are directed to creating engineered myeloid cells, wherein the engineered myeloid cells comprise at least one genetic modification, and can be directed to recognize and induce effector functions against a pathogen, a diseased cell, such as a tumor or cancer cell, such that the engineered myeloid cell is capable of recognizing, targeting, phagocytosing, killing and/or eliminating the pathogen or the diseased cell or the cancer cell, and additionally, may activate a specific immune response cascade following the phagocytosis, killing and/or eliminating the pathogen or the diseased cell.

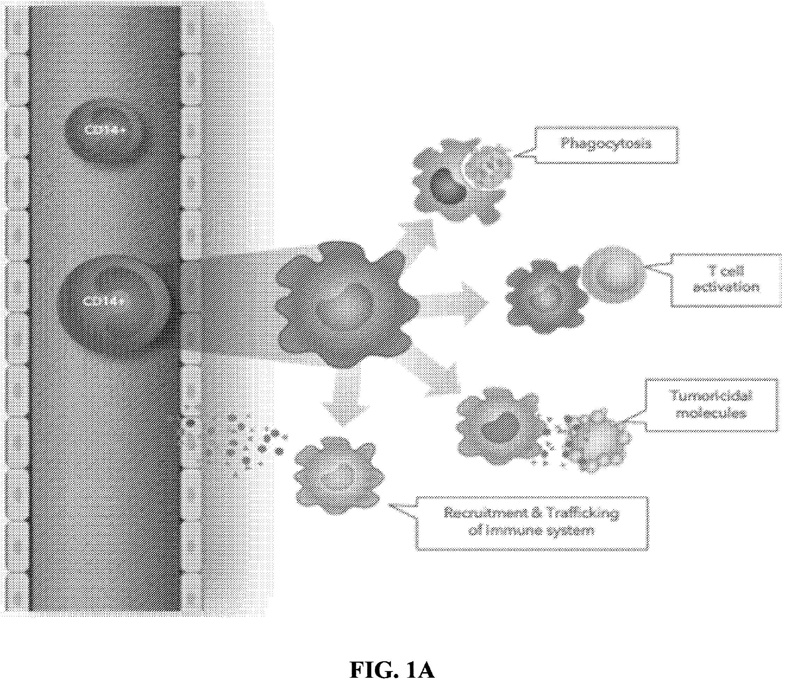

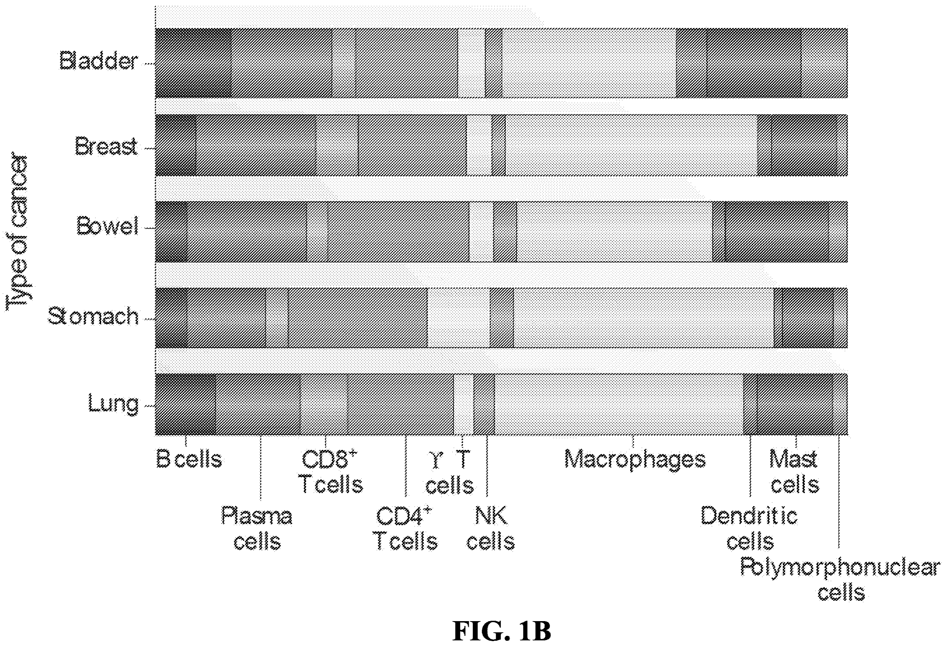

Myeloid cells appear to be the most abundant cells in a tumor (FIG. 1B). Myeloid cells are also capable of recognizing a tumor cell over a healthy normal cell of the body and mount an immune response to a tumor cell of the body. As sentinels of innate immune response, myeloid cells are able to sense non-self or aberrant cell types and clear them via a process called phagocytosis. This can be directed to a therapeutic advantage in driving myeloid cell mediated phagocytosis and lysis of tumor cells. However, these naturally occurring tumor-infiltrating myeloid cells (TIMs) may be subjected to influence of the tumor microenvironment (TME). TIMs constitute a heterogeneous population of cells. Many TIMs originate from circulating monocytes and granulocytes, which in turn stem from bone marrow-derived hematopoietic stem cells. However, in the presence of persistent stimulation by tumor-derived factors the monocyte and granulocyte progenitors divert from their intrinsic pathway of terminal differentiation into mature macrophages, DCs or granulocytes, and may become tumor promoting myeloid cell types. Differentiation into pathological, alternatively activated immature myeloid cells is favored. These immature myeloid cells include tumor-associated DCs (TADCs), tumor-associated neutrophils (TANs), myeloid-derived suppressor cells (MDSCs), and tumor-associated macrophages (TAMs). Alternative to this emergency myelopoiesis, TAMs may also originate from tissue-resident macrophages, which in turn can be of embryonic or monocytic origin. These tissue-resident macrophages undergo changes in phenotype and function during carcinogenesis, and proliferation may help to maintain TAMs derived from tissue-resident macrophages. A tumor microenvironment may drive a tumor infiltrating myeloid cell to become myeloid derived suppressor cells and acquire the ability to suppress T cells. As a result, innovative methods are necessary to create therapeutically effective TIMs that can infiltrate a tumor, and can target tumor cells for phagocytic uptake and killing.

In one aspect, provided herein are engineered myeloid cells that are capable of targeting specific target cells, for example, tumor cells or pathogenic cells. In some embodiments, engineered myeloid cells provided herein are potent in infiltrating, targeting, and killing tumor cells. An engineered myeloid/phagocytic cell described herein is designed to comprise a recombinant nucleic acid, which encodes one or more proteins that help target the phagocytic cell to a target cell, for example a tumor cell or a cancer cell. In one embodiment, the engineered myeloid cell is capable of readily infiltrating a tumor. In one embodiment, the engineered myeloid cell has high specificity for the target cell, with none or negligible cross-reactivity to a non-tumor, non-diseased cell of the subject while in circulation. In one embodiment, the engineered myeloid/phagocytic cell described herein is designed to comprise a recombinant nucleic acid, which will help the cell to overcome/bypass the TME influence and mount a potent anti-tumor response. In one embodiment, the engineered myeloid/phagocytic cell described herein is designed to comprise a recombinant nucleic acid, which augments phagocytosis of the target cell. In another embodiment, the engineered myeloid/phagocytic cell described herein is designed to comprise a recombinant nucleic acid, which augments reduce or eliminate trogocytosis and/or enhance phagocytic lysis or of the target cell.

Accordingly, in some embodiments, the compositions disclosed herein comprise a myeloid cell, comprising a recombinant nucleic acid encoding a chimeric receptor fusion protein (CFP), for example, a phagocytic receptor (PR) fusion protein (PFP). The recombinant nucleic acid can comprise a sequence encoding a PR subunit comprising: (i) a transmembrane domain, and (ii) an intracellular domain comprising a PR intracellular signaling domain; and an extracellular antigen binding domain specific to an antigen of a target cell; wherein the transmembrane domain and the extracellular antigen binding domain are operatively linked; wherein the PR intracellular signaling domain is derived from a receptor with a signal transduction domain. The recombinant nucleic acid further encodes for one or more polypeptides that constitute one or more plasma membrane receptors that helps engage the phagocytic cell to the target cell, and enhance its phagocytic activity.

In some embodiments, the myeloid cell described herein comprises one or more recombinant proteins comprising a chimeric receptor, wherein the chimeric receptor is capable of responding to a first phagocytic signal directed to a target cell, which may be a diseased cell, a tumor cell or a pathogen, and a second signal, which is an inflammatory signal, that augments the phagocytic and killing response to target initiated by the first signal.

### *Phagocytes*

Provided herein are methods and compositions for immunotherapy, comprising 'improving' or 'modifying' or 'engineering' a phagocytic cell and targeting it towards a specific target, which can be a specific cell type or class of cells in a patient or a subject. In some embodiments, the subject is a patient having a disease. The terms subject and patient may often be used interchangeably in this section. In some embodiments, the phagocytic cell is derived from the subject having a disease, wherein the disease is, for example, cancer. The autologous cells from the subject may be modified *in vitro* and administered into the cell, where the modified phagocytic cell is redesigned to specifically attack and kill the cancer cells in the subject.

In some embodiments, the subject has a disease that is not a cancer.

In some embodiments, the subject has a disease that is an infection. In some embodiments, the methods and compositions for immunotherapy provided herein are for 'improving' or 'modifying' or 'engineering' a phagocytic cell and targeting it towards an infection, for example an infected cell within the subject.

In some embodiments, the subject has a disease that is a viral, a bacterial, a fungal or a protozoal infection. In some embodiments, the methods and compositions for immunotherapy provided herein are for 'improving' or 'modifying' or 'engineering' a phagocytic cell and targeting it towards a virus infected cell, a bacteria infected cell, a fungus infected cell or a protozoa infected cell inside the infected subject. In some embodiments the methods and compositions for immunotherapy provided herein are for 'improving' or 'modifying' or 'engineering' a phagocytic cell and targeting it towards a virus, a bacteria, a fungus or any pathogen in a subject, such that the virus, the bacteria, the fungus or the pathogen in a subject is phagocytosed, and/or killed. In some embodiments the methods and compositions for immunotherapy provided herein are for 'improving' or 'modifying' or 'engineering' a phagocytic cell and targeting it towards a viral antigen, a bacterial antigen, a fungal antigen or an antigen of a pathogen in a subject, such that there is at least one improved immune response within the subject to the virus, the bacteria, the fungus or the pathogen in the subject.

In some embodiments, the myeloid cells, such as phagocytic cells, are allogeneic In some embodiments, the methods and compositions for immunotherapy provided herein comprises obtaining myeloid cells, such as phagocytic cells, derived from an allogeneic source. The myeloid cells, such as phagocytic cells, can thereafter be modified or engineered and introduced into a diseased subject, such that the modified or engineered cells from the allogeneic source are capable of attacking a diseased cell of the subject, phagocytose the diseased cell and/or kill the diseased cell directly or indirectly, or improve at least one immune response of the subject to the disease. In some embodiments, the allogeneic source is a human. In some embodiments, the allogeneic source is a healthy human.

Phagocytes are the natural sentinels of the immune system and form the first line of defense in the body. They engulf a pathogen, a pathogen infected cell a foreign body or a cancerous cell and remove it from the body. Most potential pathogens are rapidly neutralized by this system before they can cause, for example, a noticeable infection. This can involve receptor-mediated uptake through the clathrin coated pit system, pinocytosis, particularly macropinocytosis as a consequence of membrane ruffling and phagocytosis. The phagocytes therefore can be activated by a variety of non-self (and self) elements and exhibit a level of plasticity in recognition of their "targets".

Mononuclear phagocytic system (MPS), comprised of monocytes, macrophages, and dendritic cells, is essential in tissue homeostasis and in determining the balance of an immune response through its role in antigen presentation. The MPS is a cell lineage which originates from bone marrow progenitor cells and gives rise to blood monocytes, tissue macrophages and dendritic cells. Thus, the process of generating a macrophage from the MPS begins with a promonocyte in the BM which undergoes a differentiation process into a monocyte that is ready to enter the systemic circulation. After a short period (<48h) in the circulation, these newly formed monocytes rapidly infiltrate into peripheral tissues where a majority of them differentiate into macrophages or dendritic cells (DC). Anti-microbe phagocytosis clears and degrades disease-causing microbes, induces pro-inflammatory signaling through cytokine and chemokine secretion, and recruits immune cells to mount an effective inflammatory response. This type of phagocytosis is often referred to as "inflammatory phagocytosis" (or "immunogenic phagocytosis"). However, in some instances, such as with certain persistent infections, anti-inflammatory responses may follow microbial uptake. Anti-microbe phagocytosis is commonly performed by professional phagocytes of the myeloid lineage, such as immature dendritic cells (DCs) and macrophages and by tissue-resident immune cells. Phagocytosis of damaged, apoptotic cells or cell is typically a non-inflammatory (also referred to as a "nonimmunogenic") process. Transformed or malignant cells (self-cells), and cells are phagocytosed and apoptotic cells are removed promptly without causing damage to the surrounding tissues or inducing a pro-inflammatory immune response. This type of apoptotic cell clearance is non-inflammatory and include release of "find me" signals from apoptotic cells to recruit phagocytes to the location of apoptotic cells; accompanied by "eat me" signals exposed on the surface of apoptotic cells are bound by phagocytes via specific receptors; cytoskeletal rearrangement to engulf the apoptotic cell; followed by the ingested apoptotic cell is digested and specific phagocytic responses are elicited (e.g., secretion of anti-inflammatory cytokines).

Phagocytosis, the cellular uptake of particulates, e.g. particles >0.5 $\mu$m within a plasma-membrane envelope, is closely related to and partly overlaps the endocytosis of soluble ligands by fluid-phase macropinocytic and receptor pathways. Variants associated with the uptake of apoptotic cells, also known as efferocytosis, and that of necrotic cells arising from infection and inflammation (necroptosis and pyroptosis). The uptake of exogenous particles (heterophagy) has features in common with autophagy, an endogenous process of sequestration and lysosomal disposal of damaged intracellular organelles There is a spectrum of uptake mechanisms depending on the particle size, multiplicity of receptor-ligand interactions, and involvement of the cytoskeleton. Once internalized, the phagosome vacuole can fuse selectively with primary lysosomes, or the product of the endoplasmic reticulum (ER) and Golgi complex, to form a secondary phagolysosome (Russell, D.G. (2011). Immunol. Rev. 240, 252-268). This pathway is dynamic in that it undergoes fusion and fission with endocytic and secretory vesicles macrophages, DCs, osteoclasts, and eosinophils. Anti-microbe phagocytosis clears and degrades disease-causing microbes, induces pro-inflammatory signaling through cytokine and chemokine secretion, and recruits immune cells to mount an effective inflammatory response. This type of phagocytosis is often referred to as "inflammatory phagocytosis" (or "immunogenic phagocytosis"). However, in some instances, such as with certain persistent infections, anti-inflammatory responses may follow microbial uptake. Anti-microbe phagocytosis is commonly performed by professional phagocytes of the myeloid lineage, such as immature dendritic cells (DCs) and macrophages and by tissue-resident immune cells. Phagocytosis of damaged, self-derived apoptotic cells or cell debris (e.g., efferocytosis), in contrast, is typically a non-inflammatory (also referred to as a "nonimmunogenic") process. Billions of damaged, dying, and unwanted cells undergo apoptosis each day. Unwanted cells include, for example, excess cells generated during development, senescent cells, infected cells (intracellular bacteria or viruses), transformed or malignant cells, and cells irreversibly damaged by cytotoxic agents.

The bone marrow is the source of circulating neutrophils and monocytes that will replace selected tissue-resident macrophages and amplify tissue myeloid populations during inflammation and infection. After phagocytosis, newly recruited monocytes and tissue macrophages secrete their products by generating them from pre-existing phospholipids and arachidonates in the plasma membrane and by releasing radicals generated by activation of a respiratory burst or induction of inducible nitric oxide synthesis; apart from being achieved by synthesis of the low-molecular-weight products (arachidonate metabolites, superoxide anions, and nitric oxide) generated as above, secretion induced by phagocytosis in macrophages is mainly achieved by new synthesis of RNA and changes in pH, resulting in progressive acidification.

In some embodiments, phagocytes provided herein are monocytes or cells of the monocyte lineage.

In some embodiments, myeloid cells are phagocytic macrophages are MARCO+ SignR1+ and are found in the outer marginal zone rapidly clear capsulated bacteria. Similar CD169+ F4/80-macrophages line the subcapsular sinus in lymph nodes and have been implicated in virus infection. It was noted that endothelial macrophages, including Kupffer cells in the liver, clear microbial and antigenic ligands from blood and lymph nodes to provide a sinusoidal immune function comparable to but distinct from mucosal immunity. Not all tissue macrophages are constitutively phagocytic, even though they still express typical macrophage markers. In the marginal zone of the rodent spleen, metallophilic macrophages, which lack F4/80, strongly express CD169, sialic acid-binding immunoglobulin (Ig)-like lectin 1 (SIGLEC1 [sialoadhesin]), but are poorly phagocytic. Non-professional phagocytes include epithelial cells, and fibroblasts. Fibroblasts are "working-class phagocytes" that clear apoptotic debris by using integrins other than CD11b-CD18 through adhesion molecules ICAM and vitronectin receptors. Astrocytes have also been reported to engulf, even if not efficiently degrade, apoptotic corpses. Plasma-membrane receptors relevant to phagocytosis can be opsonic, FcRs (activating or inhibitory) for mainly the conserved domain of IgG antibodies, and complement receptors, such as CR3 for iC3b deposited by classical (IgM or IgG) or alternative lectin pathways of complement activation. CR3 can also mediate recognition in the absence of opsonins, perhaps by depositing macrophage-derived complement. Anti-microbe phagocytosis is commonly performed by professional phagocytes of the myeloid lineage, such as immature dendritic cells (DCs) and macrophages and by tissue-resident immune cells.

In some embodiments, for the purpose of the instant cellular engineering program disclosed herein, cells that are used for engineering for use in immunotherapy are potently phagocytic.

In some embodiments, for the purpose of the instant cellular engineering program disclosed herein, cells that are used for engineering for use in immunotherapy are obtained from whole blood, peripheral blood mononuclear cells, bone marrow, lymph node tissue, cord blood, thymus tissue, tissue from a site of infection, ascites, pleural effusion, spleen tissue.

In some embodiments, cells that are used for engineering for use in immunotherapy are obtained from peripheral blood.

Among the liver MPS, a variety of structural and functional distinctions have been characterized, both stimulatory and inhibitory with respect to the purpose of generation of cells for cancer immunotherapy.

**Table 1 - Exemplary phenotypic characteristics of liver monocytes, macrophages and DCs**

| | **Molecularly defined** | Other characteristics |
|---|---|---|
| Liver monocytes | CD14++CD16-CD14++CD16+ DC-like phenotype-High DR, CD80+ Macrophage-like phenotype-CD163+, CD68+ CD16+CD14dim CD14 "DC"-Postulated to be monocyte derived | CD16+ monocytes (undefined as to whether they CD14+ +CD16+ or CD16+CD14dim) possess superior phagocytosis compared to blood monocytes and can efficiently activate CD4+ T cells |
| Liver macrophages | Pan CD68 | Liver Macrophages appear to be predominantly tolerogenic in nature, with a regulatory and scavenging role |
| Liver dendritic cells | BDCA1 (CD1c+) DC BDCA2 (CD303+) DC BDCA3 (CD141hi) DC | Tolerogenic in nature; Lower expression of costimulation markers compared to spleen; Produce IL-10 on LPS stimulation; Stimulate T-cells that are IL-10 producing and hypo-responsive on re-stimulation; Produce higher numbers of FoxP3+ Treg cells on naïve T cell stimulation; Weak MLR response compared to blood. |

In some embodiments the myeloid cells that are engineered for use in immunotherapy in the instant application comprise myeloid cells selected from the group consisting of macrophages, dendritic cells, mast cells, monocytes, neutrophils, microglia, and astrocytes.

In some embodiments the myeloid cells that are engineered for use in immunotherapy are phagocytic cells. In some embodiments, the phagocytic cells are monocytes.

In some embodiments, the myeloid cells that are engineered for use in immunotherapy in the instant application are monocytes, monocyte derived macrophages, and/or dendritic cells.

In some embodiments the myeloid cells that are engineered for use in immunotherapy in the instant application are monocytes or macrophages.

In some embodiments the cells that myeloid cells obtained from the peripheral blood.

In some embodiments, the myeloid cells are selected by selection marker $CD14^+CD16^{low}$. In some embodiments the myeloid cells are selected via elutriation.

In some embodiments, the myeloid cells are isolated from leukapheresis column of the subject. In some embodiments the subject is the same subject who is administered the pharmaceutical composition comprising engineered cells.

In some embodiments the subject is not the same subject who is administered the pharmaceutical composition comprising engineered cells.

In some embodiments, the leukapheresis is performed on the same subject once a week to collect more myeloid cells. In some embodiments, the leukapheresis is performed on the same subject more than once in a span of 8-10 days to collect more myeloid cells. In some embodiments, the leukapheresis is performed on the same subject more than twice in a span of one month to collect more myeloid cells.

In some embodiments, myeloid cells are isolated from a leukapheresis sample or a peripheral blood sample. In some embodiments, the myeloid cell is a progenitor cell. In some embodiments, the myeloid cell is a monocyte precursor cell. In some embodiments, a myeloid cell described herein is not differentiated into a terminal cell and do not exhibit a terminal cell phenotype, such as tissue macrophages. In some embodiments, the myeloid cells comprise CD14+ cells. In some embodiments, the myeloid cells do not express CD16. In some embodiments the myeloid cells express low amounts of CD16. In some embodiments, the myeloid cells are pre-selected for the purpose of engineering from a biological sample, such as peripheral blood or an apheresis sample by selection of CD14+ cells. In some embodiments, the selection is performed without contacting with or engaging with the myeloid cell to be selected. In some embodiments, the myeloid cells are selected prior to engineering from a biological sample by sorting, for example a flow cytometry based cell sorter (FACS). In some embodiments, the myeloid cells expressing CD16 are captured by an antibody and the remaining myeloid cells were collected and used for engineering. In some embodiments, one or more other cell surface molecules are targeted for capturing in the negative selection process in addition to CD16, in order to obtain the myeloid cells, for example CD3, CD8, CD11c, CD40, or CD206.

In one aspect, provided herein are myeloid cells comprising at least one exogenous recombinant nucleic acid that encodes for a fusion protein. The fusion protein may be a chimeric protein comprising at least a transmembrane domain and an extracellular domain that comprises a region that can bind to a target cell. For example, the chimeric protein may bind to a target, e.g. a target antigen, an antigenic peptide, or a ligand on the target cell. In some embodiments, the target cell is a cancer cell. In some embodiments, a target is a cancer antigen. In some embodiments, the chimeric protein is expressed in the myeloid cell and activates the myeloid cell to overcome TME induced suppressive signal and act as an activated pro-inflammatory myeloid cell. In one embodiment, the chimeric protein that is expressed in the myeloid cell is capable of being responsive to a second signal other than the target (the first signal), wherein the second signal is a pro-inflammatory signal and an activating signal. In some embodiments, the chimeric protein that is expressed in the myeloid cell is capable of being responsive to multiple signals other than the target or the first signal. The chimeric protein may be able to respond to one, two, three, four, five, or more signals besides the target or the first signal.

In another embodiment, the chimeric protein that is expressed in the myeloid cell is specific for binding to a target. In some embodiments, the target is a cancer antigen. Expression of the chimeric protein endows target specificity to the myeloid cell.

In one embodiment, the chimeric protein that is expressed in the myeloid cell is capable of multiplexing, for example, has multiple domains for activation and processing of more than one signal or signal types. In some embodiments, activation of the multiple domains simultaneously leads to an augmented effector response to the myeloid cell. An effector response for the myeloid cell encompasses, for example, enhanced phagocytosis, pro-inflammatory activation, and killing of target cell. In some embodiments, the chimeric protein that is expressed in the myeloid cell, capable of multiplexing is capable of binding to more than one ligands, such as a target antigen and a helper molecule. In some embodiments, the chimeric protein is capable of binding to multiple target antigens on a cancer cell. In some embodiments, the chimeric protein is capable of multiplexing is capable of binding to multiple target antigens on multiple cells. In some embodiments, the chimeric protein may bind to a macrophage-monocyte inhibitory target on a cancer cell, and create a stimulatory signal upon contact using the pro-inflammatory domain fused to the intracellular end, a process termed as "signal switch". For example, an extracellular domain of the chimeric protein may comprise a CD47-binding domain, whereas, the chimeric fusion protein lacks the transmembrane and/or intracellular domain of the native CD47 receptor, but comprises a PI3K recruiter domain at the intracellular region, thereby converting the macrophage-monocyte inhibitory signal from contact with the tumor cell to a pro-inflammatory phagocytosis enhancing signal.

In some embodiments, the chimeric protein is capable of binding to multiple units of the expressed chimeric protein, for example, multimerizing. Multimerizing comprises dimer, trimer, tetramer, pentamer, hexamer, heptamer, octamer, nonamer, or decamer formations. In some embodiments, multimerizing can occur via association of the transmembrane region, the extracellular region or the intracellular region or combinations thereof. For example, a chimeric protein comprising a region of the collagenous domain of the phagocytic receptor MARCO may form a trimer for its effective function. In some embodiments, the chimeric protein is capable of associating with other molecules for example, another receptor. For example, the chimeric protein comprises an Fc-alpha transmembrane domain that dimerizes with $Fc\gamma$ TM domain, wherein the $Fc\gamma$ may be an endogenous receptor.

In some embodiments, the chimeric protein capable of multiplexing comprises multiple intracellular domains that can be activated by more than one signal and can in turn activate multiple intracellular signaling molecules. For example, the chimeric protein may comprise, a phagocytosis receptor domain and a pro-inflammatory domain. For example, the chimeric protein comprises a FcR signaling domain and an additional phosphorylation domain that recruits procaspases.

## *PHAGOCYTIC RECEPTOR (PR) SUBUNIT OF PFP FUSION PROTEIN*

Provided herein is recombinant nucleic acid encoding a CFP that is phagocytic receptor (PR) fusion protein (PFP). The PFP can comprise a PR subunit comprising: a transmembrane (TM) domain, and an intracellular domain (ICD) comprising a PR intracellular signaling domain. In some embodiments, the recombinant nucleic acid encoding the PFP when expressed in a cell, the PFP functionally incorporates into the cell membrane of the cell. In some embodiments, the recombinant nucleic acid encodes for a transmembrane domain that specifically incorporates in the membrane of a myeloid cell, such as a phagocytic cell, e.g., a macrophage.

In some embodiments, the suitable PR is selected after screening a library of membrane spanning proteins. The PR subunit is fused at the extracellular domain with a cancer cell binding antibody. In some embodiments, the PR may be fused with one or more additional domains at the intracellular end.

## INTRACELLULAR DOMAIN OF CFP FUSION PROTEIN

In some embodiments the CFP subunit comprises a TM domain of a phagocytic receptor.

In some embodiments the CFP subunit comprises an ICD domain of a phagocytic receptor.

In some embodiments, the phagocytic receptor is a scavenger receptor. Whilst many scavenger receptors collaborate in the detection and ingestion of materials, not all the receptors engaged in the course of phagocytosis trigger engulfment alone. The engagement of certain phagocytosis and scavenger receptors can have dramatic impacts on the downstream immune response. For example, triggering the type A scavenger receptor MARCO with 500 nm negatively charge nanoparticles is associated with an anti-inflammatory tolerogenic immune response. Whereas, particles with positive charge are engulfed by a subset of phagocytosis receptors that activate proinflammatory pathways such as NLRP3 and/or fibrotic responses. Furthermore, certain scavenger receptor pathways such as the scavenger receptor expressed by endothelial cells (SREC-I), have been shown to play a role in antigen cross presentation. Therefore, identifying and understanding potential receptors that can be harnessed to enhance macrophage activity and clinical efficacy is important step in the CFP development platform.

Non-opsonic receptors variably expressed naturally by professional phagocytes include lectin-like recognition molecules, such as CD169, CD33, and related receptors for sialylated residues. In addition, phagocytes also express Dectin-1 (a receptor for fungal β-glucan with well-defined signaling capacity), related C-type lectins (e.g., MICL, Dectin-2, Mincle, and DNGR-1), and a group of scavenger receptors. SR-A, MARCO, and CD36 vary in domain structure and have distinct though overlapping recognition of apoptotic and microbial ligands. CD36-related family member revealed that apoprotein ligands bind to receptor helical bundles, whereas their exofacial domains form a channel through which lipids such as cholesterol are translocated to the membrane bilayer.

### Table 2 - Scavenger receptors in human

| Gene names, aliases | NCBI Acc # |
|---|---|
| MSR1, SR-AI, CD204, SCARA1, SR-A1 | NM_138715 |
| Alternatively spliced form of SR-AI SR-AII SR-A1.1 | NM_002445 |
| MARCO, SCARA2, SR-A6 | NM_006770 |
| SCARA3, MSRL1, SR-A3 | NM_016240 |
| COLEC12, SCARA4, SRCLI, SRCLII, CL-P1, SR-A4 | NM_130386 |
| SCARA5, TESR, NET33 SR-A5 | NM_173833 |
| CD36 SCARB3, FAT, GPIV, PAS4 SR-B2 | NM_001001548 |
| SCARB1 SR-BI, CD36L1 SR-B1 | NM_005505 |
| CD68 gp110, SCARD1, LAMP4 SR-D1 | NM_001251 |
| OLR1 LOX-1, SCARE1, CLEC8A SR-E1 | NM_002543 |
| Alternatively spliced form of SRE-1 LOXIN SR-E1.1 | NM_001172632 |
| CLEC7A, Dectin-1, SCARE2, CD369, SR-E2 | NM_197947 |
| CD206/MRC1, Mannose receptor 1 SR-E3 | NM_002438 |
| ASGPR ASGR1, CLEC4H1, HL-1 SR-E4 | NM_001197216 |
| SCARF1, SREC-I, SR-F1 | NM_003693 |
| MEGF10, EMARDD, SR-F2 | NM_032446 |

(continued)

| Gene names, aliases | NCBI Acc # |
|---|---|
| CXCL16, SR-PSOX SR-G1 | NM_001100812 |
| STAB1, FEEL-1, SR-H1 | NM_015136 |
| STAB2, FEEL-2, SR-H2 | NM_017564 |
| CD163 M130, CD163A, SR-I1 | NM_004244 |
| CD163L1 CD163B, M160 SR-I2 | NM_001297650 |
| SCART1 CD163c-a SR-I3 | NR_002934.3 |
| RAGE (membrane form) AGER SR-J1 | NM_001136 |
| RAGE (soluble form) AGER SR-J1.1 | AB061668 |
| CD44 Pgp-1 SR-K1 | NM_000610 |
| LRP1 A2MR, APOER, CD91 SR-L1 | NM_002332 |
| LRP2 Megalin, gp330 SR-L2 | NM_004525 |
| SRCRB4D | NM_080744 |
| SSC5D | NM_001144950 |
| CD14 | NM_000591 |
| Ly75/CD205 | NM_002349 |
| CD207/Langerin | NM_015717 |
| CD209/DC-SIGN CLEC4L | NM_021155 |

**Table 3 - Selected ligands of SR family members**

| SR molecules | Ligands |
|---|---|
| SR-AI/II | Undefined protein in serum, Activated B cells, $\beta$ amyloid protein, Apoptotic cells AGE-modified proteins Ox-LDL, Ac-LDL, LPS, LTA, G$^+$ and G$^-$bacteria |
| MARCO | Splenic B cells UGRP-1 in lung clara cells, Ox-LDL, Ac-LDL, G$^+$ and G$^-$bacteria |
| SRCL-I/II | T and Tn antigen, Ox-LDL, G$^+$ and G$^-$bacteria, yeast |
| LOX-1 | Fibronectin, AGE-modified protein, Apoptotic cells, Ox-LDL, G$^+$ and G$^-$bacteria |
| SR-PSOX | Chemokine receptor, Phosphatidyl serine CXCR6, G$^+$ and G$^-$bacteria, Apoptotic cells, Ox-LDL |
| FEEL-I/II | AGE-modified protein, Ac-LDL, G$^+$ and G$^-$bacteria |
| dSR-CI | Ac-LDL, G$^+$ and G$^-$bacteria, glucan, laminarin |
| CD-36 | Thrombospondin, Collagen, AGE, Apoptotic cells, Ox-LDL, PfEMP protein on plasmodium infected RBC Diacylated lipids on bacteria |
| SR-BI | AGE-modified proteins, Apoptotic cells Ox-LDL |
| CLA-I/human SR-BI | Apoptotic cells,Ox-LDL, LPS, Hepatitis C virus E2 glycoprotein |
| gp-340 | Surfactant protein-A, surfactant protein-D, G$^+$ and G$^-$bacteria |

(continued)

| SR molecules | Ligands |
|---|---|
| | Influenza A virus, gp-120 |
| (ND, not defined) | |

In some embodiments, the recombinant nucleic acid encodes a chimeric antigenic receptor for phagocytosis (CAR-P). In some embodiments, the recombinant nucleic acid encodes a phagocytic receptor (PR) fusion protein.

In some embodiments, the ICD of a CFP encoded by the recombinant nucleic acid comprises a domain from a protein selected from the group consisting of TNFR1, CD40, MDA5, lectin, dectin 1, mannose receptor (CD206), scavenger receptor A1 (SRA1), MARCO, CD36, CD163, MSR1, SCARA3, COLEC12, SCARA5, SCARB1, SCARB2, CD68, OLR1, SCARF1, SCARF2, CXCL16, STAB1, STAB2, SRCRB4D, SSC5D, CD205, CD207, CD209, RAGE, CD14, CD64, F4/80, CCR2, CX3CR1, CSF1R, Tie2, HuCRIg(L), and CD169 receptor.

In some embodiments, the ICD comprises the signaling domain derived from any one or more of: lectin, dectin 1, mannose receptor (CD206), scavenger receptor A1 (SRA1), MARCO (Macrophage Receptor with Collagenous Structure, aliases: SRA6, SCARA2), CD36 (Thrombospondin receptor, aliases: Scavenger Receptor class B, member 3), CD163 (Scavenger receptor, cysteine rich-type 1), MSR1, SCARA3, COLEC12 (aliases: Scavenger Receptor With C-Type Lectin, SCARA4, or Collectin 12), SCARA5, SCARB1, SCARB2, CD68 (SCARD, microsialin), OLR1 (Oxidized Low Density Lipoprotein Receptor 1, LOX1, or C-Type Lectin Domain Family 8 Member A), SCARF1, SCARF2, SRCRB4D, SSC5D, and CD169 (aliases, Sialoadhesin receptor, SIGLEC1).

In some embodiments, the recombinant nucleic acid encodes, for example, an intracellular domain of human MARCO. The PR subunit can compriss an intracellular domain having a 44 amino acid ICD of human MARCO having an amino acid sequence: MRNKKILKEDELLSETQQAAFHQIAMEPFEINVPKPKRRNGVNF (SEQ ID NO: 36). In some embodiments the PR subunit comprises a variant which is at least 70%, 75%, 80%, 85%, 90% or 95% identical to the intracellular domain of MARCO. In some embodiments, the PR comprises a transmembrane region of human MARCO.

In some embodiments, the recombinant nucleic acid encodes an intracellular domain of human SRA1. The CFP comprises an intracellular domain having a 50 amino acid ICD of human SRA1 having an amino acid sequence: MEQWDHFHNQQEDTDSCSESVKFDARSMTA LLPPNPKNSPSLQEKLKSFK (SEQ ID NO: 37). In some embodiments the PR subunit comprises a variant which is at least 70%, 75%, 80%, 85%, 90% or 95% identical to the intracellular domain of human SRA1. The intracellular region of SRA has a phosphorylation site.

In some embodiments, the CFP comprises a transmembrane region of human SRA1.

In some embodiments, the recombinant nucleic acid comprises a sequence encoding an intracellular domain of CD36. In some embodiments, the recombinant nucleic acid comprises a sequence encoding TM domain of CD36. Naturally occurring full length CD36 has two TM domains and two short intracellular domains, and an extracellular domain of CD36 binds to oxidized LDL. Both of the intracellular domains contain pairs of cysteines that are fatty acid acylated. It lacks known signaling domains (e.g. kinase, phosphatase, g-protein binding, or scaffolding domains). N-terminal cytoplasmic domain is extremely short (5-7 amino acid residues) and is closely associated with the internal leaflet of the plasma membrane. The carboxy-terminal domain contains 13 amino acids, containing a CXCX5K motif homologous to a region in the intracellular domain of CD4 and CD8 that is known to interact with signaling molecules. The intracellular domain of CD36 is capable of assembling a signaling complex that activates lyn kinases, MAP kinases and Focal Adhesion Kinases (FAK), and inactivation of src homology 2-containing phosphotyrosine phosphatase (SHP-2). Members of the guanine nucleotide exchange factors (GEFs) have been identified as potential key signaling intermediates.

In some embodiments, the recombinant nucleic acid encodes for example, an intracellular domain of human SCARA3. The CFP may comprise an intracellular domain having a 56 amino acid ICD of human SCARA3 having an amino acid sequence: MKVRSAGGDGDALCVTEEDL AGDDEDMPTFPCTQKGRPGPRCSRCQKNLS LHTSVR (SEQ ID NO: 38). In some embodiments, the CFP comprises a variant which is at least 70%, 75%, 80%, 85%, 90% or 95% identical to an intracellular domain of human SCARA3. In some embodiments the CFP comprises a TM domain of SCARA3.

In some embodiments, the TM domain of a PR is about 20-30 amino acids long. In some embodiments, the TM domain comprises multiple transmembrane spans. In some embodiment, the TM domain comprises about 20-30, 30-40, 40-50, 50-60, 60-70, 70-80, 80-90, 90-100, 100-150, or more amino acids in length. In some embodiments, the TM domains of SRs are about 20-30 amino acids long.

Scavenger receptors may occur as homo or hetero dimers. MARCO, for example occurs as a homo trimer. In some embodiments, a scavenger receptor is a monomer. In some embodiments, the scavenger receptor is a homodimer. In some embodiments, the scavenger receptor is a heterodimer. In some embodiments, the scavenger receptor is a homotrimer. In some embodiments, the scavenger receptor is a heterotrimer. In some embodiments, the scavenger receptor is a homo tetramer. In some embodiments, the scavenger receptor is a hetero tetramer. In some embodiments, the scavenger receptor is multimer comprising two, three, four, five, six, seven, eight, night, ten or more subunits that are the same or different.

In some embodiments, the TM domain or the ICD domain of the PSP is not derived from FcR, Megf10, Bail or MerTK. In some embodiments, the ICD of the PR does not comprise a CD3 zeta intracellular domain.

In some embodiments, the intracellular domain and transmembrane domains are derived from FcRβ.

In one aspect the recombinant nucleic acid encodes a chimeric antigenic receptor for enhanced phagocytosis (CAR-P), which is a phagocytic scavenger receptor (PSR) fusion protein (PFP) comprising: (a) an extracellular domain comprising an extracellular antigen binding domain specific to an antigen of a target cell, (b) a transmembrane domain, and (c) a recombinant PSR intracellular signaling domain, wherein the recombinant PSR intracellular signaling domain comprises a first portion derived from a phagocytic receptor and a second portion derived from a non-phagocytic receptor.

In some embodiments, the second portion is not a PI3K recruitment domain.

The second portion derived from a non-phagocytic receptor may comprise an intracellular signaling domain that enhances phagocytosis, and/or inflammatory potential of the engineered myeloid cells, such as phagocytic cells, expressing the recombinant nucleic acid. In some embodiment, the second portion derived from non-phagocytic receptor comprises more than one intracellular domains (ICD). In some embodiments, the second portion derived from non-phagocytic receptor comprises a second ICD. In some embodiments, the second portion derived from non-phagocytic receptor comprises a second and a third ICD. In some embodiments, the second portion derived from non-phagocytic receptor comprises a second, a third and a fourth ICD, wherein the second portion is encoded by the recombinant nucleic acid. In some embodiments, the intracellular portion comprises two, three, four, five, six, seven, or more ICDs. The respective second portions comprising a second, or third or fourth ICD derived from non-phagocytic receptor are described as follows.

### *Chimeric antigen receptors for enhancing intracellular signaling and inflammation activation*

In one aspect, the recombinant nucleic acid encodes a second intracellular domain in addition to the phagocytic ICD, which confers capability of potent pro-inflammatory immune activation, such as when myeloid cells, such as macrophages, engage in fighting infection. The second intracellular domain (second ICD) is fused to the cytoplasmic terminus of the first phagocytic ICD. The second intracellular domain provides a second signal is necessary to trigger inflammasomes and pro-inflammatory signals. Nod-like receptors (NLRs) are a subset of receptors that are activated in innate immune response, and oligomerize to form multi-protein complexes that serve as platforms to recruit proinflammatory caspases and induce their cleavage and activation. This leads to direct activation of ROS, and often result in a violent cell death known as pyroptosis. There are four inflammasome complexes, NLRP1m, NLRP3, IPAF and AIM2.

The tumor microenvironment (TME) constitutes an immunosuppressive environment. Influence of IL-10, glucocorticoid hormones, apoptotic cells, and immune complexes can interfere with innate immune cell function. Immune cells, including phagocytic cells settle into a tolerogenic phenotype. In myeloid cells such as macrophages, this phenotype, commonly known as the M2 phenotype, is distinct from the M1 phenotype, where the cells are potent and capable of killing pathogens. Myeloid cells, such as macrophages, exposed to LPS or IFNγ, for example, can polarize towards an M1 phenotype, whereas myeloid cells, such as macrophages, exposed to IL-4 or IL-13 can polarize towards an M2 phenotype. LPS or IFNγ can interact with Toll-like receptor 4 (TLR4) on the surface of myeloid cells, such as macrophages, inducing the Trif and MyD88 pathways, inducing the activation of transcription factors IRF3, AP-1, and NFKB and thus activating TNFs genes, interferon genes, CXCL10, NOS2, IL-12, etc., for a pro-inflammatory M1 myeloid cell response. Similarly, IL-4 and IL-13 bind to IL-4R, activation the Jak/Stat6 pathway, which regulates the expression of CCL17, ARG1, IRF4, IL-10, SOCS3, etc., which are genes associated with an anti-inflammatory response (M2 response). Expression of CD14, CD80, D206 and low expression of CD163 are indicators of myeloid cells, such as macrophages, polarization towards the M1 phenotype.

In some embodiments, the recombinant nucleic acid encodes one or more additional intracellular domains, comprising a cytoplasmic domain for inflammatory response. In some embodiments, expression of the recombinant nucleic acid encoding the phagocytic receptor (PR) fusion protein (PFP) comprising the cytoplasmic domain for inflammatory response in the engineered myeloid cells, such as macrophages, confers potent pro-inflammatory response similar to the M1 phenotype.

In some embodiments, the cytoplasmic domain for inflammatory response comprises an intracellular signaling domain of TLR3, TLR4, TLR9, MYD88, TRIF, RIG-1, MDA5, CD40, IFN receptor, NLRP-1, NLRP-2, NLRP-3, NLRP-4, NLRP-5, NLRP-6, NLRP-7, NLRP-8, NLRP-9, NLRP-10, NLRP-11, NLRP-12, NLRP-13, NLRP-14, NOD1, NOD2, Pyrin, AIM2, NLRC4 and/or CD40.

In some embodiments, the phagocytic scavenger receptor (PR) fusion protein (PFP) comprises a pro-inflammatory cytoplasmic domain for activation of IL-1 signaling cascade.

In some embodiments, the cytoplasmic portion of the chimeric receptor (for example, phagocytic receptor (PR) fusion protein (PFP)) comprises a cytoplasmic domain from a toll-like receptor, such as the intracellular signaling domains of toll-like receptor 3 (TLR3), toll-like receptor 4 (TLR4), toll-like receptor 7 (TLR7), toll-like receptor 8 (TLR8), toll-like receptor 9 (TLR9).

In some embodiments, the cytoplasmic portion of the chimeric receptor comprises a suitable region from interleukin-1 receptor-associated kinase 1 (IRAK1).

In some embodiments, the cytoplasmic portion of the chimeric receptor comprises a suitable region from differentiation primary response protein (MYD88).

In some embodiments, the cytoplasmic portion of the chimeric receptor comprises a suitable region from myelin and lymphocyte protein (MAL).

In some embodiments, the cytoplasmic portion of the chimeric receptor comprises a suitable region from retinoic acid inducible gene (RIG-1).

In some embodiments the cytoplasmic portion of the CFP comprises a cytoplasmic domain of any one of MYD88, TLR3, TLR4, TLR7, TLR8, TLR9, MAL, or IRAK1.

In some embodiments, the recombinant CFP intracellular signaling domain comprises a first portion derived from a phagocytic and a second portion derived from non-phagocytic receptor wherein the second portion derived from non-phagocytic receptor comprises a phosphorylation site. In some embodiments, the phosphorylation site comprises amino acid sequences suitable for an autophosphorylation site. In some embodiments, the phosphorylation site comprises amino acid sequences suitable phosphorylation by Src family kinases. In some embodiments, the phosphorylation site comprises amino acid sequences, which upon phosphorylation are capable of binding to SH2 domains in a kinase. In some embodiments, a receptor tyrosine kinase domain is fused at the cytoplasmic end of the PFP in addition to the first cytoplasmic portion.

In some embodiments, the phosphorylation is a Tyrosine phosphorylation.

In some embodiments the second intracellular domain is an Immune receptor Tyrosine Activation Motif (ITAM). The ITAM motif is present in mammalian $\alpha$ and $\beta$ immunoglobulin proteins, TCR $\gamma$ receptors, FCR $\gamma$ receptors subunits, CD3 chains receptors and NFAT activation molecule.

In some embodiments the PFP intracellular domain comprises one ITAM motif. In some embodiments the PFP intracellular domain comprises more than one ITAM motifs. In some embodiments the PFP intracellular domain comprises two or more ITAM motifs. In some embodiments the PFP intracellular domain comprises three or more ITAM motifs. In some embodiments the PFP intracellular domain comprises four or more ITAM motifs. In some embodiments the PFP intracellular domain comprises five or more ITAM motifs. In some embodiments the PFP intracellular domain comprises six or more ITAM motifs. In some embodiments the PFP intracellular domain comprises seven or more ITAM motifs. In some embodiments the PFP intracellular domain comprises eight or more ITAM motifs. In some embodiments the PFP intracellular domain comprises nine or more ITAM motifs. In some embodiments the PFP intracellular domain comprises ten or more ITAM motifs.

In some embodiments one or more domains in the first phagocytic ICD comprises a mutation.

In some embodiments one or more domains in the second ICD comprises a mutation to enhance a kinase binding domain, to generate a phosphorylation site, to generate an SH2 docking site or a combination thereof.

### *Co-expression of an Inflammatory Gene*

In one aspect, the recombinant nucleic acid comprises a coding sequence for a pro-inflammatory gene, which is co-expressed with the PFP in the engineered cell. In some embodiments, the pro-inflammatory gene is a cytokine.

Examples include but not limited to TNF-$\alpha$, IL-1$\alpha$, IL-1$\square$, IL-6, CSF, GMCSF, or IL-12 or interferons.

The recombinant nucleic acid encoding the proinflammatory gene can be monocistronic, wherein the two coding sequences for (a) the PSP and (b) the proinflammatory gene are post-transcriptionally or post-translationally cleaved for independent expression.

In some embodiments, the two coding sequences comprise a self-cleavage domain, encoding a P2A sequence, for example.

In some embodiments the two coding regions are separated by a IRES site.

In some embodiments the two coding sequences are encoded by a bicistronic genetic element. The coding regions for (a) the PSP and (b) the proinflammatory gene can be unidirectional, where each is under a separate regulatory control. In some embodiments the coding regions for both are bidirectional and drive in opposite directions. Each coding sequence is under a separate regulatory control.

Coexpression of the proinflammatory gene is designed to confer strong inflammatory stimulation of the myeloid cells, such as macrophages, and activate the surrounding tissue for inflammation.

### *Integrin activation domains*

Cell-cell and cell-substratum adhesion is mediated by the binding of integrin extracellular domains to diverse protein ligands; however, cellular control of these adhesive interactions and their translation into dynamic cellular responses, such as cell spreading or migration, requires the integrin cytoplasmic tails. These short tails bind to intracellular ligands that connect the receptors to signaling pathways and cytoskeletal networks (Calderwood DA, 2004, Integrin Activation, Journal of Cell Science 117, 657-666, incorporated herein in its entirety). Integrins are heterodimeric adhesion receptors formed by the non-covalent association of $\alpha$ and $\beta$ subunits. Each subunit is a type I transmembrane glycoprotein that has relatively large extracellular domains and, with the exception of the $\beta4$ subunit, a short cytoplasmic tail. Individual integrin family members have the ability to recognize multiple ligands. Integrins can bind to a large number of extracellular matrix proteins (bone matrix proteins, collagens, fibronectins, fibrinogen, laminins, thrombospondins, vitronectin, and von Willebrand factor), reflecting the primary function of integrins in cell adhesion to extracellular matrices. Many "counter-receptors" are ligands, reflecting the role of integrins in mediating cell-cell interactions. Integrins undergo conformational changes to increase ligand affinity.

The Integrin $\beta_2$ subfamily consists of four different integrin receptors, $\alpha_M\beta_2$ (CD11b/CD18, Mac-1, CR3, Mo-1), $\alpha_L\beta_2$ (CD11a/CD18, LFA-1), $\alpha_X\beta_2$ (CD11c/CD18), and $\alpha_D\beta_2$ (CD11d/CD18). These leukocyte integrins are involved in virtually every aspect of leukocyte function, including the immune response, adhesion to and transmigration through the endothelium, phagocytosis of pathogens, and leukocyte activation.

The $\alpha$ subunits of all $\beta_2$ integrins contain an inserted region of ~200 amino acids, termed the I or A domain. Highly conserved I domains are found in several other integrin $\alpha$ subunits and other proteins, such as certain coagulation and complement proteins. I domains mediate protein-protein interactions, and in integrins, they are integrally involved in the binding of protein ligands. Although the I domains dominate the ligand binding functions of their integrins, other regions of the $\alpha$ subunits do influence ligand recognition. As examples, in $\alpha_M\beta_2$ a mAb (OKM1) recognizing an epitope outside the I domain but in the $\alpha_M$ subunit inhibits ligand binding; and the EF-hand regions in $\alpha_L\beta_2$ and $\alpha_2\beta_1$, integrins with I domains in their $\alpha$ subunits, contribute to ligand recognition. The $\alpha_M$ subunit, and perhaps other $\alpha$ subunits, contains a lectin-like domain, which is involved in engagement of non-protein ligands, and occupancy may modulate the function of the I domain.

As integrins lack enzymatic activity, signaling is instead induced by the assembly of signaling complexes on the cytoplasmic face of the plasma membrane. Formation of these complexes is achieved in two ways; first, by receptor clustering, which increases the avidity of molecular interactions thereby increasing the on-rate of binding of effector molecules, and second, by induction of conformational changes in receptors that creates or exposes effector binding sites. Within the ECM, integrins have the ability to bind fibronectin, laminins, collagens, tenascin, vitronectin and thrombospondin. Clusters of integrin/ECM interactions form focal adhesions, concentrating cytoskeletal components and signaling molecules within the cell. The cytoplasmic tail of integrins serve as a binding site for $\alpha$-actinin and talin which then recruit vinculin, a protein involved in anchoring F-actin to the membrane. Talin is activated by kinases such as protein kinase C (PKC$\alpha$).

Integrins are activated by selectins. Leucocytes express L-selectin, activated platelets express P-selectin, and activated endothelial cells express E-and P-selectin. P-selectin-mediated adhesion enables chemokine-or platelet-activating factor-triggered activation of $\beta2$ integrins, which stabilizes adhesion. It also facilitates release of chemokines from adherent leucocytes. The cytoplasmic domain of P-selectin glycoprotein ligand 1 formed a constitutive complex with Nef-associated factor 1. After binding of P-selectin, Src kinases phosphorylated Nef-associated factor 1, which recruit the phosphoinositide-3-OH kinase p85-p110$\delta$ heterodimer and result in activation of leukocyte integrins. E-selectin ligands transduce signals that also affect $\beta2$ integrin function. Selectins trigger activation of Src family kinases. SFKs activated by selectin engagement phosphorylate the immunoreceptor tyrosine-based activation motifs (ITAMs) in the cytoplasmic domains of DAP12 and FcR$\gamma$. In some respects, CD44 is sufficient to transduce signals from E-selectin. CD44 triggers the inside-out signaling of integrins. A final common step in integrin activation is binding of talin to the cytoplasmic tail of the $\beta$ subunit. Kindlins, another group of cytoplasmic adaptors, bind to a different region of integrin $\beta$ tails. Kindlins increase the clustering of talin-activated integrins. Kindlins are responsive to selectin signaling, however, kindlins are found mostly in hematopoietic cells, such as neutrophils. Selectin signaling as well as signaling upon integrin activation by chemokines components have shared components, including SFKs, Syk, and SLP-76.

In some embodiments, the intracellular domain of the recombinant CFP comprises an integrin activation domain. The integrin activation domain comprises an intracellular domain of a selectin, for example, a P-selectin, L-selectin or E-selectin.

In some embodiments, the intracellular domain of the recombinant CFP comprises an integrin activation domain of laminin.

In some embodiments, the intracellular domain of the recombinant CFP comprises an integrin activation domain for activation of Talin.

In some embodiments, the intracellular domain of the recombinant CFP comprises an integrin activation domain fused to the cytoplasmic end of the phagocytic receptor ICD domain.

## Chimeric Receptor for Enhancing Antigen Cross presentation

In some embodiments, the recombinant nucleic acid encodes a domain capable of enabling cross presentation of antigens. In general, MHC class I molecules present self-or pathogen-derived antigens that are synthesized within the cell, whereas exogenous antigens derived via endocytic uptake are loaded onto MHC class II molecules for presentation to CD4+ T cells. MHC I-restricted presentation of endogenous antigens, in which peptides are generated by the proteasome. However, in some cases, DC can process exogenous antigens into the MHC-I pathway for presentation to CD8+ T cells. This is referred to as cross presentation of antigens. Soluble or exogenous antigenic components may get degraded by lysosomal proteases in the vacuoles and cross presented by DCs, instead of following the endocytotic pathway. In some instances, chaperones, such as heat shock protein 90 (Hsp90) have shown to help cross present antigens by certain APCs. HSP-peptide complexes are known to be internalized by a distinct group of receptors compared to free polypeptides. These receptors were from the scavenger receptor families and included LOX-1, SREC-I/SCARF-I, and FEEL1/Stabilin-1. Both SREC-I and LOX-1 have been shown to mediate the cross presentation of molecular chaperone bound antigens and lead to activation of CD8+ T lymphocytes.

SREC-1 (scavenger receptor expressed by endothelial cells) has no significant homology to other types of scavenger receptors but has unique domain structures. It contains 10 repeats of EGF-like cysteine-rich motifs in the extracellular domain. Recently, the structure of SREC-I was shown to be similar to that of a transmembrane protein with 16 EGF-like repeats encoded by the *Caenorhabditis elegans* gene *ced-I,* which functions as a cell surface phagocytic receptor that recognizes apoptotic cells.

Cross presentation of cancer antigens through the Class-I MHC pathway results in enhanced CD8+ T cell response, which is associated with cytotoxicity and therefore beneficial in tumor regression. In some embodiments, the intracellular domain of the PFP comprises a SREC1 intracellular domain. In some embodiments, the intracellular domain of the PFP comprises a SRECII intracellular domain.

In some embodiments, the CFP comprises: an intracellular domain comprising a PSR intracellular signaling domain from SREC1 or SRECII.

In some embodiments, the CFP comprises: (i) a transmembrane domain, and (ii) an intracellular domain comprising a CFP intracellular signaling domain from SREC1 or SRECII.

In some embodiments, the CFP comprises: (i) a transmembrane domain, (ii) an intracellular domain comprising a intracellular signaling domain, and (iii) an extracellular domain from SREC1 or SRECII.

## TRANSMEMBRANE DOMAIN OF PFP FUSION PROTEIN

In some embodiments, the TM encoded by the recombinant nucleic acid comprises a sequence encoding a domain of a scavenger receptor (SR). In some embodiments, the TM can be the TM domain of or derived from any one or more of: lectin, dectin 1, mannose receptor (CD206), SRA1, MARCO, CD36, CD163, MSR1, SCARA3, COLEC12, SCARA5, SCARB1, SCARB2, CD68, OLR1, SCARF1, SCARF2, SRCRB4D, SSC5D, and CD169.

In some embodiments, the TM domains are about 20-30 amino acids long. TM domains of SRs are about 20-30 amino acids long.

In some embodiments, the TM domain or the ICD domain of the CFP is not derived from Megf10, Bai1 or MerTK. In some embodiments, the ICD of the CFP does not comprise a CD3ζ intracellular domain.

In some embodiments the TM is derived from the same phagocytic receptor as the ICD.

In some embodiments, the TM region is derived from a plasma membrane protein. The TM can be selected from an Fc receptor (FcR). In some embodiments, nucleic acid sequence encoding domains from specific FcRs are used for cell-specific expression of a recombinant construct. An FCR-alpha region comprising the TM domain may be used for a myeloid cell, such as a macrophage, specific expression of the construct. FcRα recombinant protein can be expressed in mast cells.

In some embodiments, the PFP comprises the TM of FcRβ.

In some embodiments, the PFP comprises both the FcRβ and ICD domains. In some embodiments, the PFP comprises both the FcRα and ICD domains.

In some embodiments, the TM domain is derived from CD8.

In some embodiments, the TM is derived from CD2.

In some embodiments the TM is derived from FcRα.

## EXTRACELLULAR DOMAIN OF PFP FUSION PROTEIN

In some embodiments, the extracellular domain of a PFP fusion protein provided herein comprises an antigen binding domain that binds to one or more targets. The binding targets may be antigens or ligands. For example, a binding target may be an antigen on a target cell. In some embodiments, the target binding domain is specific for the target. In some embodiments, the extracellular domain can include an antibody or an antigen-binding domain selected from intrabodies, peptibodies, nanobodies, single domain antibodies. SMIPs, and multispecific antibodies.

In some embodiments, an antibody fragment comprises a portion of an intact antibody, such as the antigen binding or variable region of the intact antibody. In a further aspect of the invention, an anti-HIV antibody according to any of the above embodiments is a monoclonal antibody, including a chimeric, humanized or human antibody. Antibody fragments include, but are not limited to, Fab, Fab', Fab'-SH, F(ab')$_2$, Fv, diabody, linear antibodies, multispecific formed from antibody fragments antibodies and scFv fragments, and other fragments described below. In another embodiment, the antibody is a full length antibody, e.g., an intact IgG1 antibody or other antibody class or isotype as described herein. (*See, e.g.,* Hudson et al. Nat. Med. 9:129-134 (2003); Pluckthiin, The Pharmacology of Monoclonal Antibodies, vol. 113, pp. 269-315 (1994); Hollinger et al., Proc. Natl. Acad. Sci. USA 90: 6444-6448 (1993); WO93/01161; and U.S. Pat. Nos. 5,571,894, 5,869,046, 6,248,516, and 5,587,458). A full length antibody, intact antibody, or whole antibody is an antibody having a structure substantially similar to a native antibody structure or having heavy chains that contain an Fc region as defined herein. Antibody fragments can be made by various techniques, including but not limited to proteolytic digestion of an intact antibody as well as production by recombinant host cells (*e.g., E. coli* or phage), as described herein.

An Fv is the minimum antibody fragment that contains a complete antigen-recognition and antigen-binding site. This fragment contains a dimer of one heavy-and one light-chain variable region domain in tight, non-covalent association. From the folding of these two domains emanate six hypervariable loops (three loops each from the H and L chain) that contribute the amino acid residues for antigen binding and confer antigen binding specificity to the antibody. However, even a single variable region (or half of an Fv comprising only three CDRs specific for an antigen) has the ability to recognize and bind antigen, although at a lower affinity than the entire binding site.

A single-chain Fv (sFv or scFv) is an antibody fragment that comprises the $V_H$ and $V_L$ antibody domains connected into a single polypeptide chain. The sFv polypeptide can further comprise a polypeptide linker between the $V_H$ and $V_L$ domains that enables the sFv to form the desired structure for antigen binding. (*See, e.g.,* Pluckthun in The Pharmacology of Monoclonal Antibodies, vol. 113, Rosenburg and Moore eds., Springer-Verlag, New York, pp. 269-315 (1994); Borrebaeck 1995, infra. The sFv can be used in a chimeric antigen receptor (CAR).

A diabody is a small antibody fragment prepared by constructing an sFv fragment with a short linker (about 5-10 residues) between the $V_H$ and $V_L$ domains such that inter-chain but not intra-chain pairing of the V domains is achieved, resulting in a bivalent fragment. Bispecific diabodies are heterodimers of two crossover sFv fragments in which the $V_H$ and $V_L$ domains of the two antibodies are present on different polypeptide chains. (*See, e.g.,* EP 404,097; WO 93/11161; and Hollinger et al, Proc. Natl. Acad. Sci. USA, 90:6444-6448 (1993)).

Domain antibodies (dAbs), which can be produced in fully human form, are the smallest known antigen-binding fragments of antibodies, ranging from about 11 kDa to about 15 kDa. DAbs are the robust variable regions of the heavy and light chains of immunoglobulins ($V_H$ and $V_L$, respectively). They are highly expressed in microbial cell culture, show favorable biophysical properties including, for example, but not limited to, solubility and temperature stability, and are well suited to selection and affinity maturation by in vitro selection systems such as, for example, phage display. DAbs are bioactive as monomers and, owing to their small size and inherent stability can be formatted into larger molecules to create drugs with prolonged serum half-lives or other pharmacological activities. (*See, e.g.,* WO9425591 and US20030130496).

Fv and sFv are the only species with intact combining sites that are devoid of constant regions. Thus, they are suitable for reduced nonspecific binding during in vivo use. sFv fusion proteins can be constructed to yield fusion of an effector protein at either the amino or the carboxy terminus of an sFv. The antibody fragment also can be a "linear antibody. (*See, e.g.,* U.S. Pat. No. 5,641,870). Such linear antibody fragments can be monospecific or bispecific.

In some embodiments, the extracellular domain includes a Fab binding domain. In yet other such embodiments, the extracellular domain includes a scFv.

In some embodiments the chimeric antigen receptor comprises an extracellular antigen binding domain is derived from the group consisting of an antigen-binding fragment (Fab), a single-chain variable fragment (scFv), a nanobody, a $V_H$ domain, a $V_L$ domain, a single domain antibody (sdAb), a VNAR domain, and a $V_{HH}$ domain, a bispecific antibody, a diabody, or a functional fragment of any thereof. In some embodiments, the antigen-binding fragment (Fab), a single-chain variable fragment (scFv), a nanobody, a $V_H$ domain, a $V_L$ domain, a single domain antibody (sdAb), a VNAR domain, and a $V_{HH}$ domain, a bispecific antibody, a diabody, or a functional fragment of any thereof specifically bind to one or more antigens.

In some embodiments, the antigen is a cancer antigen, and the target cell is a target cancer cell. In some embodiments, the antigen for a target cell is selected from the group consisting of CD3, CD4, CD5, CD7, CD19, CCR2, CCR4, CD30, CD37, TCRB1/2, TCR $\alpha\beta$, TCR$\alpha\delta$. CD22, HER2 (ERBB2/neu), Mesothelin, PSCA, CD123, CD30, CD171, CD138, CS-1, CLECL1, CD33, CD79b, EGFRvIII, GD2, GD3, BCMA, PSMA, ROR1, FLT3, TAG72, CD38, CD44v6, CEA, EPCAM, B7H3 (CD276), KIT (CD 117), CD213A2, IL-1 IRa, PRSS21, VEGFR2, CD24, MUC-16, PDGFR-$\beta$, SSEA-4, CD20, MUC1, EGFR, NCAM, Prostase, PAP, ELF2M, Ephrin B2, FAP, EphA2, GM3, TEM1/CD248, TEM7R, CLDN6, TSHR, GPRC5D, CD97, CD179a, ALK, and IGLL1.

In some embodiments, the target antigen is an autoimmune antigen. In some embodiments, the target cell is a B cell. In some embodiments, the target cell is a B cell that produces autoantibodies. In some embodiments, the target antigen is Dsg1 or Dsg3.

Various cancer antigen targets can be selected from cancer antigens known to one of skill in the art. Depending on the cancer and the cell type involved cancer antigens are mutated native proteins. The antigen binding domains are screened for specificity towards mutated/cancer antigens and not the native antigens.

In some embodiments, for example, the cancer antigen for a target cancer cell can be one or more of the mutated/cancer antigens: MUC16, CCAT2, CTAG1A, CTAG1B, MAGE A1, MAGEA2, MAGEA3, MAGEA4, MAGEA6, PRAME, PCA3, MAGEC1, MAGEC2, MAGED2, AFP, MAGEA8, MAGE9, MAGEA11, MAGEA12, IL13RA2, PLAC1, SDCCAG8, LSP1, CT45A1, CT45A2, CT45A3, CT45A5, CT45A6, CT45A8, CT45A10, CT47A1, CT47A2, CT47A3, CT47A4, CT47A5, CT47A6, CT47A8, CT47A9, CT47A10, CT47A11, CT47A12, CT47B1, SAGE1, and CT55.

In some embodiments, for example, the cancer antigen for a target cancer cell can be one or more of the mutated/cancer antigens: CD2, CD3, CD4, CD5, CD7, CD8, CD20, CD30, CXCR4, CD45, CD56, where the cancer is a T cell lymphoma.

In some embodiments, for example, the cancer antigen for a target cancer cell can be one or more of the mutated/cancer antigens: IDH1, ATRX, PRL3, or ETBR, where the cancer is a glioblastoma.

In some embodiments, for example, the cancer antigen for a target cancer cell can be one or more of the mutated/cancer antigens: CA125, β-hCG, urinary gonadotropin fragment, AFP, CEA, SCC, inhibin or extradiol, where the cancer is ovarian cancer.

In some embodiments the cancer antigen for a target cancer cell may be CD5.

In some embodiments the cancer antigen for a target cancer cell may be HER2.

In some embodiments the cancer antigen for a target cancer cell may be EGFR Variant III.

In some embodiments the cancer antigen for a target cancer cell may be CD19.

In some embodiments, the SR subunit region comprises an extracellular domain (ECD) of the scavenger receptor. In some embodiments, the ECD of the scavenger receptor comprises an ECD domain of the SR comprising the ICD and the TM domains. In some embodiments the target antigen is the SR-ligand on the cancer cell, for example, any one of the ligand components from Table 2 or Table 3. In some embodiments, the SR-ECD contributes to the binding of the phagocyte to the target cell, and in turn is activated, and activates the phagocytosis of the target cell.

In some embodiments, the CFP comprises an ECD or portion thereof of a scavenger receptor. In some embodiments, the CFP comprises an ICD or portion thereof of a scavenger receptor. In some embodiments, the CFP comprises a TM domain of a scavenger receptor. In some embodiments, the ECD encoded by the recombinant nucleic acid comprises a domain selected from the group consisting of lectin, dectin 1, mannose receptor (CD206), scavenger receptor A1 (SRA1), MARCO, CD36, CD163, MSR1, SCARA3, COLEC12, SCARA5, SCARB1, SCARB2, CD68, OLR1, SCARF1, SCARF2, CXCL16, STAB1, STAB2, SRCRB4D, SSC5D, CD205, CD207, CD209, RAGE, CD14, CD64, F4/80, CCR2, CX3CR1, CSF1R, Tie2, HuCRIg(L), and CD169. The extracellular domains of most scavenger receptors contain scavenger receptors with a broad binding specificity that may be used to discriminate between self and non-self in the nonspecific antibody-independent recognition of foreign substances. The type I and II class A scavenger receptors (SR-AI1 and SR-AII) are trimeric membrane glycoproteins with a small NH2-terminal intracellular domain, and an extracellular portion containing a short spacer domain, an a-helical coiled-coil domain, and a triple-helical collagenous domain. The type I receptor additionally contains a cysteine-rich COOH-terminal (SRCR) domain. These receptors are present in myeloid cells, such as macrophages, in diverse tissues throughout the body and exhibit an unusually broad ligand binding specificity. They bind a wide variety of polyanions, including chemically modified proteins, such as modified LDL, and they have been implicated in cholesterol deposition during atherogenesis. They may also play a role in cell adhesion processes in macrophage-associated host defense and inflammatory conditions.

In some embodiments, the SR ECD is designed to bind to pro-apoptotic cells. In some embodiments, the scavenger receptor ECD comprises a binding domain for a cell surface molecule of a cancer cell or an infected cell.

In some embodiments, the extracellular domain of the PR subunit is linked by a linker to a target cell binding domain, such as an antibody or part thereof, specific for a cancer antigen.

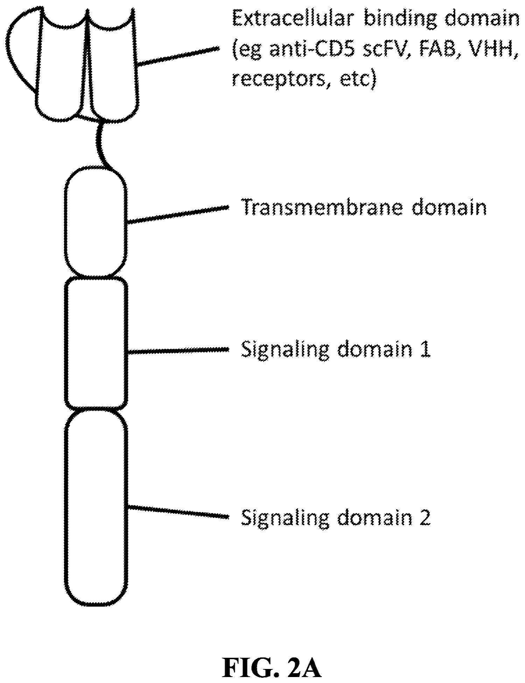

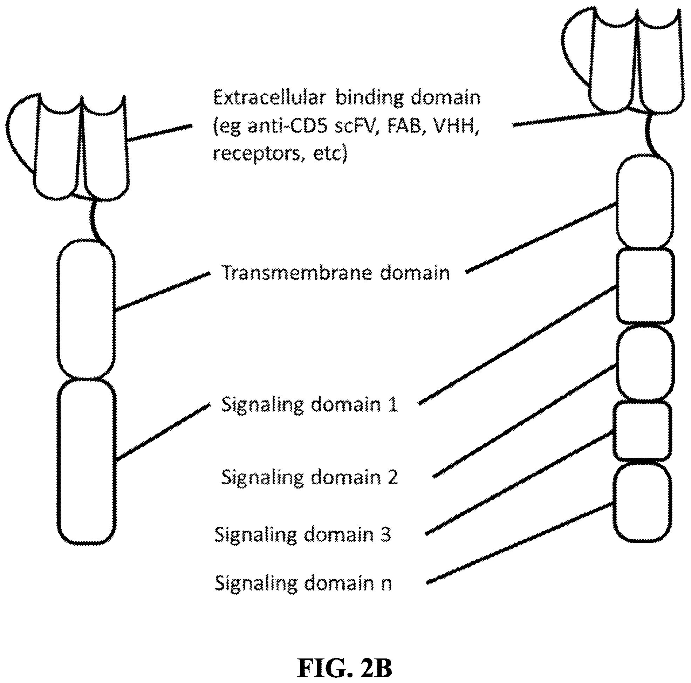

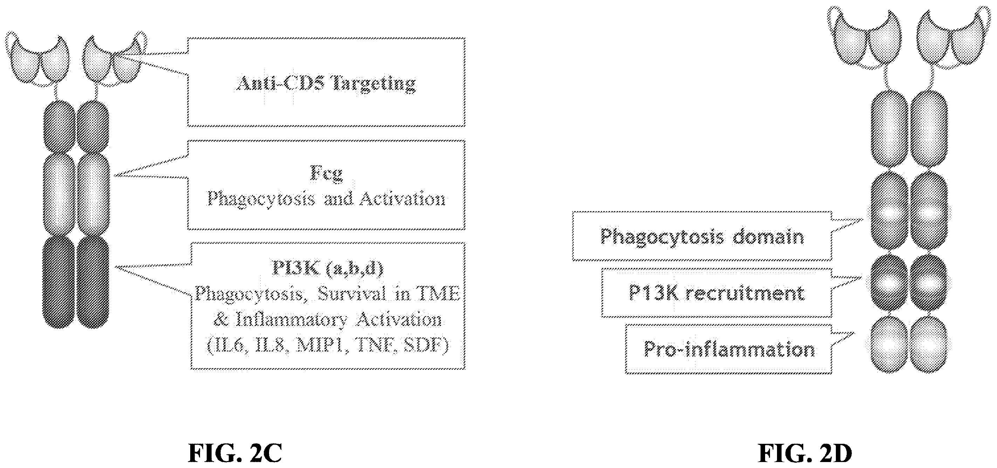

In some embodiments, the extracellular antigen binding domain comprises one antigen binding domain. In some embodiments, the extracellular antigen binding domain comprises more than one binding domain. In some embodiments the binding domain are scFvs. FIG. 2 shows a diagrammatic representation of an embodiment, where the PFP targets a single target on a cancer cell (left) or multiple targets (right). The one or more than one scFvs are fused to the recombinant PR at the extracellular domain. In some embodiments the scFv fraction and the extracellular domain of the PR are linked via a linker.

In some embodiments, the ECD antigen binding domain can bind to an intracellular antigen. In some embodiments, the intracellular antigen is a cancer antigen.

In some embodiments, the extracellular antigen binding domain binds to the target ligand with an affinity of less than 1000 nM. In some embodiments, the extracellular antigen binding domain binds to the target ligand with an affinity of less than 500 nM. In some embodiments, the extracellular antigen binding domain binds to the target ligand with an affinity of less than 450 nM. In some embodiments, the extracellular antigen binding domain binds to the target ligand with an affinity of less than 400 nM. In some embodiments, the extracellular antigen binding domain binds to the target ligand with an affinity of less than 350 nM. In some embodiments, the extracellular antigen binding domain binds to the target ligand with an affinity of less than 250 nM. In some embodiments, the extracellular antigen binding domain binds to the target ligand with an affinity of less than 200 nM. In some embodiments, the extracellular antigen binding domain binds to the target ligand with an affinity of less than 100 nM. In some embodiments, the extracellular antigen binding domain binds to the target ligand with an affinity ranging between than 200 nM to 1000 nM. In some embodiments, the extracellular antigen binding domain binds to the target ligand with an affinity ranging between than 300 nM to 1.5 mM. In some embodiments, the antigen binding domain binds to the target ligand with an affinity > 200 nM, > 300 nM or >500 nM.

In some embodiments, the extracellular antigen binding domain binds to the target ligand, where the target ligand is a T cell, the binding characteristics are such that the target T cell is not triggered to activate T cell mediated lysis of the engineered cell. In some embodiments, binding of the TCR to a ligand on the engineered cell is avoided, bypassed or inhibited.

*LINKERS*

Linkers may be used to link any of the polypeptides or peptide domains of the present disclosure. The PFP fusion proteins described herein may comprises one or more linkers. For example, one or more of the domains and subunits of a PFP fusion protein can be directly fused to another domain or subunit, or can be connected to another domain or subunit via a linker. In some embodiments, the extracellular antigen binding domains comprising the antibody specific for the antigen on a target cell, parts of an antibody that can specifically bind to an antigen on a target cell, or scFvs specific for an antigen on a target cell are linked to the TM domain or other extracellular domains by a linker. In some embodiments, where there are more than one scFv at the extracellular antigen binding domain, the more than scFvs are linked with each other by linkers.

In some embodiments, linkers are short peptide sequences.

The linker may be as simple as a covalent bond, or it may be a polymeric linker many atoms in length. In certain embodiments, the linker is a polypeptide or based on amino acids. In other embodiments, the linker is not peptide-like. In certain embodiments, the linker is a covalent bond (e.g., a carbon-carbon bond, disulfide bond, carbon-heteroatom bond, etc.).

In some embodiments, the linker is an amino acid or a plurality of amino acids (e.g., a peptide or protein). In some embodiments, the linker is a bond (e.g., a covalent bond), an organic molecule, group, polymer, or chemical moiety. In some embodiments, the linker is about 3 to about 104 (e.g., 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 55, 60, 65, 70, 75, 80, 85, 90, 95, or 100) amino acids in length. In some embodiments the linkers are stretches of Glycine and one or more Serine residues. Other amino acids preferred for short peptide linkers include but are not limited to threonine (Thr), serine (Ser), proline (Pro), glycine (Gly), aspartic acid (Asp), lysine (Lys), glutamine (Gln), asparagine (Asn), and alanine (Ala) arginine (Arg), phenylalanine (Phe), glutamic acid (Glu). Of these Pro, Thr, and Gln are frequently used amino acids for natural linkers. Pro is a unique amino acid with a cyclic side chain which causes a very restricted conformation. Pro-rich sequences are used as interdomain linkers, including the linker between the lipoyl and E3 binding domain in pyruvate dehydrogenase (GA$_2$PA$_3$PAKQEA$_3$PAPA$_2$KAEAPA$_3$PA$_2$KA (SEQ ID NO: 39)). For the purpose of the disclosure, the empirical linkers may be flexible linkers, rigid linkers, and cleavable linkers. Sequences such as (G4S)x (where x is multiple copies of the moiety, designated as 1, 2, 3, 4, and so on) (SEQ ID NO: 40) comprise a flexible linker sequence. Other flexible sequences used herein include several repeats of glycine, e.g., (Gly)6 (SEQ ID NO: 41) or (Gly)8 (SEQ ID NO: 42). On the other hand, a rigid linker may be used, for example, a linker (EAAAK)x, where x is an integer, 1, 2, 3, 4 etc. (SEQ ID NO: 43) gives rise to a rigid linker. Various linker lengths and flexibilities between domains or subunits of the fusion proteins provided herein can be employed, e.g., ranging from very flexible linkers of the form (GGGS)n (SEQ ID NO: 44), (GGGGS)n (SEQ ID NO: 45), and (G)n to more rigid linkers of the form (EAAAK)n (SEQ ID NO: 46), (SGGS)n (SEQ ID NO: 47), SGSETPGTSESATPES (SEQ ID NO: 48) (see, e.g., Guilinger JP, Thompson DB, Liu DR. Fusion of catalytically inactive Cas9 to FokI nuclease improves the specificity of genome modification. Nat. Biotechnol. 2014;32(6): 577-82; the entire contents are incorporated herein by reference) and (XP)n) in order to achieve the optimal length. In some embodiments, n is 1, 2, 3, 4, 5, 6, 7, 8, 9, 10, 11, 12, 13, 14, or 15. In some embodiments, the linker comprises a (GGS)n motif, wherein n is 1, 3, or 7 (SEQ ID NO: 49). In some embodiments, the linker comprises amino acid sequence SGGGGSG (SEQ ID NO: 11). In some embodiments, the linker comprises amino acid sequence GSGS (SEQ ID NO: 13).

In some embodiments the linkers are flexible. In some embodiments the linkers comprise a hinge region. Where included, such a spacer or linker domain may position the binding domain away from the host cell surface to further enable proper cell/cell contact, binding, and activation. The length of the extracellular spacer may be varied to optimize target molecule binding based on the selected target molecule, selected binding epitope, binding domain size and affinity. In certain embodiments, an extracellular spacer domain is an immunoglobulin hinge region (e.g., IgG1, IgG2, IgG3, IgG4, IgA, IgD). An immunoglobulin hinge region may be a wild type immunoglobulin hinge region or an altered wild type immunoglobulin hinge region. In some embodiments, a linker or a spacer used herein comprises an IgG4 hinge region, having a sequence: ESKYGPPCPPCP (SEQ ID NO: 50). In some embodiments, the hinge region comprises a hinge or a spacer comprising a sequence present in the extracellular regions of type 1 membrane proteins, such as CD8a, CD4, CD28 and CD7, which may be wild-type or variants thereof. In some embodiments, an extracellular spacer domain comprises all or a portion of an immunoglobulin Fc domain selected from: a CH1 domain, a CH2 domain, a CH3 domain, or combinations thereof. In some embodiments the spacer or the linker may be further modified by post-translation modifications, such as glycosylation.

In some embodiments, an extracellular spacer domain may comprise a stalk region of a type II C-lectin (the extracellular domain located between the C-type lectin domain and the transmembrane domain). Type II C-lectins include CD23, CD69, CD72, CD94, NKG2A, and NKG2D. In yet further embodiments, an extracellular spacer domain may be derived from scavenger receptor MERTK.

In some embodiments, the linker comprises at least 2, or at least 3 amino acids. In some embodiments, the linker comprises 4 amino acids. In some embodiments, the linker comprises 5 amino acids. In some embodiments, the linker comprises 6 amino acids. In some embodiments, the linker comprises 7 amino acids. In some embodiments, the linker comprises 8 amino acids. In some embodiments, the linker comprises 9 amino acids. In some embodiments, the linker comprises 8 amino acids. In some embodiments, the linker comprises 10 amino acids. In some embodiments the linker comprises greater than 10 amino acids. In some embodiments, the linker comprises 11, 12, 13, 14, 15, 16, 17, 18, 19, or 20 amino acids. In some embodiments there are 12 or more amino acids in the linker. In some embodiments, there are 14 or more amino acids in the linker. In some embodiments, there are 15 or more amino acids in the linker.

### *Other Fusion proteins for Enhancement of Phagocytosis*

In one aspect of the disclosure, recombinant nucleic acids are prepared which encode one or more chimeric receptors that enhance phagocytosis in myeloid cells, such as macrophages, principally by blocking inhibitory signals. Myeloid cells, such as macrophages, especially in the tumor environment encounter phagocytosis dampening or inhibitory signals, such as CD47 mediated anti-phagocytic activity on target cells, e.g., cancer cells. Chimeric receptors are generated which when expressed in a phagocytic cell blocks CD47 signaling.

In some embodiments, other CAR fusion protein may be designed for expression in a phagocytic cell that may enhance phagocytosis. In one embodiment, provided herein is a composition comprising a recombinant nucleic acid encoding a chimeric antigen receptor (CAR) fusion protein (CFP) comprising: (a) a subunit comprising: (i) an extracellular domain; and (ii) a transmembrane domain; (b) an extracellular antigen binding domain specific to CD47 of a target cell; wherein: the extracellular domain of the subunit and the extracellular antigen binding domain are operatively linked; and the subunit does not comprise a functional intracellular domain of an endogenous receptor that binds CD47, or does not comprise an intracellular domain that activates a phosphatase. In some embodiments, the extracellular antigen binding domain is derived from signal-regulatory protein alpha (SIRPα). In some embodiments, the extracellular antigen binding domain is derived from signal-regulatory protein alpha (SIRPβ). In some embodiments, the transmembrane domain is derived from SIRPα. In some embodiments, the transmembrane domain is derived from SIRPβ.

In some embodiments, the additional CAR fusion protein (CFP) may be co-transfected with the recombinant PFP described above. In some embodiments, the scavenger receptor intracellular domain comprises a second intracellular domain comprising a signaling domain that activates phagocytosis; or a proinflammatory domain at the cytoplasmic terminus, which are operably linked. The signaling domain that activates phagocytosis is derived from a receptor selected from the group consisting of the receptors listed in Table 2.

In some embodiments, the intracellular domain with a phagocytosis signaling domain comprises a domain having one or more Immunoreceptor Tyrosine-based Activation Motif (ITAM) motifs. ITAMs are conserved sequences present in the cytoplasmic tails of several receptors of the immune system, such as T cell receptors, immunoglobulins (Ig) and FcRs. They have a conserved amino acid sequence motif consisting of paired YXXL/I motifs (Y= Tyrosine, L= Lysine and I= Isoleucine) separated by a defined interval (YXXL/I-$X_{6-8}$-YXXL/I). In addition, most ITAMs contain a negatively charged amino acid (D/E) in the +2 position relative to the first ITAM tyrosine. Phosphorylation of residues within the ITAM recruits several signaling molecules that activate phagocytosis. ITAM motifs are also present in the intracellular adapter protein, DNAX Activating Protein of 12 kDa (DAP12).

In some embodiments, the phagocytic signaling domain in the intracellular region comprises a PI3kinase (PI3K) recruitment domain (also called PI3K binding domain). The PI3K binding domains used herein can be the respective PI3K binding domains of CD19, CD28, CSFR or PDGFR. PI3 kinase recruitment to the binding domain leads to the Akt mediated signaling cascade and activation of phagocytosis. The PI3K-Akt signaling pathway is important in phagocytosis, regulation of the inflammatory response, and other activities, including vesicle trafficking and cytoskeletal reorganization. The PI3kinase recruitment domain is an intracellular domain in a plasma membrane protein, which has tyrosine residues that can be phosphorylated, and which can in turn be recognized by the Src homology domain (SH2) domain of PI3Kp85.

The SH2 domain of p85 recognizes the phosphorylated tyrosines on the cytosolic domain of the receptor. This causes an allosteric activation of p110 and the production of phosphatidylinositol-3,4,5-trisphosphate ($PIP_3$) that is recognized by the enzymes Akt and the constitutively active 3'-phosphoinositide-dependent kinase 1 (PDK1) through their plekstrin homology domains. The interaction of Akt with $PIP_3$ causes a change in the Akt conformation and phosphorylation of the residues Thr308 and Ser473 by PDK1 and rictor-mTOR complex, respectively. Phosphorylation of these two residues causes the activation of Akt which in turn phosphorylates, among other substrates, the enzyme glycogen synthase kinase-3 (GSK-3). GSK-3 has two isoforms, GSK-3α and GSK-3β both of which are constitutively active. The isoforms are structurally related but functionally nonredundant. Inactivation of GSK-3 is observed when the residues Ser21 in GSK-3α or Ser9 in GSK-3β, located in their regulatory N-terminal domains, are phosphorylated by Akt and other kinases. Inhibition of GSK-3 by phosphorylation is important for the modulation of the inflammation and in phagocytosis processes.

In some embodiments, a recombinant PFP comprises (a) an extracellular CD47 binding domain SIRPα, (b) a SIRPβ transmembrane domain, and (c) an intracellular domain of SIRPβ. SIRPβ signaling can activate pro-phagocytic signaling by engaging DAP12 activation.

Various members of the family transduce checkpoint signal upon contact with sialylated glycans on membrane proteins. In some members, the intracellular domains of the Siglec proteins comprise multiple immunoreceptor tyrosine-based inhibitory motifs (ITIMs). ITIMs share a consensus amino acid sequence in their cytoplasmic tail, namely (I/V/L/S)-X-Y-X-X-(L/V), where X denotes any amino acid, I= Isoleusine, V=valine, L=Lysine, S=Serine, Y=Tyrosine. Phosphorylation of the Tyrosine residues at the ITIM motif recruit either of two SH2 domain-containing negative regulators: the inositol phosphatase SHIP (Src homology 2-containing inositol polyphosphate 5-phosphatase) or the tyrosine phosphatase SHP-1 (Src homology 2-containing protein tyrosine phosphatase-1). A leucine in the (Y+2) position favors binding to SHIP, whereas an isoleucine in the (Y-2) position favors SHP-1 binding. ITIMs can also bind to another tyrosine phosphatase, SHP-2, but evidence for SHP-2 playing a functional role in ITIM-mediated inhibition is less clear than for the other mediators. Therefore, activation of the Siglec membrane proteins at the extracellular ligand binding domain by binding with a sialic acid residue, (e.g. in sialylated membrane glycan proteins), the ITIMs receive the intracellular signals, which are phosphorylated, and initiate the SHP mediated signaling for immunomodulation, including reduction in phagocytic potential.

In some embodiments the composition described herein comprises a recombinant nucleic acid construct encoding a chimeric Siglec receptor (SgR) fusion protein (SgFP), comprising: (a) a SgR subunit which comprises: (i) a transmembrane domain, and (ii) an intracellular domain comprising an intracellular signaling domain; an (a) an extracellular domain comprising an antigen binding domain specific to a sialylated glycan of a cell surface protein of a target cell; (b) wherein the transmembrane domain and the extracellular domain are operatively linked; and wherein: (i) the SgFP does not comprise a functional intracellular domain of an endogenous receptor that binds a sialylated glycan, or (ii) the SgFP comprises an intracellular signaling domain that activates phagocytosis or an inflammatory pathway. In some embodiments, the chimeric receptor is deficient in an intracellular domain, and therefore acts as a blocker for Siglec induced immunoregulatory intracellular signaling. Such is achieved by deletion of the nucleic acid region encoding the intracellular domain and cloning the remainder of the coding sequence of the Siglec receptor. This construct can be designated as a siglec intracellular domain deletion construct [Siglec☐ ICD]. In some embodiments, the recombinant nucleic acid construct encodes a recombinant chimeric antigenic receptor comprising a cancer antigen specific scFv fused with the extracellular domain (ECD) of a siglec receptor. This allows targetability of the construct to the cancer cell. The chimeric receptor comprises the TM and the ICD of the siglec receptor, which can be the endogenous ICD, or the ICD fused with additional phagocytosis promoting domains, such as PI3K binding domain or the domains. In some embodiment, a chimeric receptor comprising an extracellular siglec domain, is co-expressed with a sialidase. The nucleic acid encoding a sialidase may be incorporated in the expression vector expressing the chimeric domain with a signal sequence for secretion. Since the sialidase is expressed by the same cell that expressed the CAR-siglec receptor, expression of sialidase deprives the ECD of the siglec from binding to its natural ligand, but is activated by the scFv binding to its receptor, thereby ensuring the specificity of action of the chimeric receptor on a cancer-antigen expressing cell.

In some embodiments, the chimeric receptors comprise one or more domains from TREM proteins, fused at the extracellular region with an antigen binding domain that can specifically bind to a cancer antigen, such as a cancer antigen-specific antibody or part or fragment thereof. In some embodiments, recombinant nucleic acids encoding a TREM chimeric antigen receptor encode a fusion proteins that comprises: (a) the at least a TREM transmembrane domain (TM) and a TREM intracellular domain (ICD); and (b) an extracellular domain (ECD) comprising an antigen binding domain that can specifically bind to a cancer antigen. The fusion proteins are designed to target cancer cells and bind to the target cancer cells via the ECD comprising the antigen binding domain, and the binding triggers and enhance phagocytosis via signaling through the TREM TM and/or the intracellular domains. The transmembrane domain of TREM trimerizes with DAP12 transmembrane domains and trigger intracellular pro-phagocytosis signaling cascade. In some embodiments, the TREM domains are contributed by TREM1, or by TREM2, or by TREM3 members. The extracellular antigen binding domain is fused to the extracellular terminus of the TREM domains through a short spacer or linker.

In some embodiments, the extracellular antigen binding domain comprises an antibody, specific to a cancer antigen. In some embodiments, the extracellular antigen binding domain comprises an antibody or an antigen binding part thereof that binds specifically to an antigen on the surface of a cancer cell.

In some embodiments the extracellular antigen binding domain is an antibody specific for a cancer antigen. In some embodiments, the extracellular antigen binding domain is a fraction of an antibody, wherein the fragment can bind specifically to the cancer antigen on a cancer cell. In some embodiments the antigen binding domain comprises a single chain variable fraction (scFv) specific for a cancer antigen binding domain.

In some embodiments, the chimeric fusion protein (CFP) comprises an extracellular domain (ECD) targeted to bind to CD5 (CD5 binding domain), for example, comprising a heavy chain variable region (VH) having an amino acid sequence as set forth in SEQ ID NO: 1. In some embodiments, the chimeric CFP comprises a CD5 binding heavy chain variable domain comprising an amino acid sequence that has at least 80%, 85%, 90%, 95%, 96%, 97%, 98%, 99% sequence identity to SEQ ID NO: 1. In some embodiments, the extracellular domain (ECD) targeted to bind to CD5 (CD5 binding domain) comprises a light chain variable domain ($V_L$) having an amino acid sequence as set forth in SEQ ID NO: 2. In some embodiments, the chimeric CFP comprises a CD5 binding light chain variable domain comprising an amino acid sequence that has at least 80%, 85%, 90%, 95%, 96%, 97%, 98%, 99% sequence identity to SEQ ID NO: 2.

In some embodiments, the CFP comprises an extracellular domain targeted to bind to HER2 (HER2 binding domain) having for example a heavy chain variable domain amino acid sequence as set forth in SEQ ID NO: 8 and a light chain variable domain amino acid sequence as set forth in SEQ ID NO: 9. In some embodiments, the CFP comprises a HER2 binding heavy chain variable domain comprising an amino acid sequence that has at least 80%, 85%, 90%, 95%, 96%, 97%, 98%, 99% sequence identity to SEQ ID NO: 8. In some embodiments, the CFP comprises a HER2 binding light chain variable domain comprising an amino acid sequence that has at least 80%, 85%, 90%, 95%, 96%, 97%, 98%, 99% sequence identity to SEQ ID NO: 9.

In some embodiments, the CFP comprises a hinge connecting the ECD to the transmembrane (TM). In some embodiments the hinge comprises the amino acid sequence of the hinge region of a CD8 receptor. In some embodiments, the CFP may comprise a hinge having the amino acid sequence set forth in SEQ ID NO: 7 (CD8α chain hinge domain). In some embodiments, the PFP hinge region comprises an amino acid sequence that is at least 80%, 85%, 90%, 95%, 96%, 97%, 98%, 99% sequence identity to SEQ ID NO: 7.

In some embodiments, the CFP comprises a CD8 transmembrane region, for example having an amino acid sequence set forth in SEQ ID NO: 6. In some embodiments, the CFP TM region comprises an amino acid sequence that is at least 80%, 85%, 90%, 95%, 96%, 97%, 98%, 99% sequence identity to SEQ ID NO: 6.

In some embodiments, the CFP comprises an intracellular domain having an FcR domain. In some embodiments, the CFP comprises an FcR domain intracellular domain comprises an amino acid sequence set forth in SEQ ID NO: 3, or at least a sequence having 80%, 85%, 90%, 95%, 96%, 97%, 98%, 99% sequence identity to SEQ ID NO: 3.

In some embodiments, the CFP comprises an intracellular domain having a PI3K recruitment domain. In some embodiments the PI3K recruitment domain comprises an amino sequence set forth in SEQ ID NO: 4. In some embodiments the PI3K recruitment domain comprises an amino acid sequence that has at least 80%, 85%, 90%, 95%, 96%, 97%, 98%, 99% sequence identity to SEQ ID NO: 4.

In some embodiments, the CFP comprises an intracellular domain having a CD40 intracellular domain. In some embodiments the CD40 ICD comprises an amino sequence set forth in SEQ ID NO: 5. In some embodiments the CD40 ICD comprises an amino acid sequence that has at least 80%, 85%, 90%, 95%, 96%, 97%, 98%, 99% sequence identity to SEQ ID NO: 5.

**Table 4** - **Sequences of chimeric PFPs and domains thereof**

| SEQ ID NO | PFP/Domain | Sequence |
|---|---|---|
| 1 | Anti-CD5 heavy chain variable domain | EIQLVQSGGGLVKPGGSVRISCAASGYTFTNYGMNWVR QAPGKGLEWMGWINTHTGEPTYADSFKGRFTFSLDDSK NTAYLQINSLRAEDTAVYFCTRRGYDWYFDVWGQGTT VTV |
| 2 | Anti-CD5 light chain variable domain | DIQMTQSPSSLSASVGDRVTITCRASQDINSYLSWFQQKP GKAPKTLIYRANRLESGVPSRFSGSGSGTDYTLTISSLQY EDFGIYYCQQYDESPWTFGGGTKLEIK |

(continued)

| SEQ ID NO | PFP/Domain | Sequence |
|---|---|---|
| 33 | Anti-CD5 scFv | EIQLVQSGGGLVKPGGSVRISCAASGYTFTNYGMNWVR QAPGKGLEWMGWINTHTGEPTYADSFKGRFTFSLDDSK NTAYLQINSLRAEDTAVYFCTRRGYDWYFDVWGQGTT VTV*SSGGGGSGGGGSGGGGS*DIQMTQSPSSLSASVGDRV TITCRASQDINSYLSWFQQKPGKAPKTLIYRANRLESGVP SRFSGSGSGTDYTLTISSLQYEDFGIYYCQQYDESPWTFG GGTKLEIK |
| 3 | FcRγ-chain intracellular signaling domain | LYCRRLKIQVRKAAITSYEKSDGVYTGLSTRNQETYETL KHEKPPQ |
| 20 | FcRγ-chain intracellular signaling domain | LYCRLKIQVRKAAITSYEKSDGVYTGLSTRNQETYETLK HEKPPQ |
| 27 | FcRγ-chain intracellular signaling domain | RLKIQVRKAAITSYEKSDGVYTGLSTRNQETYETLKHEK PPQ |
| 28 | FcRγ-chain intracellular signaling domain | RLKIQVRKAAITSYEKSDGVYTGLSTRNQETYETLKHEK PPQ |
| 4 | PI3K recruitment domain | YEDMRGILYAAPQLRSIRGQPGPNHEEDADSYENM |
| 5 | CD40 intracellular domain | KKVAKKPTNKAPHPKQEPQEINFPDDLPGSNTAAPVQET LHGCQPVTQEDGKESRISVQERQ |
| 6 | CD8α chain transmembrane domain | IYIWAPLAGTCGVLLLSLVIT |
| 29 | CD8α chain transmembrane domain | IYIWAPLAGTCGVLLLSLVITLYC |
| 7 | CD8α chain hinge domain | ALSNSIMYFSHFVPVFLPAKPTTTPAPRPPTPAPTIASQPLS LRPEACRPAAGGAVHTRGLD |
| 8 | Anti-HER2 heavy chain variable domain | DIQMTQSPSSLSASVGDRVTITCRASQDVNTAVAWYQQ KPGKAPKLLIYSASFLYSGVPSRFSGSRSGTDFTLTISSLQ PEDFATYYCQQHYTTPPTFGQGTKVEIKRTGSTSGSGKP GSGEGSEVQLVE |

(continued)

| SEQ ID NO | PFP/Domain | Sequence |
|---|---|---|
| 9 | Anti-HER2 light chain variable domain | LVQPGGSLRLSCAASGFNIKDTYIHWVRQAPGKGLEWV ARIYPTNGYTRYADSVKGRFTISADTSKNTAYLQMNSLR AEDTAVYYCSRWGGDGFYAMDVWGQGTLVTV |
| 32 | Anti-HER2 scFv | DIQMTQSPSSLSASVGDRVTITCRASQDVNTAVAWYQQ KPGKAPKLLIYSASFLYSGVPSRFSGSRSGTDFTLTISSLQ PEDFATYYCQQHYTTPPTFGQGTKVEIKRTGSTSGSGKP GSGEGSEVQLVE*SSGGGGSGGGGSGGGGS*LVQPGGSLRL SCAASGFNIKDTYIHWVRQAPGKGLEWVARIYPTNGYTR YADSVKGRFTISADTSKNTAYLQMNSLRAEDTAVYYCS RWGGDGFYAMDVWGQGTLVTV |
| 17 | GMCSF Signal peptide | MWLQSLLLLGTVACSIS |
| 18 | CD28 transmembrane domain | FWVLVVVGGVLACYSLLVTVAFIIFWV |
| 34 | CD2 Transmembrane domain | IYLIIGICGGGSLLMVFVALLVFYIT |
| 19 | CD68 transmembrane domain | ILLPLIIGLILLGLLALVLIAFCII |
| 21 | TNFR1 intracellular domain | QRWKSKLYSIVCGKSTPEKEGELEGTTTKPLAPNPSFSPT PGFTPTLGFSPVPSSTFTSSSTYTPGDCPNFAAPRREVAPP YQGADPILATALASDPIPNPLQKWEDSAHKPQSLDTDDP ATLYAVVENVPPLRWKEFVRRLGLSDHEIDRLELQNGRC LREAQYSMLATWRRRTPRREATLELLGRVLRDMDLLGC LEDIEEALCGPAALPPAPSLLR |
| 22 | TNFR2 intracellular domain | PLCLQREAKVPHLPADKARGTQGPEQQHLLITAPSSSSSS LESSASALDRRAPTRNQPQAPGVEASGAGEARASTGSSD SSPGGHGTQVNVTCIVNVCSSSDHSSQCSSQASSTMGDT DSSPSESPKDEQVPFSKEECAFRSQLETPETLLGSTEEKPL PLGVPDAGMKPS |

(continued)

| SEQ ID NO | PFP/Domain | Sequence |
|---|---|---|
| 23 | MDA5 intracellular domain | MSNGYSTDENFRYLISCFRARVKMYIQVEPVLDYLTFLP AEVKEQIQRTVATSGNMQAVELLLSTLEKGVWHLGWTR EFVEALRRTGSPLAARYMNPELTDLPSPSFENAHDEYLQ LLNLLQPTLVDKLLVRDVLDKCMEEELLTIEDRNRIAAA ENNGNESGVRELLKRIVQKENWFSAFLNVLRQTGNNEL VQELTGSDCSESNAEIEN |
| 30 | CD8α chain hinge domain + transmembrane domain | ALSNSIMYFSHFVPVFLPAKPTTTPAPRPPTPAPTIASQPLS LRPEACRPAAGGAVHTRGLDIYIWAPLAGTCGVLLLSLV ITLYC |
| 31 | CD8α chain hinge domain + transmembrane domain | ALSNSIMYFSHFVPVFLPAKPTTTPAPRPPTPAPTIASQPLS LRPEACRPAAGGAVHTRGLDIYIWAPLAGTCGVLLLSLV IT |
| 14 | CD5-FcRγ-PI3K | MWLQSLLLLGTVACSISEIQLVQSGGGLVKPGGSVRISCA ASGYTFTNYGMNWVRQAPGKGLEWMGWINTHTGEPTY ADSFKGRFTFSLDDSKNTAYLQINSLRAEDTAVYFCTRR GYDWYFDVWGQGTTVTV*SSGGGGSGGGGSGGGGS*DIQ MTQSPSSLSASVGDRVTITCRASQDINSYLSWFQQKPGK APKTLIYRANRLESGVPSRF*SGSGSG*TDYTLTISSLQYEDF GIYYCQQYDESPWTFGGGTKLEIK*SGGGGSG*ALSNSIMY FSHFVPVFLPAKPTTTPAPRPPTPAPTIASQPLSLRPEACRP AAGGAVHTRGLDIYIWAPLAGTCGVLLLSLVITLYCRRL KIQVRKAAITSYEKSDGVYTGLSTRNQETYETLKHEKPP Q*GSGS*YEDMRGILYAAPQLRSIRGQPGPNHEEDADSYEN M |

(continued)

| SEQ ID NO | PFP/Domain | Sequence |
|---|---|---|
| 15 | HER2-FcRγ-PI3K | MWLQSLLLLGTVACSISDIQMTQSPSSLSASVGDRVTITC RASQDVNTAVAWYQQKPGKAPKLLIYSASFLYSGVPSRF SGSRSGTDFTLTISSLQPEDFATYYCQQHYTTPPTFGQGT KVEIKRTGSTSGSGKPGSGEGSEVQLVE*GGGG*LVQPGGS LRLSCAASGFNIKDTYIHWVRQAPGKGLEWVARIYPTNG YTRYADSVKGRFTISADTSKNTAYLQMNSLRAEDTAVY YCSRWGGDGFYAMDVWGQGTLVTV*SSSGGGGSG*ALSN SIMYFSHFVPVFLPAKPTTTPAPRPPTPAPTIASQPLSLRPE ACRPAAGGAVHTRGLDIYIWAPLAGTCGVLLLSLVITLY CRRLKIQVRKAAITSYEKSDGVYTGLSTRNQETYETLKH EKPPQ*GSGS*YEDMRGILYAAPQLRSIRGQPGPNHEEDADS YENM |
| 16 | CD5-FcRγ-CD40 | MWLQSLLLLGTVACSISEIQLVQSGGGLVKPGGSVRISCA ASGYTFTNYGMNWVRQAPGKGLEWMGWINTHTGEPTY ADSFKGRFTFSLDDSKNTAYLQINSLRAEDTAVYFCTRR GYDWYFDVWGQGTTVTV*SSGGGGSGGGGSGGGGS*DIQ MTQSPSSLSASVGDRVTITCRASQDINSYLSWFQQKPGK APKTLIYRANRLESGVPSRFSGSGSGTDYTLTISSLQYEDF GIYYCQQYDESPWTFGGGTKLEIK*SGGGGSG*ALSNSIMY FSHFVPVFLPAKPTTTPAPRPPTPAPTIASQPLSLRPEACRP AAGGAVHTRGLDIYIWAPLAGTCGVLLLSLVITLYCRLKI QVRKAAITSYEKSDGVYTGLSTRNQETYETLKHEKPPQK KVAKKPTNKAPHPKQEPQEINFPDDLPGSNTAAPVQETL HGCQPVTQEDGKESRISVQERQ |

(continued)

| SEQ ID NO | PFP/Domain | Sequence |
|---|---|---|
| 24 | CD5-FcRγ-MDA5 | MWLQSLLLLGTVACSISEIQLVQSGGGLVKPGGSVRISCA ASGYTFTNYGMNWVRQAPGKGLEWMGWINTHTGEPTY ADSFKGRFTFSLDDSKNTAYLQINSLRAEDTAVYFCTRR GYDWYFDVWGQGTTVTVSSGGGGSGGGGSGGGGSDIQ MTQSPSSLSASVGDRVTITCRASQDINSYLSWFQQKPGK APKTLIYRANRLESGVPSRFSGSGSGTDYTLTISSLQYEDF GIYYCQQYDESPWTFGGGTKLEIKSGGGGSGALSNSIMY FSHFVPVFLPAKPTTTPAPRPPTPAPTIASQPLSLRPEACRP AAGGAVHTRGLDIYIWAPLAGTCGVLLLSLVITLYCRLKI QVRKAAITSYEKSDGVYTGLSTRNQETYETLKHEKPPQG SGSMSNGYSTDENFRYLISCFRARVKMYIQVEPVLDYLT FLPAEVKEQIQRTVATSGNMQAVELLLSTLEKGVWHLG WTREFVEALRRTGSPLAARYMNPELTDLPSPSFENAHDE YLQLLNLLQPTLVDKLLVRDVLDKCMEEELLTIEDRNRI AAAENNGNESGVRELLKRIVQKENWFSAFLNVLRQTGN NELVQELTGSDCSESNAEIEN |

(continued)

| SEQ ID NO | PFP/Domain | Sequence |
|---|---|---|
| 25 | CD5-FcRγ-TNFR1 | MWLQSLLLLGTVACSISEIQLVQSGGGLVKPGGSVRISCAASGYTFTNYGMNWVRQAPGKGLEWMGWINTHTGEPTYADSFKGRFTFSLDDSKNTAYLQINSLRAEDTAVYFCTRRGYDWYFDVWGQGTTVTVSSGGGGSGGGGSGGGGSDIQMTQSPSSLSASVGDRVTITCRASQDINSYLSWFQQKPGKAPKTLIYRANRLESGVPSRFSGSGSGTDYTLTISSLQYEDFGIYYCQQYDESPWTFGGGTKLEIKSGGGGSGALSNSIMYFSHFVPVFLPAKPTTTPAPRPPTPAPTIASQPLSLRPEACRPAAGGAVHTRGLDIYIWAPLAGTCGVLLLSLVITLYCRLKIQVRKAAITSYEKSDGVYTGLSTRNQETYETLKHEKPPQGSGSQRWKSKLYSIVCGKSTPEKEGELEGTTTKPLAPNPSFSPTPGFTPTLGFSPVPSSTFTSSSTYTPGDCPNFAAPRREVAPPYQGADPILATALASDPIPNPLQKWEDSAHKPQSLDTDDPATLYAVVENVPPLRWKEFVRRLGLSDHEIDRLELQNGRCLREAQYSMLATWRRRTPRREATLELLGRVLRDMDLLGCLEDIEEALCGPAALPPAPSLLR |

(continued)

| SEQ ID NO | PFP/Domain | Sequence |
|---|---|---|
| 26 | CD5-FcRγ-TNFR2 | MWLQSLLLLGTVACSISEIQLVQSGGGLVKPGGSVRISCAASGYTFTNYGMNWVRQAPGKGLEWMGWINTHTGEPTYADSFKGRFTFSLDDSKNTAYLQINSLRAEDTAVYFCTRRGYDWYFDVWGQGTTVTVSSGGGGSGGGGSGGGGSDIQMTQSPSSLSASVGDRVTITCRASQDINSYLSWFQQKPGKAPKTLIYRANRLESGVPSRFSGSGSGTDYTLTISSLQYEDFGIYYCQQYDESPWTFGGGTKLEIKSGGGGSGALSNSIMYFSHFVPVFLPAKPTTTPAPRPPTPAPTIASQPLSLRPEACRPAAGGAVHTRGLDIYIWAPLAGTCGVLLLSLVITLYCRLKIQVRKAAITSYEKSDGVYTGLSTRNQETYETLKHEKPPQGSGSPLCLQREAKVPHLPADKARGTQGPEQQHLLITAPSSSSSSLESSASALDRRAPTRNQPQAPGVEASGAGEARASTGSSDSSPGGHGTQVNVTCIVNVCSSSDHSSQCSSQASSTMGDTDSSPSESPKDEQVPFSKEECAFRSQLETPETLLGSTEEKPLPLGVPDAGMKPS |

**Table 5 - Linker sequences**

| SEQ ID | Sequence |
|---|---|
| 10 | SSGGGGSGGGGSGGGGS |
| 11 | SGGGGSG |
| 12 | SGGG |
| 13 | GSGS |

### *Characteristics of the PFP:*

The PFP structurally incorporates into the cell membrane of the cell in which it is expressed. Specific leader sequences in the nucleic acid construct, such as the signal peptide directs plasma membrane expression of the encoded protein. The transmembrane domain encoded by the construct incorporates the expressed protein in the plasma membrane of the cell.

In some embodiments the transmembrane domain comprises a TM domain of an FcR-alpha receptor, which dimerizes with endogenous FcRγ receptors in the myeloid cells, such as macrophages, ensuring myeloid cell specific expression.

In some embodiments, the PFP renders the cell expressing it as potently phagocytic. When the recombinant nucleic acid encoding the PFP is expressed in a cell, the cell exhibits an increased phagocytosis of a target cell having the antigen of a target cell, compared to a cell not expressing the recombinant nucleic acid. When the recombinant nucleic acid is expressed in a cell, the cell exhibits an increased phagocytosis of a target cell having the antigen of a target cell, compared to a cell not expressing the recombinant nucleic acid. In some embodiments, the recombinant nucleic acid when expressed in a cell, the cell exhibits at least 2-fold increased phagocytosis of a target cell having the antigen of a target cell, compared to a cell not expressing the recombinant nucleic acid. In some embodiments, the recombinant nucleic acid when expressed in a cell, the cell exhibits at least 3-fold, 4-fold, 5-fold, 6-fold, 7-fold, 8-fold, 9-fold, 10-fold, 20-fold 30-fold or at least 5-fold increased phagocytosis of a target cell having the antigen of a target cell, compared to a cell not expressing the recombinant nucleic acid. In some embodiments, the composition comprises a recombinant nucleic acid encoding a phagocytic or tethering receptor (PR) fusion protein (PFP) comprising: (a) a PR subunit comprising: (i) a transmembrane domain, and (ii) an intracellular domain comprising an intracellular signaling domain; and (b) an extracellular domain comprising an antigen binding domain specific to an antigen of a target cell; wherein the transmembrane domain and the extracellular domain are operatively linked; and wherein upon binding of the PFP to the antigen of the target cell, the killing or phagocytosis activity of a cell expressing the PFP is increased by at least greater than 10% compared to a cell not expressing the PFP. In some embodiments, the phagocytosis activity of a cell expressing the PFP is increased by at least greater than 10% compared to a cell not expressing the PFP. In some embodiments, the phagocytosis activity of a cell expressing the PFP is increased by at least greater than 11% compared to a cell not expressing the PFP. In some embodiments, the phagocytosis activity of a cell expressing the PFP is increased by at least greater than 12% compared to a cell not expressing the PFP. In some embodiments, the phagocytosis activity of a cell expressing the PFP is increased by at least greater than 13% compared to a cell not expressing the PFP. In some embodiments, the phagocytosis activity of a cell expressing the PFP is increased by at least greater than 14% compared to a cell not expressing the PFP. In some embodiments, the phagocytosis activity of a cell expressing the PFP is increased by at least greater than 15% compared to a cell not expressing the PFP. In some embodiments, the phagocytosis activity of a cell expressing the PFP is increased by at least greater than 16% compared to a cell not expressing the PFP. In some embodiments, the phagocytosis activity of a cell expressing the PFP is increased by at least greater than 17% compared to a cell not expressing the PFP. In some embodiments, the phagocytosis activity of a cell expressing the PFP is increased by at least greater than 18% compared to a cell not expressing the PFP. In some embodiments, the phagocytosis activity of a cell expressing the PFP is increased by at least greater than 19% compared to a cell not expressing the PFP. In some embodiments, the phagocytosis activity of a cell expressing the PFP is increased by at least greater than 20% compared to a cell not expressing the PFP. In some embodiments, the phagocytosis activity of a cell expressing the PFP is increased by at least greater than 30% compared to a cell not expressing the PFP. In some embodiments, the phagocytosis activity of a cell expressing the PFP is increased by at least greater than 40% compared to a cell not expressing the PFP. In some embodiments, the phagocytosis activity of a cell expressing the PFP is increased by at least greater than 50% compared to a cell not expressing the PFP. In some embodiments, the phagocytosis activity of a cell expressing the PFP is increased by at least greater than 60% compared to a cell not expressing the PFP. In some embodiments, the phagocytosis activity of a cell expressing the PFP is increased by at least greater than 70% compared to a cell not expressing the PFP. In some embodiments, the phagocytosis activity of a cell expressing the PFP is increased by at least greater than 80% compared to a cell not expressing the PFP. In some embodiments, the phagocytosis activity of a cell expressing the PFP is increased by at least greater than 90% compared to a cell not expressing the PFP. In some embodiments, the phagocytosis activity of a cell expressing the PFP is increased by at least greater than 100% compared to a cell not expressing the PFP.

In some embodiments, the phagocytosis activity of a cell expressing the PFP is increased by at least greater than 2-fold compared to a cell not expressing the PFP. In some embodiments, the phagocytosis activity of a cell expressing the PFP is increased by at least greater than 4-fold compared to a cell not expressing the PFP. In some embodiments, the phagocytosis activity of a cell expressing the PFP is increased by at least greater than 6-fold compared to a cell not expressing the PFP. In some embodiments, the phagocytosis activity of a cell expressing the PFP is increased by at least greater than 8-fold compared to a cell not expressing the PFP. In some embodiments, the phagocytosis activity of a cell expressing the PFP is increased by at least greater than 10-fold compared to a cell not expressing the PFP. In some embodiments, the phagocytosis activity of a cell expressing the PFP is increased by at least greater than 20-fold compared to a cell not expressing the PFP. In some embodiments, the phagocytosis activity of a cell expressing the PFP is increased by at least greater than 50-fold compared to a cell not expressing the PFP. In some embodiments, the phagocytosis activity of a cell expressing the PFP is increased by about 50-fold compared to a cell not expressing the PFP.

In some embodiments, the phagocytosis associated killing activity of a cell expressing the PFP is increased by at least greater than 10% compared to a cell not expressing the PFP. In some embodiments, the phagocytosis associated killing activity of a cell expressing the PFP is increased by at least greater than 20% compared to a cell not expressing the PFP. In some embodiments, the phagocytosis associated killing activity of a cell expressing the PFP is increased by at least greater than 30% compared to a cell not expressing the PFP. In some embodiments, the phagocytosis associated killing activity of a cell expressing the PFP is increased by at least greater than 40% compared to a cell not expressing the PFP. In some embodiments, the phagocytosis associated killing activity of a cell expressing the PFP is increased by at least greater than 50% compared to a cell not expressing the PFP. In some embodiments, the phagocytosis associated killing activity of a cell expressing the PFP is increased by at least greater than 60% compared to a cell not expressing the PFP. In some embodiments, the phagocytosis associated killing activity of a cell expressing the PFP is increased by at least greater than 70% compared to a cell not expressing the PFP. In some embodiments, the phagocytosis associated killing activity of a cell expressing the PFP is increased by at least greater than 80% compared to a cell not expressing the PFP. In some embodiments, the phagocytosis associated killing activity of a cell expressing the PFP is increased by at least greater than 90% compared to a cell not expressing the PFP. In some embodiments, the phagocytosis associated killing activity of a cell expressing the PFP is increased by at least greater than 100% compared to a cell not expressing the PFP.

In some embodiments, the phagocytosis associated killing activity of a cell expressing the PFP is increased by at least greater than 2-fold compared to a cell not expressing the PFP. In some embodiments, the phagocytosis associated killing activity of a cell expressing the PFP is increased by at least greater than 4-fold compared to a cell not expressing the PFP. In some embodiments, the phagocytosis associated killing activity of a cell expressing the PFP is increased by at least greater than 6-fold compared to a cell not expressing the PFP. In some embodiments, the phagocytosis associated killing activity of a cell expressing the PFP is increased by at least greater than 8-fold compared to a cell not expressing the PFP. In some embodiments, the phagocytosis associated killing activity of a cell expressing the PFP is increased by at least greater than 10-fold compared to a cell not expressing the PFP. In some embodiments, the phagocytosis associated killing activity of a cell expressing the PFP is increased by at least greater than 20-fold compared to a cell not expressing the PFP. In some embodiments, the phagocytosis associated killing activity of a cell expressing the PFP is increased by at least greater than 30-fold compared to a cell not expressing the PFP. In some embodiments, the phagocytosis associated killing activity of a cell expressing the PFP is increased by at least greater than 40-fold compared to a cell not expressing the PFP. In some embodiments, the phagocytosis associated killing activity of a cell expressing the PFP is increased by at least greater than 50-fold compared to a cell not expressing the PFP. In some embodiments, the phagocytosis associated killing activity of a cell expressing the PFP is increased by at least greater than 100-fold compared to a cell not expressing the PFP. In some embodiments, the phagocytosis associated killing activity of a cell expressing the PFP is increased by about 100-fold compared to a cell not expressing the PFP.

In some embodiments, the phagocytosis associated killing activity of a cell expressing the PFP is increased by at least greater than 2-fold compared to a cell not expressing the PFP.

In some embodiments, when the recombinant nucleic acid is expressed in a cell, the cell exhibits an increased cytokine production. The cytokine can comprise any one of: IL-1, IL-6, IL-12, IL-23, TNF, CXCL9, CXCL10, CXCL11, IL-18, IL-23, IL-27 and interferons.

In some embodiments, when the recombinant nucleic acid is expressed in a cell, the cell exhibits an increased cell migration. Enhanced cell migration may be detected in cell culture by standard motility assays. In some embodiments, actin filament rearrangements may be detected and monitored using phalloidin staining and fluorescent microscopy. In some instances, time-lapsed microscopy is used for the purpose.

In some embodiments, when the recombinant nucleic acid is expressed in a cell, the cell exhibits an increased immune activity. In some embodiments, when the recombinant nucleic acid is expressed in a cell, the cell exhibits an increased expression of MHC II. In some embodiments, when the recombinant nucleic acid is expressed in a cell, the cell exhibits an increased expression of CD80. In some embodiments, when the recombinant nucleic acid is expressed in a cell, the cell exhibits an increased expression of CD86. In some embodiments, when the recombinant nucleic acid is expressed in a cell, the cell exhibits an increased iNOS production.

In some embodiments, when the recombinant nucleic acid is expressed in a cell, the cell exhibits increased trogocytosis of a target cell expressing the antigen of a target cell compared to a cell not expressing the recombinant nucleic acid. In some embodiments, when the recombinant nucleic acid is expressed in a cell, the cell exhibits less trogocytosis of a target cell expressing the antigen of a target cell as compared to phagocytosis of the cell expressing the recombinant nucleic acid.

In embodiments, the chimeric receptors may be glycosylated, pegylated, and/or otherwise post-translationally modified. In further embodiments, glycosylation, pegylation, and/or other posttranslational modifications may occur in vivo or in vitro and/or may be performed using chemical techniques. In additional embodiments, any glycosylation, pegylation and/or other posttranslational modifications may be N-linked or O-linked. In embodiments any one of the chimeric receptors may be enzymatically or functionally active such that, when the extracellular domain is bound by a ligand, a signal is transduced to polarize myeloid cells, such as macrophages.

## Methods for preparing CFPs and engineered myeloid cells

The method for preparing CAR-Ps comprise the steps of (1) screening for PSR subunit framework; (2) screening for antigen binding specificity; (3) CAR-P recombinant nucleic acid constructs; (4) engineering cells and validation.

Screening for PSR subunit framework: As described above, the design of the receptor comprises at least of one phagocytic receptor domain, which enables the enhanced signaling of phagocytosis. In essence a large body of plasma membrane proteins can be screened for novel phagocytic functions or enhancements domains. Methods for screening phagocytic receptor subunits are known to one of skill in the art. Additional information can be found in The Examples section. In general, functional genomics and reverse engineering is often employed to obtain a genetic sequence encoding a functionally relevant protein polypeptide or a portion thereof. In some embodiments, primers and probes are constructed for identification, and or isolation of a protein, a polypeptide or a fragment thereof or a nucleic acid fragment encoding the same. In some embodiments, the primer or probe may be tagged for experimental identification. In some embodiments, tagging of a protein or a peptide may be useful in intracellular or extracellular localization.

Potential antibodies are screened for selecting specific antigen binding domains of high affinity. Methods of screening for antibodies or antibody domains are known to one of skill in the art. Specific examples provide further information. Examples of antibodies and fragments thereof include, but are not limited to IgAs, IgDs, IgEs, IgGs, IgMs, Fab fragments, F(ab')2 fragments, monovalent antibodies, scFv fragments, scRv-Fc fragments, IgNARs, hcIgGs, $V_{HH}$ antibodies, nanobodies, and alphabodies.

Commercially available antibodies can be adapted to generate extracellular domains of a chimeric receptor. Examples of commercially available antibodies include, but are not limited to: anti-HGPRT, clone 13H11.1 (EMD Millipore), anti-ROR1 (ab135669) (Abcam), anti-MUC1 [EP1024Y] (ab45167) (Abcam), anti-MUC16 [X75] (ab1107) (Abcam), anti-EGFRvIII [L8A4] (Absolute antibody), anti-Mesothelin [EPR2685 (2)] (ab134109) (Abcam), HER2 [3B5] (ab16901) (Abcam), anti-CEA (LS-C84299-1000) (LifeSpan BioSciences), anti-BCMA (ab5972) (Abcam), anti-Glypican 3 [9C2] (ab129381) (Abcam), anti-FAP (ab53066) (Abcam), anti-EphA2 [RM-0051-8F21] (ab73254) (Abcam), anti-GD2 (LS-0546315) (LifeSpan BioSciences), anti-CD19 [2E2B6B10] (ab31947) (Abcam), anti-CD20 [EP459Y] (ab78237) (Abcam), anti-CD30 [EPR4102] (ab134080) (Abcam), anti-CD33 [SP266](ab199432) (Abcam), anti-CD123 (ab53698) (Abcam), anti-CD133 (BioLegend), anti-CD123 (1A3H4) ab181789 (Abcam), and anti-CD171 (L1.1) (Invitrogen antibodies). Techniques for creating antibody fragments, such as scFvs, from known antibodies are routine in the art.

The recombinant nucleic acid can be generated following molecular biology techniques known to one of skill in the art. The methods include but are not limited to designing primers, generating PCR amplification products, restriction digestion, ligation, cloning, gel purification of cloned product, bacterial propagation of cloned DNA, isolation and purification of cloned plasmid or vector. General guidance can be found in: Molecular Cloning of PCR Products: by Michael Finney, Paul E. Nisson, Ayoub Rashtchian in Current Protocols in Molecular Biology, Volume 56, Issue 1 (First published: 01 November 2001); Recombinational Cloning by Jaehong Park, Joshua LaBaer in Current Protocols in Molecular Biology Volume 74, Issue 1 (First published: 15 May 2006) and others. In some embodiments specific amplification techniques may be used, such as TAS technique (Transcription-based Amplification System), described by Kwoh et al. in 1989; the 3SR technique, which are hereby incorporated by reference. (Self-Sustained Sequence Replication), described by Guatelli et al. in 1990; the NASBA technique (Nucleic Acid Sequence Based Amplification), described by Kievitis et al. in 1991; the SDA technique (Strand Displacement Amplification) (Walker et al., 1992); the TMA technique (Transcription Mediated Amplification).

In some embodiments the recombinant nucleic acid sequence is optimized for expression in human.

DNA, mRNA and Circular RNA: In some embodiments, naked DNA or messenger RNA (mRNA) may be used to introduce the nucleic acid inside the cell. In some embodiments, DNA or mRNA encoding the PFP is introduced into the phagocytic cell by lipid nanopaticle (LNP) encapsulation. mRNA is single stranded and may be codon optimized. In some embodiments the mRNA may comprise one or more modified or unnatural bases such as 5'-Methylcytosine, or Pseudouridine. mRNA may be 50-10,000 bases long. In one aspect the transgene is delivered as an mRNA. The mRNA may comprise greater than about 100, 200, 300, 400, 500, 600, 700, 800, 900, 1000, 1100, 1200, 1300, 1400, 1500, 1600, 1700, 1800, 1900, 2000, 3000, 4000, 5000, 6000, 7000, 8000, 9000, 10,000 bases. In some embodiments, the mRNA may be more than 10,000 bases long. In some embodiments, the mRNA may be about 11,000 bases long. In some embodiments, the mRNA may be about 12,000 bases long. In some embodiments, the mRNA comprises a transgene sequence that encodes a fusion protein. LNP encapsulated DNA or RNA can be used for transfecting myeloid cells, such as macrophages, or can be administered to a subject.

In some embodiments, circular RNA (circRNAs) encoding the PFP is used. In circular RNAs (circRNAs) the 3' and 5' ends are covalently linked, constitute a class of RNA. CircRNA may be delivered inside a cell or a subject using LNPs.

## MECHANISMS OF FURTHER ENHANCEMENT MYELOID CELLS FUNCTION EXPRESSING CHIMERIC ANTIGEN RECEPTOR PROTEIN

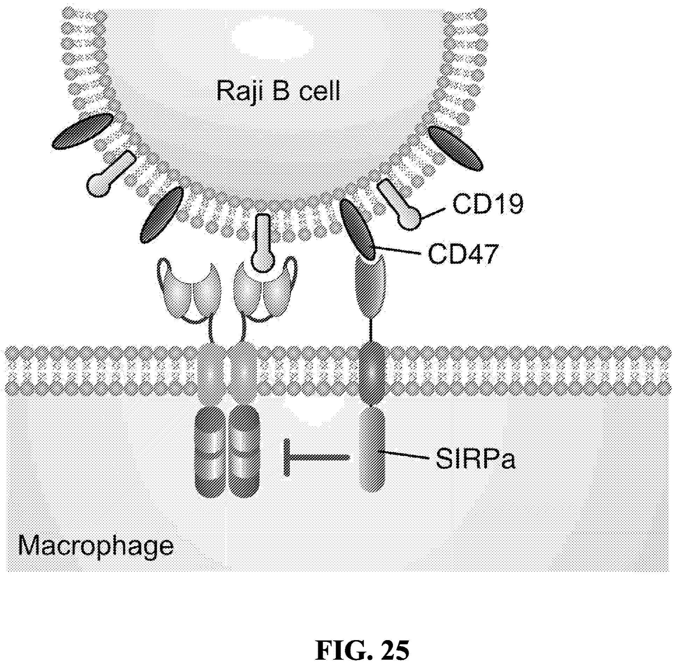

Myeloid cells, such as macrophages, especially in the tumor microenvironment encounter phagocytosis dampening or inhibitory signals, such as CD47 mediated anti-phagocytic activity by target cells, e.g., cancer cells, as graphically represented in FIG. 25. Cluster of Differentiation 47 (CD47) is a receptor belonging to the immunoglobulin superfamily. It can bind to integrins, and thrombospondin 1 (TSP-1), and is ubiquitously expressed in human cells. Target cells, including tumor cells express CD47 as the 'don't eat me' signals to evade phagocytosis mediated killing and removal. Phagocytic cells can express signal regulatory protein alpha receptors, (SIRP) which bind to CD47. SIRP family members are receptor-type transmembrane glycoproteins involved in the negative regulation of receptor tyrosine kinase-coupled signaling processes. SIRP-$\alpha$ can be phosphorylated by tyrosine kinases. The phospho-tyrosine residues of this PTP have been shown to recruit SH2 domain containing tyrosine phosphatases (PTP), and serve as substrates of PTPs. SIRP-□ is another member of the SIRP family, which is found to interact with TYROBP/DAP12, a protein bearing immunoreceptor tyrosine-based activation motifs. It can trigger activation of myeloid cells when associated with TYROBD. This protein was also reported to participate in the recruitment of tyrosine kinase SYK.

In one aspect, provided herein are chimeric receptors generated to functionally block CD47 signaling when expressed in a phagocytic cell.

In another aspect, provided herein are compositions and methods for phagocytic enhancement of the engineered myeloid cells, such as macrophages, by blocking CD47 signal. In some embodiments the recombinant nucleic acids encoding the CFP receptors described herein are transfected or transduced into myeloid cells, such as phagocytic cells, alone or in combination with other recombinant phagocytic receptors for creating engineered macrophages for use in immunotherapy. In some other embodiments, the recombinant phagocytic receptors comprise an intracellular domain of a scavenger receptor.

In some embodiments, provided herein is a composition comprising a recombinant nucleic acid encoding a CFP comprising: (a) a subunit comprising: (i) an extracellular domain that can specifically bind to CD47 on a target cell; and (ii) a transmembrane domain; wherein the extracellular domain of the subunit and the extracellular antigen binding domain are operably linked; and the subunit does not comprise a functional intracellular domain of an endogenous receptor that binds CD47, or does not comprise an intracellular domain that activates a phosphatase. In some embodiments, the extracellular antigen binding domain is derived from signal-regulatory protein alpha (SIRPα). In some embodiments, the extracellular antigen binding domain is derived from signal-regulatory protein beta (SIRPβ). SIRPβ does not bind to CD47. In some embodiments, the transmembrane domain is derived from SIRPα. In some embodiments, the transmembrane domain is derived from SIRPβ. The recombinant nucleic acid of this category lacks any intracellular domain, and is therefore a non-signaling receptor and blocker of CD47 signaling. This construct is referred to as SIRP-ΔICD. With the extracellular ligand binding domain of either the SIRPα or the SIRPβ ECD of this chimeric receptor, the receptor binds to CD47 on the target cell, but renders it's signaling inert by not having a functional SIRPα intracellular domain, thereby reducing CD47 signaling and inhibition of phagocytosis of the CD47+ cells by myeloid cells, such as macrophages, that express the CFP. Overexpression of this construct can largely reduce CD47 mediated anti-phagocytic activity of myeloid cells, such as macrophages, by cancer cells.

In some embodiments endogenous SIRPα may be further inhibited, in addition to overexpression of the SIRP-ΔICD. siRNA can be designed specifically targeting the portion of the mRNA encoding the ICD of SIRPα, such that the siRNA does not reduce or affect the expression of the SIRP-ΔICD.

In some embodiments the SIRP-☐ICD and or the inhibitor of endogenous SIRPα may be expressed in a cell expressing a fusion protein comprising a phagocytic receptor and an extracellular antigen binding domain specific for cancer antigen.

### *Chimeric antigen receptor for blocking anti-phagocytic signal and activating phagocytosis*

### *A. Alteration of CD47-binding Signal Transduction*

In one aspect, a recombinant nucleic acid is generated, comprising a recombinant nucleic acid encoding a chimeric antigen receptor (CAR) fusion protein (CFP) comprising: (a) a transmembrane domain; (b) an extracellular antigen binding domain specific to CD47 of a target cell; wherein: the transmembrane domain and extracellular antigen binding domain specific to CD47 are operatively linked; and the CFP does not comprise a functional intracellular domain of an endogenous receptor that binds CD47, or does not comprise an intracellular domain that activates a phosphatase, and, (b) an intracellular domain from a phagocytic receptor, that is capable of activating intracellular signaling to enhance phagocytosis. In some embodiments the recombinant nucleic acid construct comprises a nucleic acid sequence encoding an intracellular signaling domain of a scavenger receptor. This class of receptors are termed herein the "switch receptor", because they are designed to convert (or switch) a phagocytosis inhibitory signal to a phagocytosis promoting signal. In some embodiments, the intracellular domain of the chimeric receptor may comprise the intracellular domain of a scavenger receptor, selected from lectin, dectin 1, mannose receptor (CD206), scavenger receptor A1 (SRA1), MARCO, CD36, CD163, MSR1, SCARA3, COLEC12, SCARAS, SCARB1, SCARB2, CD68, OLR1, SCARF1, SCARF2, CXCL16, STAB1, STAB2, SRCRB4D, SSCSD, CD205, CD207, CD209, RAGE, CD14, CD64, F4/80, CCR2, CX3CR1, CSF1R, Tie2, HuCRIg(L), and CD169 receptor, which is fused at the extracellular terminus with the extracellular domain comprising the SIRPα CD47 binding domain.

In some embodiments, the intracellular domain with a phagocytosis signaling domain comprises a domain having one or more Immunoreceptor Tyrosine-based Activation Motif (ITAM) motifs. ITAMs are conserved sequences present in the cytoplasmic tails of several receptors of the immune system, such as T cell receptors, immunoglobulins (Ig) and FcRs. They have a conserved amino acid sequence motif consisting of paired YXXL/I motifs (Y= Tyrosine, L= Lysine and I= Isoleucine) separated by a defined interval (YXXL/I-X$_{6-8}$-YXXL/I). In addition, most ITAMs contain a negatively charged amino acid (D/E) in the +2 position relative to the first ITAM tyrosine. Phosphorylation of residues within the ITAM recruits several signaling molecules that activate phagocytosis. ITAM motifs are also present in the intracellular adapter protein, DNAX Activating Protein of 12 kDa (DAP12).

In some embodiments, the phagocytic signaling domain in the intracellular region can comprise a PI3kinase (PI3K) recruitment domain (also called PI3K binding domain). The PI3K binding domains used herein can be the respective PI3K binding domains of CD19, CD28, CSFR or PDGFR. PI3 kinase recruitment to the binding domain leads to the Akt meadiated signaling cascade and activation of phagocytosis. The PI3K-Akt signaling pathway is important in phagocytosis, regulation of the inflammatory response, and other activities, including vesicle trafficking and cytoskeletal reorganization. The PI3kinase recruitment domain is an intracellular domain in a plasma membrane protein, which has tyrosine residues that can be phosphorylated, and which can in turn be recognized by the Src homology domain (SH2) domain of PI3Kp85. The SH2 domain of p85 recognizes the phosphorylated tyrosines on the cytosolic domain of the receptor. This causes an allosteric activation of p110 and the production of phosphatidylinositol-3,4,5-trisphosphate ($PIP_3$) that is recognized by the enzymes Akt and the constitutively active 3'-phosphoinositide-dependent kinase 1 (PDK1) through their plekstrin homology domains. The interaction of Akt with $PIP_3$ causes a change in the Akt conformation and phosphorylation of the residues Thr308 and Ser473 by PDK1 and rictor-mTOR complex, respectively. Phosphorylation of these two residues causes the activation of Akt which in turn phosphorylates, among other substrates, the enzyme glycogen synthase kinase-3 (GSK-3). GSK-3 has two isoforms, GSK-3$\alpha$ and GSK-3$\beta$ both of which are constitutively active. The isoforms are structurally related but functionally nonredundant. Inactivation of GSK-3 is observed when the residues Ser21 in GSK-3$\alpha$ or Ser9 in GSK-3$\beta$, located in their regulatory N-terminal domains, are phosphorylated by Akt and other kinases. Inhibition of GSK-3 by phosphorylation is important for the modulation of the inflammation and in phagocytosis processes.

In some embodiments, a recombinant PFP comprises (a) an extracellular CD47 binding domain SIRP$\alpha$, (b) a SIRP$\beta$ transmembrane domain, and (c) an intracellular domain of SIRP$\beta$. SIRP$\beta$ signaling can activate pro-phagocytic signaling by engaging DAP12 activation.

### B. Alteration of Sialic Acid-binding Signal Transduction

In one aspect, disclosed herein are compositions and methods of switching a phagocytosis regulatory signal transduction by members of the Siglec family of membrane proteins that are expressed on immune cells. Various members of the family transduce checkpoint signal upon contact with sialylated glycans on membrane proteins. In some members, the intracellular domains of the Siglec proteins comprise multiple immunoreceptor tyrosine-based inhibitory motifs (ITIMs). ITIMs share a consensus amino acid sequence in their cytoplasmic tail, namely (I/V/L/S)-X-Y-X-X-(L/V), where X denotes any amino acid, I= Isoleusine, V=valine, L=Lysine, S=Serine, Y=Tyrosine. Phosphorylation of the Tyrosine residues at the ITIM motif recruit either of two SH2 domain-containing negative regulators: the inositol phosphatase SHIP (Src homology 2-containing inositol polyphosphate 5-phosphatase) or the tyrosine phosphatase SHP-1 (Src homology 2-containing protein tyrosine phosphatase-1). A leucine in the (Y+2) position favors binding to SHIP, whereas an isoleucine in the (Y-2) position favors SHP-1 binding. ITIMs can also bind to another tyrosine phosphatase, SHP-2, but evidence for SHP-2 playing a functional role in ITIM-mediated inhibition is less clear than for the other mediators. Therefore, activation of the Siglec membrane proteins at the extracellular ligand binding domain by binding with a sialic acid residue, (e.g. in sialylated membrane glycan proteins), the ITIMs receive the intracellular signals, which are phosphorylated, and initiate the SHP mediated signaling for immunomodulation, including reduction in phagocytic potential.

In some embodiments the composition described herein comprises a recombinant nucleic acid construct encoding a chimeric Siglec receptor (SgR) fusion protein (SgFP), comprising: (a) a SgR subunit which comprises: (i) a transmembrane domain, and (ii) an intracellular domain comprising an intracellular signaling domain; an (a) an extracellular domain comprising an antigen binding domain specific to a sialylated glycan of a cell surface protein of a target cell; (b) wherein the transmembrane domain and the extracellular domain are operatively linked; and wherein: (i) the SgFP does not comprise a functional intracellular domain of an endogenous receptor that binds a sialylated glycan, or (ii) the SgFP comprises an intracellular signaling domain that activates phagocytosis or an inflammatory pathway.

Siglec family receptors comprise the membrane proteins, siglec 1 (CD169), siglec 2 (CD22), siglec 3 (CD33), siglec 4 (MAG), siglec 5, siglec 6, siglec 7, siglec 8, siglec 9, siglec 10, siglec 11, siglec 12, siglec 13, siglec 14, siglec 15, siglec 16.

In some embodiments the recombinant nucleic acid construct encodes a recombinant chimeric antigenic receptor comprising an extracellular domain (ECD) of a Siglec receptor that can bind to sialylated residues on membrane proteins of a target cell, which comprises any one of the siglec family members. In some embodiments, the recombinant nucleic acid construct encodes a recombinant chimeric antigenic receptor comprising a transmembrane protein (TM) domain of a Siglec receptor. In some embodiments, the chimeric receptor is deficient in an intracellular domain, and therefore acts as a blocker for Siglec induced immunoregulatory intracellular signaling. Such is achieved by deletion of the nucleic acid region encoding the intracellular domain and cloning the remainder of the coding sequence of the Siglec receptor. This construct can be designated as a siglec intracellular domain deletion construct [SiglecΔICD].

In some embodiments the recombinant nucleic acid construct encodes a recombinant chimeric antigenic receptor comprising an extracellular domain (ECD) of a Siglec receptor that can bind to sialylated residues on membrane proteins of a target cell. In some embodiments, the recombinant nucleic acid construct encodes a recombinant chimeric antigenic receptor comprising a transmembrane protein (TM) domain of a Siglec receptor.

In some embodiments, the chimeric receptor comprises a TM domain of an unrelated membrane protein, for example CD8 TM or CD2 TM domains. In some embodiments the chimeric antigenic receptor comprising the Siglec ECD and/or Siglec TM is deficient in endogenous Siglec intracellular domain (ICD) (e.g., achieved by a deletion of the intracellular domain [Siglec□ICD]), and wherein an intracellular domain of an unrelated protein is fused to the cytoplasmic end of the construct.

Of note, Siglec 2, 3, 5, 6, 7, 8, 9, 10, 11, and 12 family members comprise 2 or more intracellular ITIM motifs. In some embodiments, the intracellular domains of the siglec proteins comprising the ITIM motifs are deleted to generate Siglec□ICD, and fused with an ICD of a phagocytosis promoting protein, thereby altering the inhibitory signal generated by binding of the siglec to its ligand (sialylated glycan) on a cancer cell, into a pro-inflammatory and phagocytosis promoting signal.

The unrelated protein can comprise an intracellular domain that can generate phagocytosis activation signals or pro-inflammatory signals, such as the intracellular domains of the proteins: MRC1, ItgB5, MERTK, ELMO, BAIL Tyro3, Axl, Traf6, Syk, MyD88, Zap70, PI3K, FeyR1, FcγR2A, FcγR2B2, FcγR2C, FcγR3A, FcER1, FcaRl, BAFF-R, DAP12, NFAM1, and CD79b intracellular domains.

In some embodiments, the intracellular domain of the chimeric receptor may comprise the intracellular domain of a scavenger receptor, selected from lectin, dectin 1, mannose receptor (CD206), scavenger receptor A1 (SRA1), MARCO, CD36, CD163, MSR1, SCARA3, COLEC12, SCARA5, SCARB1, SCARB2, CD68, OLR1, SCARF1, SCARF2, CXCL16, STAB1, STAB2, SRCRB4D, SSCSD, CD205, CD207, CD209, RAGE, CD14, CD64, F4/80, CCR2, CX3CR1, CSF1R, Tie2, HuCRIg(L), and CD169 receptor, which is fused at the extracellular terminus with the extracellular domain comprising the Siglec sialylated glycan binding domain.

In some embodiments, the phagocytic signaling domain in the intracellular region can comprise a PI3kinase (PI3K) recruitment domain (also called PI3K binding domain). The PI3K binding domains used herein can be the respective PI3K binding domains of CD19, CD28, CSFR or PDGFR.

In some embodiments, the intracellular domain with a phagocytosis signaling domain comprises a domain having one or more Immunoreceptor Tyrosine-based Activation Motif (ITAM) motifs.

### *C. Chimeric Siglec Constructs and Sialidase Co-expression*

In some embodiments, the recombinant nucleic acid construct encodes a recombinant chimeric antigenic receptor comprising a cancer antigen specific scFv fused with the extracellular domain (ECD) of a siglec receptor. This allows targetability of the construct to the cancer cell. The chimeric receptor comprises the TM and the ICD of the siglec receptor, which can be the endogenous ICD, or the ICD fused with additional phagocytosis promoting domains, such as PI3K binding domain or the domains. The siglec ECD region is prevented from activation by a concurrently expressed sialidase. The sialidase may be encoded by the construct in a monocistronic design, where the entire construct is expressed as a single polypeptide and them is readily cleaved by an endogenous T2A cleavage; or may be bicistronic, where the coding sequence of the sialidase enzyme is transcribed as a separate mRNA.

The siglec receptors bind to sialylated residues ubiquitously present on membrane proteins, and can activate the downstream signals. In some embodiments, the chimeric construct described herein encodes an extracellular domain, a transmembrane domain and an intracellular domain of the siglec protein, but is fused with a cancer targeting scFv at the extracellular terminus, and encodes for a sialidase at the intracellular terminus.

In some embodiments, irrespective of whether the recombinant construct is mono- or polycistronic, the sialidase coding sequence is fused with the coding sequence of an N-terminal signal peptide that signals for secretion of the protein sialidase upon translation. Upon expression of the sialidase and its release into the extracellular environment, the enzyme removes the digests sialic acid residues from the extracellular membrane proteins in the environment proximal to the membrane of the cell expressing the construct. Since the sialidase is expressed by the same cell that expressed the CAR-siglec receptor, expression of sialidase deprives the ECD of the siglec from binding to its natural ligand, but is activated by the scFv binding to its receptor, thereby ensuring the specificity of action of the chimeric receptor on a cancer-antigen expressing cell.

In some embodiments, the chimeric antigenic receptor functions as an anti-inflammatory and phagocytosis regulatory signaling protein by activation of the endogenous ICD domains of the siglec receptor through activation of the ITIMs.

In some embodiments, the chimeric antigenic receptor comprises endogenous ICD domains that have a pro-inflammatory signaling moiety, and/or a depletion of the endogenous siglec ITIM comprising ICDs, each of which are described in the previous section, and can be combined in modular ways with the scFv comprising CAR described herein.

### Chimeric Antigenic Receptor Domains for Enhancing Phagocytosis

*TREM Domains*

In one aspect, presented herein are recombinant nucleic acids encoding chimeric receptors, having one or more domains from a Triggering Receptor Expressed on Myeloid cells (TREM) receptor, a TREM chimeric receptor. TREM receptors are important regulators of the immune response, due to their ability to either amplify or decrease PRR-induced signals. This family of proteins includes the members: TREM 1, 2, 3. TREMs share common structural properties, including the presence of a single extracellular immunoglobulin-like domain of the V-type, a trans-membrane domain and a short cytoplasmic tail. In particular, the TREM trans-membrane domain (TM) possesses negatively charged residues that interact with the positively charged residues of the DNAX Activating Protein of 12 kDa (DAP12), a trans-membrane adaptor containing an immunoreceptor tyrosine-based activation motif (ITAM).

In some embodiments, recombinant nucleic acids encoding a chimeric antigen receptor comprises sequences that encode at least the TREM TM domain, such that the chimeric receptor interacts with DAP12 and enhance phagocytosis via phosphorylation of residues within the ITAM in DAP12, which recruits several signaling molecules that activate phagocytosis.

In some embodiments, the chimeric receptors comprise one or more domains from TREM proteins, fused at the extracellular region with an antigen binding domain that can specifically bind to a cancer antigen, such as a cancer antigen-specific antibody or part or fragment thereof.

In some embodiments, recombinant nucleic acids encoding a TREM chimeric antigen receptor encode a fusion proteins that comprises: (a) the at least a TREM transmembrane domain (TM) and a TREM intracellular domain (ICD); and (b) an extracellular domain (ECD) comprising an antigen binding domain that can specifically bind to a cancer antigen. The fusion proteins are designed to target cancer cells and bind to the target cancer cells via the ECD comprising the antigen binding domain, and the binding triggers and enhance phagocytosis via signaling through the TREM TM and/or the intracellular domains. The transmembrane domain of TREM trimerizes with DAP12 transmembrane domains and trigger intracellular pro-phagocytosis signaling cascade. In some embodiments, the TREM domains are contributed by TREM1, or by TREM2, or by TREM3 members. The extracellular antigen binding domain is fused to the extracellular terminus of the TREM domains through a short spacer or linker.

In some embodiments, the extracellular antigen binding domain comprises an antibody, specific to a cancer antigen. In some embodiments, the extracellular antigen binding domain comprises an antibody or an antigen binding part thereof that binds specifically to an antigen on the surface of a cancer cell.

In some embodiments the extracellular antigen binding domain is an antibody specific for a cancer antigen. In some embodiments, the extracellular antigen binding domain is a fraction of an antibody, wherein the fragment can bind specifically to the cancer antigen on a cancer cell. In some embodiments the antigen binding domain comprises a single chain variable fraction (scFv) specific for a cancer antigen binding domain.

### FcR domains

In some aspects, described herein are recombinant nucleic acids encoding chimeric antigen receptors that comprise an FcR domain. In some embodiments, the chimeric receptors described herein may comprise a FcR$\alpha$ (FcR$\alpha$1) domain. The FeR$\alpha$1 transmembrane domain heteromerizes with FcR$\gamma$ transmembrane domains in the myeloid cells, such as macrophages, and other phagocytic cells, such as mast cells, and the heterodimerization is required for expression of the protein on the cell surface. In some embodiments, expression of a recombinant protein that comprises the FcR$\alpha$1 TM domain is restricted to expression in cells that naturally express the FcyR. In this respect, a recombinant chimeric protein having the FcR$\alpha$1 TM domain is precluded for expressing in any cells other than the phagocytic cells that express the FcR$\gamma$. Similarly, FcR$\beta$ expression is restricted to mast cells. In some embodiments, the chimeric receptors are designed with one or more domains comprising the FcR$\alpha$1 TM domain for myeloid cell specific expression of the chimeric protein. In some embodiments, the chimeric receptors are designed with one or more domains comprising the FcR$\beta$ TM domain for mast-cell specific expression of the chimeric protein.

### Pro-caspases domains

In some aspects, described herein are recombinant nucleic acids encoding chimeric antigen receptors and a fusion protein comprising: an Src Homology 2 domain (SH2) 1 linked at the C terminus with a caspase cleavage sequence and a sequence encoding a Procaspase (SH2-ccs-Procaspase). In some embodiments, the recombinant nucleic acid encodes chimeric antigen receptor which comprises a first polypeptide (a) comprising: an extracellular antigen binding domain that can specifically bind to a cancer antigen, a transmembrane domain and an intracellular signaling domain comprising an ITAM motif that can trigger or enhance phagocytosis, and a second polypeptide (b) an intracellular polypeptide comprising the Src Homology 2 domain (SH2) 1 linked at the C terminus with a caspase cleavage sequence and a sequence encoding a Procaspase (SH2-ccs-Procaspase). The recombinant nucleic acid may be monocistronic or polycistronic. In some embodiments the recombinant nucleic acid is monocistronic and the sequence encoding (a) and the sequence encoding (b) are separated by a T2A sequence that cleaves the two polypeptides endogenously after translation. The Procaspase can be a Procaspase 1, Procaspase 2 or Procaspase 3. Upon expression of and cleavage of (a) and (b), the SH2 domain of the SH2-ccs-Procaspase directs the Procaspase to the phosphorylated intracellular domain of the ITAM motif of (a), and is activated. Activation of the SH2 domain in contact with the phosphorylated ITAM residues activates the proteolytic cleavage of Procaspase to generate activated caspase which are required to digest phagocytosed cells.

### *Ubiquitylation Activation Domains*

In some embodiments, intracellular signaling domains may be added to the chimeric receptor to enhance phagocytotic signaling. Ubiquitin signaling is involved in triggering of phagocytosis. Monoubiquitylation of endocytic receptors can target them for lysosomal degradation. IL-4 signaling mediated polyubiquitination of scavenger receptor MSR1 at the intracellular domain can lead to activation of the receptor, and trigger its interaction with MAP kinase pathway proteins that are involved in inducing inflammatory gene activation. For example, K63 polyubiquitylation of the MSR1 protein leads to its interaction with TAK1/MKK7/JNK in the phagosomes. Ubiquitylation of K27 residue of MSR1 is also implicated in MSR signaling and pro-inflammatory gene activation, such as expression of pro-inflammatory cytokines. Ubiquitin E3 ligase can promote ligation of ubiquitin to specific lysine residues on the intracellular signaling domain of MSR1, which is activated upon IL4 activation, and which in turn can activate intracellular signaling by binding of TAK1, MKK7 and JNK, and triggering expression of pro-inflammatory genes, such as TNF-alpha and IL-1□.

In some embodiments the recombinant chimeric antigen receptors described herein may comprise an intracellular domain which comprises residues that can be ubiquitylated and activated to generate a proinflammatory signal.

In some embodiments the intracellular domain of a chimeric antigen receptors described herein comprises the intracellular domain of MSR1 comprising the residues that can undergo E3 ligase mediated ubiquitylation.

In some embodiments, the intracellular domain of a chimeric antigen receptors described herein comprises the intracellular domain which can be ubiquitylated, and which upon ubiquitylation can bind to TAK1. In some embodiments, the intracellular domain of a chimeric antigen receptors described herein comprises the intracellular domain which can be ubiquitylated, and which upon ubiquitylation can bind to a MAP kinase protein, such as MKK7. In some embodiments, the intracellular domain of a chimeric antigen receptors described herein comprises the intracellular domain which can be ubiquitylated, and which upon ubiquitylation can bind to a kinase or a protein complex that comprises a MAP kinase protein, such as MKK7. In some embodiments, the intracellular domain of a chimeric antigen receptors described herein comprises an intracellular domain which can be ubiquitylated, and which upon ubiquitylation can bind to a kinase or a protein complex that comprises a JNK. In some embodiments, the intracellular domain of a chimeric antigen receptors described herein comprises an intracellular domain which can be ubiquitylated, and which upon ubiquitylation can bind to a kinase or a protein complex that can activate pro-inflammatory gene transcription.

As contemplated herein, a suitable ubiquitylation domain can be ligated at the intracellular portion of any one of the CARP receptors described in the disclosure.

### *Coexpression with other chimeric receptors for phagocytosis*

In some embodiments, one or more CFPs described herein may be co-expressed with a recombinant phagocytic receptor fusion protein (PFP) that has an extracellular domain comprising an antigen binding domain that can specifically bind to a cancer antigen on the surface of a cancer cell. The fusion protein further comprises a transmembrane domain and an intracellular phagocytosis receptor domain, may comprise and further phagocytosis signaling enhancement domains, such as kinase binding domains. The PFP is specifically designed to target a cancer cell and activate phagocytosis upon binding to the target. The phagocytosis enhancement by PFP is augmented by co-expression of the CFP. For example, cells coexpressing the PFP with the SIRP-CFPs enhance the phagocytic potential of the cells, wherein additionally the PFPs provide specific cancer targeting to the cells. For example, cells coexpressing the PFP with SH2-ccs-Procaspase have specific targeting to cancer cells with the PFP, whereas the phagocytic activity is enhanced by the PFP intracellular domains, and cancer cell killing activity is enhanced due to clearance of apoptotic cells by SH2-ccs-Procaspase via Procaspase activation to caspase.

In some embodiments, the scavenger receptor intracellular domain comprises a second intracellular domain comprising a signaling domain that activates phagocytosis; or a proinflammatory domain at the cytoplasmic terminus, which are operably linked. As the CD47 binding domain is operably linked with the one or more intracellular signaling domains of the phagocytic receptor, the signaling event originating from the engagement of the CD47 ligand at the extracellular end is altered upon passage through the intracellular domains to phagocytosis enhancing signal at the intracellular end of the recombinant receptor instead of the phagocytosis inhibition signal of a native CD47-binding SIRPα receptor. The phagocytic potential of the cell expressing the recombinant receptor is highly enhanced, or over a cell expressing SIRP-□ICD.

In some embodiments, the intracellular phagocytosis signaling domain may comprise domains selected from MRC1, ItgB5, MERTK, ELMO, BAIL Tyro3, Axl, Traf6, Syk, MyD88, Zap70, PI3K, FcyR1, FcyR2A, FcyR2B2, FcyR2C, FcyR3A, FcER1, FcaRI, BAFF-R, DAP12, NFAM1, and CD79b intracellular domains.

In one aspect, the function of the chimeric receptor may be further augmented by expressing an additional recombinant protein in the myeloid cell concurrently with or independent of the CFP expression. In some embodiments the additional recombinant protein is co-expressed with the CFP described above, wherein the CFP can bind to an antigen that is expressed in the target cell, and the CFP enhances phagocytosis and killing of the target cell by the myeloid cell expressing the CFP. In some embodiments, the additional recombinant protein is designed to further enhance the functioning of the CFP expressing cell.

In some embodiments, the additional recombinant protein is a second chimeric protein, such as a chimeric fusion protein, for example, a second chimeric protein. In some embodiments, the second chimeric protein is expressed in population of myeloid cells that expresses the cancer antigen targeting CFP. In some embodiments the additional recombinant protein is a second chimeric fusion protein or phagocytosis receptor fusion protein (PFP). In some embodiments, the second chimeric protein is a truncated protein.

*Exemplary additional recombinant constructs for augmenting phagocytosis and killing by myeloid cells:*

In one aspect, provided herein is a composition comprising a recombinant nucleic acid encoding a chimeric CD47 receptor (CR) fusion protein comprising: (a) a CR subunit comprising: (i) a transmembrane domain, and (ii) an intracellular domain comprising an intracellular signaling domain; an (b) an extracellular domain comprising an antigen binding domain specific to CD47 of a target cell; wherein the transmembrane domain and the extracellular domain are operatively linked; and wherein: (i) the CR does not comprise a functional intracellular domain of an endogenous receptor that binds CD47, (ii) the CR comprises a phosphatase inactivation domain or does not comprise an intracellular domain that activates a phosphatase, or (iii) the CR comprises an intracellular signaling domain derived from a phagocytic receptor. In some embodiments, the CR does not comprise a functional intracellular domain of an endogenous receptor that binds CD47.

In some embodiments, the CR comprises a phosphatase inactivation domain or does not comprise an intracellular domain that activates a phosphatase. In some embodiments, the CR comprises an intracellular signaling domain derived from a phagocytic or tethering receptor.

In some embodiments, the antigen binding domain specific to CD47 comprises an antibody domain specific to CD47.

In some embodiments, the antigen binding domain specific to CD47 comprises an extracellular domain derived from SIRPα or SIRPβ.

In some embodiments, the CR comprises an intracellular domain comprising an intracellular signaling domain.

In one aspect, provided herein is a composition comprising a recombinant nucleic acid encoding a phagocytic receptor (PR) fusion protein (PFP) comprising: (a) a PR subunit comprising: (i) a transmembrane domain, and (ii) an intracellular domain comprising an intracellular signaling domain; an (b) an extracellular domain comprising an antigen binding domain specific to an antigen of a target cell; wherein the transmembrane domain and the extracellular domain are operatively linked; wherein the intracellular signaling domain is derived from a phagocytic receptor; and wherein the recombinant nucleic acid encodes: (A) encodes a pro-inflammatory polypeptide, (B) comprises a binding motif for a molecule, such that upon binding of the molecule to the binding motif translation of the recombinant nucleic acid is inhibited (C) a procaspase domain, or (D) a procaspase binding domain, or (E) a sialidase.

In one aspect, provided herein is a composition comprising a recombinant nucleic acid encoding a phagocytic receptor (PR) fusion protein (PFP) comprising: (a) a PR subunit comprising: a transmembrane domain, and an intracellular domain comprising an intracellular signaling domain; an (b) an extracellular domain comprising an antigen binding domain specific to an antigen of a target cell; wherein the transmembrane domain and the extracellular domain are operatively linked; an wherein the PFP forms a functional complex with FcRγ when expressed in a cell.

In one aspect, provided herein is a composition comprising a recombinant nucleic acid encoding a phagocytic receptor (PR) fusion protein (PFP) comprising: (a) a PR subunit comprising: a transmembrane domain, and (ii) an intracellular domain comprising an intracellular signaling domain; an (b) an extracellular domain comprising an antigen binding domain specific to an antigen of a target cell; wherein the transmembrane domain and the extracellular domain are operatively linked; and wherein the PFP forms a functional complex with DAP12 when expressed in a cell.

In some embodiments, the recombinant nucleic acid encodes: (A) encodes a pro-inflammatory polypeptide, (B) comprises a binding motif for a molecule, such that upon binding of the molecule to the binding motif translation of the recombinant nucleic acid is inhibited, (C) a procaspase domain, or (D) a procaspase binding domain.

In some embodiments, the PFP forms a functional complex with FcRγ when expressed in a cell.

In some embodiments, the PFP forms a functional complex with DAP12 when expressed in a cell.

In some embodiments, the PFP comprises an antigen binding domain that binds to a CD47 ligand, but does not comprise a functional intracellular domain of an endogenous receptor that binds CD47, (i) the PFP comprises a phosphatase inactivation domain or does not comprise an intracellular domain that activates a phosphatase, or (ii) the PFP comprises an intracellular signaling domain derived from a phagocytic receptor.

In some embodiments, the CFP or the PFP forms a functional complex with an FcR.

In some embodiments, the CFP or the PFP forms a functional complex with FcRγ.

In some embodiments, the CFP or the PFP comprises a transmembrane domain of FcR-alpha or FcRβ.

In some embodiments, the CFP or the PFP forms a functional complex with a TREM.

In some embodiments, the CFP or the PFP comprises an intracellular domain comprising an ITAM.

In some embodiments, the CFP or the PFP comprises an intracellular domain comprising an ITIM.

In some embodiments, a cell expressing the CFP or the PFP exhibits inhibits CD47 mediated anti-phagocytosis activity.

In some embodiments, the truncated SIRPα binds to CD47 and inhibits.

In some embodiments, the binding motif is in a UTR region of the recombinant nucleic acid. In some embodiments, the binding motif is an ARE sequence.

In some embodiments, the transmembrane domain binds to a transmembrane domain of DAP12.

In some embodiments, the transmembrane domain that binds to a transmembrane domain of DAP12 is derived from TREM.

In some embodiments, the CFP or the PFP comprises an intracellular domain derived from a DAP12 monomer.

In some embodiments, the recombinant polynucleotide further comprises a sequence encoding a first modified signal regulatory protein α (SIRPα).

In some embodiments, the first modified SIRPα lacks an intracellular signaling domain of a wild type SIRPα.

In some embodiments, the first modified SIRPα lacks an intracellular signaling domain of a wild type SIRPβ.

In some embodiments, the CFP or the PFP does not transmit a signal to block phagocytosis.

In some embodiments, the composition further comprises a polynucleotide encoding a second modified signal regulatory protein α (SIRPα), wherein the second modified SIRPα comprises a PI3K binding domain

In some embodiments, the PI3K binding domain is derived from CD19, CD28, CSFR or PDGFR.

In some embodiments, the composition further comprises a polynucleotide encoding a third modified signal regulatory protein α (SIRPα), wherein the third modified SIRPα comprises a pro-inflammatory domain.

In some embodiments, the composition further comprises a polynucleotide encoding a recombinant SIRP, wherein the recombinant SIRP comprises an extracellular domain derived from SIRPα, a transmembrane domain derived from SIRPβ, and an intracellular domain derived from SIRPβ.

In some embodiments, the composition further comprises when the recombinant SIRP binds to an antigen CD47, the third modified SIRPα does not transmit a signal to block phagocytosis.

In some embodiments, the anti-CD47 binding domain is derived from signal-regulatory protein alpha (SIRPα) or signal-regulatory protein beta (SIRPβ).

In some embodiments, upon binding of the PFP to the antigen of the target cell, the killing activity of a cell expressing the PFP is increased by at least greater than 20% compared to a cell not expressing the PFP.

In some embodiments, the intracellular signaling domain is derived from a phagocytic or tethering receptor.

In some embodiments, the intracellular signaling domain is derived from a receptor other than a phagocytic receptor selected from any one of the receptors listed in Table 2.

In some embodiments, the intracellular signaling domain is derived from a receptor selected from the group consisting of lectin, dectin 1, CD206, scavenger receptor A1 (SRA1), MARCO, CD36, CD163, MSR1, SCARA3, COLEC12, SCARA5, SCARB1, SCARB2, CD68, OLR1, SCARF1, SCARF2, CXCL16, STAB1, STAB2, SRCRB4D, SSCSD, CD205, CD207, CD209, RAGE, CD14, CD64, F4/80, CCR2, CX3CR1, CSF1R, Tie2, HuCRIg(L), and CD169.

In some embodiments, wherein the intracellular signaling domain comprises a pro-inflammatory signaling domain.

In some embodiments, the intracellular signaling domain comprises a pro-inflammatory signaling domain that is not a PI3K recruitment domain.

In some embodiments, the intracellular signaling domain is derived from a phagocytic receptor other than a phagocytic receptor selected from Megf10, MerTk, FcR-alpha, or Bai1.

In some embodiments, upon binding of the PFP to the antigen of the target cell, the killing activity of a cell expressing the PFP is increased by at least greater than 20% compared to a cell not expressing the PFP.

In some embodiments, the intracellular signaling domain is derived from a phagocytic receptor selected from the group consisting of any one of the proteins listed in Table 1.

In some embodiments, the intracellular signaling domain comprises a PI3K recruitment domain.

In some embodiments, the CFP or the PFP functionally incorporates into a cell membrane of a cell when the CFP or the PFP is expressed in the cell.

In some embodiments, a cell expressing the CFP or the PFP exhibits an increase in phagocytosis of a target cell expressing the antigen compared to a cell not expressing the CFP or the PFP.

In some embodiments, a cell expressing the CFP or the PFP exhibits at least a 1.1-fold increase in phagocytosis of a target cell expressing the antigen compared to a cell not expressing the CFP or the PFP.

In some embodiments, a cell expressing the CFP or the PFP exhibits at least a 2-fold, 3-fold, 4-fold, 5-fold, 6-fold, 7-fold, 8-fold, 9-fold, 10-fold, 20-fold, 30-fold or 50-fold increase in phagocytosis of a target cell expressing the antigen compared to a cell not expressing the CFP or the PFP.

In some embodiments, the target cell expressing the antigen is a cancer cell.

In some embodiments, the target cell expressing the antigen is at least 0.8 microns in diameter.

In some embodiments, the intracellular signaling domain is derived from a scavenger receptor.

In some embodiments, a cell expressing the CFP or the PFP exhibits an increase in production of a cytokine compared to a cell not expressing the CFP or the PFP.

In some embodiments, the cytokine is selected from the group consisting of IL-1, IL3, IL-6, IL-12, IL-13, IL-23, TNF, CCL2, CXCL9, CXCL10, CXCL11, IL-18, IL-23, IL-27, CSF, MCSF, GMCSF, IL17, IP-10, RANTES, an interferon and combinations thereof.

In some embodiments, a cell expressing the CFP or the PFP exhibits an increase in effector activity compared to a cell not expressing the CFP or the PFP.

In some embodiments, a cell expressing the CFP or the PFP exhibits an increase in cross-presentation compared to a cell not expressing the CFP or the PFP.

In some embodiments, a cell expressing the CFP or the PFP exhibits an increase in expression of an MHC class II protein compared to a cell not expressing the CFP or the PFP.

In some embodiments, a cell expressing the CFP or the PFP exhibits an increase in expression of CD80 compared to a cell not expressing the CFP or the PFP.

In some embodiments, a cell expressing the CFP or the PFP exhibits an increase in expression of CD86 compared to a cell not expressing the CFP or the PFP.

In some embodiments, a cell expressing the CFP or the PFP exhibits an increase in expression of MHC class I protein compared to a cell not expressing the CFP or the PFP.

In some embodiments, a cell expressing the CFP or the PFP exhibits an increase in expression of TRAIL/TNF Family death receptors compared to a cell not expressing the CFP or the PFP.

In some embodiments, a cell expressing the CFP or the PFP exhibits an increase in expression of B7-H2 compared to a cell not expressing the CFP or the PFP.

In some embodiments, a cell expressing the CFP or the PFP exhibits an increase in expression of LIGHT compared to a cell not expressing the CFP or the PFP.

In some embodiments, a cell expressing the CFP or the PFP exhibits an increase in expression of HVEM compared to a cell not expressing the CFP or the PFP.

In some embodiments, a cell expressing the CFP or the PFP exhibits an increase in expression of CD40 compared to a cell not expressing the CFP or the PFP.

In some embodiments, a cell expressing the CFP or the PFP exhibits an increase in expression of TL1A compared to a cell not expressing the CFP or the PFP.

In some embodiments, a cell expressing the CFP or the PFP exhibits an increase in expression of 41BBL compared to a cell not expressing the CFP or the PFP.

In some embodiments, a cell expressing the CFP or the PFP exhibits an increase in expression of OX40L compared to a cell not expressing the CFP or the PFP.

In some embodiments, a cell expressing the CFP or the PFP exhibits an increase in expression of GITRL death receptors compared to a cell not expressing the CFP or the PFP.

In some embodiments, a cell expressing the CFP or the PFP exhibits an increase in expression of CD30L compared to a cell not expressing the CFP or the PFP.

In some embodiments, a cell expressing the CFP or the PFP exhibits an increase in expression of TIM4 compared to a cell not expressing the CFP or the PFP.

In some embodiments, a cell expressing the CFP or the PFP exhibits an increase in expression of TIM1 Ligand compared to a cell not expressing the CFP or the PFP.

In some embodiments, a cell expressing the CFP or the PFP exhibits an increase in expression of SLAM compared to a cell not expressing the CFP or the PFP.

In some embodiments, a cell expressing the CFP or the PFP exhibits an increase in expression of CD48 compared to a cell not expressing the CFP or the PFP.

In some embodiments, a cell expressing the CFP or the PFP exhibits an increase in expression of CD58 compared to a cell not expressing the CFP or the PFP.

In some embodiments, a cell expressing the CFP or the PFP exhibits an increase in expression of CD155 compared to a cell not expressing the CFP or the PFP.

In some embodiments, a cell expressing the CFP or the PFP exhibits an increase in expression of CD112 compared to a cell not expressing the CFP or the PFP.

In some embodiments, a cell expressing the CFP or the PFP exhibits an increase in expression of PDL1compared to a cell not expressing the CFP or the PFP.

In some embodiments, provided herein is the composition of any of B7-DC compared to a cell not expressing the CFP or the PFP.

In some embodiments, a cell expressing the CFP or the PFP exhibits an increase in respiratory burst compared to a cell not expressing the CFP or the PFP.

In some embodiments, a cell expressing the CFP or the PFP exhibits an increase in ROS production compared to a cell not expressing the CFP or the PFP.

In some embodiments, a cell expressing the CFP or the PFP exhibits an increase in iNOS production compared to a cell not expressing the CFP or the PFP.

In some embodiments, a cell expressing the CFP or the PFP exhibits an increase in iNOS production compared to a cell not expressing the CFP or the PFP.

In some embodiments, a cell expressing the CFP or the PFP exhibits an increase in extra-cellular vesicle production compared to a cell not expressing the CFP or the PFP.

In some embodiments, a cell expressing the CFP or the PFP exhibits an increase in trogocytosis with a target cell expressing the antigen compared to a cell not expressing the CFP or the PFP.

In some embodiments, a cell expressing the CFP or the PFP exhibits an increase in resistance to CD47 mediated inhibition of phagocytosis compared to a cell not expressing the CFP or the PFP.

In some embodiments, a cell expressing the CFP or the PFP exhibits an increase in resistance to LILRB1 mediated inhibition of phagocytosis compared to a cell not expressing the CFP or the PFP.

In some embodiments, the intracellular domain comprises a Rac inhibition domain, a Cdc42 inhibition domain or a GTPase inhibition domain.

In some embodiments, the Rac inhibition domain, the Cdc42 inhibition domain or the GTPase inhibition domain inhibits Rac, Cdc42 or GTPase at a phagocytic cup of a cell expressing the CFP or the PFP.

In some embodiments, the intracellular domain comprises an F-actin disassembly activation domain, a ARHGAP12 activation domain, a ARHGAP25 activation domain or a SH3BP1 activation domain.

In some embodiments, a cell expressing the CFP or the PFP exhibits an increase in phosphatidylinositol 3,4,5-trisphosphate production.

In some embodiments, the extracellular domain comprises an Ig binding domain.

In some embodiments, the extracellular domain comprises an IgA, IgD, IgE, IgG, IgM, Fc$\gamma$RI, Fc$\gamma$RIIA, Fc$\gamma$RIIB, Fc$\gamma$RIIC, Fc$\gamma$RIIIA, Fc$\gamma$RIIIB, FcRn, TRIM21, FcRLS binding domain.

In some embodiments, the extracellular domain comprises an FcR extracellular domain.

In some embodiments, the extracellular domain comprises an FcR$\alpha$, FcR$\beta$, FcR$\epsilon$ or FcR$\gamma$ extracellular domain.

In some embodiments, the extracellular domain comprises FcR$\alpha$ (FCAR) extracellular domain.

In some embodiments, the extracellular domain comprises an FcR$\beta$ extracellular domain.

In some embodiments, the extracellular domain comprises an FcR$\epsilon$ (FCER1A) extracellular domain.

In some embodiments, the extracellular domain comprises an FcR$\gamma$ (FDGR1A, FCGR2A, FCGR2B, FCGR2C, FCGR3A, FCGR3B) extracellular domain The composition of any one of the preceding embodiments, wherein the extracellular domain comprises an integrin domain.

In some embodiments, the extracellular domain comprises one more integrin $\alpha1, \alpha2, \alpha$IIb, $\alpha3, \alpha4, \alpha5, \alpha6, \alpha7, \alpha8, \alpha9, \alpha10, \alpha11, \alpha$IIb, $\alpha$D, $\alpha$E, $\alpha$L, $\alpha$M, $\alpha$V, $\alpha$X, $\beta1, \beta2, \beta3, \beta4, \beta5, \beta6, \beta7, \beta8$ domain.

In some embodiments, the intracellular domain comprises a CD47 inhibition domain.

In some embodiments, the PSR subunit further comprises an extracellular domain operatively linked to the transmembrane domain and the extracellular antigen binding domain. In some embodiments, the extracellular domain further comprises an extracellular domain of a receptor, a hinge, a spacer or a linker. In some embodiments, the extracellular domain comprises an extracellular portion of a PSR. In some embodiments, the extracellular portion of the PSR is derived from the same PSR as the PSR intracellular signaling domain. In some embodiments, the extracellular domain comprises an extracellular domain of a scavenger receptor or an immunoglobulin domain.

In some embodiments, the immunoglobulin domain comprises an extracellular domain of an immunoglobulin or an immunoglobulin hinge region. In some embodiments, the extracellular domain comprises a phagocytic engulfment marker.

In some embodiments, the extracellular domain comprises a structure capable of multimeric assembly. In some embodiments, the extracellular domain comprises a scaffold for multimerization.

In some embodiments, the extracellular domain is at least 10, 20, 30, 40, 50, 60, 70, 80, 90, 100, 150, 200, 300, 300, 400, or 500 amino acids in length. In some embodiments, the extracellular domain is at most 500, 400, 300, 200, or 100 amino acids in length.

In some embodiments, the extracellular antigen binding domain specifically binds to the antigen of a target cell. In some embodiments, the extracellular antigen binding domain comprises an antibody domain. In some embodiments, the extracellular antigen binding domain comprises a receptor domain, antibody domain, wherein the antibody domain comprises a functional antibody fragment, a single chain variable fragment (scFv), an Fab, a single-domain antibody (sdAb), a nanobody, a $V_H$ domain, a $V_L$ domain, a VNAR domain, a $V_{HH}$ domain, a bispecific antibody, a diabody, or a functional fragment or a combination thereof.

In some embodiments, the extracellular antigen binding domain comprises a ligand, an extracellular domain of a receptor or an adaptor.

In some embodiments, the extracellular antigen binding domain comprises a single extracellular antigen binding domain that is specific for a single antigen.

In some embodiments, the extracellular antigen binding domain comprises at least two extracellular antigen binding domains, wherein each of the at least two extracellular antigen binding domains is specific for a different antigen. In some embodiments, the antigen is a cancer antigen or a pathogenic antigen or an autoimmune antigen. In some embodiments, the antigen comprises a viral antigen. In some embodiments, the antigen is a T-lymphocyte antigen. In some embodiments, the antigen is an extracellular antigen. In some embodiments, the antigen is an intracellular antigen.

In some embodiments, the antigen is selected from the group consisting of Thymidine Kinase (TK1), Hypoxanthine-Guanine Phosphoribosyltransferase (HPRT), Receptor Tyrosine Kinase-Like Orphan Receptor 1 (ROR1), Mucin-1, Mucin-16 (MUC16), MUC1, Epidermal Growth Factor Receptor vIII (EGFRvIII), Mesothelin, Human Epidermal Growth Factor Receptor 2 (HER2), Mesothelin, EBNA-1, LEMD1, Phosphatidyl Serine, Carcinoembryonic Antigen (CEA), B-Cell Maturation Antigen (BCMA), Glypican 3 (GPC3), Follicular Stimulating Hormone receptor, Fibroblast Activation Protein (FAP), Erythropoietin-Producing Hepatocellular Carcinoma A2 (EphA2), EphB2, a Natural Killer Group 2D (NKG2D) ligand, Disialoganglioside 2 (GD2), CD2, CD3, CD4, CD5, CD7, CD8, CD19, CD20, CD22, CD24, CD30, CD33, CD38, CD44v6, CD45, CD56CD79b, CD97, CD117, CD123, CD133, CD138, CD171, CD179a, CD213A2, CD248, CD276, PSCA, CS-1, CLECL1, GD3, PSMA, FLT3, TAG72, EPCAM, IL-1, an integrin receptor, PRSS21, VEGFR2, PDGFR-β, SSEA-4, EGFR, NCAM, prostase, PAP, ELF2M, GM3, TEM7R, CLDN6, TSHR, GPRC5D, ALK, IGLL1 and combinations thereof. In some embodiments, the antigen is selected from the group consisting of CD2, CD3, CD4, CD5, CD7, CCR4, CD8, CD30, CD45, CD56.

In some embodiments, the antigen is an ovarian cancer antigen or a T lymphoma antigen. In some embodiments, the antigen is an integrin receptor. In some embodiments, the antigen is an integrin receptor selected from the group consisting of α1, α2, αIIb, α3, α4, a5, α6, α7, α8, α9, α10, α11, αD, αE, αL, αM, αV, αX, β1, β2, β3, β4, β5, β6, β7, and β8.

In some embodiments, the transmembrane domain and the extracellular antigen binding domain is operably linked through a linker. In some embodiments, the transmembrane domain and the extracellular antigen binding domain is operatively linked through a linker such as the hinge region of CD8α □ □ IgG1 or IgG4.

In some embodiments, the extracellular domain comprises a multimerization scaffold.

In some embodiments, the transmembrane domain comprises an FcR transmembrane domain.

In some embodiments, the transmembrane domain comprises an FcR-alpha, FcRβ or FcRγ transmembrane domain.

In some embodiments, the transmembrane domain comprises an FcαR (FCAR) transmembrane domain. In some embodiments, the transmembrane domain comprises an FcRε (FCER1A) transmembrane domain. In some embodiments, the transmembrane domain comprises an FcRγ (FDGR1A, FCGR2A, FCGR2B, FCGR2C, FCGR3A, and FCGR3B) transmembrane domain.

In some embodiments, the transmembrane domain comprises a T cell Receptor subunit, CD3 epsilon, CD3 gamma and CD3 delta, CD45, CD2 CD4, CD5, CD8, CD9, CD16, CD19, CD22, CD33, CD28, CD30, CD37, CD64, CD80, CD86, CD134, CD137 and CD 154, or a functional fragment thereof, or an amino acid sequence having at least one, two or three modifications but not more than 20, 10 or 5 modifications transmembrane domain.

In some embodiments, the transmembrane domain comprises a transmembrane domain from a syntaxin such as syntaxin 3 or syntaxin 4 or syntaxin 5.

In some embodiments, the transmembrane domain oligomerizes with a transmembrane domain of an endogenous receptor when the CR or the PFP is expressed in a cell.

In some embodiments, the transmembrane domain oligomerizes with a transmembrane domain of an exogenous receptor when the CR or the PFP is expressed in a cell.

In some embodiments, the transmembrane domain dimerizes with a transmembrane domain of an endogenous receptor when the CFP or the PFP is expressed in a cell.

In some embodiments, the transmembrane domain dimerizes with a transmembrane domain of an exogenous receptor when the CFP or the PFP is expressed in a cell.

In some embodiments, the transmembrane domain is derived from a protein that is different than the protein from which the intracellular signaling domain is derived.

In some embodiments, the transmembrane domain is derived from a protein that is different than the protein from which the extracellular domain is derived.

In some embodiments, the transmembrane domain comprises a transmembrane domain of a phagocytic receptor.

In some embodiments, the transmembrane domain and the extracellular domain are derived from the same protein.

In some embodiments, the transmembrane domain is derived from the same protein as the intracellular signaling domain.

In some embodiments, the recombinant nucleic acid encodes a DAP12 recruitment domain.

In some embodiments, the transmembrane domain comprises a transmembrane domain that oligomerizes with DAP12.

In some embodiments, the transmembrane domain is at least 12, 13, 14, 15, 16, 17, 18, 19, 20, 21, 22, 23, 24, 25, 26, 27, 28, 29, 30, 31 or 32 amino acids in length.

In some embodiments, the transmembrane domain is at most 12, 13, 14, 15, 16, 17, 18, 19, 20, 21, 22, 23, 24, 25, 26, 27, 28, 29, 30, 31 or 32 amino acids in length.

In some embodiments, the intracellular domain comprises a phosphatase inhibition domain.

In some embodiments, the intracellular domain comprises an ARP2/3 inhibition domain.

In some embodiments, the intracellular domain comprises at least one ITAM domain.

In some embodiments, the intracellular domain comprises at least 1, 2, 3, 4, 5, 6, 7, 8, 9, or 10 ITAM domains.

In some embodiments, the intracellular domain further comprises at least one ITAM domain. In some embodiments, the intracellular domain further comprises at least one ITAM domain select from a group CD3 zeta TCR subunit, CD3 epsilon TCR subunit, CD3 gamma TCR subunit, CD3 delta TCR subunit, TCR zeta chain, Fc epsilon receptor 1 chain, Fc epsilon receptor 2 chain, Fc gamma receptor 1 chain, Fc gamma receptor 2a chain, Fc gamma receptor 2b 1 chain, Fc gamma receptor 2b2 chain, Fc gamma receptor 3a chain, Fc gamma receptor 3b chain, Fc beta receptor 1 chain, TYROBP (DAP12), CD5, CD16a, CD16b, CD22, CD23, CD32, CD64, CD79a, CD79b, CD89, CD278, CD66d, functional fragments thereof, and amino acid sequences thereof having at least one but not more than 20 modifications thereto.

In some embodiments, the at least one ITAM domain comprises a Src-family kinase phosphorylation site. In some embodiments, the at least one ITAM domain comprises a Syk recruitment domain. In some embodiments, the intracellular domain comprises an F-actin depolymerization activation domain.

In some embodiments, the intracellular domain comprises residues that can be ubiquitiylated. In some embodiments, the intracellular domain can bind to an E3 ubiquitin ligase.

In some embodiments, the intracellular domain can bind to a TAK1 kinase.

In some embodiments, the intracellular domain can bind to a MAP kinase or a MAP kinase family member.

In some embodiments, the intracellular domain can be activated upon ubiquitylation and can activate intracellular signaling resulting in pro-inflammatory gene transcription.

In some embodiments, the intracellular domain lacks enzymatic activity.

In some embodiments, the intracellular domain does not comprise a domain derived from a CD3 zeta intracellular domain.

In some embodiments, the intracellular domain comprises a CD47 inhibition domain

In some embodiments the intracellular signaling domain comprises a domain that activates integrin such as the intracellular region of PSGL-1.

In some embodiments, the intracellular signaling domain comprises a domain that activates Rap1 GTPase, such as that from EPAC and C3G.

In some embodiments, the intracellular signaling domain is from Paxillin. In some embodiments, the intracellular signaling domain activates focal adhesion kinase. In some embodiments, the intracellular signaling domain is derived from a single phagocytic receptor. In some embodiments, the intracellular signaling domain is derived from a single scavenger receptor. In some embodiments, the intracellular domain further comprises a phagocytosis enhancing domain. In some embodiments, the intracellular domain comprises a pro-inflammatory signaling domain. In some embodiments, the pro-inflammatory signaling domain comprises a kinase activation domain or a kinase binding domain. In some embodiments, the pro-inflammatory signaling domain comprises an IL-1 signaling cascade activation domain. In some embodiments, the pro-inflammatory signaling domain comprises an intracellular signaling domain derived from TLR3, TLR4, TLR7, TLR 9, TRIF, RIG-1, MYD88, MAL, IRAK1, MDA-5, an IFN-receptor, an NLRP family member, NLRP1-14, NOD1, NOD2, Pyrin, AIM2, NLRC4, FCGR3A, FCERIG, CD40 or any combination thereof. In some embodiments, the CFP or the PFP does not comprise a full length intracellular signaling domain.

In some embodiments, the intracellular domain is at least 5, 10, 20, 30, 40, 50, 60, 70, 80, 90, 100, 150, 200, 300, 300, 400, or 500 amino acids in length.

In some embodiments, the intracellular domain is at most 10, 20, 30, 40, 50, 60, 70, 80, 90, 100, 150, 200, 300, 300, 400, or 500 amino acids in length.

In some embodiments, the recombinant nucleic acid encodes an FcRα chain extracellular domain, an FcRα chain transmembrane domain and/or an FcRα chain intracellular domain. In some embodiments, the recombinant nucleic acid encodes an FcRβ chain extracellular domain, an FcRβ chain transmembrane domain and/or an FcRβ chain intracellular domain. In some embodiments, the FcRα chain or the FcRβ chain forms a complex with FcRγ when expressed in a cell. In some embodiments, the FcRα chain or FcRβ chain forms a complex with endogenous FcRγ when expressed in a cell. In some embodiments, the FcRα chain or the FcRβ chain does not incorporate into a cell membrane of a cell that does not express FcRγ. In some embodiments, the CFP or the PFP does not comprise an FcRα chain intracellular signaling domain. In some embodiments, the CFP or the PFP does not comprise an FcRβ chain intracellular signaling domain.

In some embodiments, the recombinant nucleic acid encodes a TREM extracellular domain, a TREM transmembrane domain and/or a TREM intracellular domain. In some embodiments, the TREM is TREM1, TREM 2 or TREM 3.

In some embodiments, the recombinant nucleic acid comprises a sequence encoding a pro-inflammatory polypeptide. In some embodiments, the composition further comprises a pro-inflammatory polypeptide.

In some embodiments, the pro-inflammatory polypeptide is a chemokine, cytokine and nucleotides. In some embodiments, In some embodiments, the chemokine is selected from the group consisting of CCL2, CXCL1, CXCL12, CXCL9, CXCL10, CXCL11.

In some embodiments, the cytokine is selected from the group consisting of IL-1, IL3, IL5, IL-6, IL-12, IL-13, IL-23, TNF, IL-18, IL-23, IL-27, CSF, MCSF, GMCSF, IL17, IP-10, RANTES, an interferon.

In some embodiments, the recombinant nucleic acid comprises a homeostatic regulator of inflammation.

In some embodiments, the homeostatic regulator of inflammation is a sequence in an untranslated region (UTR) of an mRNA. In some embodiments, the sequence in the UTR is a sequence that binds to an RNA binding protein. In some embodiments, translation is inhibited or prevented upon binding of the RNA binding protein to the sequence in an untranslated region (UTR). In some embodiments, the sequence in the UTR comprises a consensus sequence of WWWU(AUUUA)UUUW (SEQ ID NO: 35), wherein W is A or U.

In some embodiments, the recombinant nucleic acid is expressed on a bicistronic vector.

In some embodiments, the target cell is a mammalian cell. In some embodiments, the target cell is a human cell. In some embodiments, the target cell comprises a cell infected with a pathogen. In some embodiments, the target cell is a cancer cell. In some embodiments, the target cell is a cancer cell that is a lymphocyte. In some embodiments, the target cell is a cancer cell that is an ovarian cancer cell. In some embodiments, the target cell is a cancer cell that is an ovarian pancreatic cell. In some embodiments, the target cell is a cancer cell that is a glioblastoma cell.

In some embodiments, the recombinant nucleic acid is DNA. In some embodiments, the recombinant nucleic acid is RNA. In some embodiments, the recombinant nucleic acid is mRNA. In some embodiments, the recombinant nucleic acid is a circRNA. In some embodiments, the recombinant nucleic acid is a tRNA. In some embodiments, the recombinant nucleic acid is a microRNA.

Provided herein is a vector comprising the composition described above. In some embodiments, vector is viral vector. In some embodiments, the viral vector is retroviral vector or a lentiviral vector. In some embodiments, the vector further comprises a promoter operably linked to at least one nucleic acid sequence encoding one or more polypeptides. In some embodiments, the vector is polycistronic. In some embodiments, each of the at least one nucleic acid sequence is operably linked to a separate promoter. In some embodiments, the vector further comprises one or more internal ribosome entry sites (IRESs). In some embodiments, the vector further comprises a 5'UTR and/or a 3'UTR flanking the at least one nucleic acid sequence encoding one or more polypeptides. In some embodiments, the vector further comprises one or more regulatory regions.

Provided herein is a polypeptide encoded by the recombinant nucleic acid of the composition described above.

In one aspect, provided herein is a cell comprising a vector described above or the polypeptide described above. In some embodiments, the cell is a phagocytic cell. In some embodiments, the cell is a stem cell derived cell, myeloid cell, macrophage, a dendritic cell, lymphocyte, mast cell, monocyte, neutrophil, microglia, or an astrocyte. In some embodiments, the cell is an autologous cell. In some embodiments, the cell is an allogeneic cell. In some embodiments, the cell is an M1 myeloid cell, such as a macrophage. In some embodiments, the cell is an M2 myeloid cell, such as a macrophage. In some embodiments, the cell is a precursor cell of myeloid lineage. In some embodiments, the myeloid cell is a CD14+ cell. In some embodiments, the myeloid cell is a CD16- cell. In some embodiments, the myeloid cell is a CD14+ and CD16- cell.

Provided herein is a pharmaceutical composition comprising (a) the nucleic acid composition or the vector or the polypeptide or the cell as described above; and (b) a pharmaceutically acceptable excipient. In some embodiments, the pharmaceutical composition further comprising an additional therapeutic agent. In some embodiments, the pharmaceutical composition comprises additional therapeutic agent which is selected from the group consisting of a CD47 agonist, an agent that inhibits Rac, an agent that inhibits Cdc42, an agent that inhibits a GTPase, an agent that promotes F-actin disassembly, an agent that promotes PI3K recruitment to the CFP or the PFP, an agent that promotes PI3K activity, an agent that promotes production of phosphatidylinositol 3,4,5-trisphosphate, an agent that promotes ARHGAP12 activity, an agent that promotes ARHGAP25 activity, an agent that promotes SH3BP1 activity and any combination thereof.

In some embodiments, the pharmaceutically acceptable excipient comprises serum free media, a lipid, or a nanoparticle.

Provided herein is a method of treating a disease in a subject in need thereof comprising administering to the subject the pharmaceutical composition described herein.

In some embodiments, the disease is cancer. In some embodiments, the cancer is a solid cancer. In some embodiments, the solid cancer is selected from the group consisting of ovarian cancer, suitable cancers include ovarian cancer, renal cancer, breast cancer, prostate cancer, liver cancer, brain cancer, lymphoma, leukemia, skin cancer, pancreatic cancer, colorectal cancer, lung cancer. In some embodiments, the cancer is a liquid cancer. In some embodiments, the liquid cancer is a leukemia or a lymphoma. In some embodiments, the liquid cancer is a T cell lymphoma. In some embodiments, the disease is a T cell malignancy. In some embodiments the method further comprises administering an additional therapeutic agent to the subject. In some embodiments, the additional therapeutic agent is selected from the group consisting of a CD47 agonist, an agent that inhibits Rac, an agent that inhibits Cdc42, an agent that inhibits a GTPase, an agent that promotes F-actin disassembly, an agent that promotes PI3K recruitment to the CFP or the PFP, an agent that promotes PI3K activity, an agent that promotes production of phosphatidylinositol 3,4,5-trisphosphate, an agent that promotes ARHGAP12 activity, an agent that promotes ARHGAP25 activity, an agent that promotes SH3BP1 activity and any combination thereof.

In some embodiments, administering comprises infusing or injecting. In some embodiments, administering comprises administering directly to the solid cancer.

In some embodiments, administering comprises a circRNA, mRNA, viral-, particle-, liposome-, or exosome-based delivery procedure.

In some embodiments, a CD4+ T cell response or a CD8+ T cell response is elicited in the subject.

In some embodiments, method comprising contacting a cell with the composition described above, the vector or the polypeptide described above. In some embodiments, contacting comprises transducing. In some embodiments, transducing comprises chemical transfection, electroporation, nucleofection, or viral infection.

In some embodiments, provided herein is a method of preparing a pharmaceutical composition comprising contacting a lipid to the composition described herein or the vector described herein.

In some embodiments, contacting comprises forming a lipid nanoparticle.

Also provided is a method of preparing a pharmaceutical composition comprising contacting an antibody to the composition as described herein or the vector described herein. In some embodiments, contacting comprises forming a lipid nanoparticle.

## _TRANSCRIPTION REGULATORY ELEMENTS IN THE RECOMBINANT NUCLEIC ACID CONSTRUCT_

In some embodiments, the recombinant nucleic comprises one or more regulatory elements within the noncoding regions that can be manipulated for desired expression profiles of the encoded proteins. In some embodiments, the noncoding region may comprise suitable enhancer. In some embodiments, the enhancer comprises a binding region for a regulator protein or peptide may be added to the cell or the system comprising the cell, for commencement of expression of the protein encoded under the influence of the enhancer. Conversely, a regulatory element may comprise a protein binding domain that remains bound with the cognate protein and continue to inhibit transcription and/or translation of recombinant protein until an extracellular signal is provided for the protein to decouple from the bound position to allow commencement of the protein synthesis. Examples include but are not limited to Tetracyclin-inducible (Tet-Inducible or Tet-on) and Tetracyclin repressible (Tet-off) systems known to one of skill in the art.

### _Construct comprising metabolic switch:_

In some embodiments the 5' and 3' untranslated regions flanking the coding regions of the construct may be manipulated for regulation of expression of the recombinant protein encoded by the nucleic acid constructs described above. For instance, the 3'UTR may comprise one or more elements that are inserted for stabilizing the mRNA. In some embodiments, AU-Rich Elements (ARE) sequences are inserted in the 3' UTR that result in binding of RNA binding proteins that stabilize or destabilize the mRNA, allowing control of the mRNA half-life.

In some embodiments the 3'UTR may comprise a conserved region for RNA binding proteins (eg GAPDH) binding to mature mRNA strand preventing translation. In some embodiments, glycolysis results in the uncoupling of the RNA binding proteins (eg GAPDH) allowing for mRNA strand translation. The principle of the metabolic switch is to trigger expression of target genes when a cell enters a certain metabolic state. In resting cells, for example, GAPDH is a RNA binding protein (RBP). It binds to ARE sequences in the 3'UTR, preventing translation of mRNA. When the cell enters glycolysis, GAPDH is required to convert glucose into ATP, coming off the mRNA allowing for translation of the protein to occur. In some embodiments, the environment in which the cell comprising the recombinant nucleic acid is present, provides the metabolic switch to the gene expression. For example, hypoxic condition can trigger the metabolic switch inducing the disengaging of GAPDH from the mRNA. The expression of the mRNA therefore can be induced when the myeloid cell, such as a macrophage, leaves the circulation and enters into a tumor environment, which is hypoxic. This allows for systemic administration of the nucleic acid or a cell comprising the nucleic acid, but ensures a local expression, specifically targeting the tumor environment.

In some embodiments the nucleic acid construct can be a split construct, for example, allowing a portion of the construct to be expressed under the control of a constitutive expression system whereas another portion of the nucleic acid is expressed under control of a metabolic switch, as described above. In some embodiments the nucleic acid may be under bicistronic control. In some embodiments, the bicistronic vector comprises a first coding sequence under a first regulatory control, comprising the coding sequence of a target recognition moiety which may be under constitutive control; and a second coding sequence encoding an inflammatory gene expression which may be under the metabolic switch. In some embodiments the bicistronic vector may be unidirectional. In some embodiments the bicistronic vector may be bidirectional.

In some embodiments, the ARE sequences comprise protein binding motifs for binding ARE sequence that bind to ADK, ALDH18A1, ALDH6A1, ALDOA, ASS1, CCBL2, CS, DUT, ENO1, FASN, FDPS, GOT2,HADHB, HK2, HSD17B10, MDH2, NME1, NQ01, PKM2, PPP1CC, SUCLGI1, TP11, GAPDH, or LDH.

### *Delivery of nucleic acids into a cell:*

Nucleic acids encoding the CFP or PFP as described herein may be introduced to a cell, e.g. a myeloid cell, via different delivery approaches. A recombinant nucleic acid as described herein may be introduced to a cell in vitro, ex vivo or in vivo. In some embodiments, a nucleic acid is introduced into a myeloid cell in the form of a plasmid or a vector. In some embodiments, the vector is a viral vector. In some embodiments, the vector is an expression vector, for example, a vector comprising one or more promoters, and other regulatory components, including enhancer binding sequence, initiation and terminal codons, a 5'UTR, a 3'UTR comprising a transcript stabilization element, optional conserved regulatory protein binding sequences and others. In some embodiments, the vector is a phage, a cosmid, or an artificial chromosome.

In some embodiments, a vector is introduced or incorporated in the cell by known methods of transfection, such as using lipofectamine, or calcium phosphate, or via physical means such as electroporation or nucleofection. In some embodiments the vector is introduced or incorporated in the cell by infection, a process commonly known as viral transduction.

In some embodiments, the vector for expression of the CFP is of a viral origin. In some embodiments, the recombinant nucleic acid is encoded by a viral vector capable of replicating in non-dividing cells. In some embodiments, the nucleic acid encoding the recombinant nucleic acid is encoded by a lentiviral vector, e.g. HIV and FIV-based vectors. In some embodiments the lentiviral vector is prepared in-house and manufactured in large scale for the purpose. In some embodiments, commercially available lentiviral vectors are utilized, as is known to one of skill in the art. In some embodiments, the recombinant nucleic acid is encoded by a herpes simplex virus vector, a vaccinia virus vector, an adenovirus vector, or an adeno-associated virus (AAV) vector.

In some embodiments, a stable integration of transgenes into myeloid cells, such as macrophages, and other phagocytic cells may be accomplished via the use of a transposase and transposable elements, in particular, mRNA-encoded transposase. In one embodiment, Long Interspersed Element-1 (L1) RNAs may be contemplated for retrotransposition of the transgene and stable integration into myeloid cells, such as macrophages or phagocytic cells. Retrotransposon may be used for stable integration of a recombinant nucleic acid encoding a phagocytic or tethering receptor (PR) fusion protein (PFP).

In some embodiments, the myeloid cell may be modified by expressing a transgene via incorporation of the transgene in a transient expression vector. In some embodiments expression of the transgene may be temporally regulated by a regulator from outside the cell. Examples include the Tet-on Tet-off system, where the expression of the transgene is regulated via presence or absence of tetracycline.

In some embodiments, the recombinant nucleic acid described herein is a circular RNA (circRNA). A circular RNA comprises a RNA molecule where the 5' end and the 3' end of the RNA molecule are joined together. Without wishing to be bound by any theory, circRNAs have no free ends and may have longer half-life as compared to some other forms of RNAs or nucleic acid and may be resistant to digestion with RNase R exonuclease and turn over more slowly than its counterpart linear RNA in vivo. In some embodiments, the half-life of a circRNA is more than 20 hours. In some embodiments, the half-life of a circRNA is more than 30 hours. In some embodiments, the half-life of a circRNA is more than 40 hours. In some embodiments, the half-life of a circRNA is more than 48 hours. In certain embodiments, a circRNA comprises an internal ribosome entry site (IRES) element that engages a eukaryotic ribosome and an RNA sequence element encoding a polypeptide operatively linked to the IRES for insertion into cells in order to produce a polypeptide of interest.

circRNAs can be prepared by methods known to those skilled in the art. For example, circRNAs may be chemically synthesized or enzymatically synthesized. In some embodiments, a linear primary construct or linear mRNA may be cyclized, or concatemerized to create a circRNA. The mechanism of cyclization or concatemerization may occur through methods such as, but not limited to, chemical, enzymatic, or ribozyme catalyzed methods. The newly formed 5'-/3'-linkage may be an intramolecular linkage or an intermolecular linkage. In some embodiments, a linear primary construct or linear mRNA may be cyclized, or concatemerized using the chemical method to form a circRNA. In the chemical method, the 5'-end and the 3'-end of the nucleic acid (e.g., linear primary construct or linear mRNA) contain chemically reactive groups that, when close together, form a new covalent linkage between the 5'-end and the 3'-end of the molecule. The 5'-end may contain an NHS-ester reactive group and the 3'-end may contain a 3'-amino-terminated nucleotide such that in an organic solvent the 3'-amino-terminated nucleotide on the 3'-end of a linear RNA molecule will undergo a nucleophilic attack on the 5'-NHS-ester moiety forming a new 5'-/3'-amide bond. In some embodiments, a DNA or RNA ligase, e.g. a T4 ligase, may be used to enzymatically link a 5'-phosphorylated nucleic acid molecule (e.g., a linear primary construct or linear mRNA) to the 3'-hydroxyl group of a nucleic acid forming a new phosphorodiester linkage. In some embodiments, a linear primary construct or linear mRNA may be cyclized or concatermerized by using at least one non-nucleic acid moiety. For example, the at least one non-nucleic acid moiety may react with regions or features near the 5' terminus and/or near the 3' terminus of the linear primary construct or linear mRNA in order to cyclize or concatermerize the linear primary construct or linear mRNA. In some embodiments, a linear primary construct or linear mRNA may be cyclized or concatermerized due to a non-nucleic acid moiety that causes an attraction between atoms, molecules surfaces at, near or linked to the 5' and 3' ends of the linear primary construct or linear mRNA. For example, a linear primary construct or linear mRNA may be cyclized or concatermized by intermolecular forces or intramolecular forces. Non-limiting examples of intermolecular forces. In some embodiments, a linear primary construct or linear mRNA may comprise a ribozyme RNA sequence near the 5' terminus and near the 3' terminus. In some embodiments, a circRNA may be synthesized by inserting DNA fragments into a plasmid containing sequences having the capability of spontaneous cleavage and self-circularization. In some embodiments, a circRNA is produced by making a DNA construct encoding an RNA cyclase ribozyme, expressing the DNA construct as an RNA, and then allowing the RNA to self-splice, which produces a circRNA free from intron in vitro. In some embodiments, a circRNA is produced by synthesizing a linear polynucleotide, combining the linear nucleotide with a complementary linking oligonucleotide under hybridization conditions, and ligating the linear polynucleotide.

The circRNA may be modified or unmodified. In some embodiments, the circRNA is chemically modified. For example, an A, G, U or C ribonucleotide of a circRNA may comprise chemical modifications. In some embodiments, any region of a circRNA, e.g. the coding region of the CFP or PFP, the flanking regions and/or the terminal regions may contain one, two, or more (optionally different) nucleoside or nucleotide modifications. In some embodiments, a modified circRNA introduced to a cell may exhibit reduced degradation in the cell, as compared to an unmodified circRNA. Modifications such as to the sugar, the nucleobase, or the internucleoside linkage (e.g. to a linking phosphate/to a phosphodiester linkage/to the phosphodiester backbone) are also encompassed. In some embodiments, one or more atoms of nucleobase, e.g. a pyrimidine nucleobase may be replaced or substituted with optionally substituted amino, optionally substituted thiol, optionally substituted alkyl (e.g., methyl or ethyl), or halo (e.g., chloro or fluoro). In certain embodiments, modifications (e.g., one or more modifications) are present in each of the sugar and the internucleoside linkage. Additional modifications to circRNAs are described in US20170204422, the entire content of which is incorporated herein by reference.

In some embodiments, the circRNA is conjugated to other polynucleotides, dyes, intercalating agents (e.g. acridines), cross-linkers (e.g. psoralene, mitomycin C), porphyrins (TPPC4, texaphyrin, Sapphyrin), polycyclic aromatic hydrocarbons (e.g., phenazine, dihydrophenazine), artificial endonucleases (e.g. EDTA), alkylating agents, phosphate, amino, mercapto, PEG (e.g., PEG-40K), MPEG, [MPEG]2, polyamino, alkyl, substituted alkyl, radiolabeled markers, enzymes, haptens (e.g. biotin), transport/absorption facilitators (e.g., aspirin, vitamin E, folic acid), synthetic ribonucleases, proteins, e.g., glycoproteins, or peptides, e.g., molecules having a specific affinity for a co-ligand, or antibodies e.g., an antibody, that binds to a specified cell type such as a cancer cell, endothelial cell, or bone cell, hormones and hormone receptors, non-peptidic species, such as lipids, lectins, carbohydrates, vitamins, or cofactors.

In some embodiments, the circRNA is administered directly to tissues of a subject. Additional description of circRNAs in U.S. Patent No.s 5,766,903, 5,580,859, 5,773,244, 6,210,931, PCT publication No. WO1992001813, Hsu et al., Nature (1979) 280:339-340, Harland & Misher, Development (1988) 102:837-852, Memczak et al. Nature (2013) 495:333-338, Jeck et al., and RNA (2013) 19:141-157, each of which is incorporated herein by reference in its entirety.

In some embodiments, a nucleic acid is introduced into a myeloid cell with a nanoparticle (NP). A nanoparticle may be of various shapes or sizes and may harbor the nucleic acid encoding the CFP or PFP. In some embodiments, the NP is a lipid nanoparticle (LNP). In some embodiments, the NP comprises poly(amino acids), polysaccharides and poly(alpha-hydroxy acids), gold, silver, carbon, iron, silica, or any combination thereof. In some embodiments, the NP comprises a polylactide-co-glycolide (PGLA) particle. In some embodiments, the nucleic acid is encapsulated in the NP, for example, via water/oil emulsion or water-oil-water emulsion. In some embodiments, the nucleic acid is conjugated to the NP.

NPs may be delivered to a cell in vitro, ex vivo or in vivo. In some embodiments, a NP is delivered to a phagocytic cell ex vivo. In some embodiments, a NP is delivered to a phagocytic cell in vivo. In some embodiments, the NP is less than 100nm in diameter. In some embodiments, the NP is more than 100nm in diameter. In some embodiments, the NP is a rod-shaped NP. In some embodiments, the NP is a spherical NP. In particular embodiments, the NP is a spherical NP for delivery to a phagocytic cell. In additional embodiments, the NP is at least 100nm in diameter and does not trigger or triggers reduced toxicity when delivered to a cell.

In some embodiments, the NP is positively charged. In some embodiments, the NP is negatively charged. In some embodiments, the NP is a cationic NP that is delivered and taken up by a myeloid cell ex vivo or in vivo.

Stiffness may affect the biological impact of NPs. NPs made of rigid materials may be associated with increased potential for embolism, while flexible polymer-based NPs that can more easily deform may gain better access to tissues during the complex vascular changes associated with inflammation. The fluidity of NPs, too, affects the ability of antigen-loaded NP to stimulate immune responses. Thus, intramuscular, solid-phase, antigen-containing liposome immunization may elicit a more robust Th1/Th17 response than similarly administered fluid-phase liposomes. Without wishing to be bound by any theory, solid-phase particles may result from the formation of an immobilized antigen particle depot and may result in a prolonged supply of antigen for APCs also associated with upregulation of positive costimulatory molecules such as CD80, which support efficient T cell priming.

In some embodiments, a protein corona may form around NPs. A protein corona may form in a two-step process. In the first step, high-affinity proteins rapidly bind to NPs to form a primary corona. In the second step, proteins of lower affinity bind either directly to the NP or to the proteins in the primary corona forming a secondary corona. Constituents of the protein corona may thus be impacted by the protein content of the serum and thus by the homeostatic or immune responses that regulate it. In some embodiments, proteins with high abundance, such as albumin, comprise a significant proportion of the primary corona. In some embodiments, NPs with different charges bind significant amounts of less-abundant proteins in particular environments, e.g. in plasma with certain antigen or antibody. In vivo formation of a protein corona may alter NP charge or mask functional groups important for NP targeting to certain receptors and/or enhance clearance of NPs by phagocytes. In some embodiments, NPs are engineered to reduce changes to NP charges or masking of functional groups, and/or increase the serum half-life of the NPs. In some embodiments, NP surface coating are designed to modulate opsonization events. For example, the NP's surface may be coated with polymeric ethylene glycol (PEG) or its low molecular weight derivative polyethylene oxide (PEO). Without wishing to be bound by any theory, PEG increases surface hydrophilicity, resulting in improved circulating NP half-life due to reduced serum protein binding. In some embodiments, the NP coated with PEG or PEO are engineered to result in reduced toxicity or increased biocompatibility of the NPs. Additional NP design and NP targeting for myeloid cells are described in Getts et al., Trends Immunol. 36(7): 419-427 (2015), the entirety of which is incorporated herein by reference.

NPs described herein may be used to introduce the recombinant nucleic acid into a cell in in vitro/ex vivo cell culture or administered in vivo. In some embodiments, the NP is modified for in vivo administration. For example, the NP may comprise surface modification or attachment of binding moieties to bind specific toxins, proteins, ligands, or any combination thereof, before being taken up by liver or spleen phagocytes. In recent rodent proof-of-concept studies, infused highly negatively charged 'immune-modifying NPs' (IMPs) can absorb certain blood proteins, including S100 family and heat shock proteins, before finally being removed and destroyed by cells of the mononuclear phagocyte system. Furthermore, this mechanism may also be used to capture and concentrate certain circulating proteins. IMPs have been shown to bind Annexin 1. The accumulation of Annexin 1 and its presentation to particular leukocyte subsets can have broad immune outcomes. For example, Annexin 1-loaded NPs may reduce neutrophils via induction of apoptosis and/or promote T cell activation. In some embodiments, the NP is designed to target a cell surface receptor, e.g. a scavenger receptor. In some embodiments, an NP is a particle with highly negative surface charge.

In some embodiments, the NP encapsulates the nucleic acid wherein the nucleic acid is a naked DNA molecule. In some embodiments, the NP encapsulates the nucleic acid wherein the nucleic acid is an mRNA molecule. In some embodiments, the NP encapsulates the nucleic acid wherein the nucleic acid is a circular RNA (circRNA) molecule. In some embodiments, the NP encapsulates the nucleic acid wherein the nucleic acid is a vector, a plasmid, or a portion or fragment thereof.

In some embodiments, the NP is a Lipid nanoparticle (LNP). LNPs may comprise a polar and or a nonpolar lipid. In some embodiments cholesterol is present in the LNPs for efficient delivery. LNPs are 100-300 nm in diameter provide efficient means of mRNA delivery to various cell types, including myeloid cells, such as macrophages. In some embodiments, LNP may be used to introduce the recombinant nucleic acids into a cell in in vitro cell culture. In some embodiments, the LNP encapsulates the nucleic acid wherein the nucleic acid is a naked DNA molecule. In some embodiments, the LNP encapsulates the nucleic acid wherein the nucleic acid is an mRNA molecule. In some embodiments, the LNP encapsulates the nucleic acid wherein the nucleic acid is inserted in a vector, such as a plasmid vector. In some embodiments, the LNP encapsulates the nucleic acid wherein the nucleic acid is a circRNA molecule.

In some embodiments, the LNP is used to deliver the nucleic acid into the subject. LNP can be used to deliver nucleic acid systemically in a subject. It can be delivered by injection. In some embodiments, the LNP comprising the nucleic acid is injected by intravenous route. In some embodiments the LNP is injected subcutaneously.

## Pharmaceutical Composition

Provided herein is a pharmaceutical composition, comprising engineered myeloid cells, such as macrophages, comprising a recombinant nucleic acid encoding the CFP and a pharmaceutically acceptable excipient.

Also provided herein is a pharmaceutical composition, comprising a recombinant nucleic acid encoding the CFP and a pharmaceutically acceptable excipient. The pharmaceutical composition may comprise DNA, mRNA or circRNA or a liposomal composition of any one of these. The liposome is a LNP.

Also provided herein is a pharmaceutical composition comprising a vector comprising the recombinant nucleic acid encoding the CFP and a pharmaceutically acceptable excipient. The pharmaceutical composition may comprise DNA, mRNA or circRNA inserted in a plasmid vector or a viral vector.

In some embodiments the engineered myeloid cells, such as macrophages, are grown in cell culture sufficient for a therapeutic administration dose, and washed, and resuspended into a pharmaceutical composition.

In some embodiments the excipient comprises a sterile buffer, (e.g. HEPES or PBS) at neutral pH. In some embodiment, the pH of the pharmaceutical composition is at 7.5. In some embodiments, the pH may vary within an acceptable range. In some embodiments, the engineered cells may be comprised in sterile enriched cell suspension medium comprising complement deactivated or synthetic serum. In some embodiments the pharmaceutic composition further comprises cytokines, chemokines or growth factors for cell preservation and function.

In some embodiments, the pharmaceutical composition may comprise additional therapeutic agents, co-administered with the engineered cells.

## Treatment Methods

Provided herein are methods for treating cancer in a subject using a pharmaceutical composition comprising engineered myeloid cells, such as phagocytic cells (e.g., macrophages), expressing a recombinant nucleic acid encoding a CFP, such as a phagocytic receptor (PR) fusion protein (PFP), to target, attack and kill cancer cells directly or indirectly. The engineered myeloid cells, such as phagocytic cells, are also designated as CAR-P cells in the descriptions herein.

Cancers include, but are not limited to T cell lymphoma, cutaneous lymphoma, B cell cancer (e.g., multiple myeloma, Waldenstrom's macroglobulinemia), the heavy chain diseases (such as, for example, alpha chain disease, gamma chain disease, and mu chain disease), benign monoclonal gammopathy, and immunocytic amyloidosis, melanomas, breast cancer, lung cancer, bronchus cancer, colorectal cancer, prostate cancer (e.g., metastatic, hormone refractory prostate cancer), pancreatic cancer, stomach cancer, ovarian cancer, urinary bladder cancer, brain or central nervous system cancer, peripheral nervous system cancer, esophageal cancer, cervical cancer, uterine or endometrial cancer, cancer of the oral cavity or pharynx, liver cancer, kidney cancer, testicular cancer, biliary tract cancer, small bowel or appendix cancer, salivary gland cancer, thyroid gland cancer, adrenal gland cancer, osteosarcoma, chondrosarcoma, cancer of hematological tissues, and the like. Other non-limiting examples of types of cancers applicable to the methods encompassed by the present disclosure include human sarcomas and carcinomas, e.g., fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, colorectal cancer, pancreatic cancer, breast cancer, ovarian cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, liver cancer, choriocarcinoma, seminoma, embryonal carcinoma, Wilms' tumor, cervical cancer, bone cancer, brain tumor, testicular cancer, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, melanoma, neuroblastoma, retinoblastoma; leukemias, e.g., acute lymphocytic leukemia and acute myelocytic leukemia (myeloblastic, promyelocytic, myelomonocytic, monocytic and erythroleukemia); chronic leukemia (chronic myelocytic (granulocytic) leukemia and chronic lymphocytic leukemia); and polycythemia vera, lymphoma (Hodgkin's disease and non-Hodgkin's disease), multiple myeloma, Waldenstrom's macroglobulinemia, and heavy chain disease. In some embodiments, the cancer is an epithelial cancer such as, but not limited to, bladder cancer, breast cancer, cervical cancer, colon cancer, gynecologic cancers, renal cancer, laryngeal cancer, lung cancer, oral cancer, head and neck cancer, ovarian cancer, pancreatic cancer, prostate cancer, or skin cancer. In other embodiments, the cancer is breast cancer, prostate cancer, lung cancer, or colon cancer. In still other embodiments, the epithelial cancer is non-small-cell lung cancer, nonpapillary renal cell carcinoma, cervical carcinoma, ovarian carcinoma (e.g., serous ovarian carcinoma), or breast carcinoma. The epithelial cancers can be characterized in various other ways including, but not limited to, serous, endometrioid, mucinous, clear cell, or undifferentiated. In some embodiments, the present disclosure is used in the treatment, diagnosis, and/or prognosis of lymphoma or its subtypes, including, but not limited to, mantle cell lymphoma. Lymphoproliferative disorders are also considered to be proliferative diseases.

In general, cellular immunotherapy comprises providing the patient a medicament comprising live cells. In some aspects a patient or a subject having cancer, is treated with autologous cells, the method comprising, isolation of PBMC-derived myeloid cells, such as macrophages, modifying the cells ex vivo to generate phagocytic myeloid cells capable of tumor lysis by introducing into the cells a recombinant nucleic acid encoding a CFP, and administering the modified myeloid cells into the subject.

In some aspects, a subject is administered one or more doses of a pharmaceutical composition comprising therapeutic myeloid cells, such as phagocytic cells, wherein the cells are allogeneic. An HLA may be matched for compatibility with the subject, and such that the cells do not lead to graft versus Host Disease, GVHD. A subject arriving at the clinic is HLA typed for determining the HLA antigens expressed by the subject.

HLA-typing is conventionally carried out by either serological methods using antibodies or by PCR-based methods such as Sequence Specific Oligonucleotide Probe Hybridization (SSOP), or Sequence Based Typing (SBT).

The sequence information may be identified by either sequencing methods or methods employing mass spectrometry, such as liquid chromatography-mass spectrometry (LC-MS or LC-MS/MS, or alternatively HPLC-MS or HPLC-MS/MS). These sequencing methods may be well-known to a skilled person and are reviewed in Medzihradszky KF and Chalkley RJ. Mass Spectrom Rev. 2015 Jan-Feb;34(1):43-63.

In some aspects, the phagocytic cell is derived from the subject, transfected or transduced with the recombinant nucleic acid in vitro, expanded in cell culture in vitro for achieving a number suitable for administration, and then administered to the subject. In some embodiments, the steps of transfected or transduced with the recombinant nucleic acid in vitro, expanded in cell culture in vitro for achieving a number suitable for administration takes 2 days, or 3 days, or 4 days or 5 days or 6 days or 7 days or 8 days or 9 days or 10 days.

In some embodiments, sufficient quantities of transfected or transduced myeloid cells, such as macrophages, comprising the recombinant nucleic acid are preserved aseptically, which are administered to the subject as "off the shelf" products after HLA typing and matching the product with the recipients HLA subtypes. In some embodiments, the engineered phagocytes are cryopreserved. In some embodiments, the engineered phagocytes are cryopreserved in suitable media to withstand thawing without considerable loss in cell viability.

In some embodiment, the subject is administered a pharmaceutical composition comprising the DNA, or the mRNA or the circRNA in a vector, or in a pharmaceutically acceptable excipient described above.

In some embodiments the administration of the off the shelf cellular products may be instantaneous, or may require 1 day, 2 days or 3 days or 4 days or 5 days or 6 days or 7 days or more prior to administration. The pharmaceutical composition comprising cell, or nucleic acid may be preserved over time from preparation until use in frozen condition. In some embodiments, the pharmaceutical composition may be thawed once. In some embodiments, the pharmaceutical composition may be thawed more than once. In some embodiments, the pharmaceutical composition is stabilized after a freeze-thaw cycle prior administering to the subject. In some embodiments the pharmaceutical composition is tested for final quality control after thawing prior administration.

In some embodiments, a composition comprising $10^6$ engineered cells are administered per administration dose. In some embodiments, a composition comprising $10^7$ engineered cells are administered per administration dose. In some embodiments, a composition comprising $5 \times 10^7$ engineered cells are administered per administration dose. In some embodiments, a composition comprising $10^8$ engineered cells are administered per administration dose. In some embodiments, a composition comprising $2 \times 10^8$ engineered cells are administered per administration dose. In some embodiments, a composition comprising $5 \times 10^8$ engineered cells are administered per administration dose. In some embodiments, a composition comprising $10^9$ engineered cells are administered per administration dose. In some embodiments, a composition comprising $10^{10}$ engineered cells are administered per administration dose.

In some embodiments, the engineered myeloid cells, such as phagocytic cells, are administered once.

In some embodiments, the engineered myeloid cells, such as phagocytic cells, are administered more than once.

In some embodiments, the engineered myeloid cells, such as phagocytic cells, are twice, thrice, four times, five times, six times, seven times, eight times, nine times, or ten times or more to a subject over a span of time comprising a few months, a year or more.

In some embodiments, the engineered myeloid cells, such as phagocytic cells, are administered twice weekly.

In some embodiments, the engineered myeloid cells, such as phagocytic cells, are administered once weekly.

In some embodiments, the engineered myeloid cells, such as phagocytic cells, are administered once every two weeks.

In some embodiments, the engineered myeloid cells, such as phagocytic cells, are administered once every three weeks.

In some embodiments, the engineered myeloid cells, such as phagocytic cells, are administered once monthly.

In some embodiments, the engineered phagocytic cells are administered once in every 2 months, once in every 3 months, once in every 4 months, once in every 5 months or once in every 6 months.

In some embodiments, the engineered myeloid cells, such as phagocytic cells, are administered by injection.

In some embodiments, the engineered myeloid cells, such as phagocytic cells, are administered by infusion.

**EP 4 678 184 A2**

In some embodiments, the engineered myeloid cells, such as phagocytic cells, are administered by intravenous infusion.

In some embodiments, the engineered myeloid cells, such as phagocytic cells, are administered by subcutaneous infusion.

The pharmaceutical composition comprising the recombinant nucleic acid or the engineered cells may be administered by any route which results in a therapeutically effective outcome. These include, but are not limited to enteral (into the intestine), gastroenteral, epidural (into the dura mater), oral (by way of the mouth), transdermal, peridural, intracerebral (into the cerebrum), intracerebroventricular (into the cerebral ventricles), epicutaneous (application onto the skin), intradermal, (into the skin itself), subcutaneous (under the skin), nasal administration (through the nose), intravenous (into a vein), intravenous bolus, intravenous drip, intraarterial (into an artery), intramuscular (into a muscle), intracardiac (into the heart), intraosseous infusion (into the bone marrow), intrathecal (into the spinal canal), intraperitoneal, (infusion or injection into the peritoneum), intravesical infusion, intravitreal, (through the eye), intracavernous injection (into a pathologic cavity), intracavitary (into the base of the penis), intravaginal administration, intrauterine, extra-amniotic administration, transdermal (diffusion through the intact skin for systemic distribution), transmucosal (diffusion through a mucous membrane), transvaginal, insufflation (snorting), sublingual, sublabial, enema, eye drops (onto the conjunctiva), in ear drops, auricular (in or by way of the ear), buccal (directed toward the cheek), conjunctival, cutaneous, dental (to a tooth or teeth), electro-osmosis, endocervical, endosinusial, endotracheal, extracorporeal, hemodialysis, infiltration, interstitial, intra-abdominal, intra-amniotic, intra-articular, intrabiliary, intrabronchial, intrabursal, intracartilaginous (within a cartilage), intracaudal (within the cauda equine), intracisternal (within the cisterna magna cerebellomedularis), intracorneal (within the cornea), dental intracomal, intracoronary (within the coronary arteries), intracorporus cavernosum (within the dilatable spaces of the corporus cavernosa of the penis), intradiscal (within a disc), intraductal (within a duct of a gland), intraduodenal (within the duodenum), intradural (within or beneath the dura), intraepidermal (to the epidermis), intraesophageal (to the esophagus), intragastric (within the stomach), intragingival (within the gingivae), intraileal (within the distal portion of the small intestine), intralesional (within or introduced directly to a localized lesion), intraluminal (within a lumen of a tube), intralymphatic (within the lymph), intramedullary (within the marrow cavity of a bone), intrameningeal (within the meninges), intraocular (within the eye), intraovarian (within the ovary), intrapericardial (within the pericardium), intrapleural (within the pleura), intraprostatic (within the prostate gland), intrapulmonary (within the lungs or its bronchi), intrasinal (within the nasal or periorbital sinuses), intraspinal (within the vertebral column), intrasynovial (within the synovial cavity of a joint), intratendinous (within a tendon), intratesticular (within the testicle), intrathecal (within the cerebrospinal fluid at any level of the cerebrospinal axis), intrathoracic (within the thorax), intratubular (within the tubules of an organ), intratumor (within a tumor), intratympanic (within the aurus media), intravascular (within a vessel or vessels), intraventricular (within a ventricle), iontophoresis (by means of electric current where ions of soluble salts migrate into the tissues of the body), irrigation (to bathe or flush open wounds or body cavities), laryngeal (directly upon the larynx), nasogastric (through the nose and into the stomach), occlusive dressing technique (topical route administration which is then covered by a dressing which occludes the area), ophthalmic (to the external eye), oropharyngeal (directly to the mouth and pharynx), parenteral, percutaneous, periarticular, peridural, perineural, periodontal, rectal, respiratory (within the respiratory tract by inhaling orally or nasally for local or systemic effect), retrobulbar (behind the pons or behind the eyeball), soft tissue, subarachnoid, subconjunctival, submucosal, topical, transplacental (through or across the placenta), transtracheal (through the wall of the trachea), transtympanic (across or through the tympanic cavity), ureteral (to the ureter), urethral (to the urethra), vaginal, caudal block, diagnostic, nerve block, biliary perfusion, cardiac perfusion, photopheresis or spinal. In specific embodiments, compositions may be administered in a way which allows them cross the blood-brain barrier, vascular barrier, or other epithelial barrier.

In some embodiments, the subject is administered a pharmaceutical composition comprising the nucleic acid encoding the CFP or PFP as described herein. In some embodiments, the subject is administered a pharmaceutical composition comprising DNA, mRNA, or circRNA. In some embodiments, the subject is administered a vector harboring the nucleic acid, e.g., DNA, mRNA, or circRNA. In some embodiments, the nucleic acid is administered or in a pharmaceutically acceptable excipient described above.

In some embodiments, the subject is administered a nanoparticle (NP) associated with the nucleic acid, e.g. a DNA, an mRNA, or a circRNA encoding the CFP or PFP as described herein. In some embodiments, the nucleic acid is encapsulated in the nanoparticle. In some embodiments, the nucleic acid is conjugated to the nanoparticle. In some embodiments, the NP is a polylactide-co-glycolide (PGLA) particle. In some embodiments, the NP is administered subcutaneously. In some embodiments, the NP is administered intravenously. In some embodiments, the NP is engineered in relation to the administration route. For example, the size, shape, or charges of the NP maybe engineered according to the administration route. In some embodiments, subcutaneously administered NPs are less than 200nm in size. In some embodiments, subcutaneously administered NPs are more than 200nm in size. In some embodiments, subcutaneously administered NPs are at least 30nm in size. In some embodiments, the NPs are intravenously infused. In some embodiments, intravenously infused NPs are at least 5nm in diameter. In some embodiments, intravenously infused NPs are at least 30nm in diameter. In some embodiments, intravenously infused NPs are at least 100nm in diameter. In certain embodiments, the administered NPs, e.g. intravenously administered NPs, are engulfed by circulating monocytes. Additional NP design and administration approaches are described in Getts et al., Trends Immunol. 36(7): 419-427 (2015), the entirety of which is incorporated herein by reference.

In some embodiments, the subject is administered a pharmaceutical composition comprising a circRNA encoding the CFP or PFP as described herein. The circRNA may be administered in any route as described herein. In some embodiments, the circRNA may be directly infused. In some embodiments, the circRNA may be in a formulation or solution comprising one or more of sodium chloride, calcium chloride, phosphate and/or EDTA. In some embodiment, the circRNA solution may include one or more of saline, saline with 2 mM calcium, 5% sucrose, 5% sucrose with 2 mM calcium, 5% Mannitol, 5% Mannitol with 2 mM calcium, Ringer's lactate, sodium chloride, sodium chloride with 2 mM calcium and mannose. In some embodiments, the circRNA solution is lyophilized. The amount of each component may be varied to enable consistent, reproducible higher concentration saline or simple buffer formulations. The components may also be varied in order to increase the stability of circRNA in the buffer solution over a period of time and/or under a variety of conditions. In some embodiments, the circRNA is formulated in a lyophilized gel-phase liposomal composition. In some embodiments, the circRNA formulation comprises a bulking agent, e.g. sucrose, trehalose, mannitol, glycine, lactose and/or raffinose, to impart a desired consistency to the formulation and/or stabilization of formulation components. Additional formulation and administration approaches for circRNA as described in US Publications No. US2012060293, and US20170204422 are herein incorporated by reference in entirety.

In some embodiments, the subject is administered a pharmaceutical composition comprising a mRNA encoding the CFP or PFP as described herein. In some embodiments, the mRNA is co-formulated into nanoparticles (NPs), such as lipid nanoparticles (LNPs). For example, the LNP may comprise cationic lipids or ionizable lipids. In some embodiments, the mRNA is formulated into polymeric particles, for example, polyethyleneimine particles, poly(glycoamidoamine), ly($\beta$-amino)esters (PBAEs), PEG particles, ceramide-PEGs, polyamindoamine particles, or polylactic-co-glycolic acid particles (PLGA). In some embodiments, the mRNA is administered by direct injection. In some embodiments, the mRNA is complexed with transfection agents, e.g. Lipofectamine 2000, jetPEI, RNAiMAX, or Invivofectamine.

The mRNA may be a naked mRNA. The mRNA may be modified or unmodified. For example, the mRNA may be chemically modified. In some embodiments, nucleobases and/or sequences of the mRNA are modified to increase stability and half-life of the mRNA. In some embodiments, the mRNA is glycosylated. Additional mRNA modification and delivery approaches as described in Flynn et al., BioRxiv 787614 (2019) and Kowalski et al. Mol. Ther. 27(4): 710-728 (2019) are each incorporated herein by reference in its entirety.

## EMBODIMENTS

1. A composition comprising a recombinant nucleic acid encoding a phagocytic or tethering receptor (PR) fusion protein (PFP) comprising: (a) a PR subunit comprising: (i) a transmembrane domain, and (ii) an intracellular domain comprising an intracellular signaling domain; and (b) an extracellular domain comprising an antigen binding domain specific to an antigen of a target cell; wherein the transmembrane domain and the extracellular domain are operatively linked; and wherein upon binding of the PFP to the antigen of the target cell, the killing or phagocytosis activity of a cell expressing the PFP is increased by at least greater than 20% compared to a cell not expressing the PFP.

2. The composition of embodiment 1, wherein the intracellular signaling domain is derived from a phagocytic or tethering receptor or wherein the intracellular signaling domain comprises a phagocytosis activation domain.

3. The composition of embodiment 1 or 2, wherein the intracellular signaling domain is derived from a receptor other than a phagocytic receptor selected from Megf10, MerTk, FcR-alpha, or Bail.

4. The composition of any one of embodiments 1-3, wherein the intracellular signaling domain is derived from a receptor selected from the group consisting of the receptors listed in Table 2.

5. The composition of any one of embodiments 1-4, wherein the intracellular signaling domain comprises a pro-inflammatory signaling domain.

6. The composition of embodiment 5, wherein the intracellular signaling domain comprises a pro-inflammatory signaling domain that is not a PI3K recruitment domain.

7. A composition comprising a recombinant nucleic acid encoding a phagocytic or tethering receptor (PR) fusion protein (PFP) comprising: (a) a PR subunit comprising: (i) a transmembrane domain, and (ii) an intracellular domain comprising an intracellular signaling domain; and (b) an extracellular domain comprising an antigen binding domain specific to an antigen of a target cell; wherein the transmembrane domain and the extracellular domain are operatively linked; and wherein the intracellular signaling domain is derived from a phagocytic receptor other than a phagocytic receptor selected from Megf10, MerTk, FcR-alpha, or Bail.

8. The composition of embodiment 7, wherein upon binding of the PFP to the antigen of the target cell, the killing activity of a cell expressing the PFP is increased by at least greater than 20% compared to a cell not expressing the PFP.

9. The composition of embodiment 7 or 8, wherein the intracellular signaling domain is derived from a phagocytic receptor selected from the group consisting of lectin, dectin 1, CD206, scavenger receptor A1 (SRA1), MARCO, CD36, CD163, MSR1, SCARA3, COLEC12, SCARA5, SCARB1, SCARB2, CD68, OLR1, SCARF1, SCARF2, CXCL16, STAB1, STAB2, SRCRB4D, SSC5D, CD205, CD207, CD209, RAGE, CD14, CD64, F4/80, CCR2, CX3CR1, CSF1R, Tie2, HuCRIg(L), CD64, CD32a, CD16a, CD89, Fc-alpha receptor I, CR1, CD35, CD3ζ, CR3, CR4, Tim-1, Tim-4 and CD169.

10. The composition of any one of embodiments 7-9, wherein the intracellular signaling domain comprises a pro-inflammatory signaling domain.

11. A composition comprising a recombinant nucleic acid encoding a phagocytic or tethering receptor (PR) fusion protein (PFP) comprising: (a) a PR subunit comprising: (i) a transmembrane domain, and (ii) an intracellular domain comprising an intracellular signaling domain; and (b) an extracellular domain comprising an antigen binding domain specific to an antigen of a target cell; wherein the transmembrane domain and the extracellular domain are operatively linked; and wherein the intracellular signaling domain is derived from a phagocytic receptor selected from the group consisting of lectin, dectin 1, CD206, scavenger receptor A1 (SRA1), MARCO, CD36, CD163, MSR1, SCARA3, COLEC12, SCARA5, SCARB1, SCARB2, CD68, OLR1, SCARF1, SCARF2, CXCL16, STAB1, STAB2, SRCRB4D, SSC5D, CD205, CD207, CD209, RAGE, CD14, CD64, F4/80, CCR2, CX3CR1, CSF1R, Tie2, HuCRIg(L), CD64, CD32a, CD16a, CD89, Fc-alpha receptor I, CR1, CD35, CD3ζ, CR3, CR4, Tim-1, Tim-4 and CD169.

12. The composition of embodiment 11, wherein upon binding of the PFP to the antigen of the target cell, the killing activity of a cell expressing the PFP is increased by at least greater than 55% compared to a cell not expressing the PFP.

13. The composition of embodiment 11 or 12, wherein the intracellular signaling domain is derived from a phagocytic receptor other than a phagocytic receptor selected from Megf10, MerTk, FcR-alpha, or Bail.

14. The composition of any one of embodiments 11-13, wherein the intracellular signaling domain comprises a pro-inflammatory signaling domain

15. The composition of embodiment 14, wherein the intracellular signaling domain comprises a pro-inflammatory signaling domain that is not a PI3K recruitment domain.

16. A composition comprising a recombinant nucleic acid encoding a phagocytic or tethering receptor (PR) fusion protein (PFP) comprising: (a) a PR subunit comprising: (i) a transmembrane domain, and (ii) an intracellular domain comprising an intracellular signaling domain; and (b) an extracellular domain comprising an antigen binding domain specific to an antigen of a target cell; wherein the transmembrane domain and the extracellular domain are operatively linked; and wherein the intracellular signaling domain comprises a pro-inflammatory signaling domain that is not a PI3K recruitment domain.

17. The composition of embodiment 16, wherein upon binding of the PFP to the antigen of the target cell, the killing activity of a cell expressing the PFP is increased by at least greater than 20% compared to a cell not expressing the PFP.

18. The composition of embodiment 16 or 17, wherein the intracellular signaling domain is derived from a phagocytic receptor.

19. The composition of any one of embodiments 16-18, wherein the intracellular signaling domain is derived from a phagocytic receptor other than a phagocytic receptor selected from Megf10, MerTk, FcR-alpha, or Bai1.

20. The composition of any one of embodiments 16-19, wherein the intracellular signaling domain is derived from a phagocytic receptor selected from the group consisting of lectin, dectin 1, CD206, scavenger receptor A1 (SRA1), MARCO, CD36, CD163, MSR1, SCARA3, COLEC12, SCARA5, SCARB1, SCARB2, CD68, OLR1, SCARF1, SCARF2, CXCL16, STAB1, STAB2, SRCRB4D, SSC5D, CD205, CD207, CD209, RAGE, CD14, CD64, F4/80, CCR2, CX3CR1, CSF1R, Tie2, HuCRIg(L), CD64, CD32a, CD16a, CD89, Fc-alpha receptor I, CR1, CD35, CD3ζ, CR3, CR4, Tim-1, Tim-4 and CD169.

21. The composition of any one of embodiments 1-15, wherein the intracellular signaling domain comprises a PI3K recruitment domain.

22. The composition of any one of the preceding embodiments, wherein the PFP functionally incorporates into a cell membrane of a cell when the PFP is expressed in the cell.

23. The composition of any one of the preceding embodiments, wherein a cell expressing the PFP exhibits an increase in phagocytosis of a target cell expressing the antigen compared to a cell not expressing the PFP.

24. The composition of embodiment 23, wherein a cell expressing the PFP exhibits at least a 1.1-fold increase in phagocytosis of a target cell expressing the antigen compared to a cell not expressing the PFP.

25. The composition of any one of the preceding embodiments, wherein a cell expressing the PFP exhibits at least a 2-fold, 3-fold, 4-fold, 5-fold, 6-fold, 7-fold, 8-fold, 9-fold, 10-fold, 20-fold, 30-fold or 50-fold increase in phagocytosis of a target cell expressing the antigen compared to a cell not expressing the PFP.

26. The composition of any one of the preceding embodiments, wherein the target cell expressing the antigen is a cancer cell.

27. The composition of any one of the preceding embodiments, wherein the target cell expressing the antigen is at least 0.8 microns in diameter.

28. The composition of any one of the preceding embodiments, wherein the intracellular signaling domain is derived from a scavenger receptor.

29. The composition of any one of the preceding embodiments, wherein a cell expressing the PFP exhibits an increase in production of a cytokine compared to a cell not expressing the PFP.

30. The composition according to embodiment 29, wherein the cytokine is selected from the group consisting of IL-1, IL3, IL-6, IL-12, IL-13, IL-23, TNF, CCL2, CXCL9, CXCL10, CXCL11, IL-18, IL-23, IL-27, CSF, MCSF, GMCSF, IL17, IP-10, RANTES, an interferon and combinations thereof.

31. The composition of any one of the preceding embodiments, wherein a cell expressing the PFP exhibits an increase in effector activity compared to a cell not expressing the PFP.

32. The composition of any one of the preceding embodiments, wherein a cell expressing the PFP exhibits an increase in cross-presentation compared to a cell not expressing the PFP.

33. The composition of any one of the preceding embodiments, wherein a cell expressing the PFP exhibits an increase in expression of an MHC class II protein compared to a cell not expressing the PFP

34. The composition of any one of the preceding embodiments, wherein a cell expressing the PFP exhibits an increase in expression of CD80 compared to a cell not expressing the PFP.

35. The composition of any one of the preceding embodiments, wherein a cell expressing the PFP exhibits an increase in expression of CD86 compared to a cell not expressing the PFP.

36. The composition of any one of the preceding embodiments, wherein a cell expressing the PFP exhibits an increase in expression of MHC class I protein compared to a cell not expressing the PFP.

37. The composition of any one of the preceding embodiments, wherein a cell expressing the PFP exhibits an increase in expression of TRAIL/TNF Family death receptors compared to a cell not expressing the PFP.

38. The composition of any one of the preceding embodiments, wherein a cell expressing the PFP exhibits an increase in expression of B7-H2 compared to a cell not expressing the PFP.

39. The composition of any one of the preceding embodiments, wherein a cell expressing the PFP exhibits an increase in expression of LIGHT compared to a cell not expressing the PFP.

40. The composition of any one of the preceding embodiments, wherein a cell expressing the PFP exhibits an increase in expression of HVEM compared to a cell not expressing the PFP.

41. The composition of any one of the preceding embodiments, wherein a cell expressing the PFP exhibits an increase in expression of CD40 compared to a cell not expressing the PFP.

42. The composition of any one of the preceding embodiments, wherein a cell expressing the PFP exhibits an increase in expression of TL1A compared to a cell not expressing the PFP.

43. The composition of any one of the preceding embodiments, wherein a cell expressing the PFP exhibits an increase in expression of 41BBL compared to a cell not expressing the PFP.

44. The composition of any one of the preceding embodiments, wherein a cell expressing the PFP exhibits an increase in expression of OX40L compared to a cell not expressing the PFP.

45. The composition of any one of the preceding embodiments, wherein a cell expressing the PFP exhibits an increase in expression of GITRL death receptors compared to a cell not expressing the PFP.

46. The composition of any one of the preceding embodiments, wherein a cell expressing the PFP exhibits an increase in expression of CD30L compared to a cell not expressing the PFP.

47. The composition of any one of the preceding embodiments, wherein a cell expressing the PFP exhibits an increase in expression of TIM4 compared to a cell not expressing the PFP.

48. The composition of any one of the preceding embodiments, wherein a cell expressing the PFP exhibits an increase in expression of TIM1 ligand compared to a cell not expressing the PFP.

49. The composition of any one of the preceding embodiments, wherein a cell expressing the PFP exhibits an increase in expression of SLAM compared to a cell not expressing the PFP.

50. The composition of any one of the preceding embodiments, wherein a cell expressing the PFP exhibits an increase in expression of CD48 compared to a cell not expressing the PFP.

51. The composition of any one of the preceding embodiments, wherein a cell expressing the PFP exhibits an increase in expression of CD58 compared to a cell not expressing the PFP.

52. The composition of any one of the preceding embodiments, wherein a cell expressing the PFP exhibits an increase in expression of CD155 compared to a cell not expressing the PFP.

53. The composition of any one of the preceding embodiments, wherein a cell expressing the PFP exhibits an increase in expression of CD112 compared to a cell not expressing the PFP.

54. The composition of any one of the preceding embodiments, wherein a cell expressing the PFP exhibits an increase in expression of PDL1 compared to a cell not expressing the PFP.

55. The composition of any one of the preceding embodiments, wherein a cell expressing the PFP exhibits an increase in expression of B7-DC compared to a cell not expressing the PFP.

56. The composition of any one of the preceding embodiments, wherein a cell expressing the PFP exhibits an increase in respiratory burst compared to a cell not expressing the PFP.

57. The composition of any one of the preceding embodiments, wherein a cell expressing the PFP exhibits an increase in ROS production compared to a cell not expressing the PFP.

58. The composition of any one of the preceding embodiments, wherein a cell expressing the PFP exhibits an increase in iNOS production compared to a cell not expressing the PFP.

59. The composition of any one of the preceding embodiments, wherein a cell expressing the PFP exhibits an increase in iNOS production compared to a cell not expressing the PFP.

60. The composition of any one of the preceding embodiments, wherein a cell expressing the PFP exhibits an increase in extra-cellular vesicle production compared to a cell not expressing the PFP.

61. The composition of any one of the preceding embodiments, wherein a cell expressing the PFP exhibits an increase in trogocytosis with a target cell expressing the antigen compared to a cell not expressing the PFP.

62. The composition of any one of the preceding embodiments, wherein a cell expressing the PFP exhibits an increase in resistance to CD47 mediated inhibition of phagocytosis compared to a cell not expressing the PFP.

63. The composition of any one of the preceding embodiments, wherein a cell expressing the PFP exhibits an increase in resistance to LILRB1 mediated inhibition of phagocytosis compared to a cell not expressing the PFP.

64. The composition of any one of the preceding embodiments, wherein the intracellular domain comprises a Rac inhibition domain, a Cdc42 inhibition domain or a GTPase inhibition domain.

65. The composition of embodiment 64, wherein the Rac inhibition domain, the Cdc42 inhibition domain or the GTPase inhibition domain inhibits Rac, Cdc42 or GTPase at a phagocytic cup of a cell expressing the PFP.

66. The composition of any one of the preceding embodiments, wherein the intracellular domain comprises an F-actin disassembly activation domain, a ARHGAP12 activation domain, a ARHGAP25 activation domain or a SH3BP1 activation domain

67. The composition of any one of the preceding embodiments, wherein a cell expressing the PFP exhibits an increase in phosphatidylinositol 3,4,5-trisphosphate production.

68. The composition of any one of the preceding embodiments, wherein the extracellular domain comprises an Ig binding domain.

69. The composition of any one of the preceding embodiments, wherein the extracellular domain comprises an IgA, IgD, IgE, IgG, IgM, FcRγI, FcRγIIA, FcRγIIB, FcRγIIC, FcRγIIIA, FcRγIIIB, FcRn, TRIM21, FcRLS binding domain.

70. The composition of any one of the preceding embodiments, wherein the extracellular domain comprises an FcR extracellular domain.

71. The composition of any one of the preceding embodiments, wherein the extracellular domain comprises an FcR-alpha, FcRβ , FcRε or FcRγ extracellular domain.

72. The composition of any one of the preceding embodiments, wherein the extracellular domain comprises an FcRα (FCAR) extracellular domain.

73. The composition of any one of the preceding embodiments, wherein the extracellular domain comprises an FcRβ extracellular domain.

74. The composition of any one of the preceding embodiments, wherein the extracellular domain comprises an FcRε (FCER1A) extracellular domain.

75. The composition of any one of the preceding embodiments, wherein the extracellular domain comprises an FcRγ (FDGR1A, FCGR2A, FCGR2B, FCGR2C, FCGR3A, FCGR3B) extracellular domain.

76. The composition of any one of the preceding embodiments, wherein the extracellular domain comprises an integrin domain.

77. The composition of any one of the preceding embodiments, wherein the extracellular domain comprises one or more integrin α1, α2, αIIb, α3, α4, α5, α6, α7, α8, α9, α10, α11, αD, αE, αL, αM, αV, αX, β1, β2, β3, β4, β5, β6, β7, or β8 domains.

78. The composition of any one of the preceding embodiments, wherein the intracellular domain comprises a CD47 inhibition domain.

79. The composition of any one of the preceding embodiments, wherein the PSR subunit further comprises an extracellular domain operatively linked to the transmembrane domain and the extracellular antigen binding domain.

80. The composition of embodiment 79, wherein the extracellular domain further comprises an extracellular domain of a receptor, a hinge, a spacer or a linker.

81. The composition of embodiment 80, wherein the extracellular domain comprises an extracellular portion of a PSR.

82. The composition of embodiment 81, wherein the extracellular portion of the PSR is derived from the same PSR as the PSR intracellular signaling domain.

83. The composition of any one of the embodiments 79-82, wherein the extracellular domain comprises an extracellular domain of a scavenger receptor or an immunoglobulin domain.

84. The composition of embodiment 83, wherein the immunoglobulin domain comprises an extracellular domain of an immunoglobulin or an immunoglobulin hinge region.

85. The composition of any one of the embodiments 79-84, wherein the extracellular domain comprises a phagocytic engulfment marker.

86. The composition of any one of the embodiments 79-85, wherein the extracellular domain comprises a structure capable of multimeric assembly.

87. The composition of any one of the embodiments 79-86, wherein the extracellular domain comprises a scaffold for multimerization.

88. The composition of any one of the preceding embodiments, wherein the extracellular domain is at least 10, 20, 30, 40, 50, 60, 70, 80, 90, 100, 150, 200, 300, 300, 400, or 500 amino acids in length.

89. The composition of any one of the preceding embodiments, wherein the extracellular domain is at most 500, 400, 300, 200, or 100 amino acids in length.

90. The composition of any one of the preceding embodiments, wherein the extracellular antigen binding domain specifically binds to the antigen of a target cell.

91. The composition of any one of the preceding embodiments, wherein the extracellular antigen binding domain comprises an antibody domain.

92. The composition of any one of the preceding embodiments, wherein the extracellular antigen binding domain comprises a receptor domain, antibody domain, wherein the antibody domain comprises a functional antibody fragment, a single chain variable fragment (scFv), an Fab, a single-domain antibody (sdAb), a nanobody, a $V_H$ domain, a $V_L$ domain, a VNAR domain, a $V_{HH}$ domain, a bispecific antibody, a diabody, or a functional fragment or a combination thereof.

93. The composition of any one of any one of the preceding embodiments, wherein the extracellular antigen binding domain comprises a ligand, an extracellular domain of a receptor or an adaptor.

94. The composition of any one of the preceding embodiments, wherein the extracellular antigen binding domain comprises a single extracellular antigen binding domain that is specific for a single antigen.

95. The composition of any one of any one of the preceding embodiments, wherein the extracellular antigen binding domain comprises at least two extracellular antigen binding domains, wherein each of the at least two extracellular antigen binding domains is specific for a different antigen.

96. The composition of any one of the preceding embodiments, wherein the antigen is a cancer antigen or a pathogenic antigen or an autoimmune antigen.

97. The composition of any one of the preceding embodiments, wherein the antigen comprises a viral antigen.

98. The composition of any one of the preceding embodiments, wherein the antigen is a T-lymphocyte antigen.

99. The composition of any one of the preceding embodiments, wherein the antigen is an extracellular antigen.

100. The composition of any one of the preceding embodiments, wherein the antigen is an intracellular antigen.

101. The composition of any one of the preceding embodiments, wherein the antigen is selected from the group consisting of Thymidine Kinase (TK1), Hypoxanthine-Guanine Phosphoribosyltransferase (HPRT), Receptor Tyrosine Kinase-Like Orphan Receptor 1 (ROR1), Mucin-1, Mucin-16 (MUC16), MUC1, Epidermal Growth Factor Receptor vIII (EGFRvIII), Mesothelin, Human Epidermal Growth Factor Receptor 2 (HER2), Mesothelin, EBNA-1, LEMD1, Phosphatidyl Serine, Carcinoembryonic Antigen (CEA), B-Cell Maturation Antigen (BCMA), Glypican 3 (GPC3), Follicular Stimulating Hormone receptor, Fibroblast Activation Protein (FAP), Erythropoietin-Producing Hepatocellular Carcinoma A2 (EphA2), EphB2, a Natural Killer Group 2D (NKG2D) ligand, Disialoganglioside 2 (GD2), CD2, CD3, CD4, CD5, CD7, CD8, CD19, CD20, CD22, CD24, CD30, CD33, CD38, CD44v6, CD45, CD56CD79b, CD97, CD117, CD123, CD133, CD138, CD171, CD179a, CD213A2, CD248, CD276, PSCA, CS-1, CLECL1, GD3, PSMA, FLT3, TAG72, EPCAM, IL-1, an integrin receptor, PRSS21, VEGFR2, PDGFR-$\beta$, SSEA-4, EGFR, NCAM, prostase, PAP, ELF2M, GM3, TEM7R, CLDN6, TSHR, GPRC5D, ALK, IGLL1 and combinations thereof.

102. The composition of any one of the preceding embodiments, wherein the antigen is selected from the group consisting of CD2, CD3, CD4, CD5, CD7, CCR4, CD8, CD30, CD45, CD56.

103. The composition of any one of the preceding embodiments, wherein the antigen is an ovarian cancer antigen or a T lymphoma antigen.

104. The composition of any one of the preceding embodiments, wherein the antigen is an integrin receptor.

105. The composition of any one of the preceding embodiments, wherein the antigen is an integrin receptor selected from the group consisting of $\alpha 1$, $\alpha 2$, $\alpha IIb$, $\alpha 3$, $\alpha 4$, $\alpha 5$, $\alpha 6$, $\alpha 7$, $\alpha 8$, $\alpha 9$, $\alpha 10$, $\alpha 11$, $\alpha D$, $\alpha E$, $\alpha L$, $\alpha M$, $\alpha V$, $\alpha X$, $\beta 1$, $\beta 2$, $\beta 3$, $\beta 4$, $\beta 5$, $\beta 6$, $\beta 7$, and $\beta 8$.

106. The composition of any one of the preceding embodiments, wherein the antigen comprises 2 or more antigens.

107. The composition of any one of the preceding embodiments, wherein the transmembrane domain and the extracellular antigen binding domain is operatively linked through a linker.

108. The composition of any one of the preceding embodiments, wherein the transmembrane domain and the extracellular antigen binding domain is operatively linked through a linker such as the hinge region of CD8α, IgG1 or IgG4.

109. The composition of any one of the preceding embodiments, wherein the extracellular domain comprises a multimerization scaffold.

110. The composition of any one of the preceding embodiments, wherein the transmembrane domain comprises an FcR transmembrane domain.

111. The composition of any one of the preceding embodiments, wherein the transmembrane domain comprises an FcR-ε with no more than 20, 10 or 5 modifications transmembrane domain.

112. The composition of any one of the preceding embodiments, wherein the transmembrane domain comprises a transmembrane domain from a syntaxin such as syntaxin 3 or syntaxin 4 or syntaxin 5.

113. The composition of any one of the preceding embodiments, wherein the transmembrane domain oligomerizes with a transmembrane domain of an endogenous receptor when the PFP is expressed in a cell.

114. The composition of any one of the preceding embodiments, wherein the transmembrane domain oligomerizes with a transmembrane domain of an exogenous receptor when the PFP is expressed in a cell.

115. The composition of any one of the preceding embodiments, wherein the transmembrane domain dimerizes with a transmembrane domain of an endogenous receptor when the PFP is expressed in a cell.

116. The composition of any one of the preceding embodiments, wherein the transmembrane domain dimerizes with a transmembrane domain of an exogenous receptor when the PFP is expressed in a cell.

117. The composition of any one of the preceding embodiments, wherein the transmembrane domain is derived from a protein that is different than the protein from which the intracellular signaling domain is derived.

118. The composition of any one of the preceding embodiments, wherein the transmembrane domain is derived from a protein that is different than the protein from which the extracellular domain is derived.

119. The composition of any one of the preceding embodiments, wherein the transmembrane domain comprises a transmembrane domain of a phagocytic receptor.

120. The composition of any one of the preceding embodiments, wherein the transmembrane domain and the extracellular domain are derived from the same protein.

121. The composition of any one of the preceding embodiments, wherein the transmembrane domain is derived from the same protein as the intracellular signaling domain.

122. The composition of any one of the preceding embodiments, wherein the recombinant nucleic acid encodes a DAP12 recruitment domain.

123. The composition of any one of the preceding embodiments, wherein the transmembrane domain comprises a transmembrane domain that oligomerizes with DAP12.

124. The composition of any one of the preceding embodiments, wherein the transmembrane domain is at least 12, 13, 14, 15, 16, 17,18, 19, 20, 21, 22, 23, 24, 25, 26, 27, 28, 29, 30, 31 or 32 amino acids in length.

125. The composition of any one of the preceding embodiments, wherein the transmembrane domain is at most 12, 13, 14, 15, 16, 17,18, 19, 20, 21, 22, 23, 24, 25, 26, 27, 28, 29, 30, 31 or 32 amino acids in length.

126. The composition of any one of the preceding embodiments, wherein the intracellular domain comprises a phosphatase inhibition domain.

127. The composition of any one of the preceding embodiments, wherein the intracellular domain comprises an ARP2/3 inhibition domain.

128. The composition of any one of the preceding embodiments, wherein the intracellular domain comprises at least one ITAM domain.

129. The composition of any one of the preceding embodiments, wherein the intracellular domain comprises at least 1, 2, 3, 4, 5, 6, 7, 8, 9, or 10 ITAM domains.

130. The composition of any one of the preceding embodiments, wherein the intracellular domain further comprises at least one ITAM domain.

131. The composition of any one of the preceding embodiments, wherein the intracellular domain further comprises at least one ITAM domain select from a group CD3 zeta TCR subunit, CD3 epsilon TCR subunit, CD3 gamma TCR subunit, CD3 delta TCR subunit, TCR zeta chain, Fc epsilon receptor 1 chain, Fc epsilon receptor 2 chain, Fc gamma receptor 1 chain, Fc gamma receptor 2a chain, Fc gamma receptor 2b 1 chain, Fc gamma receptor 2b2 chain, Fc gamma receptor 3a chain, Fc gamma receptor 3b chain, Fc beta receptor 1 chain, TYROBP (DAP12), CD5, CD16a, CD16b, CD22, CD23, CD32, CD64, CD79a, CD79b, CD89, CD278, CD66d, functional fragments thereof, and amino acid sequences thereof having at least one but not more than 20 modifications thereto.

132. The composition of embodiment 129, wherein the at least one ITAM domain comprises a Src-family kinase phosphorylation site.

133. The composition of embodiment 129, wherein the at least one ITAM domain comprises a Syk recruitment domain.

134. The composition of any one of the preceding embodiments, wherein the intracellular domain comprises a F-actin depolymerization activation domain.

135. The composition of any one of the preceding embodiments, wherein the intracellular domain lacks enzymatic activity.

136. The composition of any one of the preceding embodiments, wherein the intracellular domain does not comprise a domain derived from a CD3 zeta intracellular domain.

137. The composition of any one of the preceding embodiments, wherein the intracellular domain comprises a CD47 inhibition domain.

138. The composition of any one of the preceding embodiments, wherein the intracellular signaling domain comprises a domain that activate integrin such as the intracellular region of PSGL-1.

139. The composition of any one of the preceding embodiments, wherein the intracellular signaling domain comprises a domain that activate Rap1 GTPase, such as that from EPAC and C3G.

140. The composition of any one of the preceding embodiments, wherein the intracellular signaling domain are from paxillin.

141. The composition of any one of the preceding embodiments, wherein the intracellular signaling domain activates focal adhesion kinase.

142. The composition of any one of the preceding embodiments, wherein the intracellular signaling domain is derived from a single phagocytic receptor.

143. The composition of any one of the preceding embodiments, wherein the intracellular signaling domain is derived from a single scavenger receptor.

144. The composition of any one of the preceding embodiments, wherein the intracellular domain further comprises a phagocytosis enhancing domain.

145. The composition of any one of the preceding embodiments, wherein the intracellular domain comprises a pro-inflammatory signaling domain.

146. The composition of embodiment 145, wherein the pro-inflammatory signaling domain comprises a kinase activation domain or a kinase binding domain.

147. The composition of embodiment 145 or 146, wherein the pro-inflammatory signaling domain comprises an IL-1 signaling cascade activation domain.

148. The composition of any one of embodiments 145-147, the pro-inflammatory signaling domain comprises an intracellular signaling domain derived from TLR3, TLR4, TLR7, TLR 9, TRIF, RIG-1, MYD88, MAL, IRAK1, MDA-5, an IFN-receptor, an NLRP family member, NLRP1-14, NOD1, NOD2, Pyrin, AIM2, NLRC4, FCGR3A, FCERIG, CD40, a caspase domain or a procaspase binding domain or any combination thereof.

149. The composition of any one of the preceding embodiments, wherein the PFP does not comprise a full length intracellular signaling domain.

150. The composition of any one of the preceding embodiments, wherein the intracellular domain is at least 5, 10, 20, 30, 40, 50, 60, 70, 80, 90, 100, 150, 200, 300, 300, 400, or 500 amino acids in length.

151. The composition of any one of the preceding embodiments, wherein the intracellular domain is at most 10, 20, 30, 40, 50, 60, 70, 80, 90, 100, 150, 200, 300, 300, 400, or 500 amino acids in length.

152. The composition of any one of the preceding embodiments, wherein the recombinant nucleic acid encodes an FcRα chain extracellular domain, an FcRα chain transmembrane domain and/or an FcRα chain intracellular domain.

153. The composition of any one of the preceding embodiments, wherein the recombinant nucleic acid encodes an FcRβ chain extracellular domain, an FcRβ chain transmembrane domain and/or an FcRβ chain intracellular domain.

154. The composition of embodiment 152 or 153, wherein the FcRα chain or the FcRβ chain forms a complex with FcRγ when expressed in a cell.

155. The composition of embodiment 154, wherein the FcRα chain or FcRβ chain forms a complex with endogenous FcRγ when expressed in a cell.

156. The composition of any one of embodiments 152-155, wherein the FcRα chain or the FcRβ chain does not incorporate into a cell membrane of a cell that does not express FcRγ.

157. The composition of any one of embodiments 152-156, wherein the PFP does not comprise an FcRα chain intracellular signaling domain.

158. The composition of any one of embodiments 152-157, wherein the PFP does not comprise an FcRβ chain intracellular signaling domain.

159. The composition of any one of the preceding embodiments, wherein the recombinant nucleic acid encodes a TREM extracellular domain, a TREM transmembrane domain and/or a TREM intracellular domain.

160. The composition of embodiment 159, wherein the TREM is TREM1, TREM 2 or TREM 3.

161. The composition of any one of the preceding embodiments, wherein the recombinant nucleic acid comprises a sequence encoding a pro-inflammatory polypeptide.

162. The composition of any one of the preceding embodiments, wherein the composition further comprises a pro-inflammatory polypeptide.

163. The composition of embodiment 162, wherein the pro-inflammatory polypeptide is a chemokine, cytokine and nucleotides.

164. The composition of embodiment 163, wherein the chemokine is selected from the group consisting of IL-1, IL3, IL5, IL-6, i18, IL-12, IL-13, IL-23, TNF, CCL2, CXCL9, CXCL10, CXCL11, IL-18, IL-23, IL-27, CSF, MCSF, GMCSF, IL17, IP-10, RANTES, an interferon.

165. The composition of embodiment 163, wherein the cytokine is selected from the group consisting of IL-1, IL3, IL5, IL-6, IL-12, IL-13, IL-23, TNF, CCL2, CXCL9, CXCL10, CXCL11, IL-18, IL-23, IL-27, CSF, MCSF, GMCSF, IL17, IP-10, RANTES, an interferon.

166. The composition of embodiment 163, wherein the nucleotide is selected from ATP, ADP, UTP, UDP, and/or UDP-glucose.

167. The composition of any one of the preceding embodiments, wherein the recombinant nucleic acid comprises a sequence encoding a homeostatic regulator of inflammation.

168. The composition of embodiment 167, wherein the homeostatic regulator of inflammation is a sequence in an untranslated region (UTR) of an mRNA.

169. The composition of embodiment 168, wherein the sequence in the UTR is a sequence that binds to an RNA binding protein.

170. The composition of embodiment 168 or 169, wherein translation is inhibited or prevented upon binding of the RNA binding protein to the sequence in an untranslated region (UTR).

171. The composition of embodiment 169 or 170, wherein the sequence in the UTR comprises a consensus sequence of WWWU(AUUUA)UUUW (SEQ ID NO: 35), wherein W is A or U.

172. The composition of any one of the preceding embodiments, wherein the recombinant nucleic acid is expressed on a bicistronic vector.

173. The composition of any one of the preceding embodiments, wherein the target cell is a mammalian cell.

174. The composition of any one of the preceding embodiments, wherein the target cell is a human cell.

175. The composition of any one of the preceding embodiments, wherein the target cell comprises a cell infected with a pathogen.

176. The composition of any one of the preceding embodiments, wherein the target cell is a cancer cell.

177. The composition of any one of the preceding embodiments, wherein the target cell is a cancer cell that is a lymphocyte.

178. The composition of any one of the preceding embodiments, wherein the target cell is a cancer cell that is an ovarian cancer cell.

179. The composition of any one of the preceding embodiments, wherein the target cell is a cancer cell that is an ovarian pancreatic cell.

180. The composition of any one of the preceding embodiments, wherein the target cell is a cancer cell that is an glioblastoma cell.

181. The composition of any one of the preceding embodiments, wherein the recombinant nucleic acid is DNA.

182. The composition of any one of the preceding embodiments, wherein the recombinant nucleic acid is RNA.

183. The composition of any one of the preceding embodiments, wherein the recombinant nucleic acid is mRNA.

184. The composition of any one of the preceding embodiments, wherein the recombinant nucleic acid is a circRNA.

185. The composition of any one of the preceding embodiments, wherein the recombinant nucleic acid is a tRNA.

186. The composition of any one of the preceding embodiments, wherein the recombinant nucleic acid is a microRNA.

187. A vector comprising the composition of any one of embodiments 1-186.

188. The vector of embodiment 187, wherein the vector is viral vector.

189. The vector of embodiment 188, wherein the viral vector is retroviral vector or a lentiviral vector.

190. The vector of any one of embodiments 187-189, wherein the vector further comprises a promoter operably linked to at least one nucleic acid sequence encoding one or more polypeptides.

191. The vector of any one of embodiments 187-190, wherein the vector is polycistronic.

192. The vector of embodiment 190 or 191, wherein each of the at least one nucleic acid sequence is operably linked to a separate promoter.

193. The vector of any one of embodiments 187-192, wherein the vector further comprises one or more internal ribosome entry sites (IRESs).

194. The vector of any one of embodiments 187-192, wherein the vector further comprises a 5'UTR and/or a 3'UTR flanking the at least one nucleic acid sequence encoding one or more polypeptides.

195. The vector of any one of embodiments 187-192, wherein the vector further comprises one or more regulatory regions.

196. A polypeptide encoded by the recombinant nucleic acid of the composition of any one of embodiments 1-186.

197. A cell comprising the composition of any one of embodiments 1-186, the vector of any one of embodiments 187-195 or the polypeptide of embodiment 196.

198. The cell of embodiment 197, wherein the cell is a phagocytic cell.

199. The cell of embodiment 197 or 198, wherein the cell is a stem cell derived cell, myeloid cell, macrophage, a dendritic cell, lymphocyte, mast cell, monocyte, neutrophil, microglia, or an astrocyte.

200. The cell of any one of embodiments 197-199, wherein the cell is an autologous cell.

201. The cell of any one of embodiments 197-199, wherein the cell is an allogeneic cell.

202. The cell of any one of embodiments 197-201, wherein the cell is an M1 cell.

203. The cell of any one of embodiments 197-201, wherein the cell is an M2 cell.

204. A pharmaceutical composition comprising (a)the composition of any one of embodiments 1-186, the vector of any one of embodiments 187-195, the polypeptide of embodiment 196 or the cell of any one of embodiments 197-203; and (b)a pharmaceutically acceptable excipient.

205. The pharmaceutical composition of embodiment 204, further comprising an additional therapeutic agent.

206. The pharmaceutical composition of embodiment 204 or 205, wherein the additional therapeutic agent is selected from the group consisting of a CD47 agonist, an agent that inhibits Rac, an agent that inhibits Cdc42, an agent that inhibits a GTPase, an agent that promotes F-actin disassembly, an agent that promotes PI3K recruitment to the PFP, an agent that promotes PI3K activity, an agent that promotes production of phosphatidylinositol 3,4,5-trisphosphate, an agent that promotes ARHGAP12 activity, an agent that promotes ARHGAP25 activity, an agent that promotes SH3BP1 activity and any combination thereof.

207. The pharmaceutical composition of any one of embodiments 204-206, wherein the pharmaceutically acceptable excipient comprises serum free media, a lipid, or a nanoparticle.

208. A method of treating a disease in a subject in need thereof comprising administering to the subject the pharmaceutical composition of any one of embodiments 204-207.

209. The method of embodiment 208, wherein the disease is cancer.

210. The method of embodiment 209, wherein the cancer is a solid cancer.

211. The method of embodiment 210, wherein the solid cancer is selected from the group consisting of ovarian cancer, suitable cancers include ovarian cancer, renal cancer, breast cancer, prostate cancer, liver cancer, brain cancer, lymphoma, leukemia, skin cancer, pancreatic cancer, colorectal cancer, lung cancer

212. The method of embodiment 209, wherein the cancer is a liquid cancer.

213. The method of embodiment 212, wherein the liquid cancer is leukemia or a lymphoma.

214. The method of embodiment 212, wherein the liquid cancer is a T cell lymphoma.

215. The method of embodiment 208, wherein the disease is a T cell malignancy.

216. The method of any one of embodiments 208-215, wherein the method further comprises administering an additional therapeutic agent to the subject.

217. The method of embodiment 216, wherein the additional therapeutic agent is selected from the group consisting of a CD47 agonist, an agent that inhibits Rac, an agent that inhibits Cdc42, an agent that inhibits a GTPase, an agent that promotes F-actin disassembly, an agent that promotes PI3K recruitment to the PFP, an agent that promotes PI3K activity, an agent that promotes production of phosphatidylinositol 3,4,5-trisphosphate, an agent that promotes ARHGAP12 activity, an agent that promotes ARHGAP25 activity, an agent that promotes SH3BP1 activity and any combination thereof.

218. The method of any one of embodiments 208-217, wherein administering comprises infusing or injecting.

219. The method of any one of embodiments 208-218, wherein administering comprises administering directly to the solid cancer.

220. The method of any one of embodiments 208-219, wherein administering comprises a circRNA, mRNA, viral-, particle-, liposome-, or exosome-based delivery procedure.

221. The method of any one of embodiments 208-220, wherein a CD4+ T cell response or a CD8+ T cell response is elicited in the subject.

222. A method of preparing a cell, the method comprising contacting a cell with the composition of any one of embodiments 1-186, the vector of any one of embodiments 187-195 or the polypeptide of embodiment 196.

223. The method of embodiment 222, wherein contacting comprises transducing.

224. The method of embodiment 223, where transducing comprises chemical transfection, electroporation, nucleofection, or viral infection.

225. A method of preparing a pharmaceutical composition comprising contacting a lipid to the composition of any one of embodiments 1-186 or the vector of any one of embodiments 187-195. 226. The method of embodiment 225, where contacting comprises forming a lipid nanoparticle. 227. A method of preparing a pharmaceutical composition comprising contacting an antibody to the composition of any one of embodiments 1-186 or the vector of any one of embodiments 187-195. 228. The method of embodiment 225, where contacting comprises forming a lipid nanoparticle.

## EXAMPLES

### Example 1. Generation of novel chimeric receptors fusion proteins (CFP) constructs

In this section, an exemplary design for identification of useful CFP ECD, TM, ICD and antigen binding domains for the generation of novel CFPs is described. Briefly, a large number of potential candidate proteins are screened for enhanced phagocytic properties and their respective phagocytosis related intracellular signaling. The useful domains are then used for generation of novel CFPs. The screen can be divided in two parts: *A. Screening for the PR domains; B. Screening for the extracellular antigen binding domains.*

*Screening for the PR domains:*

5,800 plasma membrane proteins are screened for their phagocytic potential. J774 macrophage cells are transiently transfected with the library of 5800 plasma proteins. High-throughput multiplex assays (ranging from 6-well plate assay set up to up to 384-well plate assay with robotic handling) are set up to evaluate various potential functions of the plasma membranes. Exemplary assays include, but are not limited to phagocytosis assay, cytokine production assay, inflammasome activation assay, and iNOS activation assay. Exemplary simplified methods are described in the following paragraphs. Variations of each method are also used and are understood by a skilled artisan. Variations of each method are also used and are understood by a skilled artisan. Exemplary intracellular signaling domains tested for include but are not limited to CD40-FcRγ; FcRγ-CD40; NLRP3; FcRγ-SH2-Procaspase; FcRγ-Myd88; FeRγ-IFN receptor; FcR-TNFR1; FcRγ-TNFR2; FcR-AIM2; FcRγ-TRIFN; FcRy-Procaspase; TRIFC; RIG1; MDA5; TBK; CD64; CD16A; CD89; FcRε; SIRPβ; (two consecutive intracellular domains are represented as hyphenated terms, for example, FcRγ-Myd88 refers to an intracellular domain comprising an FcRγ intracellular signaling domain as signaling domain 1; and an Myd88 intracellular signaling domain as signaling domain 2). The extracellular linker domains screened include but are not limited to CD64, CD16A, CD89, SIRPα, FcRε, CD8 hinge. The transmembrane domains tested include but are not limited to CD8, CD64, CD16A, CD89, FcRε, SIRPα, TNFR1 and CD40. MDA5 domains were also screened.

### Phagocytosis assay:

Antigen-linked silica or polysterene beads ranging in diameters 1 nm, 5 nm or 10 nm were used for a screen of macrophages. Inert beads are coated in a supported lipid bilayer and the antigens are ligated to the lipid bilayer. J774 macrophage cell lines are prepared, each cell line expressing a cloned recombinant plasma membrane protein. The recombinant plasma membrane protein may also express a fluorescent tag. The cell lines are maintained and propagated in complete RPMI media with heat inactivated serum and antibiotics (Penicillin/Streptomycin). On the day of the assay, cells are plated at a density of $1 \times 10^6$ cells/ml per well in 6 well plates or in a relative proportion in 12 or 24 well plates, and incubated for 2-6 hours. The cells are then washed once in Phosphate Buffer Saline, and the beads are added in serum depleted or complement depleted nutrient media. Cells are visualized by light microscopy at 30 minutes and 2 hours after addition of the beads. Immunofluorescence reaction may be performed using tagged antibody, and fluorescent confocal microscopy is used to detect the interaction and co-localization of cellular proteins at engulfment. Confidence levels are determined by Kruskal-Wallis test with Dunn's multiple comparison correction.

In some examples, dye loaded tumor cells are fed to macrophage cell lines and phagocytosis is assessed by microscopy.

### Cytokine production:

Macrophage cell lines are cultured as above. In one assay, each J774 cell line expressing a plasma membrane protein is plated in multi-wells and challenged with antigen-linked beads and cytokine production was assayed by collecting the supernatants at 4 hours and 24 hours. Cytokines are assayed from the supernatant by ELISA. In another fraction, cells are collected at 4 and 24 hours after incubation with the beads and flow cytometry is performed for detection of cytokines. In each case, multiple cytokines are assayed in a multiplex format, which can be selected from: IL-1α, IL-1β, IL-6, IL-12, IL-23, TNF-α, GMCSF, CXCL1, CXCL3, CXCL9, CXCL-10, MIP1-α and MIP-2. Macrophage inflammatory cytokine array kit (R&D Systems) is used.

Intracellular signaling pathway for inflammatory gene and cytokine activation can be identified by western blot analysis for phosphorylation of MAP kinases, JNK, Akt signaling pathway, Interferon activation pathway including phosphorylation and activation of STAT-1.

Inflammasome activation assay:

Activation of NLRP3 inflammasome is assayed by ELISA detection of increased IL-1 production and detection caspase-1 activation by western blot, detecting cleavage of procaspase to generate the shorter caspase. In a microwell plate multiplex setting, Caspase-Glo (Promega Corporation) is used for faster readout of Caspase 1 activation.

iNOS activation assay:

Activation of the oxidative burst potential is measured by iNOS activation and NO production using a fluorimetric assay NOS activity assay kit (AbCAM).

Cancer cell killing assay:

Raji B cells are used as cancer antigen presenting cells. Raji cells are incubated with whole cell crude extract of cancer cells, and co-incubated with J774 macrophage cell lines. The macrophages can destroy the cells after 1 hour of infection, which can be detected by microscopy or detected by cell death assay.

Screening for high affinity antigen binding domains:

Cancer ligands are subjected to screening for antibody light chain and heavy chain variable domains to generate extracellular binding domains for the CFPs. Human full length antibodies or scFv libraries are screened. Also potential ligands are used for immunizing llama for development of novel immunoglobulin binding domains in llama, and preparation of single domain antibodies.

Specific useful domains identified from the screens are then reverse transcribed, and cloned into lentiviral expression vectors to generate the CFP constructs. A recombinant nucleic acid encoding a CFP can generated using one or more domains from the extracellular, TM and cytoplasmic regions of the highly phagocytic receptors generated from the screen. Briefly plasma membrane receptors showing high activators of pro-inflammatory cytokine production and inflammasome activation are identified. Bioinformatics studies are performed to identify functional domains including extracellular activation domains, transmembrane domains and intracellular signaling domains, for example, specific kinase activation sites, SH2 recruitment sites. These screened functional domains are then cloned in modular constructions for generating novel CFPs. These are candidate CFPs, and each of these chimeric construct is tested for phagocytic enhancement, production of cytokines and chemokines, and/or tumor cell killing in vitro and/or in vivo. A microparticle based phagocytosis assay was used to examine changes in phagocytosis. Briefly, streptavidin coupled fluorescent polystyrene microparticles (6 $\mu$m diameter) are conjugated with biotinylated recombinantly expressed and purified cancer ligand. Myeloid cells expressing the novel CFP were incubated with the ligand coated microparticles for 1-4 h and the amount of phagocytosis was analyzed and quantified using flow cytometry. Plasmid or lentiviral constructions of the designer CFPs are then prepared and tested in macrophage cells for cancer cell lysis.

Exemplary functional domain containing CFPs are described in the following sections.

**Example 2. Generation of recombinant CFP having scavenger receptor ECD, TM and ICD (SR-CAR)**

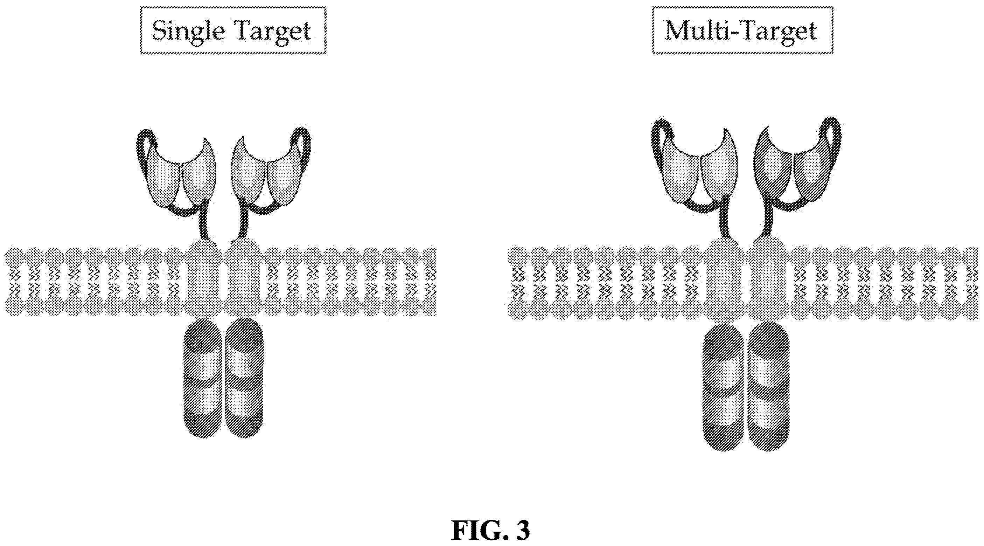

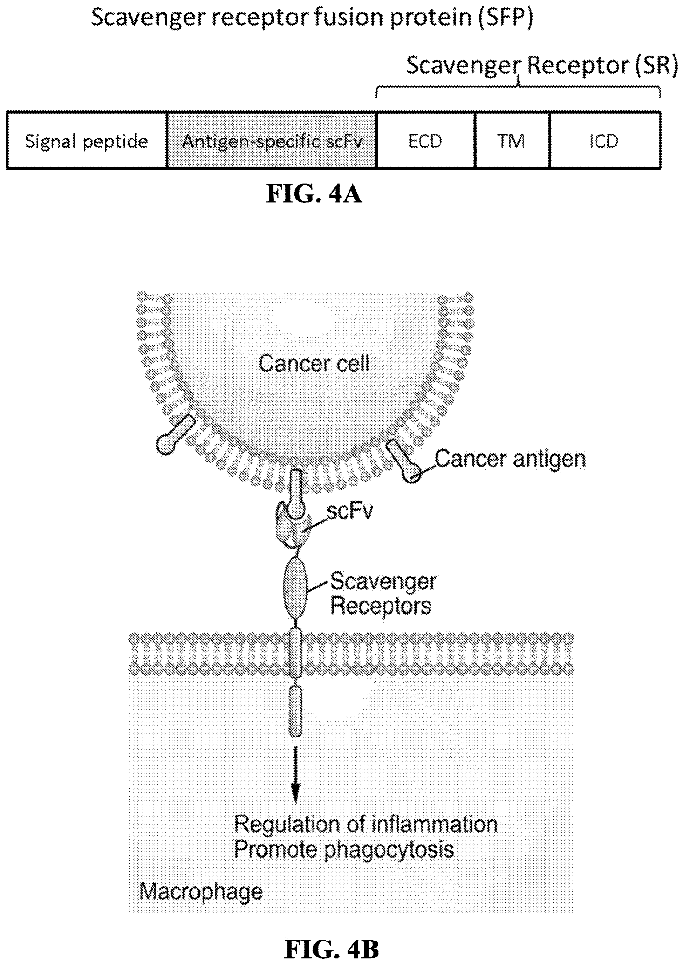

A CFP designed for the purpose of the present application is modular, having an extracellular target binding domain primarily comprising of an scFv, or an Fab region or $V_{HH}$ domain, that can bind to a target, e.g. CD5, a short hinge, a transmembrane domain, and an intracellular domain comprising one or two or more signaling domains (FIGs. 2A-2C). Additionally, the extracellular domain can be designed to bind to a single or a multiple target (FIG. 3). An exemplary design of a phagocytic scavenger receptor is illustrated in FIGs 4A and 4B. The recombinant nucleic acid encoding the CFP is constructed as follows: a signal peptide sequence which encodes for the membrane localization signal for the recombinant protein is placed upstream of the coding sequence of the extracellular antigen binding domain. Then the nucleic acid sequence encoding extracellular antigen binding scFv domain is synthesized and cloned into an expression vector, downstream of the signal peptide sequence. The CFP is made up of the sequence encoding the extracellular domain, the TM domain and the intracellular domain of the scavenger receptor of choice is ligated at the 3'end of the scFv, and preferably with a linker peptide sequence in between the 3'end of the scFv and the 5'end of the scavenger receptor ECD. An exemplary linker peptide is GGGS (SEQ ID NO: 44), and optionally the linker is a sequence that has two or more repeats of the tetramer. Once expressed, the scavenger receptor TM domain is incorporated in the cell membrane.

Lentiviral constructs of SR-CAR are prepared and purified for use in transduction studies.

**Example 3. Expression and functional analysis of the recombinant CFG (SR-CAR)**

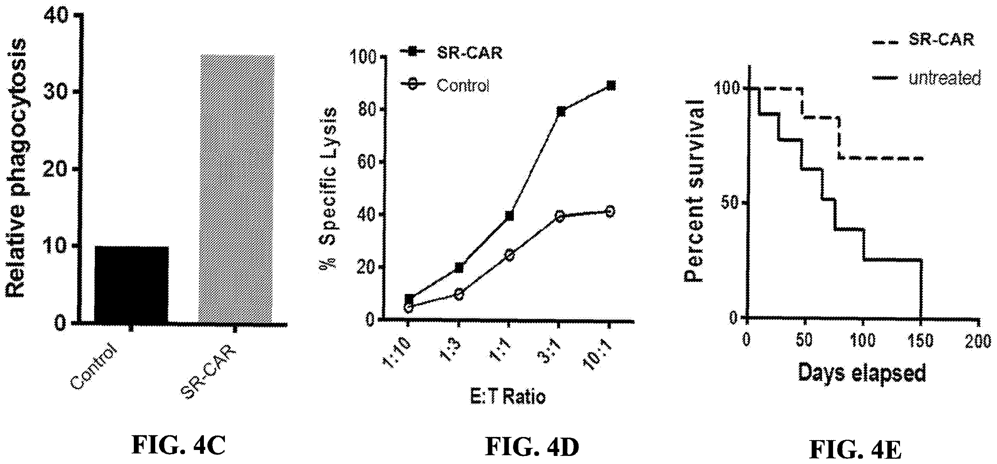

To test the function of the CFP, human macrophages are transduced with pCMV-SRCAR using lipofectamine. In parallel, control cells are transfected with an empty vector. After stabilization of the cells for 48 hours, the cells are subjected to phagocytosis assay. **FIG. 4C** shows the expected result in an in vitro phagocytosis assay. Human primary macrophage transduced with control empty vector or SR-CAR are co-cultured with dye loaded tumor cells, and phagocytosis is quantified using flow cytometry. The cells with the SR CAR plasmid show increased phagocytosis over control cells.

**FIG. 4D** shows the expected result in an in vitro cell lysis assay. Human primary macrophage transduced with control vector or SR-CAR are co-cultured with tumor cells expressing luciferase at different E:T ratio, and specific lysis is quantified using luciferase assay.

**FIG. 4E** shows the expected result in a mouse xenograft model. On day 0, NSG mice were injected with tumor cells expressing luciferase. Mice are either untreated or injected with human primary macrophage transduced with SR-CAR, and survival curve is generated.

**Example 4. Generation of recombinant CFP protein having a second intracellular domain-inflammatory response domain (IR-CAR)**

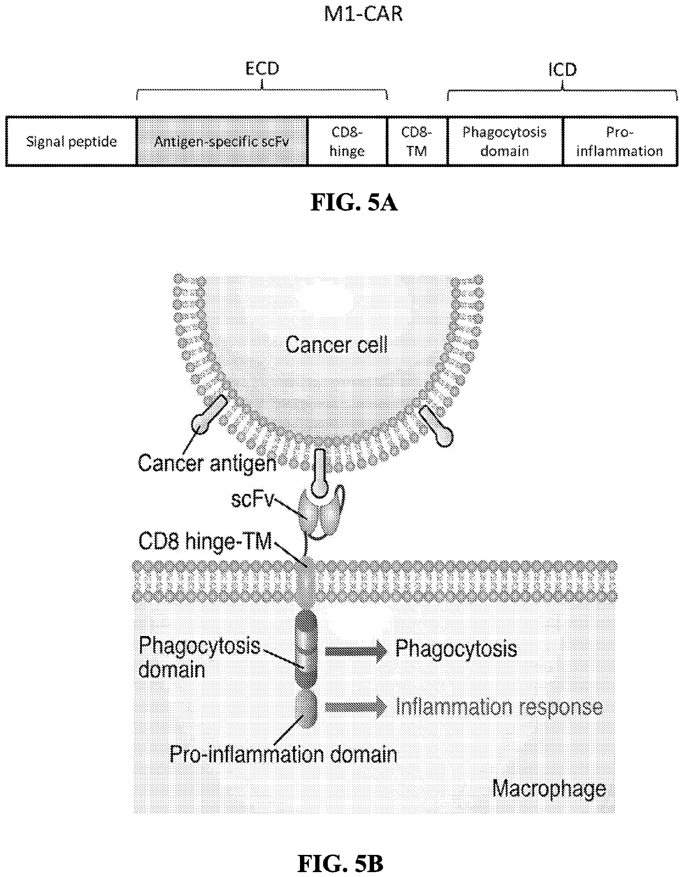

This example shows an exemplary PFP design with an extracellular scFv domain, a linker with a hinge, a CD8 transmembrane domain an intracellular phagocytic receptor domain, and additionally another intracellular inflammatory response (IR) domain from a pro-inflammatory protein (**FIGs 5A-5B**). The recombinant nucleic acid encoding the PFP is constructed as follows: a signal peptide sequence which encodes for the membrane localization signal for the recombinant protein is placed upstream of the coding sequence of the extracellular antigen binding domain. Then the nucleic acid sequence encoding extracellular antigen binding scFv domain is synthesized and cloned into an expression vector, downstream of the signal peptide sequence. The PR subunit is made up of the sequence encoding an extracellular and transmembrane domain of CD8 receptor. The scFv and the CD8 region are connected by a hinge, contributed by the CD8 region proximal to the extracellular domain. The 3'end of the CD8 TM encoding region is ligated to the intracellular domain of a phagocytic receptor of choice. To the 3'end of the coding sequence of the intracellular phagocytic domain, the 5'end of the pro-inflammatory intracellular response domain is ligated.

For testing, the recombinant construct is inserted in a Lentiviral expression vector, and purified for use in cell expression. )

**Example 5. Expression and functional analysis of recombinant CFP (IR-CAR**

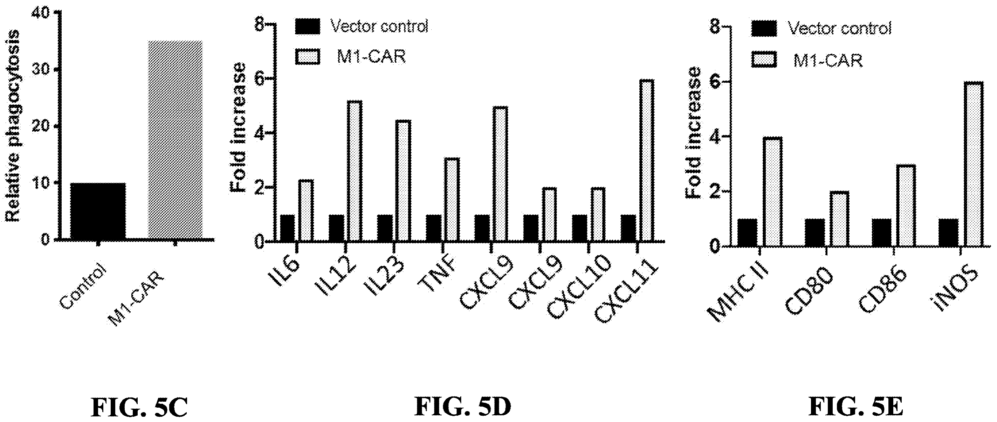

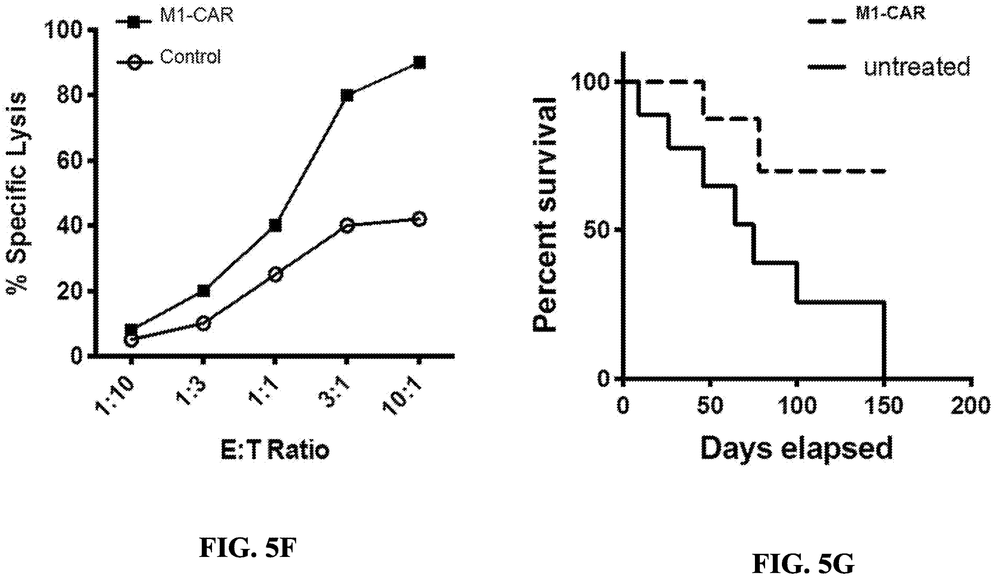

Human primary myeloid cells transduced with control empty vector or CFP (M1-CAR) are co-cultured with target tumor cells. **FIG. 5C** shows the expected result of relative phagocytoses of the dye loaded target tumor cells. **FIG. 5D** shows the expected result of expression of cytokines when M1-CAR myeloid cells are co-cultured with target tumor cells. Cytokine profiling with ELISA shows increased secretion of pro-inflammatory cytokines and chemokines compared to vector control. **FIG. 5E** shows expected result of flow cytometry of surface antigens (MHCII, CD80, CD86) shows an increase of M1 state marker expression compared with vector control, and similarly, iNOS expression (intracellular) was upregulated. **FIGs. 5F** and **5G** indicate expected results.

**Example 6. Generation of recombinant CFP having integrin activation domain (Integrin-CAR)**

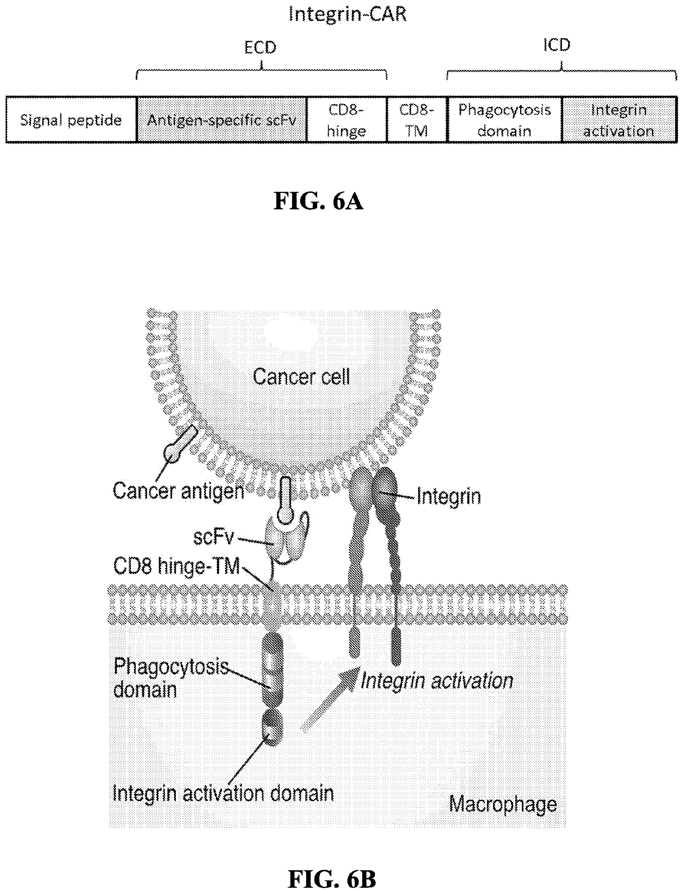

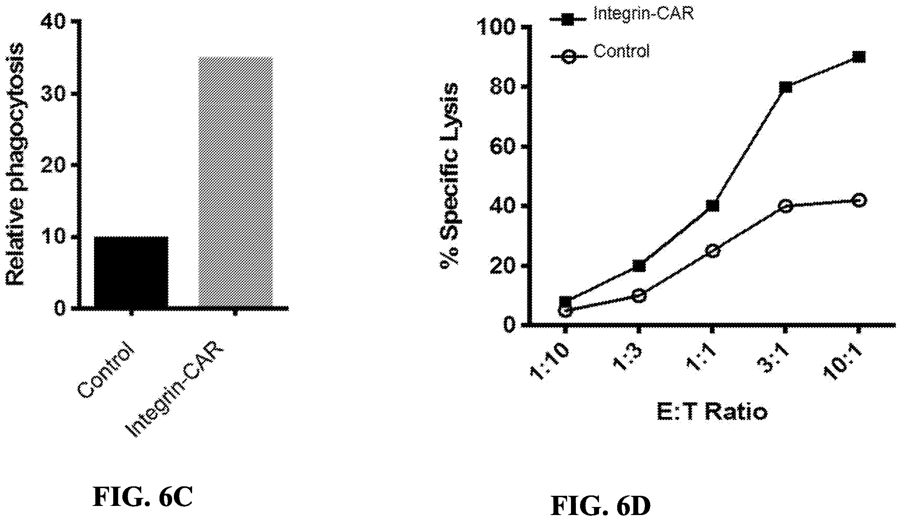

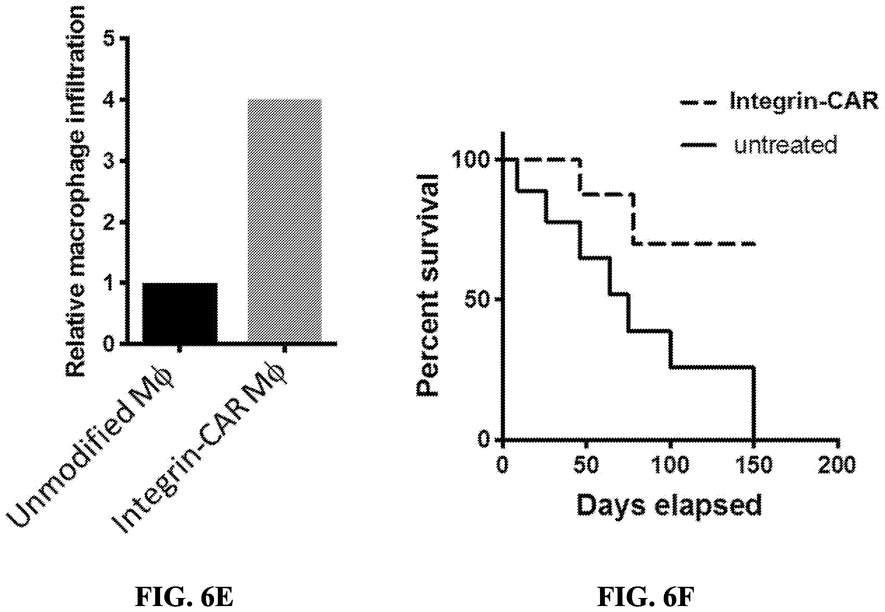

This example shows an exemplary design with an extracellular scFv domain, a transmembrane domain and an intracellular phagocytic domain, and additionally an intracellular integrin activation domain (**FIG. 6A, 6B**). The recombinant nucleic acid encoding the PFP is constructed as follows: a signal peptide sequence which encodes for the membrane localization signal for the recombinant protein is placed upstream of the coding sequence of the extracellular antigen binding domain. Then the nucleic acid sequence encoding extracellular antigen binding scFv domain is synthesized and cloned into an expression vector, downstream of the signal peptide sequence. The PSR subunit is made up of the sequence encoding an extracellular and transmembrane domain of CD8 receptor. The scFv and the CD8 region are connected by a hinge, contributed by the CD8 region proximal to the extracellular domain. The 3'end of the CD8 TM encoding region is ligated to the phagocytosis domain of a phagocytic receptor of choice. To the 3'end of the coding sequence of the intracellular phagocytic domain, the 5'end of a P-selectin intracellular integrin activation domain is ligated. The basic design of the recombinant nucleic acid is shown in **FIG. 5A.** A diagrammatic depiction of the structural layout of the exemplary receptor is shown in **FIG. 5B. FIG. 5B** shows graphical representation of integrin activation, where integrins are endogenous, and form clusters upon activation. When expressed in macrophages, binding of scFv to tumor specific antigen leads to activation of phagocytosis signaling as well as activation of integrin. This leads to stronger phagocytosis as well as improved macrophage trafficking.

The construct is inserted in a lentiviral vector and purified for functional studies.

## Example 7. Expression and functional analysis of the recombinant Integrin-CAR.

Human primary macrophage transduced with control empty vector or integrin-CAR are co-cultured with target tumor cells. **FIG 5C** shows expected results of increased phagocytosis by integrin-CAR transduced macrophages compared to control macrophages. **FIG 5D** shows expected results of increased lysis of tumor cells by cells expressing integrin-CAR. **FIG 5E** shows expected results of increased migration and tumor infiltration of integrin-CAR transduced macrophages compared to control macrophages. **FIG 5F** shows expected survival curve in mouse xenograft model of a tumor after treatment with integrin-CAR transduced macrophages, or no treatment controls.

## Example 8. Generation of recombinant CFP having an SREC-1 cross presentation domain

In this example, an exemplary design of a vector expressing the CFP, with an extracellular scFv domain, a transmembrane domain and an intracellular phagocytic domain, and additionally an signaling domain. **FIG. 6A** provides a schematic diagram of the intracellular signaling pathways involving SREC and antigen cross presentation. The recombinant nucleic acid encoding the CFP is constructed as follows: a signal peptide sequence which encodes for the membrane localization signal for the recombinant protein is placed upstream of the coding sequence of the extracellular antigen binding domain. Then the nucleic acid sequence encoding extracellular antigen binding scFv domain is synthesized and cloned into an expression vector, downstream of the signal peptide sequence. The PR subunit is made up of the sequence encoding an extracellular and transmembrane domain of phagocytic receptor. The 3'end of the TM encoding region is ligated to the phagocytosis domain of a phagocytic receptor. To the 3'end of the coding sequence of the intracellular phagocytic domain, the 5'end of the intracellular signaling domain for cross presentation is ligated. A diagrammatic depiction of the structural layout of the exemplary receptor is shown in **FIG. 6B. FIGs. 6C-6F** show expected functional characteristics as described earlier.

## Example 9. Manufacturing Protocol for Myeloid and Macrophage Cell Preparation from a subject

Myeloid/Macrophage cell isolation from PBMCs:

Peripheral blood mononuclear cells are separated from normal donor buffy coats by density centrifugation using Histopaque 1077 (Sigma). After washing, CD14+ monocytes are isolated from the mononuclear cell fraction using CliniMACS GMP grade CD14 microbeads and LS separation magnetic columns (Miltenyi Biotec). Briefly, cells are resuspended to appropriate concentration in PEA buffer (phosphate-buffered saline [PBS] plus 2.5 mmol/L ethylenediaminetetraacetic acid [EDTA] and human serum albumin [0.5% final volume of Alburex 20%, Octopharma]), incubated with CliniMACS CD14 beads per manufacturer's instructions, then washed and passed through a magnetized LS column. After washing, the purified monocytes are eluted from the demagnetized column, washed and re-suspended in relevant medium for culture. Isolation of CD14+ cells from leukapheresis: PBMCs are collected by leukapheresis from cirrhotic donors who gave informed consent to participate in the study. Leukapheresis of peripheral blood for mononuclear cells (MNCs) is carried out using an Optia apheresis system by sterile collection. A standard collection program for MNC is used, processing 2.5 blood volumes. Isolation of CD14 cells is carried out using a GMP-compliant functionally closed system (CliniMACS Prodigy system, Miltenyi Biotec). Briefly, the leukapheresis product is sampled for cell count and an aliquot taken for pre-separation flow cytometry. The percentage of monocytes (CD14+) and absolute cell number are determined, and, if required, the volume is adjusted to meet the required criteria for selection ($\leq$ 20 x $10^9$ total white blood cells; < 400 x $10^6$ white blood cells/mL; $\leq$ 3.5 x $10^9$ CD14 cells, volume 50-300 mL). CD14 cell isolation and separation is carried out using the CliniMACS Prodigy with CliniMACS CD14 microbeads (medical device class III), TS510 tubing set and LP-14 program. At the end of the process, the selected CD14+ positive monocytes are washed in PBS/EDTA buffer (CliniMACS buffer, Miltenyi) containing pharmaceutical grade 0.5% human albumin (Alburex), then re-suspended in TexMACS (or comparator) medium for culture.

*Cell count and purity:*

Cell counts of total MNCs and isolated monocyte fractions are performed using a Sysmex XP-300 automated analyzer (Sysmex). Assessment of macrophage numbers is carried out by flow cytometry with TruCount tubes (Becton Dickinson) to determine absolute cell number, as the Sysmex consistently underestimated the number of monocytes. The purity of the separation is assessed using flow cytometry (FACSCanto II, BD Biosciences) with a panel of antibodies against human leukocytes (CD45-VioBlue, CD15-FITC, CD14-PE, CD16-APC), and product quality is assessed by determining the amount of neutrophil contamination (CD45int, CD15pos).

*Cell culture-development of cultures with healthy donor samples*

Optimal culture medium for macrophage differentiation is investigated, and three candidates are tested using for the cell product. In addition, the effect of monocyte cryopreservation on deriving myeloid cells and macrophages for therapeutic use is examined. Functional assays are conducted to quantify the phagocytic capacity of myeloid cells and macrophages and their capacity for further polarization, and phagocytic potential as described elsewhere in the disclosure.

*Full-scale process validation with subject samples*

Monocytes cultured from leukapheresis from Prodigy isolation are cultured at $2 \times 10^6$ monocytes per $cm^2$ and per mL in culture bags (MACS GMP differentiation bags, Miltenyi) with GMP-grade TexMACS (Miltenyi) and 100 ng/mL M-CSF. Monocytes are cultured with 100 ng/mL GMP-compliant recombinant human M-CSF (R&D Systems). Cells are cultured in a humidified atmosphere at 37°C, with 5% $CO_2$ for 7 days. A 50% volume media replenishment is carried out twice during culture (days 2 and 4) with 50% of the culture medium removed, then fed with fresh medium supplemented with 200 ng/mL M-CSF (to restore a final concentration of 100 ng/mL).

*Cell harvesting:*

For normal donor-derived macrophages, cells are removed from the wells at day 7 using Cell Dissociation Buffer (Gibco, Thermo Fisher) and a pastette. Cells are resuspended in PEA buffer and counted, then approximately $1 \times 10^6$ cells per test are stained for flow cytometry. Leukapheresis-derived macrophages are removed from the culture bags at day 7 using PBS/EDTA buffer (CliniMACS buffer, Miltenyi) containing pharmaceutical grade 0.5% human albumin from serum (HAS; Alburex). Harvested cells are resuspended in excipient composed of two licensed products: 0.9% saline for infusion (Baxter) with 0.5% human albumin (Alburex).

*Flow cytometry characterization:*

Monocyte and macrophage cell surface marker expression is analyzed using either a FACSCanto II (BD Biosciences) or MACSQuant 10 (Miltenyi) flow cytometer. Approximately 20,000 events are acquired for each sample. Cell surface expression of leukocyte markers in freshly isolated and day 7 matured cells is carried out by incubating cells with specific antibodies (final dilution 1:100). Cells are incubated for 5 min with FcR block (Miltenyi) then incubated at 4°C for 20 min with antibody cocktails. Cells are washed in PEA, and dead cell exclusion dye DRAQ7 (BioLegend) is added at 1:100. Cells are stained for a range of surface markers as follows: CD45-VioBlue, CD14-PE or CD14-PerCP-Vio700, CD163-FITC, CD169-PE and CD16-APC (all Miltenyi), CCR2-BV421, CD206-FITC, CXCR4-PE and CD115-APC (all BioLegend), and 25F9-APC and CD115-APC (eBioscience). Both monocytes and macrophages are gated to exclude debris, doublets and dead cells using forward and side scatter and DRAQ7 dead cell discriminator (BioLegend) and analyzed using FlowJo software (Tree Star). From the initial detailed phenotyping, a panel is developed as Release Criteria (CD45-VB/CD206-FITC/CD14-PE/25F9 APC/DRAQ7) that defined the development of a functional macrophage from monocytes. Macrophages are determined as having mean fluorescence intensity (MFI) five times higher than the level on day 0 monocytes for both 25F9 and CD206. A second panel is developed which assessed other markers as part of an Extended Panel, composed of CCR2-BV421/CD163-FITC/CD169-PE/CD14-PerCP-Vio700/CD16-APC/DRAQ7), but is not used as part of the Release Criteria for the cell product.

Both monocytes and macrophages from buffy coat CD14 cells are tested for phagocytic uptake using pHRodo beads, which fluoresce only when taken into acidic endosomes. Briefly, monocytes or macrophages are cultured with 1-2 uL of pHRodo *Escherichia coli* bioparticles (LifeTechnologies, Thermo Fisher) for 1 h, then the medium is taken off and cells washed to remove non-phagocytosed particles. Phagocytosis is assessed using an EVOS microscope (Thermo Fisher), images captured and cellular uptake of beads quantified using ImageJ software (NIH freeware). The capacity to polarize toward defined differentiated macrophages is examined by treating day 7 macrophages with IFNγ (50 ng/mL) or IL-4 (20 ng/mL) for 48 h to induce polarization to M1 or M2 phenotype (or M[IFNγ] versus M[IL-4], respectively). After 48 h, the cells are visualized by EVOS bright-field microscopy, then harvested and phenotyped as before. Further analysis is performed on the cytokine and growth factor secretion profile of macrophages after generation and in response to inflammatory stimuli. Macrophages are generated from healthy donor buffy coats as before, and either left untreated or stimulated with TNFα (50 ng/mL, Peprotech) and polyinosinic:polycytidylic acid (poly I:C, a viral homolog which binds TLR3, 1 g/mL, Sigma) to mimic the conditions present in the inflamed liver, or lipopolysaccharide (LPS, 100 ng/mL, Sigma) plus IFNγ (50 IU/mL, Peprotech) to produce a maximal macrophage activation. Day 7 macrophages are incubated overnight and supernatants collected and spun down to remove debris, then stored at -80°C until testing. Secretome analysis is performed using a 27-plex human cytokine kit and a 9-plex matrix metalloprotease kit run on a Magpix multiplex enzyme linked immunoassay plate reader (BioRad).

*Product stability:*

Various excipients are tested during process development including PBS/EDTA buffer; PBS/EDTA buffer with 0.5% HAS (Alburex), 0.9% saline alone or saline with 0.5% HAS. The 0.9% saline (Baxter) with 0.5% HAS excipient is found to maintain optimal cell viability and phenotype (data not shown). The stability of the macrophages from cirrhotic donors after harvest is investigated in three process optimization runs, and a more limited range of time points assessed in the process validation runs (n = 3). After harvest and re-suspension in excipient (0.9% saline for infusion, 0.5% human serum albumin), the bags are stored at ambient temperature (21-22°C) and samples taken at 0, 2, 4, 6, 8, 12, 24, 30 and 48 h postharvest. The release criteria antibody panel is run on each sample, and viability and mean fold change from day 0 is measured from geometric MFI of 25F9 and CD206.

*Statistical analysis:*

Results are expressed as mean $\pm$ SD. The statistical significance of differences is assessed where possible with the unpaired two-tailed t-test using GraphPad Prism 6. Results are considered statistically significant when the P value is < 0.05.

## Example 10. CD5-FcR-PI3K CFP construct

In this example, a CD5-targeted CFP was constructed using known molecular biology techniques. The CFP has an extracellular domain comprising a signal peptide fused to an scFv containing a heavy chain variable domain linked to a light chain variable domain that binds to CD5 on a target cell, attached to a CD8$\alpha$ chain hinge and CD8$\alpha$ chain TM domain via a short linker. The TM domain is fused at the cytosolic end with an FcR$\gamma$ cytosolic portion, and a PI3K recruitment domain. The construct was prepared in a vector having a fluorescent marker and a drug (ampicillin) resistance and amplified by transfecting a bacterial host. The sequence is provided below:

*CD5-FcR-PI3K*

MWLQSLLLLGTVACSISEIQLVQSGGGLVKPGGSVRISCAASGYTFTNYGMNWVRQAPGKG LEWMGWINTHTGEPTYADSFKGRFTFSLDDSKNTAYLQINSLRAEDTAVYFCTRRGYDWYF DVWGQGTTVTV*SSGGGGSGGGGSGGGGS*DIQMTQSPSSLSASVGDRVTITCRASQDINSYLS

WFQQKPGKAPKTLIYRANRLESGVPSRF*SGSGSG*TDYTLTISSLQYEDFGIYYCQQYDESPWT FGGGTKLEIK*SGGGGSG*ALSNSIMYFSHFVPVFLPAKPTTTPAPRPPTPAPTIASQPLSLRPEAC RPAAGGAVHTRGLDIYIWAPLAGTCGVLLLSLVITLYCRRLKIQVRKAAITSYEKSDGVYTG LSTRNQETYETLKHEKPPQ*GSGS*YEDMRGILYAAPQLRSIRGQPGPNHEEDADSYENM  (SEQ ID NO: 14).

mRNA was generated by in vitro reverse transcription of the purified plasmids using suitable primers. The purified mRNA was transduced into a cell line for expression analysis.

## Example 11. HER2-FcR-PI3K CFP construct

In this example, a HER2-targeted CFP was constructed using known molecular biology techniques. The CFP has an extracellular domain comprising a signal peptide fused to an scFv containing a heavy chain variable domain linked to a light chain variable domain that binds to HER2 on a target cell, attached to a CD8$\alpha$ chain hinge and CD8$\alpha$ chain TM domain via a short linker. The TM domain is fused at the cytosolic end with an FcR$\gamma$ cytosolic portion, and a PI3K recruitment domain as in the previous example. The sequence is provided below:

*HER2-FcR-PI3K*

MWLQSLLLLGTVACSISDIQMTQSPSSLSASVGDRVTITCRASQDVNTAVAWYQQKPGKAP
KLLIYSASFLYSGVPSRFSGSRSGTDFTLTISSLQPEDFATYYCQQHYTTPPTFGQGTKVEIKR
TGSTSGSGKPGSGEGSEVQLVE*SGGG*LVQPGGSLRLSCAASGFNIKDTYIHWVRQAPGKGLE
WVARIYPTNGYTRYADSVKGRFTISADTSKNTAYLQMNSLRAEDTAVYYCSRWGGDGFYA
MDVWGQGTLVTV*SSSGGGGSG*ALSNSIMYFSHFVPVFLPAKPTTTPAPRPPTPAPTIASQPLS
LRPEACRPAAGGAVHTRGLDIYIWAPLAGTCGVLLLSLVITLYCRRLKIQVRKAAITSYEKSD
GVYTGLSTRNQETYETLKHEKPPQ*GSGS*YEDMRGILYAAPQLRSIRGQPGPNHEEDADSYEN
M (SEQ ID NO: 15).

## Example 12. CD5-FcR-CD40 CFP construct

In this example, a CD5-targeted CFP was constructed using known molecular biology techniques having an intracellular domain comprising CD40 sequence. The CFP has an extracellular domain comprising a signal peptide fused to an scFv containing a heavy chain variable domain linked to a light chain variable domain that binds to CD5 on a target cell, attached to a CD8α chain hinge and CD8α chain TM domain via a short linker. The TM domain is fused at the cytosolic end with an FcRγ cytosolic portion, followed by a CD40 cytosolic portion. The sequence is provided below:
*CD5-FcR-CD40*

MWLQSLLLLGTVACSISEIQLVQSGGGLVKPGGSVRISCAASGYTFTNYGMNWVRQAPGKG
LEWMGWINTHTGEPTYADSFKGRFTFSLDDSKNTAYLQINSLRAEDTAVYFCTRRGYDWYF

DVWGQGTTVTV*SSGGGGSGGGGSGGGGS*DIQMTQSPSSLSASVGDRVTITCRASQDINSYLS
WFQQKPGKAPKTLIYRANRLESGVPSRFSGSGSGTDYTLTISSLQYEDFGIYYCQQYDESPW
TFGGGTKLEIK*SGGGGSG*ALSNSIMYFSHFVPVFLPAKPTTTPAPRPPTPAPTIASQPLSLRPEA
CRPAAGGAVHTRGLDIYIWAPLAGTCGVLLLSLVITLYCRLKIQVRKAAITSYEKSDGVYTG
LSTRNQETYETLKHEKPPQKKVAKKPTNKAPHPKQEPQEINFPDDLPGSNTAAPVQETLHGC
QPVTQEDGKESRISVQERQ (SEQ ID NO: 16).

## Example 13. Expression of anti-CD5 and anti-HER2 CFPs

In this example, cells from monocytic cell line THP-1, were electroporated with individual anti-CD5 CFP (CD5 CAR) constructs with either no intracellular domain (No ICD); or intracellular domain (ICD) having a CD40 signaling domain, or a FcR signaling domain; or with PI3kinase (PI3K) recruitment signaling domain; or with a first CD40 signaling domain and a second signaling domain from FcRγ intracellular domain or vice versa; with a first FcRγ signaling domain and a second PI3K recruitment signaling domain or vice versa, and expression of the CAR construct was determined by flow cytometry as indicated in **FIG. 8.** In each case, a robust expression of greater than 95% cells was observed. **FIG. 9** shows expression of some of these constructs in primary human myeloid cells. Table 6 describes the domains of the constructs in the figures.

**Table 6 - CD5-CFP constructs and HER2 CFP constructs**

| Name | Intracellular domain | TM domain | Extracellular domain | Antigen binding domain |
|---|---|---|---|---|
| CD5-CD8h-CD8tm-CD40-FcR | CD40 and FcRγ | CD8 | CD8 | Anti-CD5 scFv |
| CD5-CD8h-CD8tm-FcR-CD40 | FcRγ and CD40 | CD8 | CD8 | Anti-CD5 scFv |
| CD5-CD8h-CD8tm-FcR-PI3K | FcRγ and PI3K | CD8 | CD8 | Anti-CD5 scFv |

(continued)

| Name | Intracellular domain | TM domain | Extracellular domain | Antigen binding domain |
|---|---|---|---|---|
| CD5-CD8h-CD8tm-FcR | FcRγ | CD8 | CD8 | Anti-CD5 scFv |
| CD5-CD8h-CD8tm-no ICD | None | CD8 | CD8 | Anti-CD5 scFv |
| CD5-CD28h-CD28tm-FcR-PI3K | FcRγ and PI3K | CD28 | CD28 | Anti-CD5 scFv |
| CD5-CD8h-CD68tm-FcR-PI3K | FcRγ and PI3K | CD68 | CD8 | Anti-CD5 scFv |
| CD5-CD8tm-FcR-PI3K | FcRγ and PI3K | CD8 | - | Anti-CD5 scFv |
| CD5-CD28tm-FcR-PI3K | FcRγ and PI3K | CD28 | - | Anti-CD5 scFv |
| CD5-CD68tm-FcR-PI3K | FcRγ and PI3K | CD68 | - | Anti-CD5 scFv |
| CD5-CD8h-CD8tm-FcR-TNFR1 | FcγR and TNFR2 | CD8 | CD8 | Anti-CD5 scFv |
| CD5-CD8h-CD8tm-FcR-TNFR2 | FcRγ and TNFR2 | CD8 | CD8 | Anti-CD5 scFv |
| HER2-CD8h-CD8tm-CD40-FcR | CD40 and FcRγ | CD8 | CD8 | Anti-HER2 scFv |
| HER2-CD8h-CD8tm-FcR-CD40 | FcRγ and CD40 | CD8 | CD8 | Anti-HER2 scFv |
| HER2-CD8h-CD8tm-FcR-PI3K | FcRγ and PI3K | CD8 | CD8 | Anti-HER2 scFv |
| HER2-CD8h-CD8tm-FcR | FcRγ | CD8 | CD8 | Anti-HER2 scFv |
| HER2-CD8h-CD8tm-no ICD | None | CD8 | CD8 | Anti-HER2 scFv |
| HER2-CD28h-CD28tm-FcR-PI3K | FcRγ and PI3K | CD28 | CD28 | Anti-HER2 scFv |
| HER2-CD8h-CD68tm-FcR-PI3K | FcRγ and PI3K | CD68 | CD8 | Anti-HER2 scFv |
| HER2-CD8tm-FcR-PI3K | FcRγ and PI3K | CD8 | - | Anti-HER2 scFv |
| HER2-CD28tm-FcR-PI3K | FcRγ and PI3K | CD28 | - | Anti-HER2 scFv |
| HER2-CD68tm-FcR-PI3K | FcRγ and PI3K | CD68 | - | Anti-HER2 scFv |
| HER2-CD8h-CD8tm-FcR-TNFR1 | FcRγ and TNFR2 | CD8 | CD8 | Anti-HER2 scFv |
| HER2-CD8h-CD8tm-FcR-TNFR2 | FcRγ and TNFR2 | CD8 | CD8 | Anti-HER2 scFv |

## Example 14. Phagocytosis and activation assays

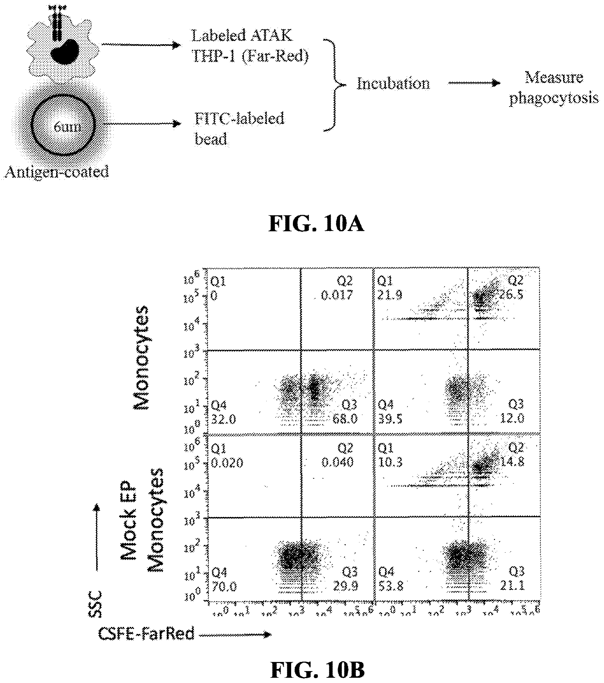

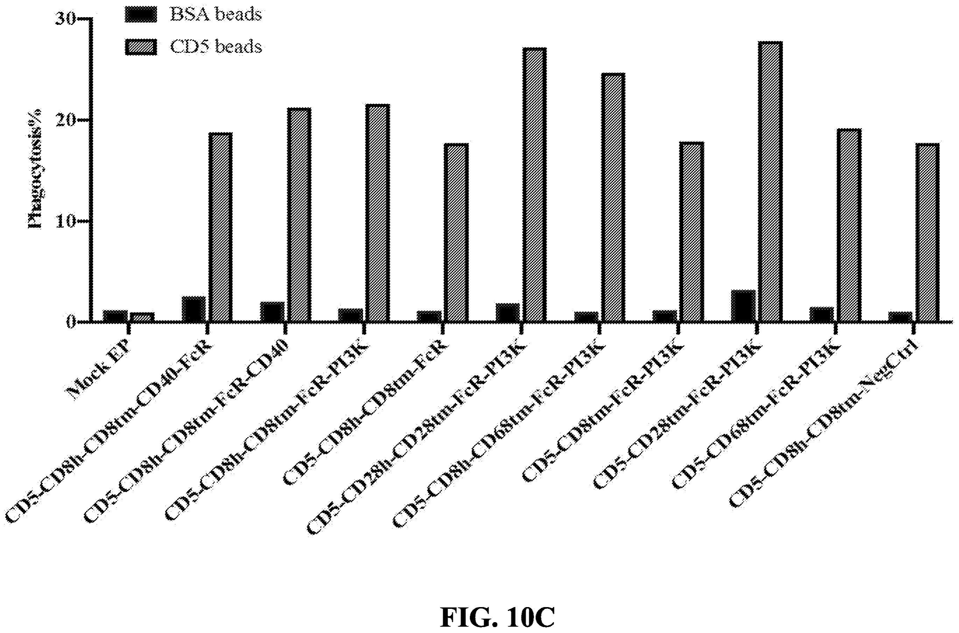

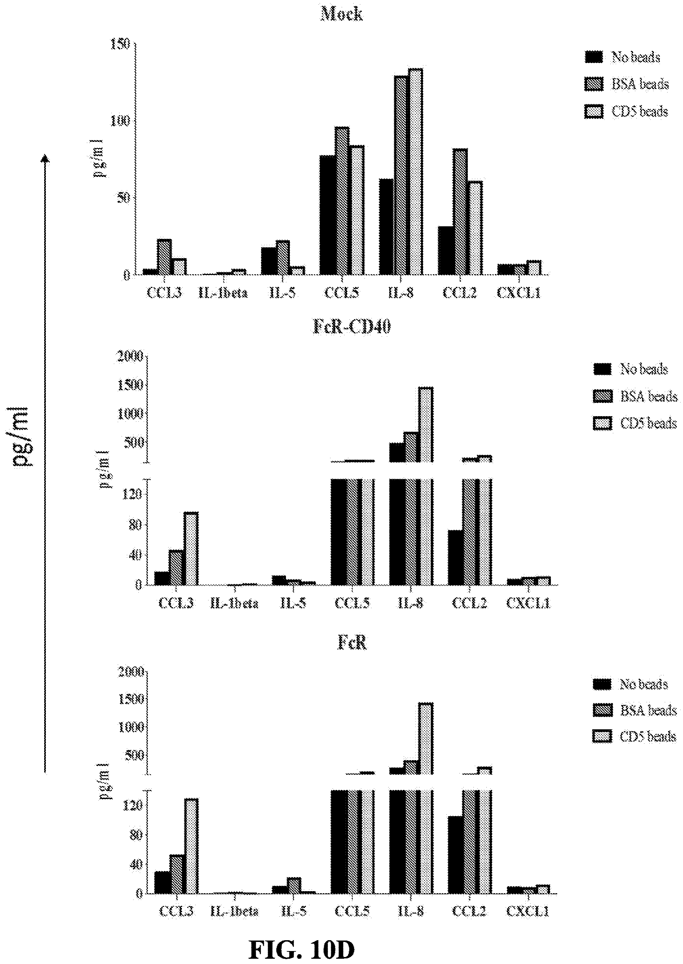

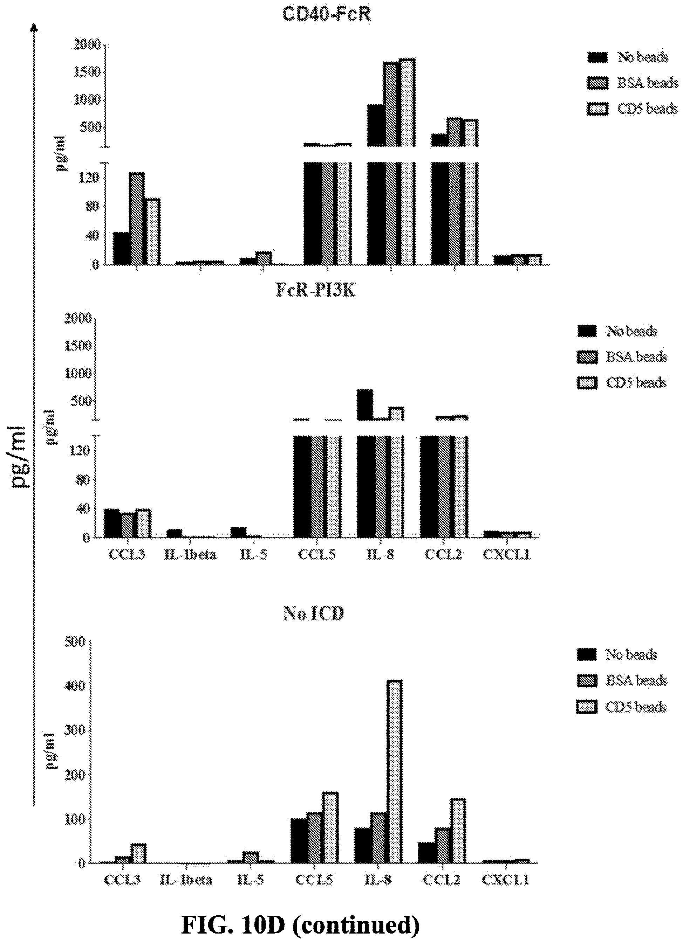

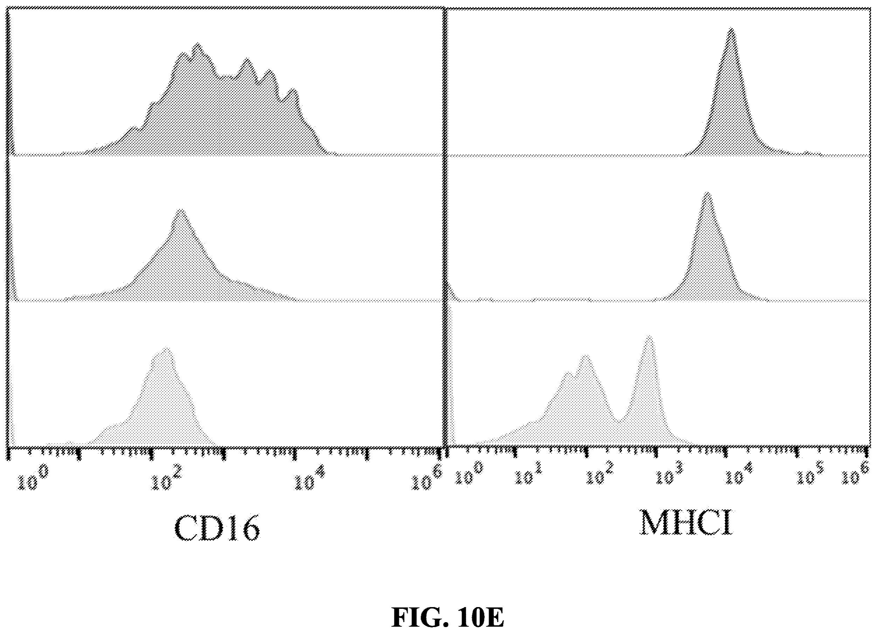

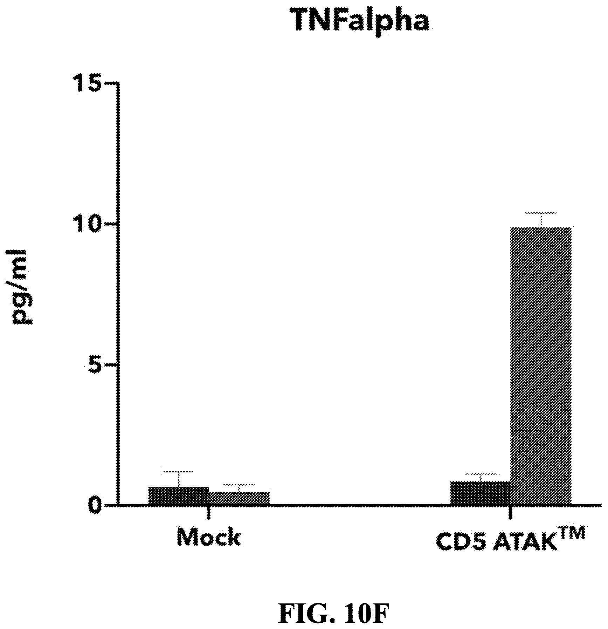

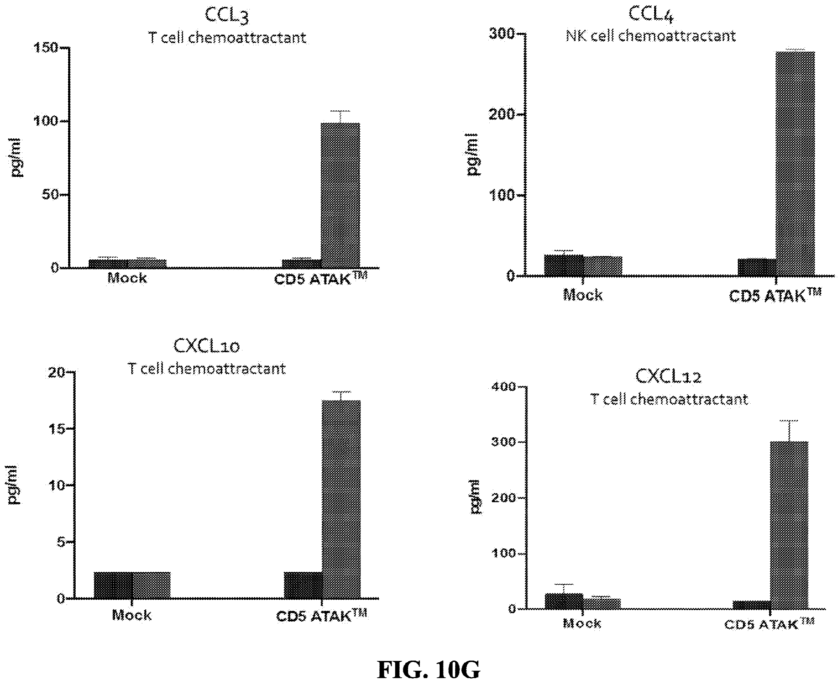

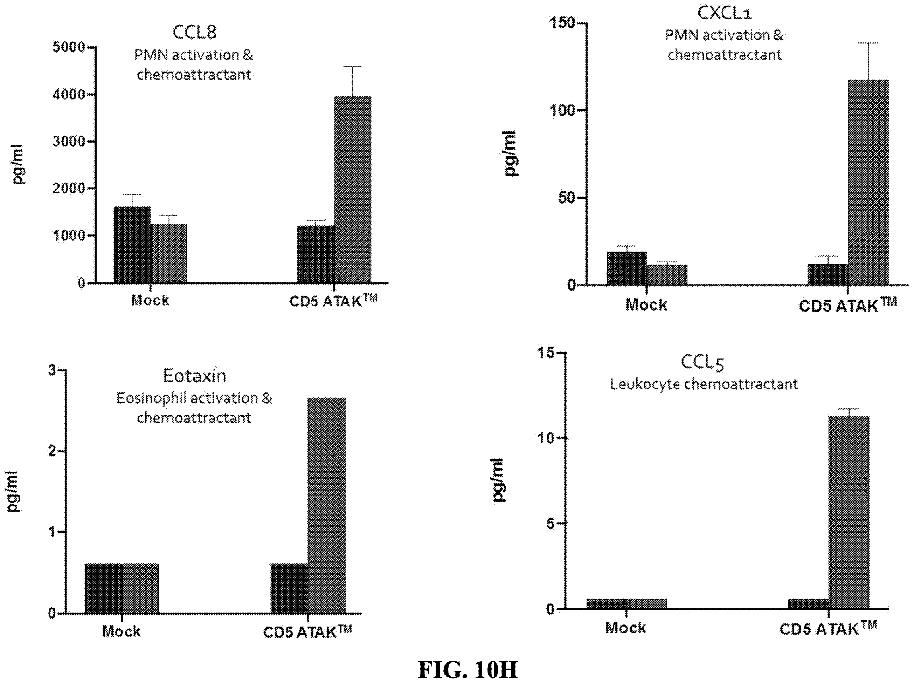

For functional analysis of the various anti-CD5 CFP expressing THP-1 macrophages, cells were fed 6 μm FITC-labeled CD5 antigen-coated beads (**FIG. 10A**) and phagocytic engulfment of the FITC beads per cell was quantitated by flow cytometry (**FIG. 10B**). Control beads were BSA coated. Experimental CD5-coated beads were readily engulfed by THP-1 cells (**FIG. 10C**). Each of the constructs showed high level of phagocytosis that was target specific, and the CD5-coated bead uptake was at least 10-fold higher compared to uptake of BSA coated beads. **FIG. 10D** shows cytokine analysis of the anti-CD5 CFP expressing cells in presence of the control BSA coated beads or experimental CD5 coated beads. Higher cytokine response was observed in the anti-CD5 CFP expressing cells, compared to mock electroporation, although the induction of cytokines were not exceedingly high in absence of any further stimuli. anti-CD5 CFP expressing cells exhibit low expression of CD16 and MHC class I molecules, which are hallmarks of non-classical monocytes (**FIG. 10E**). However, in presence of an activation stimulus, the anti-CD5 CFP expressing cells were shown to be highly activated with induction of the cytokines as shown **FIGs 10F-10H.**

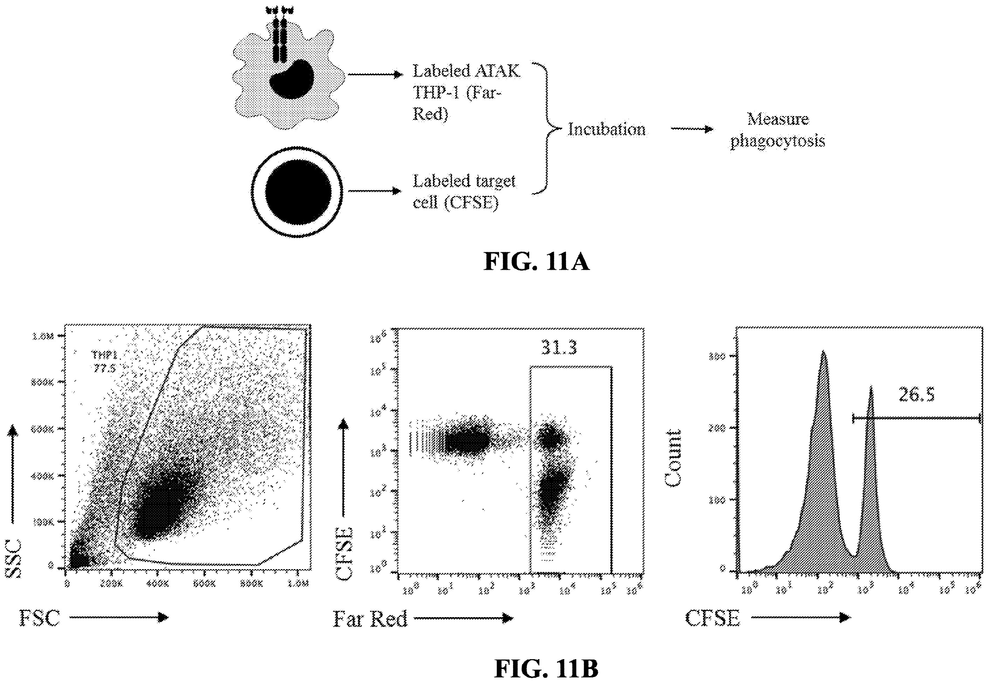

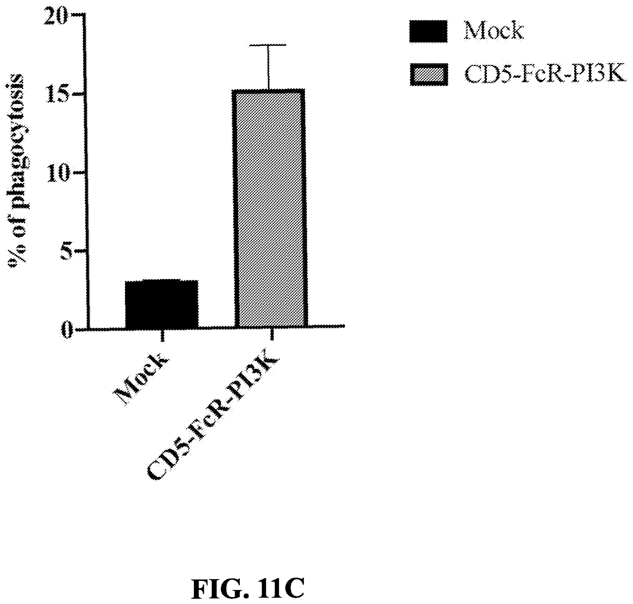

THP-1 cells were electroporated with the CFP construct encoding CD5-CD8h-CD8tm-FcR-PI3K and labelled with the intracellular FarRed dye. These cells were incubated with H9 T cell cancer cells that were pre-labelled with CFSE as a 1:3 myeloid cell:tumor cell ratio. After 4 hours phagocytosis was measured by flow cytometry (**FIG. 11A**). The cancer cells were readily phagocytosed by the anti-CD5 CFP expressing cells (**FIG. 11B, 11C**).

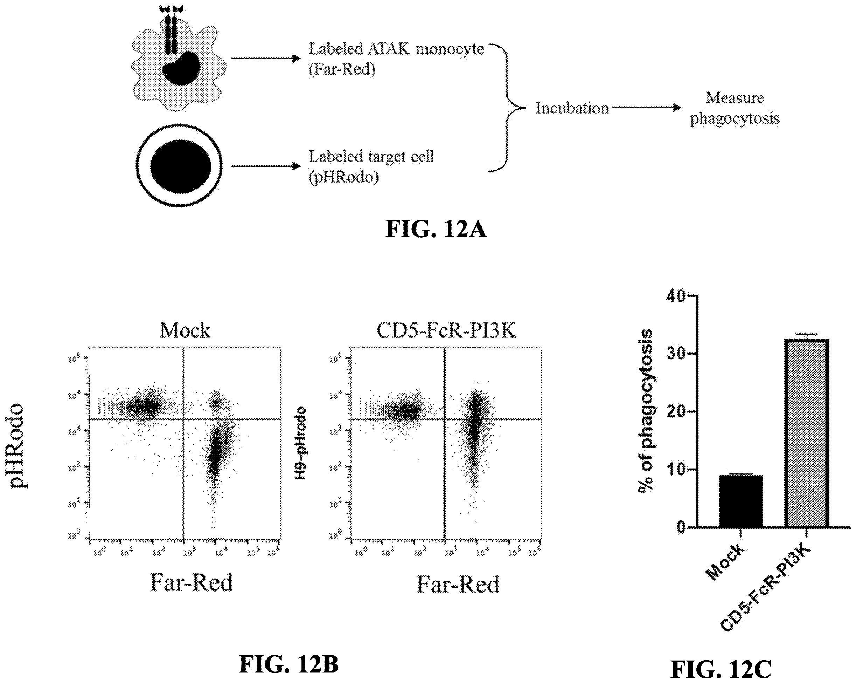

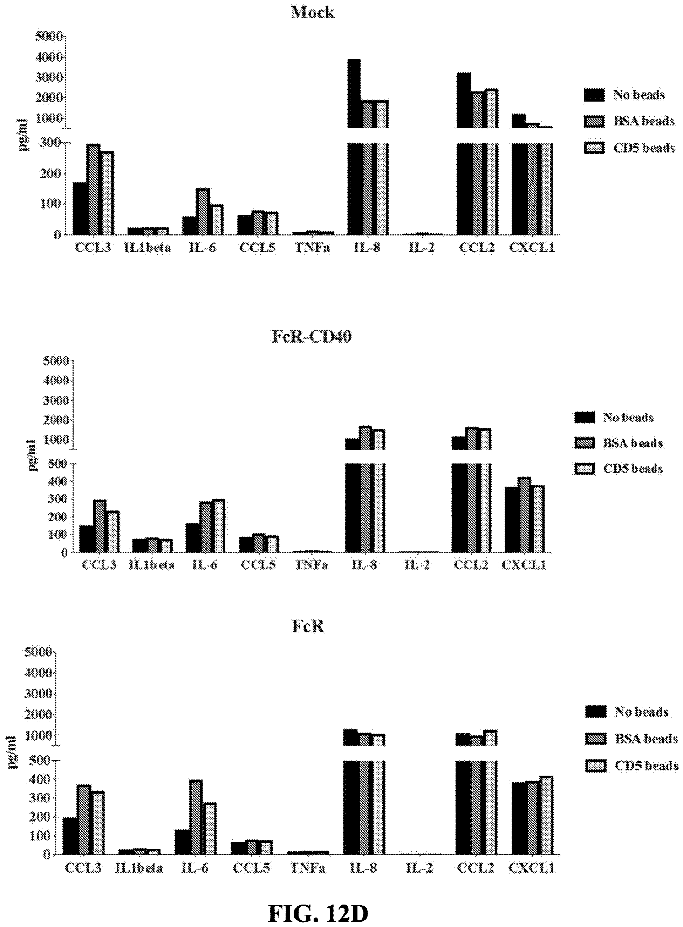

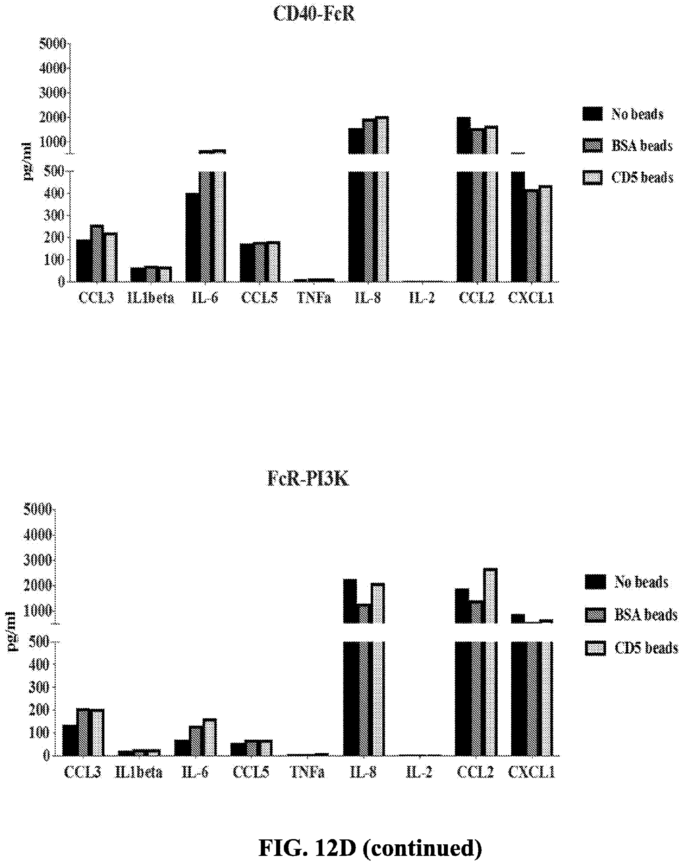

Primary monocytes electroporated with the anti-CD5-CAR construct was assayed for bead engulfment, target specificity and cytokine as above. With pHRodo labeled target cells, (**FIG. 12A**) increased phagocytic engulfment was noticed (**FIGs. 12B** and **12C**) in case of any of the monocytic cells expressing any of the CD5-binder constructs, compared to mock electroporated cells. In another experiment, primary monocytes were electroporated with an anti-CD5-CAR construct (CD5-CD8h-CD8tm-FcR-PI3K) and assayed for phagocytosis and cytokine release. Results are shown in **FIG. 12D.**

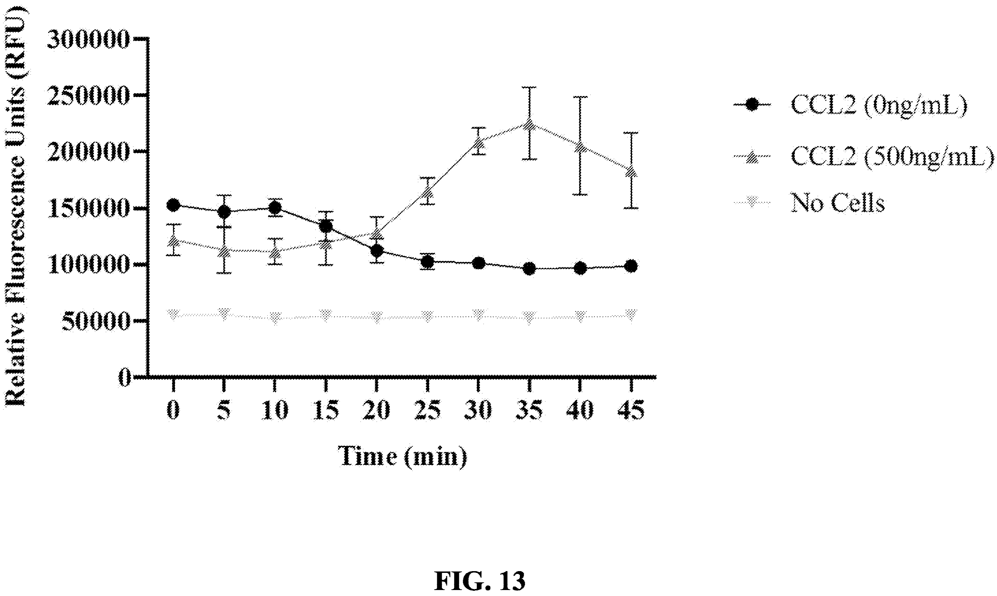

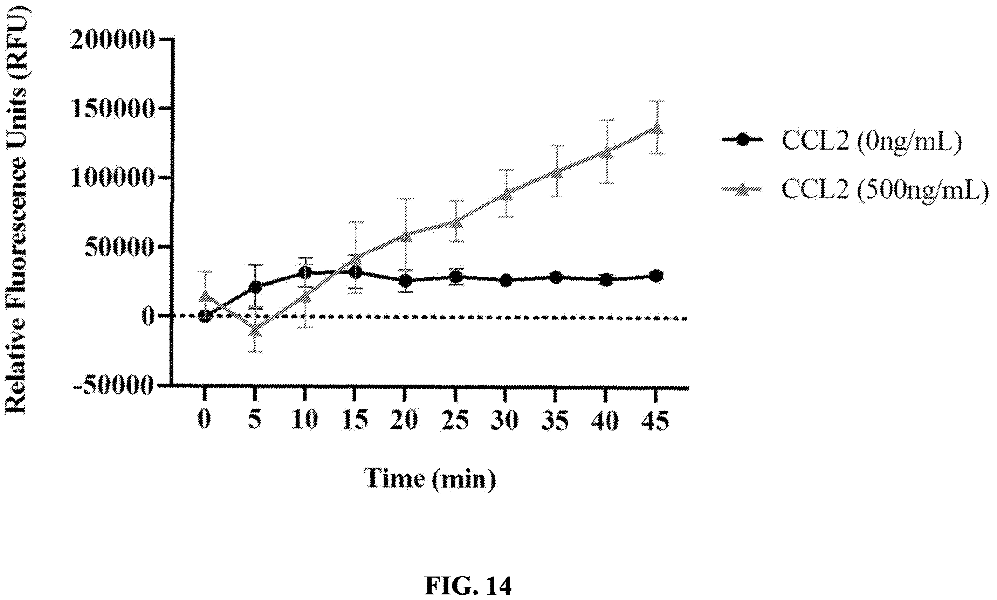

Both THP-1 cells primary monocytes were highly responsive to CCL2 administration in vitro and exhibited increased chemotaxis (**FIGs. 13** and **14** respectively).

## Example 15. In vivo efficacy of anti-CD5 chimeric antigen receptor expressing monocytes on CD5 T lymphoma model

In this example, myeloid cells expressing anti-CD5 chimeric antigen receptor generated according to the methods described in the earlier sections were examined for efficacy in reducing tumor in a mouse H9 T cell lymphoma model. CD5 is expressed on the surface of T cell lymphoma. Anti-CD5 chimeric antigen receptor expressing monocytes are capable of binding CD5-expressing cells. However, whether these anti-CD5-CAR monocytes cells could overcome the TME and exert any anti-tumor potential was tested herein. A T cell lymphoma tumor model was established, in which CD5 positive H9 cells were injected subcutaneously into NSG-SGM3 mice. NSG-SGM3 mouse (Jackson Laboratory, USA) are triple transgenic NSG-SGM3 (NSGS) mice expressing human IL3, GM-CSF and SCF, and combine the features of the highly immunodeficient NOD SCID gamma (NSG) mouse with cytokines that support the stable engraftment of myeloid lineages and regulatory T cell populations. H9-mCherry-Luciferase cell line had been derived earlier as follows: H9 cell line was derived from Hut78 Sezary syndrome T cell line; mCherry-firefly Luciferase fusion protein was stably expressed by transfection of Hut78 with pGLCherry luciferase and selected for stable integration. The mCherry positive cells were further enriched by FACS sorting and currently the cell line is over 80% mCherry positive.

Preparation of tumor cells an administration:

H9-mCherry-Luc cells were cultured in RPMI1640 with 10% FBS. On day of tumor cell injection, the cells were centrifuged at 1000 x g for 3 minutes, the supernatant was removed, and the cells were resuspended in 1:1 diluted Matrigel. $1 \times 10^6$ tumor cells were injected subcutaneously per mouse.

Myeloid cells expressing CD5-CAR (CD-CAR monocytes) were prepared as described above. 200 million cells were electroporated; Post electroporated (EP) monocytes were cultured for 1 hour and cryopreserved. Post thaw analysis showed great viability (>95%). The day of injection of CD5 CAR monocytes was 11 days after implantation of tumors. On the day of treatment with test article, animals were randomized into three groups according to tumor volume (Table 7).

**Table 7 - Dosing regimen in mice**

| | | Amount of cell needed per day ($1x10^6$) | | |
|---|---|---|---|---|
| Dose # | Injection every 3 days | Ctrl group (5 mice) | One dose (5 mice) | Three doses (5 mice) |
| 1 | Day 0 | 0 | 0.8 | 0.8 |
| 2 | Day 3 | 0 | 0 | 1.4 |
| 3 | Day 6 | 0 | 0 | 1.4 |
| 4 | Day 10 | 0 | 0 | 1.4 |
| 5 | Day 13 | 0 | 0 | 1.4 |
| 6 | Day 16 | 0 | 0 | 1.4 |

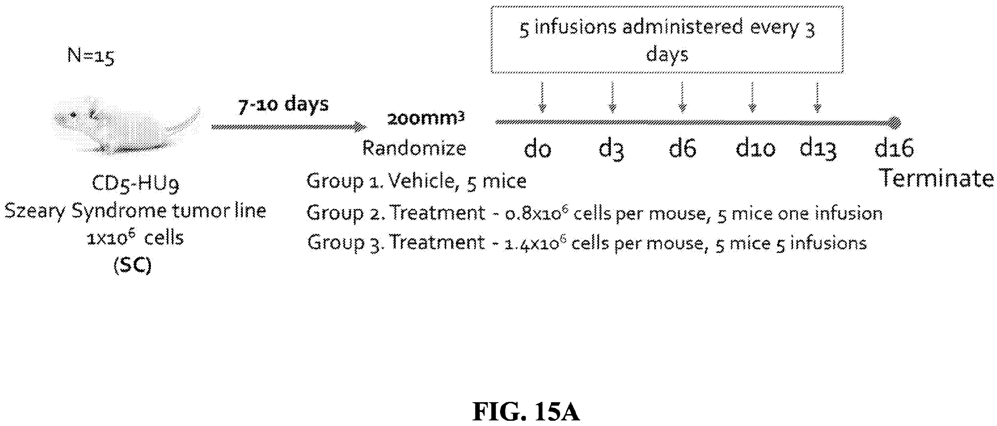

CD5-CAR monocytes were cryopreserved in CryoStor CS10 (1 ml per vial, 25M cells). Cells were thawed quickly in 37°C water bath and directly injected into animals without further processing. Prior to injection of the CD5 CAR monocytes, the areas to be injected were wiped with a 70% isopropyl or ethyl alcohol solution. CD5 CAR monocytes were administered intravenously. The day of CD5-CAR monocyte adoptive transfer was considered Day 0 of the study. Rest of the injection was performed according to Table 8. Test regime is depicted graphically in **FIG. 15A.**

**Table 8 - Injection schedule for CD5-CAR cells in mice**

| Group | # of mice | Tumor ($10^7$cells, IP) | Dose | Test article |
|---|---|---|---|---|
| 1 | 5 | H9 | NA | NA |
| 2 | 5 | H9 | $0.8 \times 10^6$ | CD5-CAR monocyte |

(continued)

| Group | # of mice | Tumor ($10^7$cells, IP) | Dose | Test article |
|-------|-----------|-------------------------|------|--------------|
| 3 | 5 | H9 | $0.8 \times 10^6$ for one dose, and $1.4 \times 10^6$ for five doses | CD5-CAR monocyte |

Tumor measurements and body weights:

Animals were observed daily for clinical signs. Tumor volume was determined by imaging using IVIS (Perkin Elmer, Boston, MA). Mice were injected IP with luciferin (Biovision, catalog # 7903) at a dose or 150 mg/kg (200 $\mu$l) and imaged 10 minutes later using IVIS. Radiance values (photons/sec/cm2) were recorded. IVIS imaging and body weight measurements were made on all animals until death or euthanasia. Tumor were removed at Day 20 post injection of the first dose and weighted.

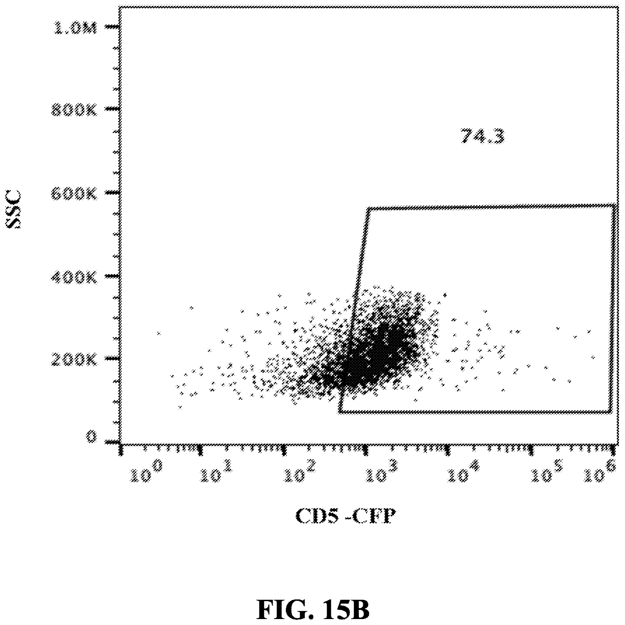

CD5-CAR monocytes were stained with Alexa488 conjugated human CD5 protein following SOP Culture and electroporation of CD14+ monocyte and binder detection at 24, 48 and 72 hours post thaw. Monocytes electroporated with HER2-CAR constructs were used as negative control to determine the position of the gate. The transfection efficiency was found to be 74% at 24hours (**FIG. 15B**). This suggest that electroporation can robustly transfect mRNA into CD14+CD16- monocytes; the expression of the CAR construct was transient with 3-4 days lifetime, potentially due to fast turnover of mRNA and receptor protein.

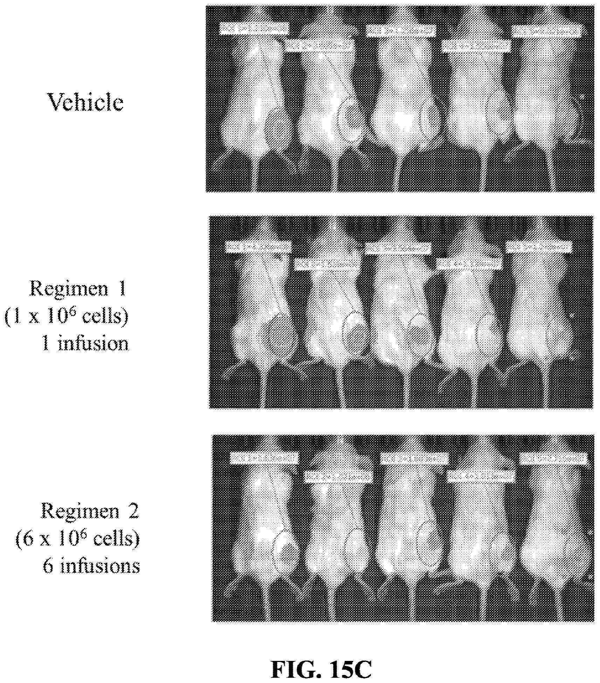

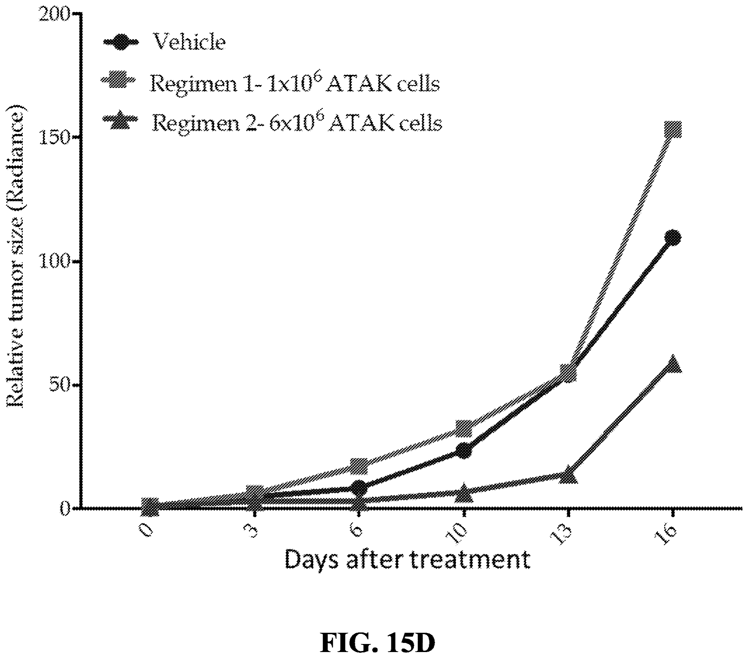

Tumor growth as measured by relative luminescence signal was significantly slower in animals that received 6 dose of $1.4 \times 10^6$ CD5-CAR cells every 3-4 days compared to untreated animals (**FIG. 15C** and **FIG. 15D**). Animals receiving only one dose of $1 \times 10^6$ CD5-CAR cells did not show such tumor stasis effect (**FIG. 15C** and **FIG. 15D**). In the 6 dose group, one animal died between day 13 and 16. At day 20 several mice have very large tumors that are clearly palpable. All animals were sacrificed at day 20 and their tumors were removed by dissection. Tumors were then weighed on a scale and data were plotted in prism.

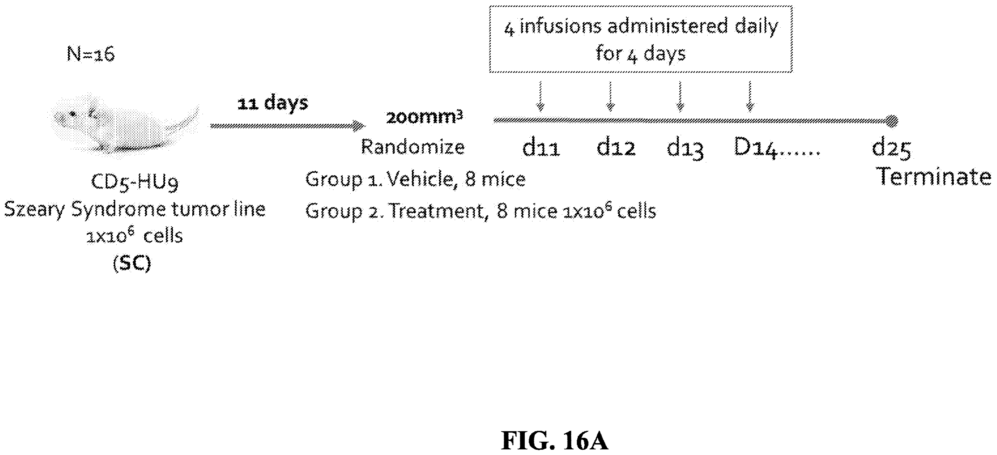

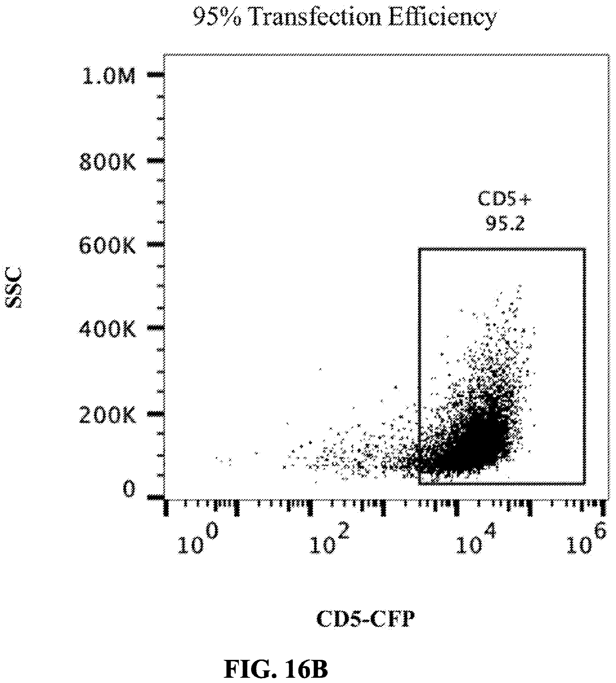

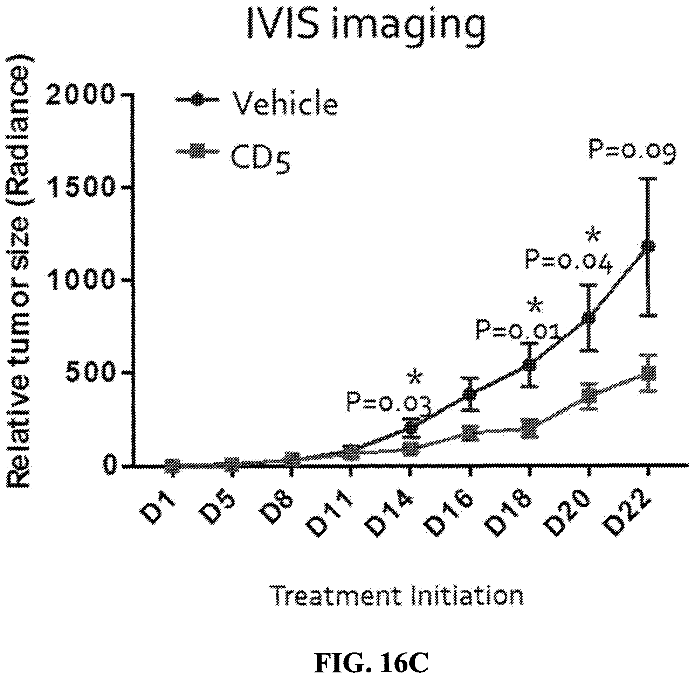

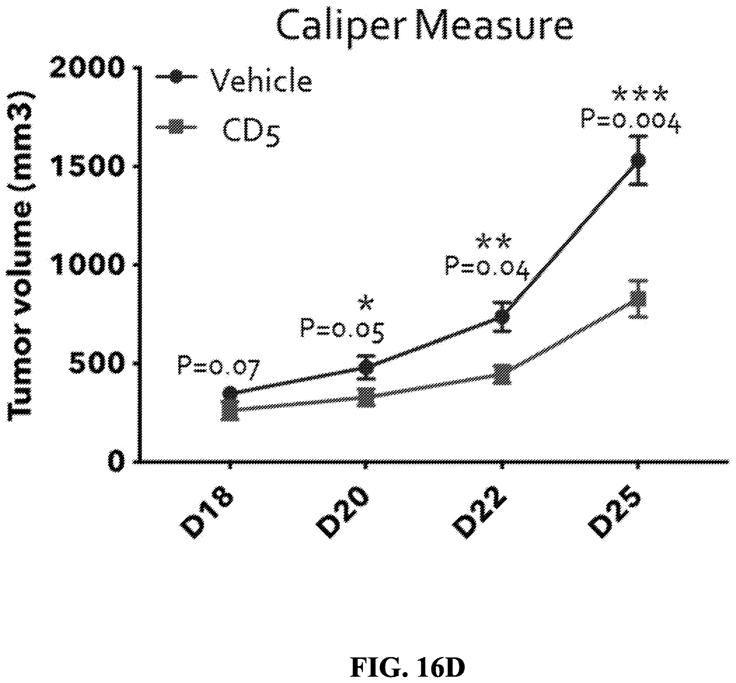

In another study, NSG-SGM3 mice were subjected to a different dosing scheme, as shown in **FIG. 16A.** In this regime, mice were administered 4 infusions at day 11, 12, 13 and 14, once daily. CD5-CAR expression was verified after electroporation and was found to be greater than 95% (**FIG. 16B**). In this assay, statistically significant reduction in tumor growth was recorded, as shown in **FIG. 16C** and **16D.**

From the study exemplified in this section, it was evident that the multiple infusion of CD5-CAR monocytes targeting CD5+ H9 can cause delay of tumor growth. Potentially a higher dose would elicit a much stronger anti-tumor response. NSG-SGM3 mice do not have functional T cells, B cells and NK cells. Therefore, the model is designed to examine the intrinsic anti-tumor activity of the C5-CAR monocytes, which includes phagocytosis and secretion of cytotoxic agents such as TNF$\alpha$ and NO/ROS. A much higher anti-tumor activity can be expected in an immune complete model in which the CAR expressing monocytes can cross-present antigen to activate T cells and to secrete inflammatory cytokine to promote lymphocyte infiltration.

## Example 16. CD5-FcR-MDA5 CFP construct

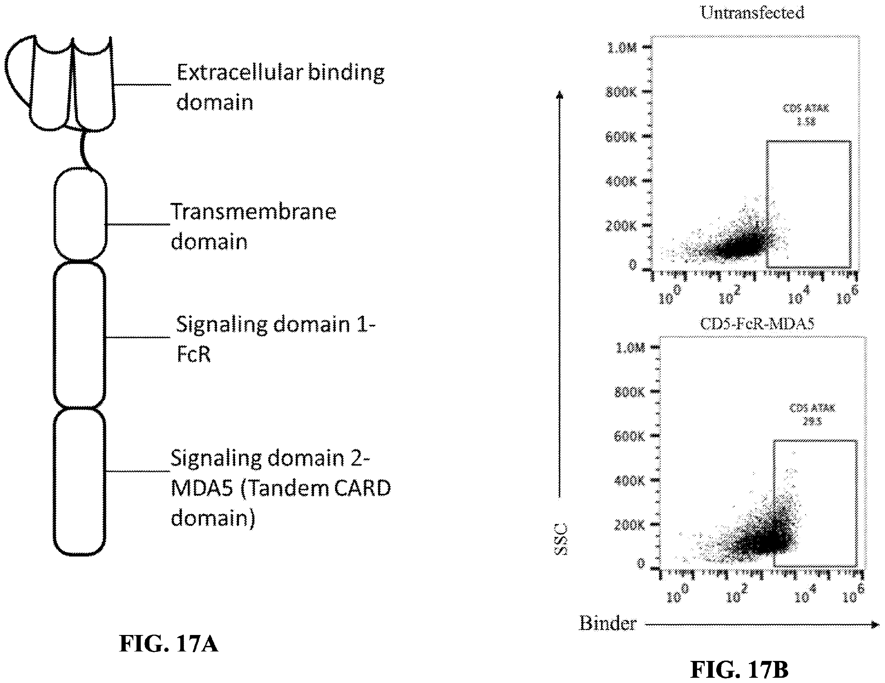

In this example, a CD5-targeted CFP was constructed using known molecular biology techniques having an intracellular domain comprising two caspase activation (CARD) domains of MDA-5 intracellular domain sequence (Tandem CARD ICD sequence as in **SEQ ID NO: 23**). As shown graphically in **FIG. 17A,** the CFP has an extracellular domain comprising a signal peptide fused to an scFv containing a heavy chain variable domain linked to a light chain variable domain that binds to CD5 on a target cell, attached to a CD8$\alpha$ chain hinge and CD8$\alpha$ chain TM domain via a short linker. The TM domain is fused at the cytosolic end with an FcR$\gamma$ cytosolic portion, followed by a MDA5 cytosolic portion (SEQ ID NO: 23).

MWLQSLLLLGTVACSISEIQLVQSGGGLVKPGGSVRISCAASGYTFTNYGMNWV
RQAPGKGLEWMGWINTHTGEPTYADSFKGRFTFSLDDSKNTAYLQINSLRAEDTAVYFCTR
RGYDWYFDVWGQGTTVTVSSGGGGSGGGGSGGGGSDIQMTQSPSSLSASVGDRVTITCRA
SQDINSYLSWFQQKPGKAPKTLIYRANRLESGVPSRFSGSGSGTDYTLTISSLQYEDFGIYYC
QQYDESPWTFGGGTKLEIKSGGGGSGALSNSIMYFSHFVPVFLPAKPTTTPAPRPPTPAPTIAS
QPLSLRPEACRPAAGGAVHTRGLDIYIWAPLAGTCGVLLLSLVITLYCRLKIQVRKAAITSYE
KSDGVYTGLSTRNQETYETLKHEKPPQGSGSMSNGYSTDENFRYLISCFRARVKMYIQVEP
VLDYLTFLPAEVKEQIQRTVATSGNMQAVELLLSTLEKGVWHLGWTREFVEALRRTGSPLA
ARYMNPELTDLPSPSFENAHDEYLQLLNLLQPTLVDKLLVRDVLDKCMEEELLTIEDRNRIA

AAENNGNESGVRELLKRIVQKENWFSAFLNVLRQTGNNELVQELTGSDCSESNAEIEN (**SEQ ID NO: 24**).

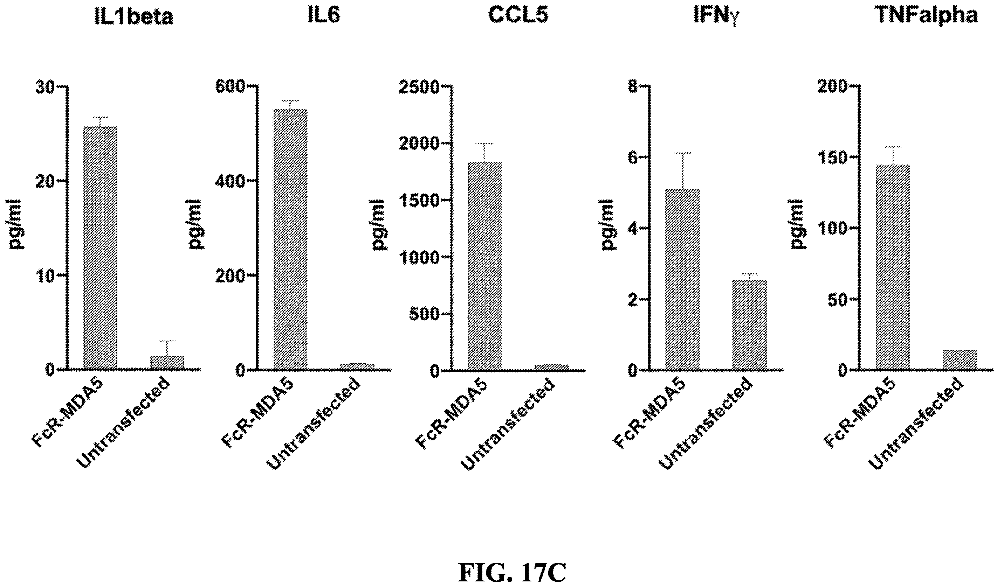

An mRNA polynucleotide having a sequence that includes a sequence encoding SEQ ID NO: 24 was prepared using in vitro transcribed mRNA, and primary macrophages were transcribed with the mRNA. Successful expression of the CD5-FcR-MDA5 chimeric antigen receptor in the primary myeloid cells was noted, as demonstrated in **FIG. 17B.** The CD5-FcR-MDA5 expressing monocytes showed higher level of IL1β, IL6, IFNγ and TNFα secretion, and chemokine CCL5 secretion, than the untransfected cells, as shown in **FIG. 17C,** measured by ELISA assay.

### Example 17. CD5-FcR-TNFR1 or TNFR2 CFP construct

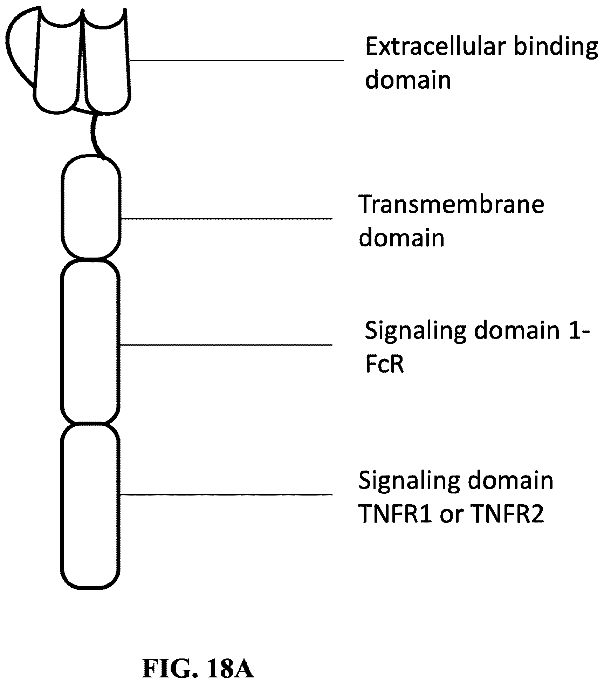

In this example, a CD5-targeted CFP was constructed using known molecular biology techniques having a TNFR1 or TNFR2 intracellular domain. As shown graphically in **FIG. 18A,** the CFP has an extracellular domain comprising a signal peptide fused to an scFv containing a heavy chain variable domain linked to a light chain variable domain that binds to CD5 on a target cell, attached to a CD8α chain hinge and CD8α chain TM domain via a short linker. The TM domain is fused at the cytosolic end with an FcRγ cytosolic portion, followed by an intracellular signaling domain (ICD) sequence TNFR1 (**SEQ ID NO: 21**) or an ICD of TNFR2 (**SEQ ID NO: 22**). The full length sequence of the CFP having a TNFR1 intracellular signaling domain is depicted below:

MWLQSLLLLGTVACSISEIQLVQSGGGLVKPGGSVRISCAASGYTFTNYGMNWVRQAPGKG
LEWMGWINTHTGEPTYADSFKGRFTFSLDDSKNTAYLQINSLRAEDTAVYFCTRRGYDWYF
DVWGQGTTVTVSSGGGGSGGGGSGGGGSDIQMTQSPSSLSASVGDRVTITCRASQDINSYL
SWFQQKPGKAPKTLIYRANRLESGVPSRFSGSGSGTDYTLTISSLQYEDFGIYYCQQYDESP
WTFGGGTKLEIKSGGGGSGALSNSIMYFSHFVPVFLPAKPTTTPAPRPPTPAPTIASQPLSLRP
EACRPAAGGAVHTRGLDIYIWAPLAGTCGVLLLSLVITLYCRLKIQVRKAAITSYEKSDGVY
TGLSTRNQETYETLKHEKPPQGSGSQRWKSKLYSIVCGKSTPEKEGELEGTTTKPLAPNPSFS
PTPGFTPTLGFSPVPSSTFTSSSTYTPGDCPNFAAPRREVAPPYQGADPILATALASDPIPNPLQ
KWEDSAHKPQSLDTDDPATLYAVVENVPPLRWKEFVRRLGLSDHEIDRLELQNGRCLREAQ
YSMLATWRRRTPRREATLELLGRVLRDMDLLGCLEDIEEALCGPAALPPAPSLLR (**SEQ ID NO: 25**) .

The full length sequence of the CFP having a TNFR2 intracellular signaling domain is depicted below:

MWLQSLLLLGTVACSISEIQLVQSGGGLVKPGGSVRISCAASGYTFTNYGMNWVRQAPGKG
LEWMGWINTHTGEPTYADSFKGRFTFSLDDSKNTAYLQINSLRAEDTAVYFCTRRGYDWYF
DVWGQGTTVTVSSGGGGSGGGGSGGGGSDIQMTQSPSSLSASVGDRVTITCRASQDINSYL

SWFQQKPGKAPKTLIYRANRLESGVPSRFSGSGSGTDYTLTISSLQYEDFGIYYCQQYDESP
WTFGGGTKLEIKSGGGGSGALSNSIMYFSHFVPVFLPAKPTTTPAPRPPTPAPTIASQPLSLRP
EACRPAAGGAVHTRGLDIYIWAPLAGTCGVLLLSLVITLYCRLKIQVRKAAITSYEKSDGVY
TGLSTRNQETYETLKHEKPPQGSGSPLCLQREAKVPHLPADKARGTQGPEQQHLLITAPSSSS
SSLESSASALDRRAPTRNQPQAPGVEASGAGEARASTGSSDSSPGGHGTQVNVTCIVNVCSS
SDHSSQCSSQASSTMGDTDSSPSESPKDEQVPFSKEECAFRSQLETPETLLGSTEEKPLPLGVP
DAGMKPS (**SEQ ID NO: 26**).

The expression of in vitro transcribed mRNA encoding the CFPs having TNFα receptor 1 or 2 intracellular domains were tested in transfected primary macrophages. Results are shown in **FIG. 18B.** Shown in **FIGs. 18C** and **18D,** expression of CFP having an TNFR1 intracellular signaling domain shows increased level of cytokine secretion. Conversely, CFP having an TNFR2 intracellular signaling domain showed cytokine levels comparable to the untransfected control cells (**FIGs. 18C, 18D**). Without wishing to be bound by a theory, TNFR2 (p75) plays a tolerogenic and immunosuppressive role in immune response pathway and is largely expressed by regulatory cells, such as certain DC subtypes, and natural Tregs. Therefore, the results shown here clearly indicate that the individual cytoplasmic domains used in the CFP construction play significant roles that individually influence the function of the cell that expresses the CFP. Also indicated in these results that the TNFR2 can be used as a negative control in the functional assays for pro-inflammatory activity of CFP expressing cells.

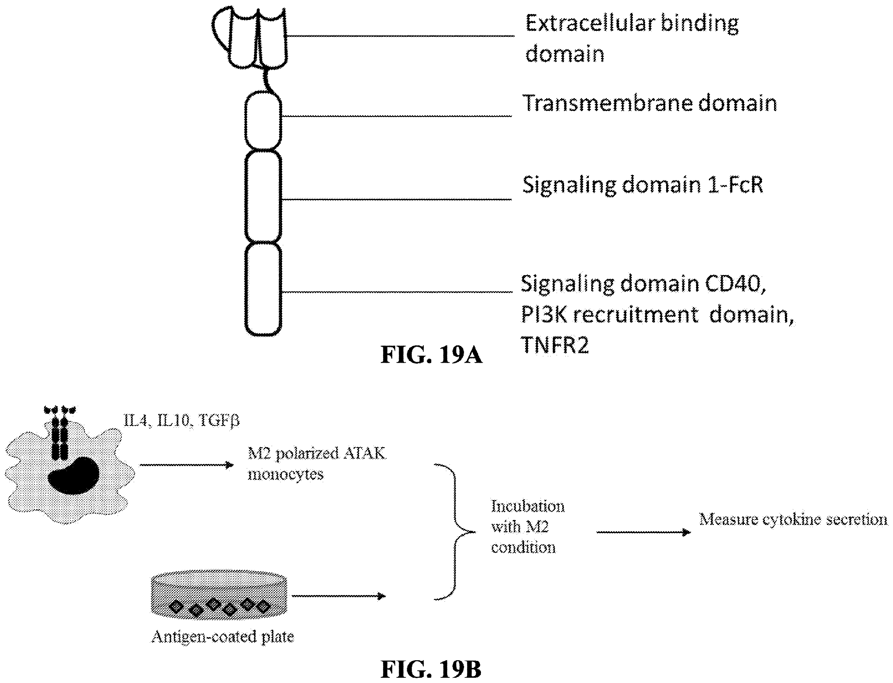

Next, several CD5 targeted constructs were tested for functional assays. Primary monocytes transfected with the polynucleotide constructs encoding respecting CFPs were subjected to culturing in presence of M2 condition (IL4, IL10, TGFβ) for 24 h, following which, these cells were plated on CD5 coated or uncoated (control) plates and cultured for 24 hours. Chemokine and cytokine secretion by the cells were measured. **FIG. 19A** shows a graphical representation of an exemplary construct that can bind CD5 and have different intracellular domains (ICDs), e.g. CD40 ICD, PI3K recruitment ICD, TNFR2 ICD. **FIG. 19B** shows a diagram depicting the experimental design. When subjected to a medium comprising IL4, IL-10 and/or TGFβ, and stimulated by CD5 antigen coated plates (or uncoated control plates) transformation of monocytic lineage cells towards M2 phenotype leading to increase in cytokine and chemokine secretion that are then measured by ELISA. Results are depicted in **FIG. 19C, 19D,** and **19E.** Cells expressing CFPs having PI3kinase (PI3K) recruitment signaling domain secrete high levels of chemokines CCL3, CXCL12, CCL4, CCL5 and KC compared to CFP with CD40 ICD, or negative control CFP TNFR2-ICD-expressing cells (**FIG. 19C**). Cells expressing CFPs having PI3K recruitment domain as well as in some cases CD40 signaling domains exhibit high secretion of the cytokines such as TNF-alpha and IL8.

### Example 18. Efficacy of anti-HER2 chimeric antigen receptor expressing monocytes

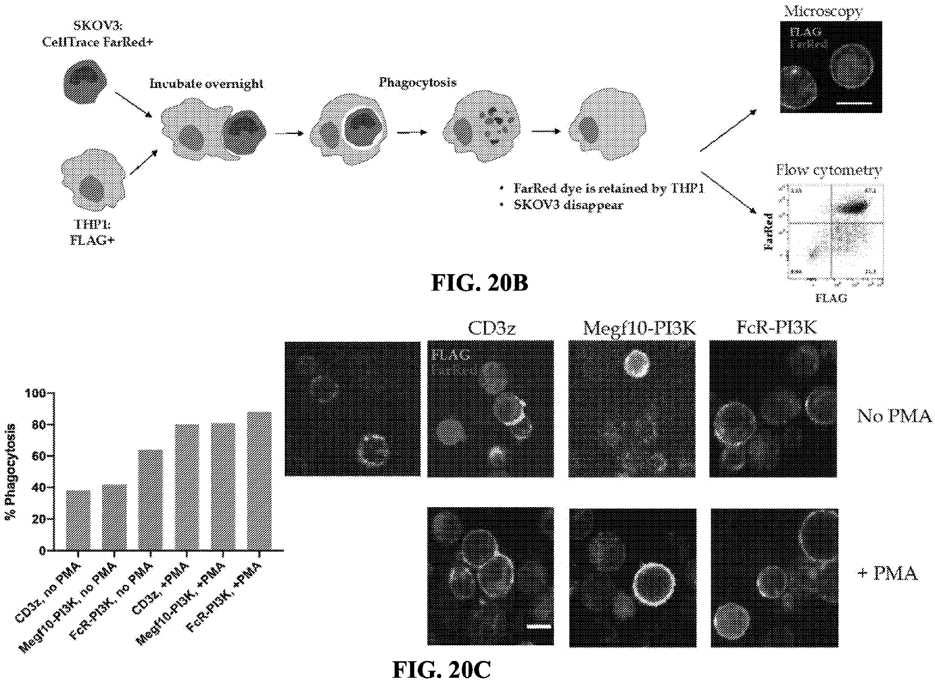

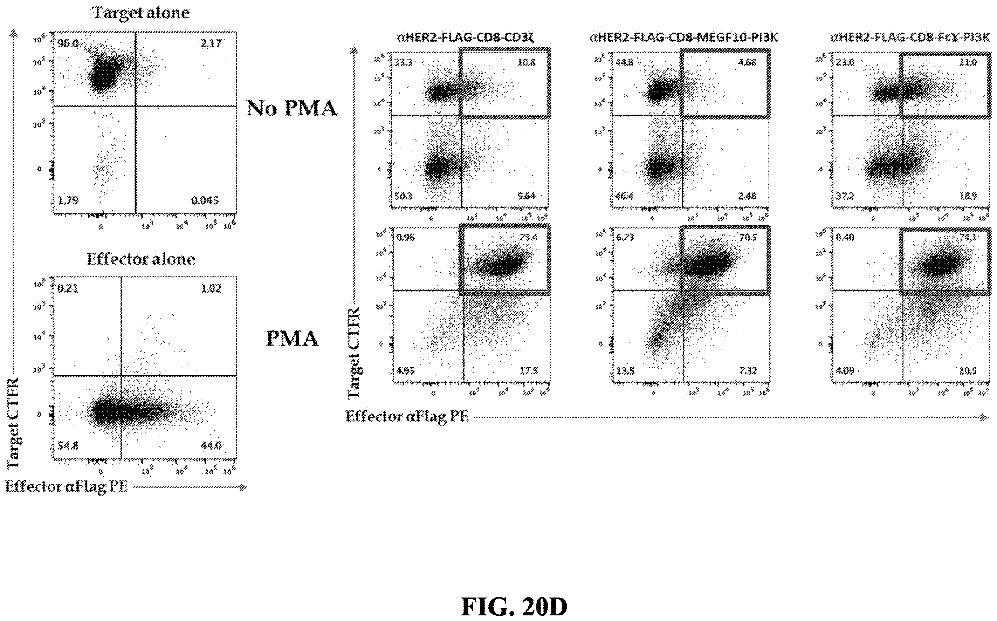

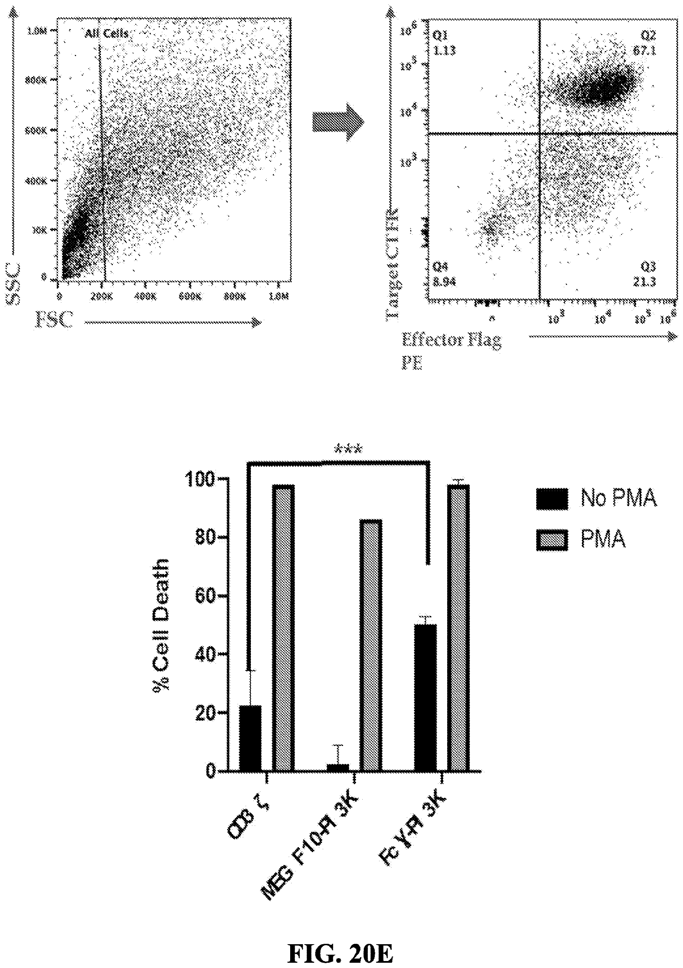

In this example, chimeric fusion proteins (CARs) having an extracellular HER2 antigen binding domain of the exemplary design described in the disclosure are analyzed for functional efficacy as potential anti-cancer agents. First generation Lentiviral vector was used to generate lentiviruses used to transduce the myeloid THP1 cell line. Transduction efficiency in PMA treated THP1 cells ranged from 67-90% (**FIG. 20A**). THP1 cells expressing HER2 targeting constructs activated with or without PMA, were incubated overnight with FarRed labelled SKOV3 ovarian tumor cells for analyzing phagocytosis. The experimental set up is depicted in the schematic diagram of **FIG. 20B.** Microscopic imaging and flow cytometry (**FIG. 20C** and **FIG. 20D**) was used was used to determine phagocytosis. The FarRed+FLAG+ cells were considered to be phagocytic events. In this experiment the FcRy-PI3K expressing construct was observed to have enhanced efficacy over the other constructs in non-activated THP-1 cells. Upon activation all receptors were associated with phagocytosis (**FIG. 20D**). Target cell death was calculated by the formula: [(# SKOV3 alone-#SKOV3 with effectors)/# SKOV3 alone] x 100; and results are shown as in **FIG. 20E.**

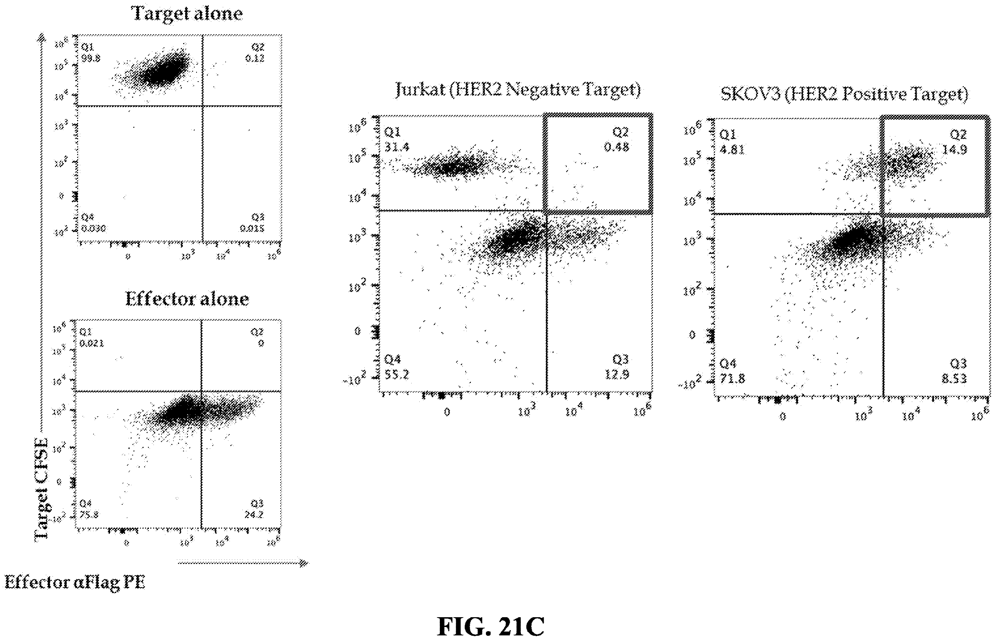

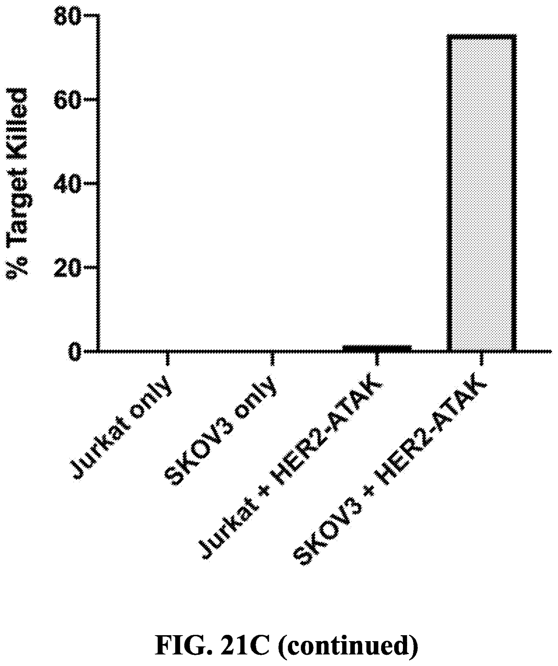

CD14+ cells isolated from healthy donor were transduced with lentiviral HER2 targeted CFP constructs encoding FcRγ + PI3K intracellular domain were analyzed for phagocytosis and killing of CSFE labeled SCOV3 tumor cells (**FIG. 21A**). Results are shown in **FIG. 21B** and **FIG. 21C.** Jurkat cells were used as control for target cell as these cells do not express HER2.

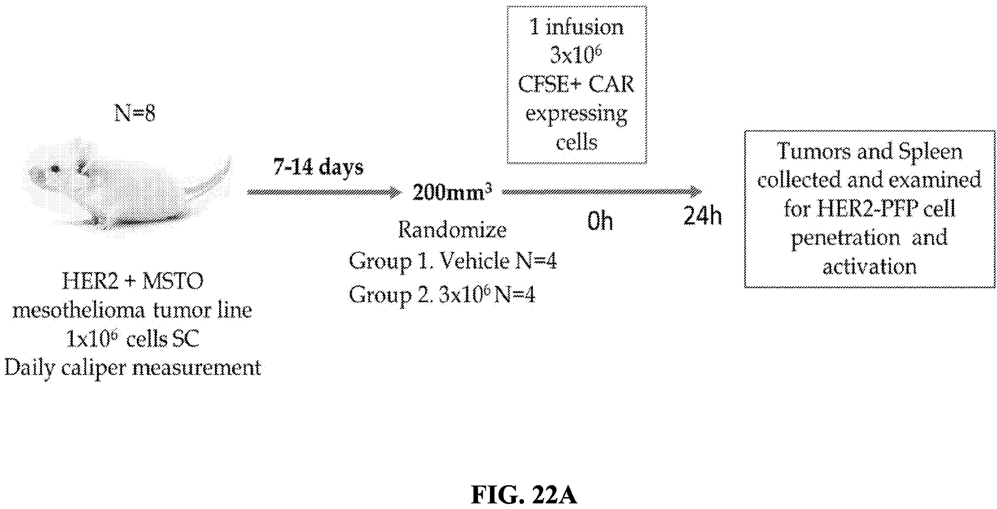

In order to test whether these cells expressing the HER2 constructs were capable of differentiating into M0, M1, M2 phenotypes in a tumor environment, an in vitro mesothelioma model with MSTO cell supernatant was developed, as outlined in the schematic diagram in **FIG. 22A.** HER2 targeted chimeric antigen receptor expressing CD14+ cells were exposed to 6 culture conditions: M0 (100ng/ml MCSF); M1 (5ng/ml LPS+100ng/ml IFNγ); M2 (100ng/ml MCSF + 20ng/ml IL-10 + 20ng/ml TGFβ); DC (100ng/ml GMCSF + 20 ng/ml IL-4); Tumor conditioned media (MSTO-condition RPMI + 100 ng/ml MCSF); and control. For some of these experiments, a sequence encoding a FLAG peptide is incorporated in between the scFv and the transmembrane domain, in the extracellular region of the chimeric HER2 construct. Cells were harvested and cell viability was tested and found to be greater than 80%. The phenotype of the cells was examined by flow cytometry at 24 hours. The expression of several cell markers at 24, 48 and 72 hours was determined. The expression of the HER-2-chimeric construct was detected using fluorescently labelled purified HER2 protein. Under all conditions the construct was found to be expressed, albeit under M1 conditions the expression was the lowest. All other conditions were associated with high levels of the chimeric receptor expression. The cells were also observed to differentially express HLA DP, DQ, DR (MHCII) and HLA ABC(MHC1). The expression of CD14, CD11c, CD379, CD303, CD45, CX3CR1 was consistent across all culture conditions, whereas, Mannose receptor (CD206), MERTK and CCR2 were associated down regulated on those cells cultured in M1 conditions, which is associated with differentiation of the cells. Taken together this data shows that HER2-CFP expressing cells will differentiate in the absence of receptor ligation based on the environmental cues.

In another extension of the assay, expression of the surface molecules on HER2-CFP expressing cells under differential culture conditions in the presence of MSTO tumor cells. Down regulation of CCR2 in the presence of tumor cells was observed and is indicative of receptor ligation and cell activation. The maintenance of HLA molecule expression may indicate maintenance of antigen processing and presentation capability. These data indicate that the receptor engagement triggers activation and activity associated with tumor destruction irrespective of polarization.

An in vivo model for a HER2 expressing tumor was utilized to investigate the tumor penetration and activation of the cells expressing a HER2-CFP. A schematic diagram of the experimental design is shown in **FIG. 22A.** Migration and penetration of the HER2-targeted CFP expressing cells was determined at 24 hours after a single infusion of the CFP expressing cells that have been labeled with cytoplasmic dye CSFE. Tumors were removed and processed for histology. As shown in **FIG. 22B,** HER2-CFP expressing myeloid cells were observed to migrate into the tumor and accumulate around tumor cells. Twenty four hours after CFSE labelled HER2-targeted CFP expressing cell administration in MSTO tumor bearing NSG mice, spleens were removed and processed for histology. As shown in **FIG. 22C,** HER2-targeted CFP expressing myeloid cells were observed to migrate into the spleen. CFSE labelled HER2-targeted CFP expressing cells were isolated from the spleen of tumor bearing mice 24 hours after the cell infusion and examined by flow cytometry. HER2-targeted CFP expressing cells in the spleen maintained expression of HLA, CD14 and CD303. CCR2 expression was observed to decrease with a concurrent increased in CD370 (CLEC9A), potentially suggesting these cells migrate into the spleen and develop into a professional APC capable of stimulating T cells responses. CD206 (Mannose) expression was observed to decrease as did CD45. The reduction of mannose receptor expression may be associated with differentiation into M1 phenotype.

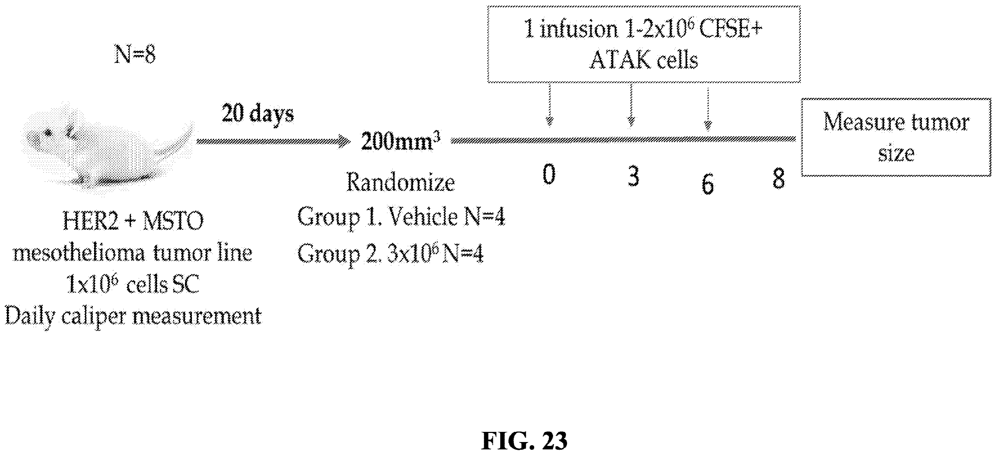

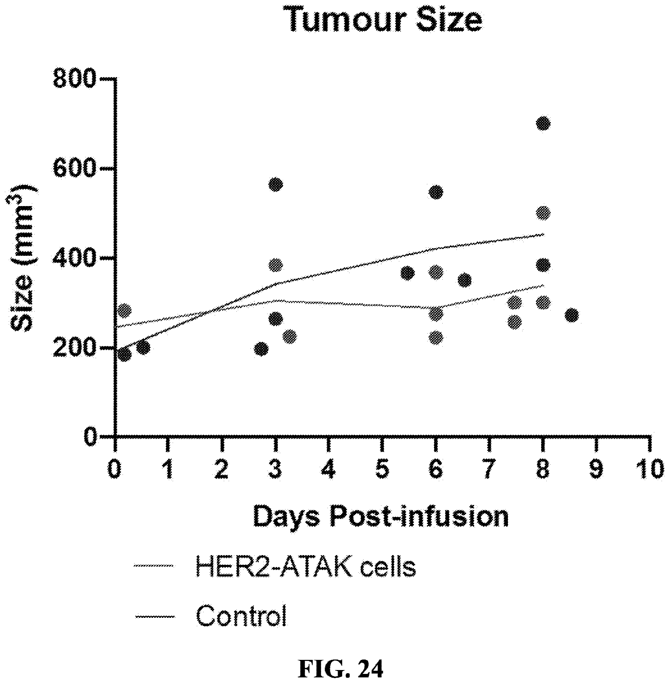

In a similar in vivo tumor model, tumor regression was analyzed after three infusions of the HER2-targeted CFP expressing cells as shown in the schematic diagram in **FIG. 23.** Three infusions of human primary monocytes expressing an anti-HER2 chimeric antigen receptor was associated with a delay in tumor progression compared to control treated animals (**FIG. 24**).

## Example 19. Materials and Methods for blocking anti-phagocytic signal and activating phagocytosis:

Dulbecco modified Eagle medium, trypsin-EDTA, wortmanni (Bradford reagent, and lysostaphin are purchased from Sigma-Aldrich, Inc. (St. Louis, MO). Reduced serum Opti-MEM I medium are purchased from Gibco-BRL (Gaithersburg, MD). SH-5 was acquired from Enzo Life Sciences (Plymouth, PA), and OSU-03012 (OSU) was purchased from Cedarlane Labs (Burlington, NC). FuGENE transfection reagent and the 50x EDTA-free protease inhibitor cocktail are purchased from Roche Applied Science (Manheim, Germany). Cells are grown in 24-well plates to 60 to 70% confluence, and the culture medium was changed to DMEM 10% FCS. Then, in order to have a similar protein expression 5 ng of pCMV5-Akt-CA or 200 ng of pCMV5-Akt-DN in 1.2 μl of FuGENE transfection reagent (ratio, 4:1 [FuGENE-plasmid]) are added to BEC in reduced serum Opti-MEM I medium according to the manufacturer's instructions.

## Example 20. Expression and functional analysis of the recombinant negative SIRPα

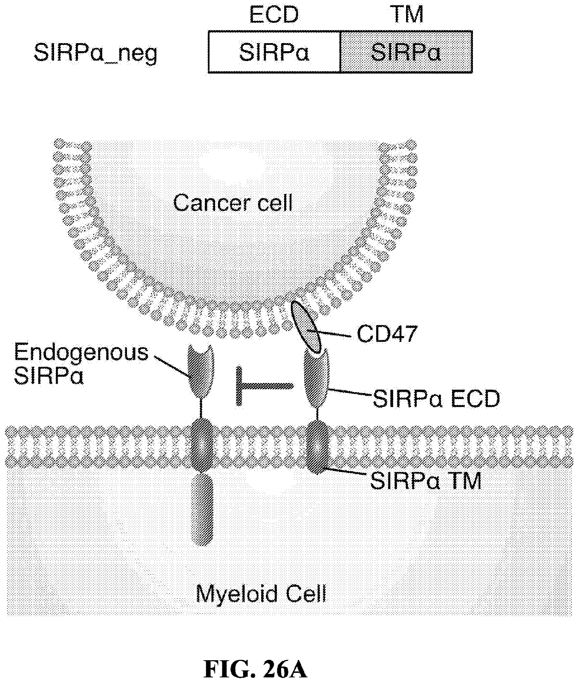

**FIG. 26A** shows illustrative schematics of dominant negative SIRPα receptor (SIRPa_neg). The receptor is composed of the ECD and TM domain of SIRPα without any intracellular domain. ECD: extracellular domain; TM: transmembrane domain. When expressed in macrophages, SIRPα_neg binds to CD47 ligand but does not signal, therefore it act as a dominant negative decoy receptor that inhibit CD47 signaling.

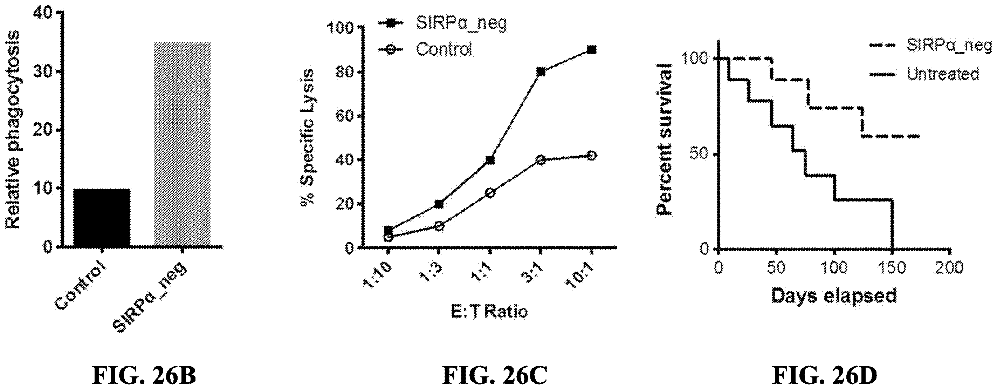

To test the function of the recombinant negative SIRPα (SIRPα_neg), human primary macrophages are transduced with a lentiviral vector expressing the recombinant negative form of SIRPα. In parallel, control cells are transfected with an empty vector or the same vector expressing GFP. **FIG. 26B** shows the expected result in an in vitro phagocytosis assay. Human primary macrophage transduced with control empty vector or SIRPα_neg are co-cultured with dye loaded tumor cells, and phagocytosis is quantified using flow cytometry. The cells with the SIRPα_neg vector show increased phagocytosis over control cells.

**FIG. 26C** shows the expected result in an in vitro cell lysis assay. Human primary macrophage are transduced with control vector or SIRPα_neg vector are co-cultured with tumor cells expressing luciferase at different E:T ratio, and specific lysis is quantified using luciferase assay.

**FIG. 26D** shows the expected result in a mouse xenograft model. On day 0, NSG mice are injected with tumor cells expressing luciferase. Mice are either untreated or injected with human primary macrophage transduced with SIRPα_neg, and a survival curve is generated.

## Example 21. Expression and functional analysis of recombinant chimeric protein expressing a SIRPα-PI3K binding domain construct

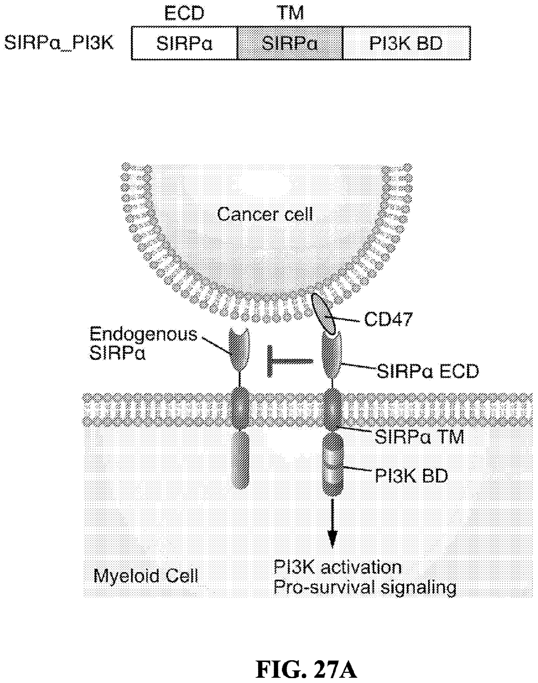

**FIG. 27A** shows illustrative schematics of SIRPα-PI3K switch receptor. The receptor is composed of the ECD and TM domain of SIRPα fused to a PI3K BD at the intracellular end. ECD: extracellular domain; TM: transmembrane domain; PI3K BD: PI3K binding domain. When expressed in macrophages, SIRPα-PI3K binds to CD47 ligand and activate PI3K mediated pro-phagocytosis, pro-survival signaling.

For testing the recombinant construct of **FIG. 27A** is inserted in a Lentiviral expression vector, and purified for use in transfection.

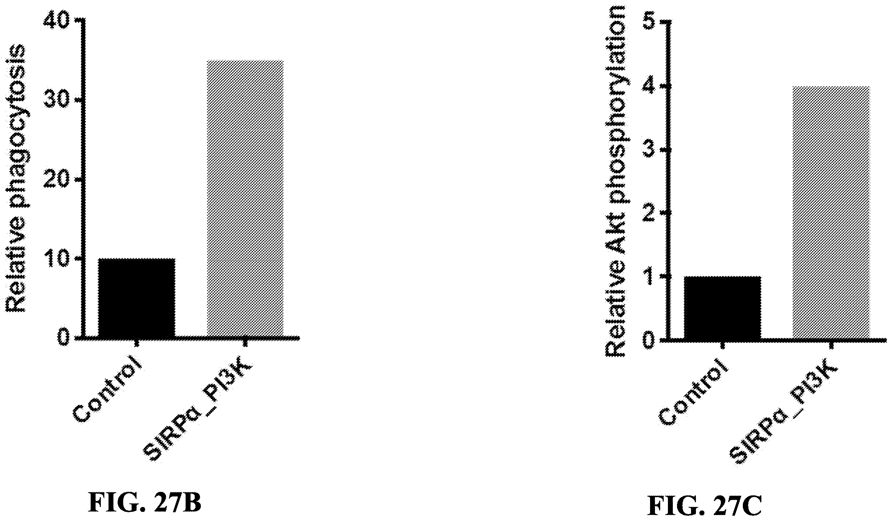

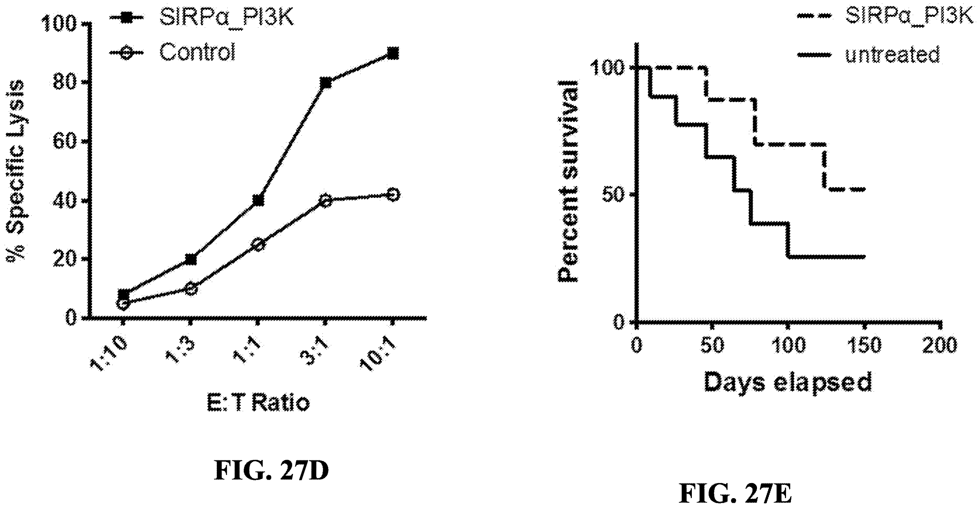

Human primary macrophage transduced with SIRPα-PI3K are co-cultured with target tumor cells. In control set, human macrophages are transduced with a control construct expressing GFP. **FIG. 27B** shows the expected result of relative phagocytoses of the dye loaded target tumor cells, quantified by flow cytometry. Cells expressing SIRPα-PI3K exhibit enhanced phagocytosis over cells expressing the control vector. **FIG. 27C** shows the expected result of measuring Akt activation level. Human primary macrophage expressing SIRPα-PI3K as well as a control construct (GFP) or SIRPα-PI3K and co-cultured with tumor cells, and the level of Akt phosphorylation downstream of PI3K activation is determined by western blot using a pAkt antibody and quantified. **FIG. 27D** shows the expected result in an in vitro cell lysis assay. Human primary macrophage expressing SIRPα-PI3K as well as a control construct (GFP) or SIRPa-PI3K are co-cultured with tumor cells expressing luciferase at different E:T ratio, and specific lysis is quantified using luciferase assay. **FIG. 27E** shows the expected result in a mouse xenograft model. On day 0, NSG mice are injected with tumor cells expressing luciferase. Mice are either untreated or injected with human primary macrophage expressing CAR-P SIRPα-PI3K.

## Example 22. Design and functional analysis of recombinant chimeric protein expressing SIRPα-M1.

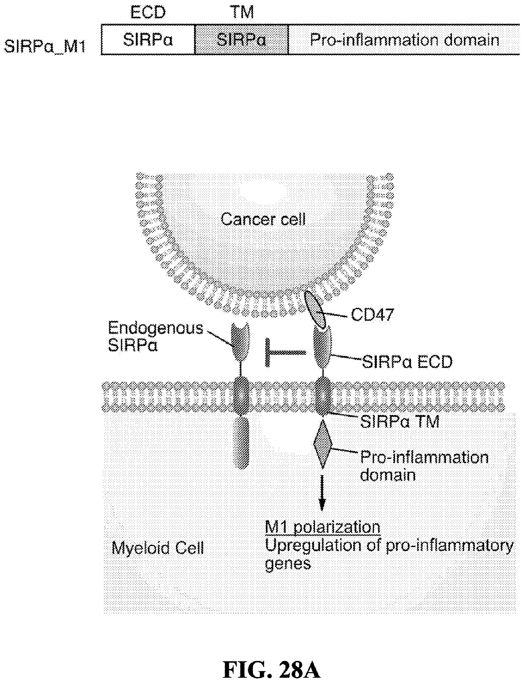

This example shows an exemplary design of a construct have an extracellular CD47 binding domain of SIRPα, and is fused with an intracellular domain that activates pro-inflammatory signaling (SIRPα-M1). **FIG. 28A** shows illustrative schematics of SIRPα switch receptor that triggers pro-inflammation signaling (SIRPα-M1). The receptor is composed of the ECD and TM domain of SIRPα fused to a pro-inflammatory domain of any one of the genes: TLR3, TLR4, TLR9, MYD88, TRIF, RIG-1, MDA5, CD40, IFN-receptor or other genes which have such pro-inflammatory intracellular signaling domains.

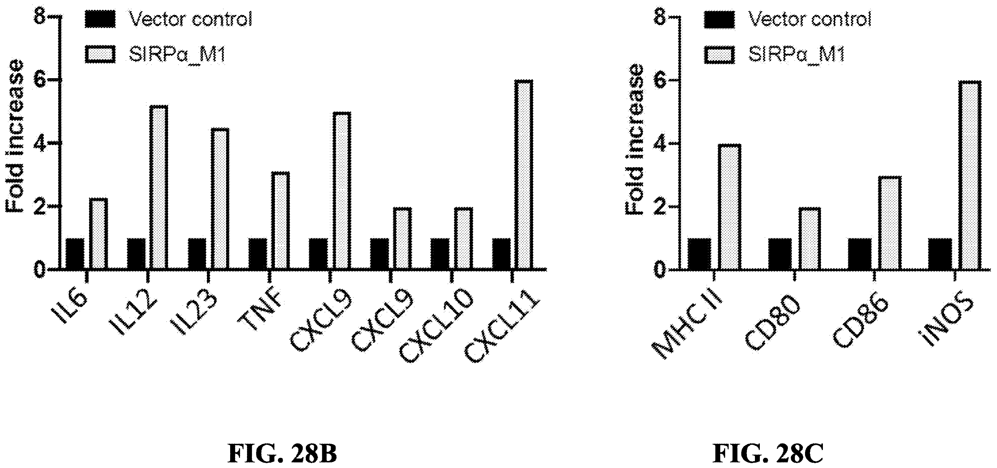

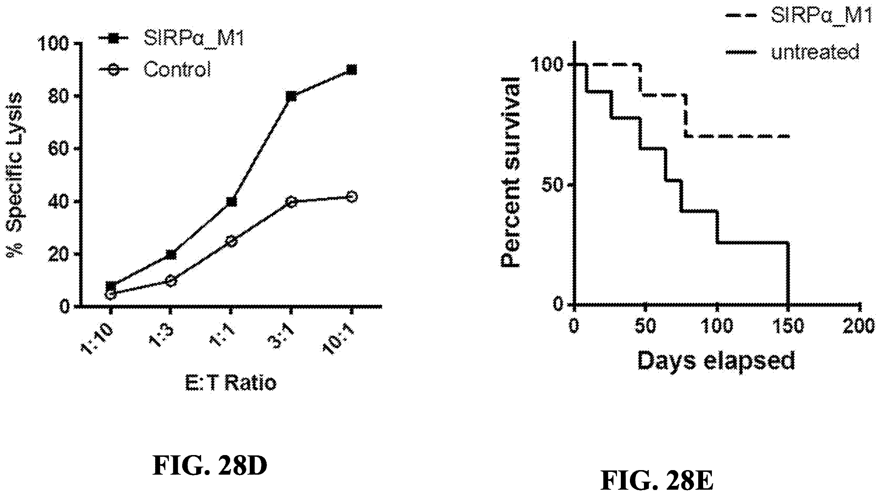

The construct is inserted in a lentiviral vector and purified for functional studies. When expressed in macrophages, binding of CD47 SIRPα-M1 leads to activation of pro-inflammatory signal. This leads to stronger phagocytosis, expression of pro-inflammatory cytokines and surface receptors, as well as enhancement of antigen crosspresentation. **FIGs. 28B** and **28C** show the expected result of induced expression of cytokines and surface antigens respectively when macrophages expressing SIRPα-M1 are co-cultured with target tumor cells. Human primary macrophage transduced with control empty vector or SIRPα-M1 are co-cultured with tumor cells. Cytokine profiling with ELISA shows increased secretion of pro-inflammatory cytokines and chemokines compared to vector control. Flow cytometry shows an increase of M1 state marker expression compared with vector control. **FIGs. 28D** and **28E** show the expected result in an in vitro cell lysis assay and in vivo xenograft mouse model. In **FIG. 28D,** human primary macrophage co-expressing CAR-P as well as SIRPα-M1 are co-cultured with tumor cells expressing luciferase at different E:T ratio, and specific lysis is quantified using luciferase assay. In **FIG. 28E,** NSG mice are injected with tumor cells expressing luciferase on day 0. Mice are either untreated or injected with human primary macrophage co-expressing CAR-P SIRPα-M1.

## Example 23. Design and functional analysis of recombinant chimeric protein expressing SIRPαβ- switch receptor

In this example, an exemplary design of a vector expressing the SIRPαβ switch receptor is described. **FIG. 29A** shows illustrative schematics of SIRPαβ switch receptor. The receptor is composed of the ECD of SIRPα fused to the TM and ICD of SIRPβ. ECD: extracellular domain; TM: transmembrane domain; ICD: intracellular domain. Unlike SIRPα, SIRPβ does not bind to CD47 but instead associate with DAP12 through its TM region and promotes phagocytosis. When expressed in macrophages, SIRPαβ binds to CD47 ligand and also associate with DAP12 to promote phagocytic signaling. Human macrophages are transduced with SIRPαβ and a control vector for functional analysis.

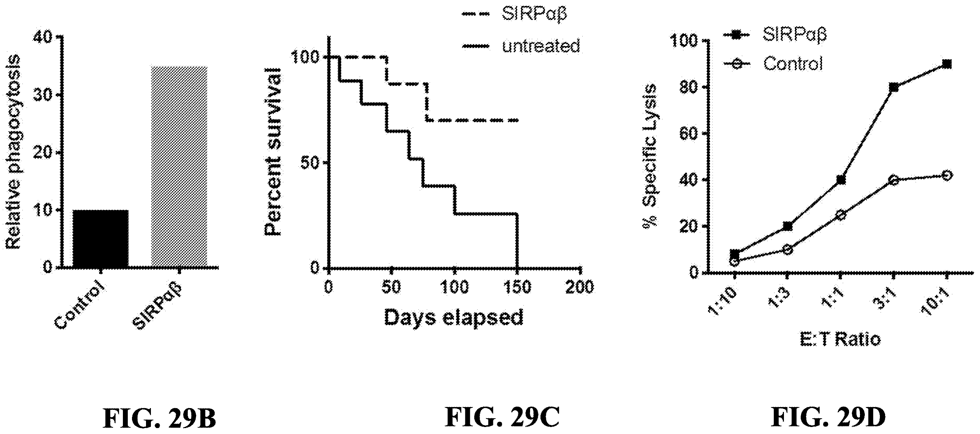

**FIG. 29B** shows the expected result of relative phagocytoses of the dye loaded target tumor cells, quantified by flow cytometry. Cells expressing SIRPαβ exhibit enhanced phagocytosis over cells expressing the control vector. **FIG. 29C** shows the expected result of relative lysis of the dye loaded target tumor cells. Cells expressing SIRPαβ exhibit higher target cell lysis over cells expressing the control vector. When NSG mice are injected with tumor cells expressing luciferase on day 0. Mice are either untreated or injected with human primary macrophage co-expressing CAR-P SIRPαβ and a survival curve is generated (**Fig. 29D**).

## Example 24. Design and functional analysis of recombinant chimeric protein expressing Siglec switch receptor.

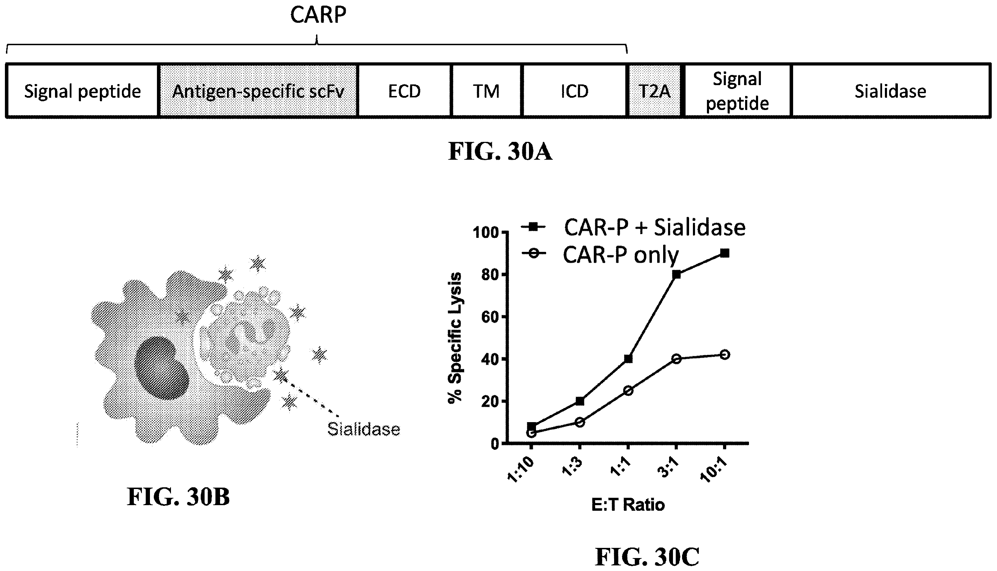

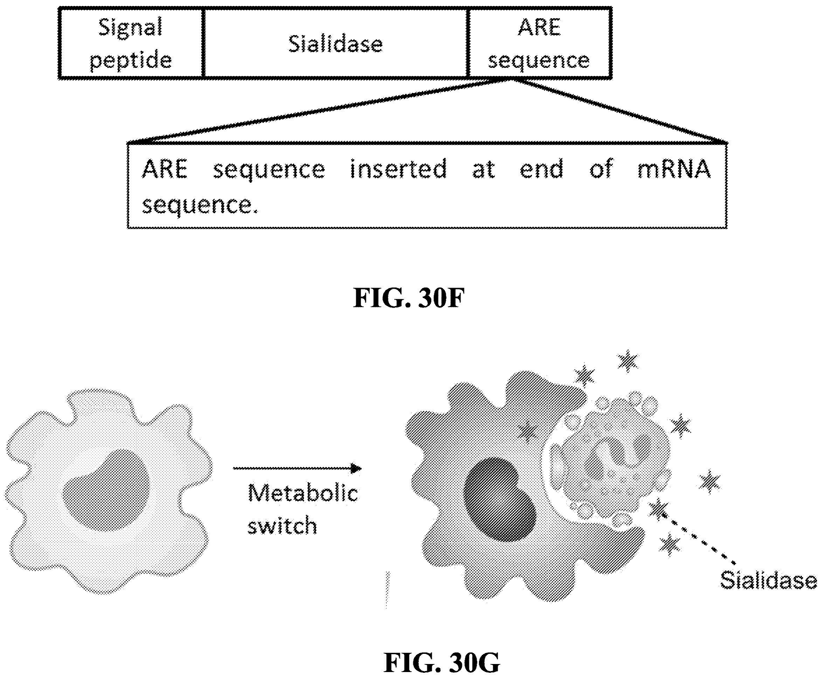

In this example, an exemplary design of a vector expressing the Siglec switch receptor is described. **FIG. 30A** shows illustrative schematics of monocistronic Siglec switch receptor. The chimeric receptor has two parts: the chimeric receptor for phagocytosis (CARP) and a sialidase. The CARP is composed of a signal peptide for membrane localization of the translated protein, an extracellular domain, which has an antigen binding domain. The antigen binding domain is a scFv specifically directed to an antigen on the target cell, e.g., a cancer cell. This antigen binding domain is fused with an ECD, and TM domains of a Siglec protein. The ICD domain can be an ICD domain that promotes phagocytosis, such as the signaling domain from a phagocytic receptor. The construct encodes a short T2A cleavage site and a downstream coding sequence for a sialidase. The sialidase has a preceding signaling sequence for extracellular release of the enzyme sialidase, which is expected to remove the sialylated residues in the extracellular environment of the cell expressing the construct. Sialylated glycans are ligands for the siglec proteins, and therefore expression of the sialidase depletes the natural ligands for siglec protein, rendering the ECD of the siglec proein in the chimeric receptor inert. ECD: extracellular domain; TM: transmembrane domain; ICD: intracellular domain. When expressed in macrophages, Siglec-Sialidase CARP enhances phagocytosis instead of inhibiting phagocytosis as with endogenous siglec signaling. Human macrophages are transduced with the Siglec-Sialidase CARP and a control vector for functional analysis.

**FIG. 30C** shows the expected result of relative lysis of the dye loaded target tumor cells. Cells expressing Siglec-Sialidase CARP higher target cell lysis over cells expressing a CARP control vector that expressed the Siglec CARP construct alone, without the sialidase.

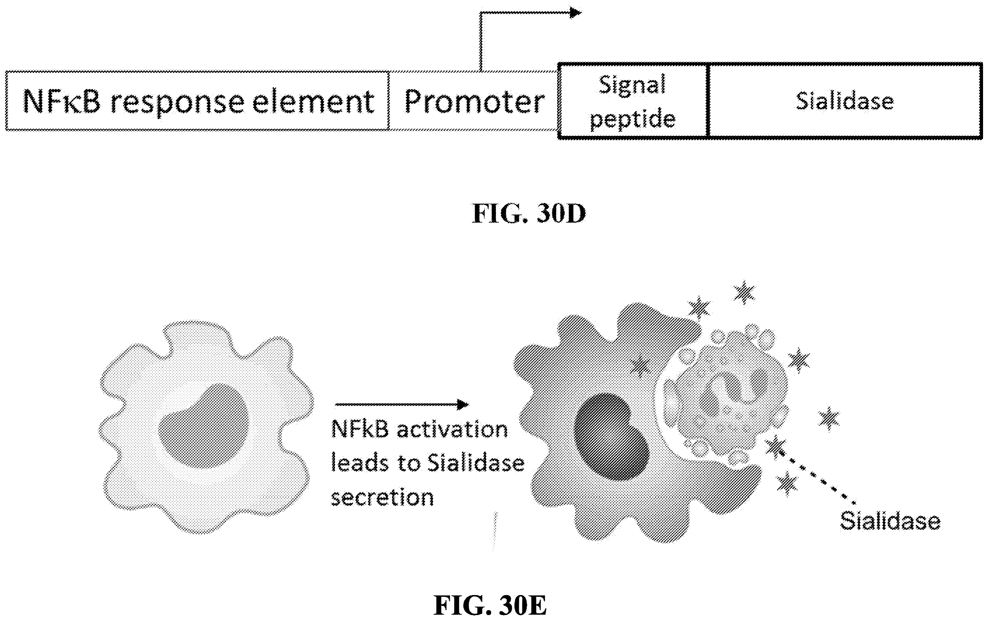

**FIGs. 30D-30G** show additional exemplary designs for the sialidase CARP construct. These could be an incorporated section within the construct described in **FIG. 30A,** where the additional elements are the regulatory elements in the 5' and 3' flanking ends of the coding sequence for the sialidase. As shown in **FIG. 30D,** the sialidase construct is under the control of a separate promoter than the CARP (this is a polycistronic construct in contrast to that in **FIG. 30A**), where the promoter is preceded by an NF-κB response element. NF-κB pathway is activated in a phagocyte. Therefore the sialydase expresses under the influence of the NF-κB response element and therefore is selectively expressed in the actively phagocytosing macrophages (as shown in **FIG. 30E**). **FIG. 30F** shows addition of the specific ARE protein binding sequences in the 3'-UTR of the sialidase construct to regulate its expression in selective macrophages. For example, inserting of sequence motif for binding of a GAPDH to the mRNA can provide a regulated expression that is triggered upon change in glycosylation state of GAPDH. Exemplary GAPDH ARE binding sequence is: WWWU(AUUUA)UUUW (SEQ ID NO: 35) (where W is A or U). GAPDH is an mRNA binding protein. When GAPDH remains bound to the mRNA, the mRNA is prevented from transcription and is therefore silent. Change of glyocylation activates the GAPDH and dissociates it from the mRNA. This triggers transcription of the mRNA. Hypoxic conditions can trigger the change in glycosylation state. Therefore this construct can be activated an expressed in hypoxic conditions, such as in tumor microenvironment.

## Example 25. Design and functional analysis of recombinant chimeric protein expressing an FcRalpha receptor.

In this example, an exemplary design of a vector expressing the cancer antigen targeted receptor with a macrophage specific expression is described. In this exemplary construct the extracellular antigen binding domain is a scFv that can specifically bind to a cancer antigen. The extracellular transmembrane and intracellular domains of an FcRα chain is fused with the scFv through a linker (CAR-FcRγ. The FcRγ chain heterodimerizes with endogenous transmembrane domain of FcRγ which are expressed specifically in macrophages. **FIG. 31A** shows illustrative schematics of the cancer targeting FcRγ receptor.

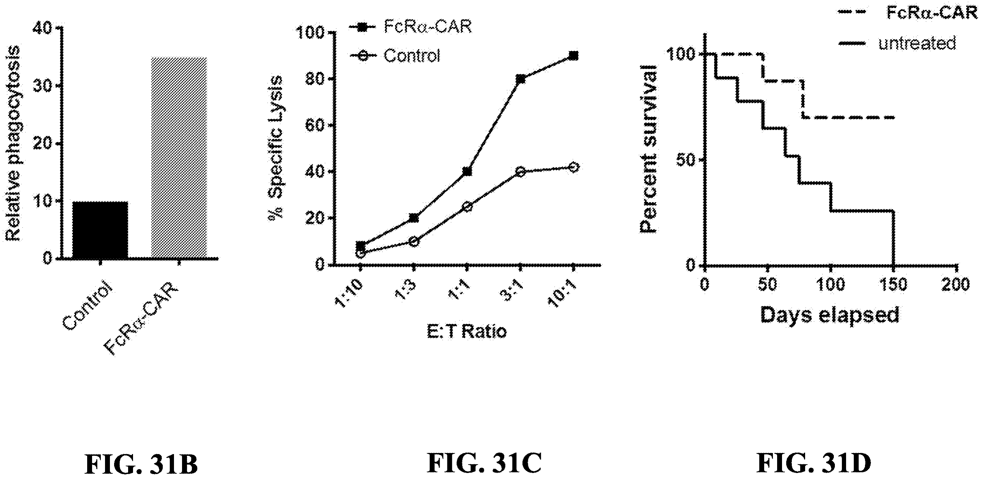

**FIG. 31B** shows the expected result of relative phagocytoses of the dye loaded target tumor cells, quantified by flow cytometry. Cells expressing the CAR-FcRγ exhibit enhanced phagocytosis over cells expressing the control vector. **FIG. 31C** shows the expected result of relative lysis of the dye loaded target tumor cells. Cells expressing FcRγ exhibit higher target cell lysis over cells expressing the control vector. When NSG mice are injected with tumor cells expressing luciferase on day 0. Mice are either untreated or injected with human primary macrophage co-expressing CAR-FcRγ and a survival curve is generated (**Fig. 31D**).

## Example 26. Design and functional analysis of recombinant CFP (CAR-TREM)

In this example, an exemplary design of a vector expressing the cancer antigen targeted receptor with a myeloid cell specific expression is described. In this exemplary construct the extracellular antigen binding domain is a scFv, which can specifically bind to a cancer antigen. The extracellular transmembrane and intracellular domains of a TREM chain are fused with the scFv through a linker (CAR-TREM□. The TREM chain heterodimerizes with endogenous DAP12 transmembrane domain of DAP12 and generates pro-inflammatory signal that promote phagocytosis. **FIG. 32A** shows illustrative schematics of the cancer targeting CAR-TREM.

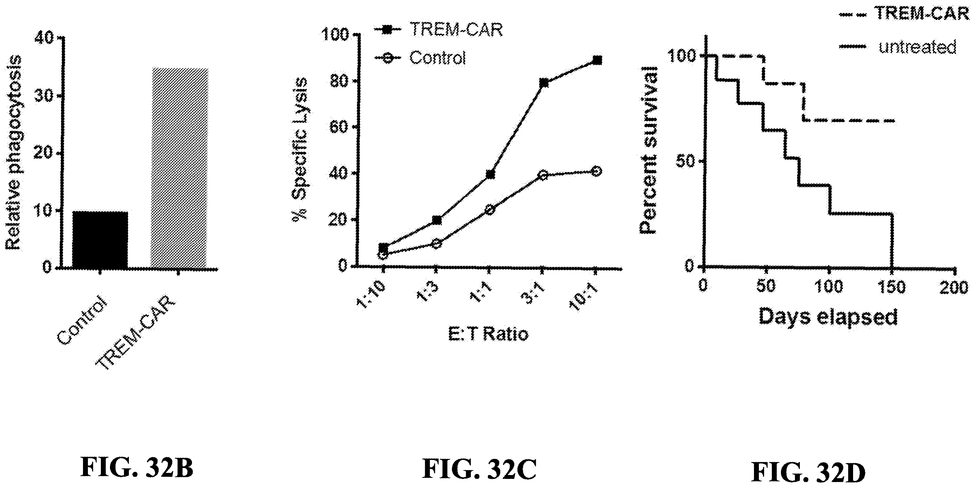

**FIG. 32B** shows the expected result of relative phagocytoses of the dye loaded target tumor cells, quantified by flow cytometry. Cells expressing the CAR-TREM exhibit enhanced phagocytosis over cells expressing the control vector. **FIG. 32C** shows the expected result of relative lysis of the dye loaded target tumor cells. Cells expressing TREM exhibit higher target cell lysis over cells expressing the control vector. When NSG mice are injected with tumor cells expressing luciferase on day 0. Mice are either untreated or injected with human primary macrophage co-expressing CAR-TREM and a survival curve is generated (**FIG. 32D**).

## Example 27. Design and functional analysis of recombinant chimeric protein expressing an intracellular caspase construct.

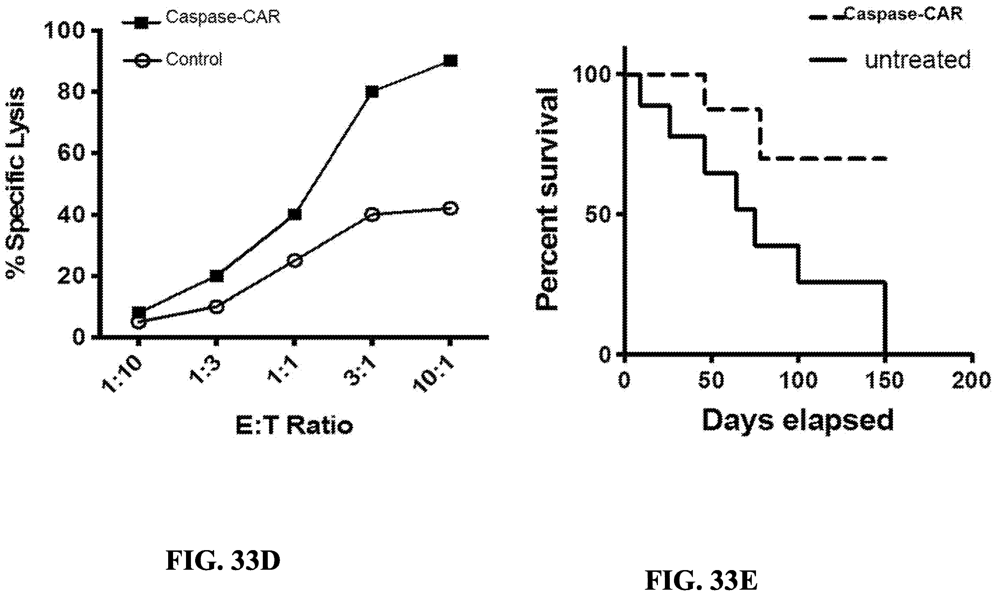

In this example, single construct comprising coding sequences for a CAR having ITAM intracellular domains and a separate coding sequence encoding a fused intracellular protein, a procaspase linked with an SH2 binding domain linked by a caspase cleavage site is designed. The construct is graphically depicted in **FIG. 33A** (upper panel). The fused procaspase coding sequence is spaced from the CAR coding sequence with a T2A domain which cleaves the two proteins after translation at the T2A site. **FIG. 33A** shows a CAR expressing an extracellular scFv specific for binding to a cancer antigen, a transmembrane domain which could be any TM described in the previous examples, and an intracellular ITAM domain comprising SH2 binding domains. As shown in the graphical depiction in **FIG. 33A,** the procaspase portion after translation and release from the remaining construct by cleavage at the T2A site, associates with the intracellular ITAM domain via the SH2 docking site, which comprises the Tyrosine phosphorylated residues at the intracellular ITAM domain of the CAR. Upon binding and phosphorylation of the SH2 domains by the ITAM, the procaspase is activated and is cleaved at the caspase cleavage site, which activates the procaspase to form caspase. This initiates the intracellular signaling pathway for phagocytosis. The construct is expressed in human primary macrophages for functional analysis. **FIGs 33B-33C** show the expected result of induced expression of cytokines and surface antigens when caspase-CAR macrophages are co-cultured with target tumor cells. Human primary macrophage transduced with control empty vector or caspase-CAR are co-cultured with target tumor cells. Cytokine profiling with ELISA shows increased secretion of pro-inflammatory cytokines and chemokines compared to vector control. Flow cytometry shows an increase of pro-inflammatory cell surface marker expression compared with vector control. **Fig. 33D-33E** show the expected result in an in vitro cell lysis assay and in vivo xenograft mouse model respectively. In **FIG. 33D,** human primary macrophage transduced with control vector or caspase-CAR are co-cultured with tumor cells expressing luciferase at different E:T ratio, and specific lysis is quantified using luciferase assay. In Fig. 33E, NSG mice are injected with tumor cells expressing luciferase on day 0. Mice are either untreated or injected with human primary macrophage transduced with caspase-CAR on day 0.

## Example 28. Modular design for vectors including metabolic switch principle.

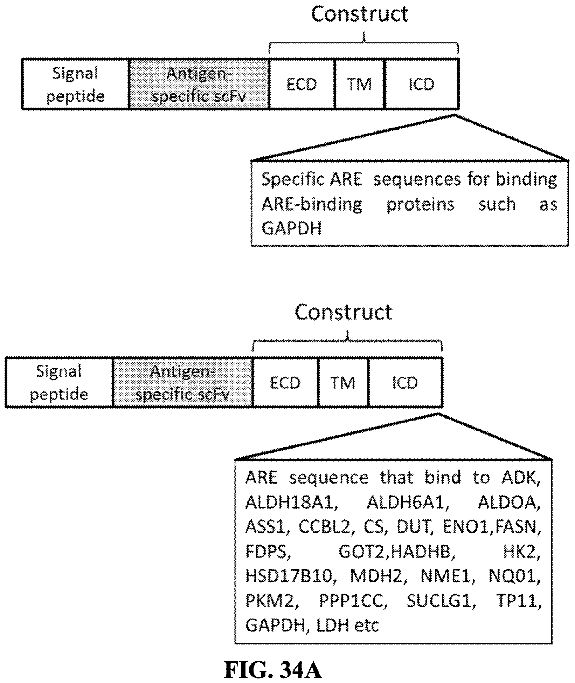

In this example, specific enhancements of the constructs are described. The designs described in this section can be adapted to any of the constructs described in the disclosure, as is understood by one of skill in the art. As shown in **FIG. 34A** (upper panel), the design exemplifies a generic construct of a CAR, with insertion of an AU rich element (ARE) sequence in the 3' UTR that result in RNA binding proteins (eg GAPDH) binding to mature mRNA strand preventing translation. The GAPDH binding sequence can be designated as WWWU(AUUUA)UUUW (SEQ ID NO: 35), where W is A or U. Glycolysis results in the uncoupling of the RNA binding proteins (eg GAPDH) allowing for mRNA strand translation.

Other exemplary ARE sequences can be used to replace the GAPDH binding sequence. Such sequences may be the ARE sequence that bind to ADK, ALDH18A1, ALDH6A1, ALDOA, ASS1, CCBL2, CS, DUT, ENO1,FASN, FDPS, GOT2,HADHB, HK2, HSD17B10, MDH2, NME1, NQ01, PKM2, PPP1CC, SUCLG1, TP11, GAPDH, LDH. The modified ARE is used at the 3'end of the expression constructs for a CARP, as shown in **FIG. 10A** (lower panel).

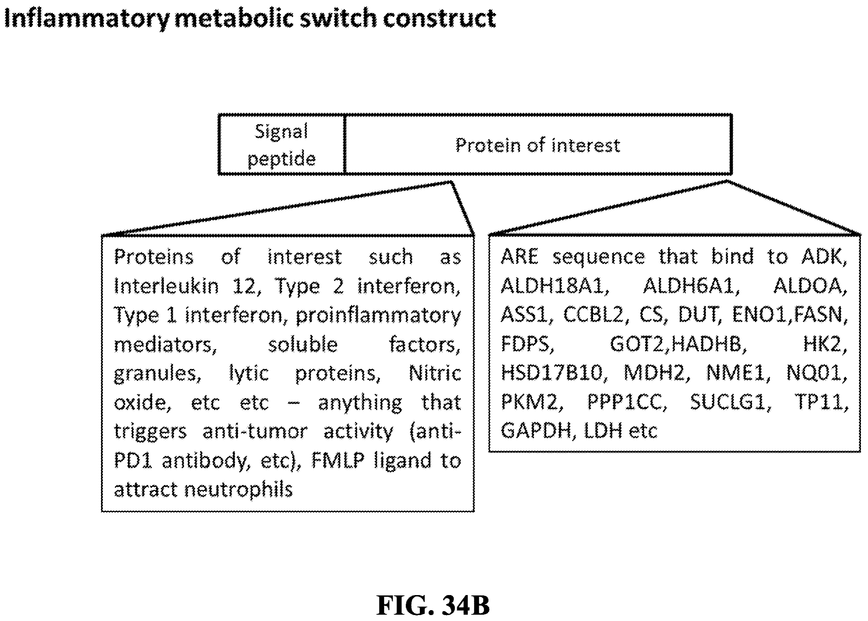

**FIG 34B** exemplifies a generic construct that is expresses a proinflammatory protein, either as part of a monocistronic construct or a polysictronic construct for expression a CARP along with the expression of the proinflammatory protein described herein; or the construct described herein can be used for co-expression with any other chimeric antigenic construct. Not shown in FIG. 34B, are the remaining parts of the construct of the section shown is used as part of the construct encoding the CAR proteins. Any protein can be expressed as "protein of interest," which includes but is not limited to Interleukin 12, Type 2 interferon, Type 1 interferon, proinflammatory mediators, soluble factors, granules, lytic proteins, Nitric oxide, etc etc - anything that triggers anti-tumor activity (anti-PD1 antibody, etc), FMLP ligand to attract neutrophils. The 3'end of the coding sequences contain one or more ARE sequences that can bind to any one of ADK, ALDH18A1, ALDH6A1, ALDOA, ASS1, CCBL2, CS, DUT, ENO1,FASN, FDPS, GOT2,HADHB, HK2, HSD17B10, MDH2, NME1, NQ01, PKM2, PPP1CC, SUCLG1, TP11, GAPDH, LDH etc

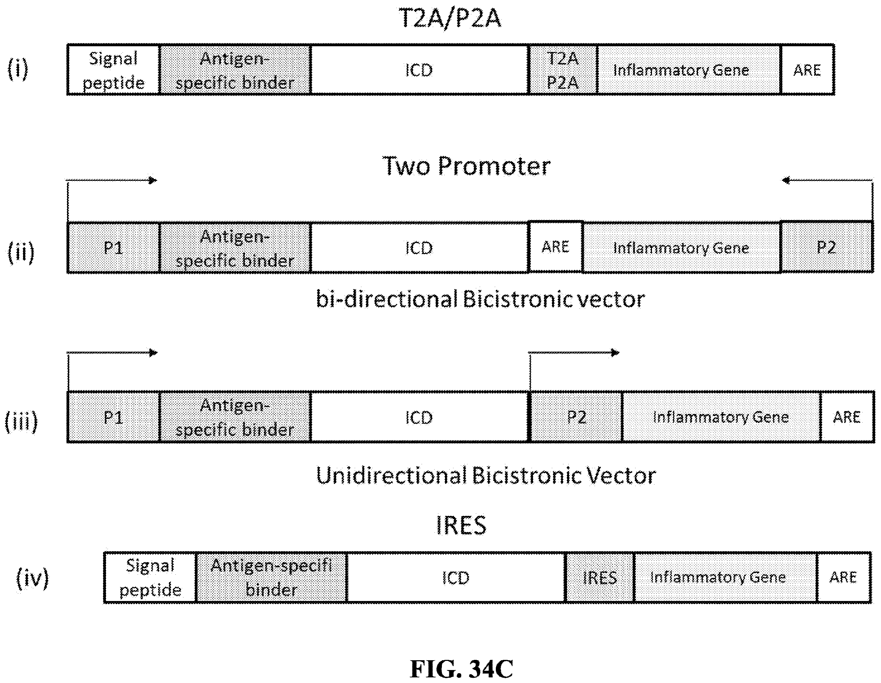

**FIG. 34C** shows several exemplary modular constructs applicable to the designs disclosed herein. **FIG. 34C** depicts an exemplary construct where two or more coding sequences are separated by a T2A or a P2A cleavage site encoded by the construct ((i)). Endogenous proteins cleave the newly translated proteins at the site, releasing two independent proteins, generated from a single construct. **FIG. 34C** also depicts an exemplary bicistronic construct comprising two coding sequences, one for the fused protein comprising the antigen specific binder and an intracellular domain (ICD), under the influence of promoter P1; and the other coding the inflammatory gene under the influence of the second promoter P2, and the 3'regulatory element. The two coding sequences are designed to be in opposite directions to each other ((ii)). **FIG. 34C** also depicts an exemplary design where two distinct genes are encoded by the bi-cistronic vector construct, and are unidirectional ((iii)). **FIG. 34C also** depicts an exemplary design where two genes are encoded by the vector construct, where the second coding sequence is preceded by an IRES construct that ensures independent ribosomal entry sites for independent translation as separate polypeptides, originating from the single nucleic acid construct ((iv)).

This invention can be further defined with reference to the following embodiments:

1. A composition comprising a recombinant nucleic acid encoding a chimeric fusion protein (CFP) comprising:

(a) a phagocytic or tethering receptor (PR) subunit comprising:

(i) a transmembrane domain, and
(ii) an intracellular domain comprising an intracellular signaling domain; and

(b) an extracellular domain comprising an antigen binding domain specific to an antigen of a target cell;

wherein the transmembrane domain and the extracellular domain are operatively linked; wherein the antigen is not a CD19 or CD22 antigen, and wherein upon binding of the CFP to the antigen of the target cell, killing or phagocytosis activity of a cell expressing the CFP is increased by at least 20% compared to a cell not expressing the CFP, wherein killing or phagocytosis activity is measured by flow cytometry.

2. A composition comprising a recombinant nucleic acid encoding a chimeric fusion protein (CFP) comprising:

(a) a phagocytic or tethering receptor (PR) subunit comprising:

(i) a transmembrane domain, and
(ii) an intracellular domain comprising an intracellular signaling domain; and

(b) an extracellular domain comprising an antigen binding domain specific to an antigen of a target cell;

wherein the transmembrane domain and the extracellular domain are operatively linked; and wherein upon binding of the CFP to the antigen of the target cell, killing or phagocytosis activity of a cell expressing the CFP is increased by at least 10 fold compared to a cell not expressing the CFP, wherein killing or phagocytosis activity is calculated as engulfment per 100 cells expressing the CFP.

3. The composition of embodiments 1 or 2, wherein the intracellular signaling domain is derived from a phagocytic or tethering receptor or wherein the intracellular signaling domain comprises a phagocytosis activation domain.

4. The composition of any one of embodiments 1-3, wherein the intracellular signaling domain is derived from a receptor other than a phagocytic receptor selected from Megf10, MerTk, an FcR, or Bai1.

5. The composition of any one of embodiments 1-4, wherein the intracellular signaling domain is derived from a receptor selected from the group consisting of the receptors listed in Table 2.

6. The composition of any one of embodiments 1-5, wherein the intracellular signaling domain comprises a pro-inflammatory signaling domain.

7. The composition of embodiment 6, wherein the intracellular signaling domain comprises a pro-inflammatory signaling domain that is not a PI3K recruitment domain.

8. A composition comprising a recombinant nucleic acid encoding a chimeric fusion protein (CFP) comprising:

 (a) a phagocytic or tethering receptor (PR) subunit comprising:

  (i) a transmembrane domain, and
  (ii) an intracellular domain comprising an intracellular signaling domain; and

 (b) an extracellular domain comprising an antigen binding domain specific to an antigen of a target cell;

wherein the transmembrane domain and the extracellular domain are operatively linked; and wherein the intracellular signaling domain is derived from a phagocytic receptor other than a phagocytic receptor selected from Megf10, MerTk, FcRα, and Bail.

9. The composition of embodiment 8, wherein upon binding of the CFP to the antigen of the target cell, the killing activity of a cell expressing the CFP is increased by at least greater than 20% compared to a cell not expressing the CFP, wherein killing or phagocytosis activity of a cell expressing the CFP is increased by at least 20% compared to a cell not expressing the CFP, wherein killing or phagocytosis activity is measured by flow cytometry.

10. The composition of embodiment 8 or 9, wherein the intracellular signaling domain is derived from protein selected from the group consisting of lectin, dectin 1, CD206, scavenger receptor A1 (SRA1), MARCO, CD36, CD163, MSR1, SCARA3, COLEC12, SCARA5, SCARB1, SCARB2, CD68, OLR1, SCARF1, SCARF2, CXCL16, STAB1, STAB2, SRCRB4D, SSC5D, CD205, CD207, CD209, RAGE, CD14, CD64, F4/80, CCR2, CX3CR1, CSF1R, Tie2, Hu-CRIg(L), CD64, CD32a, CD16a, CD89, Fc-alpha receptor I, CR1, CD35, CD3ζ, CR3, CR4, Tim-1, Tim-4, TNFR1, MDA5, CD40, and CD169.

11. The composition of any one of embodiments 8-10, wherein the intracellular signaling domain comprises a pro-inflammatory signaling domain.

12. A composition comprising a recombinant nucleic acid encoding a chimeric fusion protein (CFP) comprising:

 (a) a phagocytic or tethering receptor (PR) subunit comprising:

  (i) a transmembrane domain, and
  (ii) an intracellular domain comprising an intracellular signaling domain; and

 (b) an extracellular domain comprising an antigen binding domain specific to an antigen of a target cell;

wherein the transmembrane domain and the extracellular domain are operatively linked; and wherein the intracellular signaling domain is derived from a phagocytic receptor selected from the group consisting of lectin, dectin 1, CD206, scavenger receptor A1 (SRA1), MARCO, CD36, CD163, MSR1, SCARA3, COLEC12, SCARA5, SCARB1, SCARB2, CD68, OLR1, SCARF1, SCARF2, CXCL16, STAB1, STAB2, SRCRB4D, SSC5D, CD205, CD207, CD209, RAGE, CD14, CD64, F4/80, CCR2, CX3CR1, CSF1R, Tie2, HuCRIg(L), CD64, CD32a, CD16a, CD89, Fc-alpha receptor I, CR1, CD35, CD3ζ, CR3, CR4, Tim-1, Tim-4, TNFR1, MDA5, CD40, and CD169.

13. The composition of embodiment 12, wherein upon binding of the CFP to the antigen of the target cell, killing or phagocytosis activity of a cell expressing the CFP is increased by at least 55% compared to a cell not expressing the CFP.

14. The composition of embodiment 12 or 13, wherein the intracellular signaling domain is derived from a phagocytic receptor other than a phagocytic receptor selected from Megf10, MerTk, FcRα, or Bai1.

15. The composition of any one of embodiments 12-14, wherein the intracellular signaling domain comprises a pro-inflammatory signaling domain.

16. The composition of embodiment 15, wherein the intracellular signaling domain comprises a pro-inflammatory signaling domain that is not a PI3K recruitment domain.

17. A composition comprising a recombinant nucleic acid encoding a chimeric fusion protein (CFP) comprising:

 (a) a phagocytic or tethering receptor (PR) subunit comprising:

  (i) a transmembrane domain, and

(ii) an intracellular domain comprising an intracellular signaling domain; an

(b) an extracellular domain comprising an antigen binding domain specific to an antigen of a target cell;

wherein the transmembrane domain and the extracellular domain are operatively linked; and wherein the intracellular signaling domain comprises a pro-inflammatory signaling domain that is not a PI3K recruitment domain.

18. A composition comprising a recombinant nucleic acid encoding a chimeric fusion protein (CFP) comprising:

(a) a phagocytic or tethering receptor (PR) subunit comprising:

(i) a transmembrane domain, and
(ii) an intracellular domain comprising an intracellular signaling domain; and

(b) an extracellular domain comprising an antigen binding domain specific to an antigen of a target cell;

wherein the transmembrane domain and the extracellular domain are operatively linked; wherein the intracellular signaling domain comprises a signaling domain derived from a TFNR1 receptor.

19. A composition comprising a recombinant nucleic acid encoding a chimeric fusion protein (CFP) comprising:

(a) a phagocytic or tethering receptor (PR) subunit comprising:

(i) a transmembrane domain, and
(ii) an intracellular domain comprising an intracellular signaling domain; and

(b) an extracellular domain comprising an antigen binding domain specific to an antigen of a target cell;

wherein the transmembrane domain and the extracellular domain are operatively linked; wherein the intracellular signaling domain comprises a signaling domain derived from a CD40 receptor.

20. A composition comprising a recombinant nucleic acid encoding a chimeric fusion protein (CFP) comprising:

(a) a phagocytic or tethering receptor (PR) subunit comprising:

(i) a transmembrane domain, and
(ii) an intracellular domain comprising an intracellular signaling domain; and

(b) an extracellular domain comprising an antigen binding domain specific to an antigen of a target cell;

wherein the transmembrane domain and the extracellular domain are operatively linked; wherein the intracellular signaling domain comprises a signaling domain derived from a MDA5.

21. The composition of any one of embodiments 17-20, wherein upon binding of the CFP to the antigen of the target cell, killing or phagocytosis activity of a cell expressing the CFP is increased by at least greater than 20% compared to a cell not expressing the CFP, wherein killing or phagocytosis activity is measured by flow cytometry.

22. The composition of any one of embodiments 17-21, wherein the intracellular signaling domain is derived from a phagocytic receptor.

23. The composition of any one of embodiments 17-22, wherein the intracellular signaling domain is derived from a phagocytic receptor other than a phagocytic receptor selected from Megf10, MerTk, an FcR, or Bai1.

24. The composition of any one of embodiments 17-23, wherein the intracellular signaling domain is derived from a phagocytic receptor selected from the group consisting of lectin, dectin 1, CD206, scavenger receptor A1 (SRA1), MARCO, CD36, CD163, MSR1, SCARA3, COLEC12, SCARA5, SCARB1, SCARB2, CD68, OLR1, SCARF1, SCARF2, CXCL16, STAB1, STAB2, SRCRB4D, SSC5D, CD205, CD207, CD209, RAGE, CD14, CD64, F4/80, CCR2, CX3CR1, CSF1R, Tie2, HuCRIg(L), CD64, CD32a, CD16a, CD89, Fc-alpha receptor I, CR1, CD35, CD3$\zeta$, CR3, CR4, Tim-1, Tim-4, TNFR1, MDA5, CD40, and CD169.

25. The composition of any one of embodiments 1-15, wherein the intracellular signaling domain comprises a PI3K recruitment domain.

26. The composition of any one of the preceding embodiments, wherein the intracellular signaling domain comprises an FcR$\gamma$ intracellular domain.

27. The composition of embodiment 26, wherein the intracellular signaling domain comprises a FcR$\gamma$ intracellular domain and a CD40 receptor intracellular signaling domain in a N-to-C order.

28. The composition of embodiment 26, wherein the intracellular signaling domain comprises a FcRγ intracellular domain and a CD40 receptor intracellular signaling domain in a C-to-N order.

29. The composition of embodiment 26, wherein the intracellular signaling domain comprises a FcRγ intracellular domain and a TNFR1 receptor intracellular signaling domain in a N-to-C order.

30. The composition of embodiment 26, wherein the intracellular signaling domain comprises a FcRγ intracellular domain and a TNFR1 receptor intracellular signaling domain in a C-to-N order.

31. The composition of embodiment 26, wherein the intracellular signaling domain comprises a FcRγ intracellular domain and an MDA5 signaling domain in a N-to-C order.

32. The composition of embodiment 26, wherein the intracellular signaling domain comprises a FcRγ intracellular domain and an MDA5 signaling domain in a C-to-N order.

33. The composition of embodiment 26, wherein the intracellular signaling domain comprises a FcRγ intracellular domain and a PI3K recruitment domain in a N-to-C order.

34. The composition of embodiment 26, wherein the intracellular signaling domain comprises a FcRγ intracellular domain and a PI3K recruitment domain in a C-to-N order.

35. The composition of any one of the preceding embodiments, wherein the CFP functionally incorporates into a cell membrane of a cell when the CFP is expressed in the cell.

36. The composition of any one of the preceding embodiments, wherein a cell expressing theCFP exhibits an increase in phagocytosis of a target cell expressing the antigen compared to a cell not expressing the CFP.

37. The composition of embodiment 36, wherein a cell expressing the CFP exhibits at least a 1.1-fold increase in phagocytosis of a target cell expressing the antigen compared to a cell not expressing the CFP, wherein phagocytosis is measured by flow cytometry.

38. The composition of any one of the preceding embodiments, wherein a cell expressing the CFP exhibits at least a 1.2-fold, 1.3-fold, 1.4-fold, 1.5-fold, 1.6-fold, 1.7-fold, 1.8-fold, 1.9-fold, 2-fold, 3-fold, 4-fold, 5-fold, 6-fold, 7-fold, 8-fold, 9-fold, 10-fold, 20-fold, 30-fold, 40-fold, or 50-fold increase in phagocytosis of a target cell expressing the antigen compared to a cell not expressing the CFP, wherein phagocytosis is measured by flow cytometry.

39. The composition of any one of the preceding embodiments, wherein the target cell expressing the antigen is a cancer cell.

40. The composition of any one of the preceding embodiments, wherein the target cell expressing the antigen is at least 0.8 microns in diameter.

41. The composition of any one of the preceding embodiments, wherein the intracellular signaling domain is derived from a scavenger receptor.

42. The composition of any one of the preceding embodiments, wherein a cell expressing the CFP exhibits an increase in production of a cytokine compared to a cell not expressing the CFP.

43. The composition according to embodiment 42, wherein the cytokine is selected from the group consisting of IL-1, IL3, IL-6, IL-12, IL-13, IL-23, TNF, CCL2, CXCL9, CXCL10, CXCL11, IL-18, IL-23, IL-27, CSF, MCSF, GMCSF, IL17, IP-10, RANTES, an interferon and combinations thereof.

44. The composition of any one of the preceding embodiments, wherein a cell expressing the CFP exhibits an increase in effector activity compared to a cell not expressing the CFP.

45. The composition of any one of the preceding embodiments, wherein a cell expressing the CFP exhibits an increase in cross-presentation compared to a cell not expressing the CFP.

46. The composition of any one of the preceding embodiments, wherein a cell expressing the CFP exhibits an increase in expression of an MHC class II protein compared to a cell not expressing the CFP

47. The composition of any one of the preceding embodiments, wherein a cell expressing the CFP exhibits an increase in expression of CD80 compared to a cell not expressing the CFP.

48. The composition of any one of the preceding embodiments, wherein a cell expressing the CFP exhibits an increase in expression of CD86 compared to a cell not expressing the CFP.

49. The composition of any one of the preceding embodiments, wherein a cell expressing the CFP exhibits an increase in expression of MHC class I protein compared to a cell not expressing the CFP.

50. The composition of any one of the preceding embodiments, wherein a cell expressing the CFP exhibits an increase in expression of TRAIL/TNF Family death receptors compared to a cell not expressing the CFP.

51. The composition of any one of the preceding embodiments, wherein a cell expressing the CFP exhibits an increase in expression of B7-H2 compared to a cell not expressing the CFP.

52. The composition of any one of the preceding embodiments, wherein a cell expressing the CFP exhibits an increase in expression of LIGHT compared to a cell not expressing the CFP.

53. The composition of any one of the preceding embodiments, wherein a cell expressing the CFP exhibits an increase in expression of HVEM compared to a cell not expressing the CFP.

54. The composition of any one of the preceding embodiments, wherein a cell expressing the CFP exhibits an increase in expression of CD40 compared to a cell not expressing the CFP.

55. The composition of any one of the preceding embodiments, wherein a cell expressing the CFP exhibits an increase in expression of TL1A compared to a cell not expressing the CFP.

56. The composition of any one of the preceding embodiments, wherein a cell expressing the CFP exhibits an increase in expression of 41BBL compared to a cell not expressing the CFP

57. The composition of any one of the preceding embodiments, wherein a cell expressing the CFP exhibits an increase in expression of OX40L compared to a cell not expressing the CFP.

58. The composition of any one of the preceding embodiments, wherein a cell expressing the CFP exhibits an increase in expression of GITRL death receptors compared to a cell not expressing the CFP.

59. The composition of any one of the preceding embodiments, wherein a cell expressing the CFP exhibits an increase in expression of CD30L compared to a cell not expressing the CFP.

60. The composition of any one of the preceding embodiments, wherein a cell expressing the CFP exhibits an increase in expression of TIM4 compared to a cell not expressing the CFP.

61. The composition of any one of the preceding embodiments, wherein a cell expressing the CFP exhibits an increase in expression of TIM1 ligand compared to a cell not expressing the CFP.

62. The composition of any one of the preceding embodiments, wherein a cell expressing the CFP exhibits an increase in expression of SLAM compared to a cell not expressing the CFP.

63. The composition of any one of the preceding embodiments, wherein a cell expressing the CFP exhibits an increase in expression of CD48 compared to a cell not expressing the CFP.

64. The composition of any one of the preceding embodiments, wherein a cell expressing the CFP exhibits an increase in expression of CD58 compared to a cell not expressing the CFP.

65. The composition of any one of the preceding embodiments, wherein a cell expressing the CFP exhibits an increase in expression of CD155 compared to a cell not expressing the CFP.

66. The composition of any one of the preceding embodiments, wherein a cell expressing the CFP exhibits an increase in expression of CD112 compared to a cell not expressing the CFP.

67. The composition of any one of the preceding embodiments, wherein a cell expressing the CFP exhibits an increase in expression of PDL1 compared to a cell not expressing the CFP.

68. The composition of any one of the preceding embodiments, wherein a cell expressing the CFP exhibits an increase in expression of B7-DC compared to a cell not expressing the CFP.

69. The composition of any one of the preceding embodiments, wherein a cell expressing the CFP exhibits an increase in respiratory burst compared to a cell not expressing the CFP.

70. The composition of any one of the preceding embodiments, wherein a cell expressing the CFP exhibits an increase in ROS production compared to a cell not expressing the CFP.

71. The composition of any one of the preceding embodiments, wherein a cell expressing the CFP exhibits an increase in iNOS production compared to a cell not expressing the CFP.

72. The composition of any one of the preceding embodiments, wherein a cell expressing the CFP exhibits an increase in iNOS production compared to a cell not expressing the CFP.

73. The composition of any one of the preceding embodiments, wherein a cell expressing the CFP exhibits an increase in extra-cellular vesicle production compared to a cell not expressing the CFP.

74. The composition of any one of the preceding embodiments, wherein a cell expressing the CFP exhibits an increase in trogocytosis with a target cell expressing the antigen compared to a cell not expressing the CFP.

75. The composition of any one of the preceding embodiments, wherein a cell expressing the CFP exhibits an increase in resistance to CD47 mediated inhibition of phagocytosis compared to a cell not expressing the CFP.

76. The composition of any one of the preceding embodiments, wherein a cell expressing the CFP exhibits an increase in resistance to LILRB1 mediated inhibition of phagocytosis compared to a cell not expressing the CFP.

77. The composition of any one of the preceding embodiments, wherein the intracellular domain comprises a Rac inhibition domain, a Cdc42 inhibition domain or a GTPase inhibition domain.

78. The composition of embodiment 64, wherein the Rac inhibition domain, the Cdc42 inhibition domain or the GTPase inhibition domain inhibits Rac, Cdc42 or GTPase at a phagocytic cup of a cell expressing the CFP.

79. The composition of any one of the preceding embodiments, wherein the intracellular domain comprises an F-actin disassembly activation domain, a ARHGAP12 activation domain, a ARHGAP25 activation domain or a SH3BP1 activation domain

80. The composition of any one of the preceding embodiments, wherein a cell expressing the CFP exhibits an increase in phosphatidylinositol 3,4,5-trisphosphate production.

81. The composition of any one of the preceding embodiments, wherein the extracellular domain comprises an Ig binding domain.

82. The composition of any one of the preceding embodiments, wherein the extracellular domain comprises an IgA, IgD, IgE, IgG, IgM, FcRγI, FcRγIIA, FcRγIIB, FcRγIIC, FcRγIIIA, FcRγIIIB, FcRn, TRIM21, FcRL5 binding domain.

83. The composition of any one of the preceding embodiments, wherein the extracellular domain comprises an FcR extracellular domain.

84. The composition of any one of the preceding embodiments, wherein the extracellular domain comprises an FcR-alpha, FcRβ, FcRε or FcRγ extracellular domain.

85. The composition of any one of the preceding embodiments, wherein the extracellular domain comprises an FcRα (FCAR) extracellular domain.

86. The composition of any one of the preceding embodiments, wherein the extracellular domain comprises an FcRβ extracellular domain.

87. The composition of any one of the preceding embodiments, wherein the extracellular domain comprises an FcRε (FCER1A) extracellular domain.

88. The composition of any one of the preceding embodiments, wherein the extracellular domain comprises an FcRγ (FDGR1A, FCGR2A, FCGR2B, FCGR2C, FCGR3A, FCGR3B) extracellular domain.

89. The composition of any one of the preceding embodiments, wherein the extracellular domain comprises an integrin domain.

90. The composition of any one of the preceding embodiments, wherein the extracellular domain comprises one or more integrin α1, α2, αIIb, α3, α4, α5, α6, α7, α8, α9, α10, α11, αD, αE, αL, αM, αV, αX, β1, β2, β3, β4, β5, β6, β7, or β8 domains.

91. The composition of any one of the preceding embodiments, wherein the intracellular domain comprises a CD47 inhibition domain.

92. The composition of any one of the preceding embodiments, wherein the PR subunit further comprises an extracellular domain operatively clinked to the transmembrane domain and the extracellular antigen binding domain.

93. The composition of embodiment 92, wherein the extracellular domain further comprises an extracellular domain of a receptor, a hinge, a spacer or a linker.

94. The composition of embodiment 93, wherein the extracellular domain comprises an extracellular portion of a PR.

95. The composition of embodiment 94, wherein the extracellular portion of the PR is derived from the same PR as the PR intracellular signaling domain.

96. The composition of any one of the embodiments 92-95, wherein the extracellular domain comprises an extracellular domain of a scavenger receptor or an immunoglobulin domain.

97. The composition of embodiment 96, wherein the immunoglobulin domain comprises an extracellular domain of an immunoglobulin or an immunoglobulin hinge region.

98. The composition of any one of the embodiments 92-97, wherein the extracellular domain comprises a phagocytic engulfment marker.

99. The composition of any one of the embodiments 92-98, wherein the extracellular domain comprises a structure capable of multimeric assembly.

100. The composition of any one of the embodiments 92-98, wherein the extracellular domain comprises a scaffold for multimerization.

101. The composition of any one of the preceding embodiments, wherein the extracellular domain is at least 10, 20, 30, 40, 50, 60, 70, 80, 90, 100, 150, 200, 300, 300, 400, or 500 amino acids in length.

102. The composition of any one of the preceding embodiments, wherein the extracellular domain is at most 500, 400, 300, 200, or 100 amino acids in length.

103. The composition of any one of the preceding embodiments, wherein the extracellular antigen binding domain specifically binds to the antigen of a target cell.

104. The composition of any one of the preceding embodiments, wherein the extracellular antigen binding domain comprises an antibody domain.

105. The composition of any one of the preceding embodiments, wherein the extracellular antigen binding domain comprises a receptor domain, antibody domain, wherein the antibody domain comprises a functional antibody fragment, a single chain variable fragment (scFv), an Fab, a single-domain antibody (sdAb), a nanobody, a $V_H$ domain, a $V_L$ domain, a VNAR domain, a $V_{HH}$ domain, a bispecific antibody, a diabody, or a functional fragment or a combination thereof.

106. The composition of any one of any one of the preceding embodiments, wherein the extracellular antigen binding domain comprises a ligand, an extracellular domain of a receptor or an adaptor.

107. The composition of any one of the preceding embodiments, wherein the extracellular antigen binding domain comprises a single extracellular antigen binding domain that is specific for a single antigen.

108. The composition of any one of any one of the preceding embodiments, wherein the extracellular antigen binding domain comprises at least two extracellular antigen binding domains, wherein each of the at least two extracellular antigen binding domains is specific for a different antigen.

109. The composition of any one of the preceding embodiments, wherein the antigen is a cancer antigen or a pathogenic antigen or an autoimmune antigen.

110. The composition of any one of the preceding embodiments, wherein the antigen comprises a viral antigen.

111. The composition of any one of the preceding embodiments, wherein the antigen is a T-lymphocyte antigen.

112. The composition of any one of the preceding embodiments, wherein the antigen is an extracellular antigen.

113. The composition of any one of the preceding embodiments, wherein the antigen is an intracellular antigen.

114. The composition of any one of the preceding embodiments, wherein the antigen is selected from the group consisting of Thymidine Kinase (TK1), Hypoxanthine-Guanine Phosphoribosyltransferase (HPRT), Receptor Tyrosine Kinase-Like Orphan Receptor 1 (ROR1), Mucin-1, Mucin-16 (MUC16), MUC1, Epidermal Growth Factor Receptor vIII (EGFRvIII), Mesothelin, Human Epidermal Growth Factor Receptor 2 (HER2), Mesothelin, EBNA-1, LEMD1, Phosphatidyl Serine, Carcinoembryonic Antigen (CEA), B-Cell Maturation Antigen (BCMA), Glypican 3 (GPC3), Follicular Stimulating Hormone receptor, Fibroblast Activation Protein (FAP), Erythropoietin-Producing Hepatocellular Carcinoma A2 (EphA2), EphB2, a Natural Killer Group 2D (NKG2D) ligand, Disialoganglioside 2 (GD2), CD2, CD3, CD4, CD5, CD7, CD8, CD19, CD20, CD22, CD24, CD30, CD33, CD38, CD44v6, CD45, CD56CD79b, CD97, CD117, CD123, CD133, CD138, CD171, CD179a, CD213A2, CD248, CD276, PSCA, CS-1, CLECL1, GD3, PSMA, FLT3, TAG72, EPCAM, IL-1, an integrin receptor, PRSS21, VEGFR2, PDGFR-$\beta$, SSEA-4, EGFR, NCAM, prostase, PAP, ELF2M, GM3, TEM7R, CLDN6, TSHR, GPRC5D, ALK, IGLL1, Cutaneous lymphocyte-associated antigen (CLA) and combinations thereof.

115. The composition of any one of the preceding embodiments, wherein the antigen is selected from the group consisting of CD2, CD3, CD4, CD5, CD7, CCR4, CXCR4, CD8, CD30, CD45, CD56 and Cutaneous lymphocyte-associated antigen (CLA).

116. The composition of embodiment 114, wherein the antigen is a CD5 antigen.

117. The composition of embodiment 114, wherein the antigen is a HER2 antigen.

118. The composition of any one of the preceding embodiments, wherein the antigen is an ovarian cancer antigen or a T lymphoma antigen.

119. The composition of any one of the preceding embodiments, wherein the antigen is an integrin receptor.

120. The composition of any one of the preceding embodiments, wherein the antigen is an integrin receptor selected from the group consisting of $\alpha 1$, $\alpha 2$, $\alpha IIb$, $\alpha 3$, $\alpha 4$, $\alpha 5$, $\alpha 6$, $\alpha 7$, $\alpha 8$, $\alpha 9$, $\alpha 10$, $\alpha 11$, $\alpha D$, $\alpha E$, $\alpha L$, $\alpha M$, $\alpha V$, $\alpha X$, $\beta 1$, $\beta 2$, $\beta 3$, $\beta 4$, $\beta 5$, $\beta 6$, $\beta 7$, and $\beta 8$.

121. The composition of any one of the preceding embodiments, wherein the antigen comprises 2 or more antigens.

122. The composition of any one of the preceding embodiments, wherein the transmembrane domain and the extracellular antigen binding domain is operatively linked through a linker.

123. The composition of any one of the preceding embodiments, wherein the transmembrane domain and the extracellular antigen binding domain is operatively linked through a linker such as the hinge region of CD8$\alpha$, IgG1 or IgG4.

124. The composition of any one of the preceding embodiments, wherein the extracellular domain comprises a multimerization scaffold.

125. The composition of any one of the preceding embodiments, wherein the transmembrane domain comprises an FcR transmembrane domain.

126. The composition of any one of the preceding embodiments, wherein the transmembrane domain comprises an FcR-$\varepsilon$ with no more than 20, 10 or 5 modifications transmembrane domain.

127. The composition of any one of the preceding embodiments, wherein the transmembrane domain comprises a transmembrane domain from a syntaxin such as syntaxin 3 or syntaxin 4 or syntaxin 5.

128. The composition of any one of the preceding embodiments, wherein the transmembrane domain comprises a CD8 transmembrane domain.

129. The composition of any one of embodiments 1-127, wherein the transmembrane domain comprises a CD28 transmembrane domain or a CD2 transmembrane domain.

130. The composition of any one of embodiments 1-127, wherein the transmembrane domain comprises a CD68 transmembrane domain.

131. The composition of any one of the preceding embodiments, wherein the transmembrane domain oligomerizes with a transmembrane domain of an endogenous receptor when the CFP is expressed in a cell.

132. The composition of any one of the preceding embodiments, wherein the transmembrane domain oligomerizes with a transmembrane domain of an exogenous receptor when the CFP is expressed in a cell.

133. The composition of any one of the preceding embodiments, wherein the transmembrane domain dimerizes with a transmembrane domain of an endogenous receptor when the CFP is expressed in a cell.

134. The composition of any one of the preceding embodiments, wherein the transmembrane domain dimerizes with a transmembrane domain of an exogenous receptor when the CFP is expressed in a cell.

135. The composition of any one of the preceding embodiments, wherein the transmembrane domain is derived from a protein that is different than the protein from which the intracellular signaling domain is derived.

136. The composition of any one of the preceding embodiments, wherein the transmembrane domain is derived from a protein that is different than the protein from which the extracellular domain is derived.

137. The composition of any one of the preceding embodiments, wherein the transmembrane domain comprises a transmembrane domain of a phagocytic receptor.

138. The composition of any one of the preceding embodiments, wherein the transmembrane domain and the extracellular domain are derived from the same protein.

139. The composition of any one of the preceding embodiments, wherein the transmembrane domain is derived from the same protein as the intracellular signaling domain.

140. The composition of any one of the preceding embodiments, wherein the recombinant nucleic acid encodes a DAP12 recruitment domain.

141. The composition of any one of the preceding embodiments, wherein the transmembrane domain comprises a transmembrane domain that oligomerizes with DAP12.

142. The composition of any one of the preceding embodiments, wherein the transmembrane domain is at least 12, 13, 14, 15, 16, 17,18, 19, 20, 21, 22, 23, 24, 25, 26, 27, 28, 29, 30, 31 or 32 amino acids in length.

143. The composition of any one of the preceding embodiments, wherein the transmembrane domain is at most 12, 13, 14, 15, 16, 17,18, 19, 20, 21, 22, 23, 24, 25, 26, 27, 28, 29, 30, 31 or 32 amino acids in length.

144. The composition of any one of the preceding embodiments, wherein the intracellular domain comprises a phosphatase inhibition domain.

145. The composition of any one of the preceding embodiments, wherein the intracellular domain comprises an ARP2/3 inhibition domain.

146. The composition of any one of the preceding embodiments, wherein the intracellular domain comprises at least one ITAM domain.

147. The composition of any one of the preceding embodiments, wherein the intracellular domain comprises at least 1, 2, 3, 4, 5, 6, 7, 8, 9, or 10 ITAM domains.

148. The composition of any one of the preceding embodiments, wherein the intracellular domain further comprises at least one ITAM domain.

149. The composition of any one of the preceding embodiments, wherein the intracellular domain further comprises at least one ITAM domain select from a group CD3 zeta TCR subunit, CD3 epsilon TCR subunit, CD3 gamma TCR subunit, CD3 delta TCR subunit, TCR zeta chain, Fc epsilon receptor 1 chain, Fc epsilon receptor 2 chain, Fc gamma receptor 1 chain, Fc gamma receptor 2a chain, Fc gamma receptor 2b 1 chain, Fc gamma receptor 2b2 chain, Fc gamma receptor 3a chain, Fc gamma receptor 3b chain, Fc beta receptor 1 chain, TYROBP (DAP12), CD5, CD16a, CD16b, CD22, CD23, CD32, CD64, CD79a, CD79b, CD89, CD278, CD66d, functional fragments thereof, and amino acid sequences thereof having at least one but not more than 20 modifications thereto.

150. The composition of embodiment 149, wherein the at least one ITAM domain comprises a Src-family kinase phosphorylation site.

151. The composition of embodiment 149, wherein the at least one ITAM domain comprises a Syk recruitment domain.

152. The composition of any one of the preceding embodiments, wherein the intracellular domain comprises a F-actin depolymerization activation domain.

153. The composition of any one of the preceding embodiments, wherein the intracellular domain lacks enzymatic activity.

154. The composition of any one of the preceding embodiments, wherein the intracellular domain does not comprise a domain derived from a CD3 zeta intracellular domain.

155. The composition of any one of the preceding embodiments, wherein the intracellular domain comprises a CD47 inhibition domain.

156. The composition of any one of the preceding embodiments, wherein the intracellular signaling domain comprises a domain that activate integrin such as the intracellular region of PSGL-1.

157. The composition of any one of the preceding embodiments, wherein the intracellular signaling domain comprises a domain that activate Rap1 GTPase, such as that from EPAC and C3G.

158. The composition of any one of the preceding embodiments, wherein the intracellular signaling domain are from paxillin.

159. The composition of any one of the preceding embodiments, wherein the intracellular signaling domain activates focal adhesion kinase.

160. The composition of any one of the preceding embodiments, wherein the intracellular signaling domain is derived from a single phagocytic receptor.

161. The composition of any one of the preceding embodiments, wherein the intracellular signaling domain is derived from a single scavenger receptor.

162. The composition of any one of the preceding embodiments, wherein the intracellular domain further comprises a phagocytosis enhancing domain.

163. The composition of any one of the preceding embodiments, wherein the intracellular domain comprises a pro-inflammatory signaling domain.

164. The composition of embodiment 163, wherein the pro-inflammatory signaling domain comprises a kinase activation domain or a kinase binding domain.

165. The composition of embodiment 163 or 164, wherein the pro-inflammatory signaling domain comprises an IL-1 signaling cascade activation domain.

166. The composition of any one of embodiments 163-165, the pro-inflammatory signaling domain comprises an intracellular signaling domain derived from TLR3, TLR4, TLR7, TLR 9, TRIF, RIG-1, MYD88, MAL, IRAK1, MDA-5, an IFN-receptor, an NLRP family member, NLRP1-14, NOD1, NOD2, Pyrin, AIM2, NLRC4, FCGR3A, FCERIG, CD40,Tank-binding kinase (TNK), a caspase domain or a procaspase binding domain or any combination thereof.

167. The composition of any one of the preceding embodiments, wherein the CFP does not comprise a full length intracellular signaling domain.

168. The composition of any one of the preceding embodiments, wherein the intracellular domain is at least 5, 10, 20, 30, 40, 50, 60, 70, 80, 90, 100, 150, 200, 300, 300, 400, or 500 amino acids in length.

169. The composition of any one of the preceding embodiments, wherein the intracellular domain is at most 10, 20, 30, 40, 50, 60, 70, 80, 90, 100, 150, 200, 300, 300, 400, or 500 amino acids in length.

170. The composition of any one of the preceding embodiments, wherein the recombinant nucleic acid encodes an FcRα chain extracellular domain, an FcRα chain transmembrane domain and/or an FcRα chain intracellular domain.

171. The composition of any one of the preceding embodiments, wherein the recombinant nucleic acid encodes an FcRβ chain extracellular domain, an FcRβ chain transmembrane domain and/or an FcRβ chain intracellular domain.

172. The composition of embodiment 170 or 171, wherein the FcRα chain or the FcRβ chain forms a complex with FcRγ when expressed in a cell.

173. The composition of embodiment 172, wherein the FcRα chain or FcRβ chain forms a complex with endogenous FcRγ when expressed in a cell.

174. The composition of any one of embodiments 170-173, wherein the FcRα chain or the FcRβ chain does not incorporate into a cell membrane of a cell that does not express FcRγ.

175. The composition of any one of embodiments 170-174, wherein the CFP does not comprise an FcRα chain intracellular signaling domain.

176. The composition of any one of embodiments 170-175, wherein the CFP does not comprise an FcRβ chain intracellular signaling domain.

177. The composition of any one of the preceding embodiments, wherein the recombinant nucleic acid encodes a TREM extracellular domain, a TREM transmembrane domain and/or a TREM intracellular domain.

178. The composition of embodiment 177, wherein the TREM is TREM1, TREM 2 or TREM 3.

179. A composition comprising a recombinant nucleic acid encoding a phagocytic or tethering receptor (PR) fusion protein (CFP) comprising, from N-terminus to C-terminus:

    (a) an extracellular domain comprising an antigen binding domain specific to an antigen of a target cell; and
    (b) a PR subunit comprising:

        (i) a CD8 transmembrane domain, and
        (ii) an intracellular domain comprising a Fcγ intracellular signaling domain and a CD40 intracellular signaling domain

wherein the transmembrane domain and the extracellular domain are operatively linked by a linker.

180. A composition comprising a recombinant nucleic acid encoding a phagocytic or tethering receptor (PR) fusion protein (CFP) comprising, from N-terminus to C-terminus:

    (a) an extracellular domain comprising an antigen binding domain specific to an antigen of a target cell; and
    (b) a PR subunit comprising:

        (i) a CD8 transmembrane domain, and
        (ii) an intracellular domain comprising a Fcγ intracellular signaling domain and a TNFR1 intracellular signaling domain;

wherein the transmembrane domain and the extracellular domain are operatively linked via a linker.

181. A composition comprising a recombinant nucleic acid encoding a phagocytic or tethering receptor (PR) fusion protein (CFP) comprising, from N-terminus to C-terminus:

    (a) an extracellular domain comprising an antigen binding domain specific to an antigen of a target cell; and

(b) a PR subunit comprising:

(i) a CD8 transmembrane domain, and
(ii) an intracellular domain comprising a Fcy intracellular signaling domain and an MDA5 signaling domain;

wherein the transmembrane domain and the extracellular domain are operatively linked by a linker.

182. The composition of any one of embodiments 179-181, wherein the antigen is a CD5 antigen.

183. The composition of any one of embodiments 179-181, wherein the antigen is a HER2 antigen.

184. The composition of any one of embodiments 179-183, wherein the CFP further comprises a signal peptide.

185. The composition of embodiment 184, wherein the signal peptide is a GMCSF signal peptide.

186. The composition of embodiment 184, wherein the CFP comprises the amino acid sequence of SEQ ID NO: 24.

187. The composition of embodiment 184, wherein the CFP comprises the amino acid sequence of SEQ ID NO: 25.

188. The composition of embodiment 184, wherein the CFP comprises the amino acid sequence of SEQ ID NO: 26.

189. The composition of any one of the preceding embodiments, wherein the recombinant nucleic acid comprises a proinflammatory nucleotide or a polynucleotide sequence encoding a pro-inflammatory polypeptide.

190. The composition of any one of the preceding embodiments, wherein the composition further comprises a pro-inflammatory polypeptide.

191. The composition of embodiment 189 or 190, wherein the pro-inflammatory polypeptide is a chemokine or a cytokine.

192. The composition of embodiment 191, wherein the chemokine is selected from the group consisting of IL-1, IL3, IL5, IL-6, il8, IL-12, IL-13, IL-23, TNF, CCL2, CXCL9, CXCL10, CXCL11, IL-18, IL-23, IL-27, CSF, MCSF, GMCSF, IL17, IP-10, RANTES, an interferon.

193. The composition of embodiment 191, wherein the cytokine is selected from the group consisting of IL-1, IL3, IL5, IL-6, IL-12, IL-13, IL-23, TNF, CCL2, CXCL9, CXCL10, CXCL11, IL-18, IL-23, IL-27, CSF, MCSF, GMCSF, IL17, IP-10, RANTES, an interferon.

194. The composition of embodiment 189, wherein the nucleotide is selected from ATP, ADP, UTP, UDP, and/or UDP-glucose.

195. The composition of any one of the preceding embodiments, wherein the recombinant nucleic acid comprises a sequence encoding a homeostatic regulator of inflammation.

196. The composition of embodiment 195, wherein the homeostatic regulator of inflammation is a sequence in an untranslated region (UTR) of an mRNA.

197. The composition of embodiment 196, wherein the sequence in the UTR is a sequence that binds to an RNA binding protein.

198. The composition of embodiment 196 or 197, wherein translation is inhibited or prevented upon binding of the RNA binding protein to the sequence in an untranslated region (UTR).

199. The composition of embodiment 197 or 198, wherein the sequence in the UTR comprises a consensus sequence of WWWU(AUUUA)UUUW (SEQ ID NO: 35), wherein W is A or U.

200. The composition of any one of the preceding embodiments, wherein the recombinant nucleic acid is expressed on a bicistronic vector.

201. The composition of any one of the preceding embodiments, wherein the target cell is a mammalian cell.

202. The composition of any one of the preceding embodiments, wherein the target cell is a human cell.

203. The composition of any one of the preceding embodiments, wherein the target cell comprises a cell infected with a pathogen.

204. The composition of any one of the preceding embodiments, wherein the target cell is a cancer cell.

205. The composition of any one of the preceding embodiments, wherein the target cell is a cancer cell that is a lymphocyte.

206. The composition of any one of the preceding embodiments, wherein the target cell is a cancer cell that is an ovarian cancer cell.

207. The composition of any one of the preceding embodiments, wherein the target cell is a cancer cell that is an ovarian pancreatic cell.

208. The composition of any one of the preceding embodiments, wherein the target cell is a cancer cell that is an glioblastoma cell.

209. The composition of any one of the preceding embodiments, wherein the recombinant nucleic acid is DNA.

210. The composition of any one of the preceding embodiments, wherein the recombinant nucleic acid is RNA.

211. The composition of any one of the preceding embodiments, wherein the recombinant nucleic acid is mRNA.

212. The composition of any one of the preceding embodiments, wherein the recombinant nucleic acid is a circRNA.

213. The composition of any one of the preceding embodiments, wherein the recombinant nucleic acid is a tRNA.

214. The composition of any one of the preceding embodiments, wherein the recombinant nucleic acid is a microRNA.

215. A vector comprising the recombinant nucleic acid of the composition of any one of embodiments 1-214.

216. The vector of embodiment 215, wherein the vector is viral vector.

217. The vector of embodiment 216, wherein the viral vector is retroviral vector or a lentiviral vector.

218. The vector of any one of embodiments 215-217, wherein the vector further comprises a promoter operably linked to at least one nucleic acid sequence encoding one or more polypeptides.

219. The vector of any one of embodiments 215-218, wherein the vector is polycistronic.

220. The vector of embodiment 218 or 219, wherein each of the at least one nucleic acid sequence is operably linked to a separate promoter.

221. The vector of any one of embodiment 215-220, wherein the vector further comprises one or more internal ribosome entry sites (IRESs).

222. The vector of any one of embodiment 215-221, wherein the vector further comprises a 5'UTR and/or a 3'UTR flanking the at least one nucleic acid sequence encoding one or more polypeptides.

223. The vector of any one of embodiment 215-222, wherein the vector further comprises one or more regulatory regions.

224. A polypeptide encoded by the recombinant nucleic acid of the composition of any one of embodiment 1-214.

225. A cell comprising the composition of any one of embodiment 1-214, the vector of any one of embodiment 216-223 or the polypeptide of embodiment 224.

226. The cell of embodiment 225 wherein the cell is an immature myeloid cell.

227. The cell of embodiment 225, wherein the cell is a unpolarized or undifferentiated myeloid cell.

228. The cell of embodiment 225, wherein the cell is a CD14+/CD16$^{low}$ cell.

229. The cell of embodiment 225, wherein the cell is a CD14+/CD16- cell, a CD14-/CD16+ cell.

230. The cell of embodiment 225, wherein the cell is a phagocytic cell.

231. The cell of embodiment 225, wherein the cell is a stem cell derived cell, myeloid cell, macrophage, a dendritic cell, lymphocyte, mast cell, monocyte, neutrophil, microglia, an eosinophil, a basophil, a myeloid progenitor cell, a mosaic phenotype cell or an astrocyte.

232. The cell of any one of embodiment 225, wherein the cell is an M1 macrophage cell.

233. The cell of any one of embodiment 225, wherein the cell is an M2 macrophage cell.

234. The cell of any one of embodiments 225-233, wherein the cell is an autologous cell.

235. The cell of any one of embodiments 225-233, wherein the cell is an allogeneic cell.

236. A population of modified cells, wherein a plurality of the population of modified cells comprise the composition of any one of embodiments 1-214, the vector of any one of embodiments 216-223 or the polypeptide of embodiment 224.

237. The population of modified cells of embodiment 236, wherein the plurality comprises at least 80% of the population of the population of modified cells.

238. The population of modified cells of embodiment 237, wherein the population of cells have not been enriched.

239. The population of modified cells of embodiment 237 or 238, wherein the population of cells are CD14+/CD16- cells, CD14-/CD16+ cells or CD14+/CD16$^{low}$ cells.

240. The population of modified cells of embodiment 237 or 239, wherein the population of cells are phagocytic cells.

241. A pharmaceutical composition comprising

(a) the composition of any one of embodiments 1-214, the vector of any one of embodiments 216-223 or the polypeptide of embodiment 224, the cell of any one of embodiments 225-235, or the population of cells of any one of embodiments 236-240; and
(b) a pharmaceutically acceptable excipient.

242. The pharmaceutical composition of embodiment 241, further comprising an additional therapeutic agent.

243. The pharmaceutical composition of embodiment 241 or 242, wherein the additional therapeutic agent is selected from the group consisting of a CD47 agonist, an agent that inhibits Rac, an agent that inhibits Cdc42, an agent that inhibits a GTPase, an agent that promotes F-actin disassembly, an agent that promotes PI3K recruitment to the CFP, an agent that promotes PI3K activity, an agent that promotes production of phosphatidylinositol 3,4,5-trisphosphate, an agent that promotes ARHGAP12 activity, an agent that promotes ARHGAP25 activity, an agent that promotes SH3BP1 activity and any combination thereof.

244. The pharmaceutical composition of any one of embodiments 241-243, wherein the pharmaceutically acceptable excipient comprises serum free media, a lipid, or a nanoparticle.

245. A method of treating a disease in a subject in need thereof comprising administering to the subject the pharmaceutical composition of any one of embodiments 241-244.

246. The method of embodiment 245, wherein the disease is cancer.

247. The method of embodiment 246, wherein the cancer is a solid cancer.

248. The method of embodiment 247, wherein the solid cancer is selected from the group consisting of ovarian cancer, suitable cancers include ovarian cancer, renal cancer, breast cancer, prostate cancer, liver cancer, brain cancer, lymphoma, leukemia, skin cancer, pancreatic cancer, colorectal cancer, lung cancer.

249. The method of embodiment 246, wherein the cancer is a liquid cancer.

250. The method of embodiment 249, wherein the liquid cancer is a leukemia or a lymphoma.

251. The method of embodiment 249, wherein the liquid cancer is a T cell lymphoma.

252. The method of embodiment 245, wherein the disease is a T cell malignancy.

253. The method of any one of embodiments 245-252, wherein the method further comprises administering an additional therapeutic agent to the subject.

254. The method of embodiment 253, wherein the additional therapeutic agent is selected from the group consisting of a CD47 agonist, an agent that inhibits Rac, an agent that inhibits Cdc42, an agent that inhibits a GTPase, an agent that promotes F-actin disassembly, an agent that promotes PI3K recruitment to the CFP, an agent that promotes PI3K activity, an agent that promotes production of phosphatidylinositol 3,4,5-trisphosphate, an agent that promotes ARHGAP12 activity, an agent that promotes ARHGAP25 activity, an agent that promotes SH3BP1 activity and any combination thereof.

255. The method of any one of embodiments 245-254, wherein the administering comprises infusing or injecting.

256. The method of any one of embodiments 245-255, wherein the administering comprises administering directly to the solid cancer.

257. The method of any one of embodiments 245-256, wherein the administering comprises administering a circRNA, an mRNA, a viral vector, a particle, a nanoparticle, a liposome-, an exosome or a cell.

258. The method of any one of embodiments 245-257, wherein a CD4+ T cell response or a CD8+ T cell response is elicited in the subject.

259. A method of preparing a cell, the method comprising contacting a cell with the composition of any one of embodiments 1-214, the vector of any one of embodiments 216-223, or the polypeptide of embodiment 224.

260. The method of embodiment 259, wherein contacting comprises transducing.

261. The method of embodiment 260, where transducing comprises chemical transfection, electroporation, nucleofection, or viral infection.

262. A method of preparing a pharmaceutical composition comprising contacting a lipid to the composition of any one of embodiments 1-214, the vector of any one of embodiments 216-223 or the polypeptide of embodiment 224.

263. The method of embodiment 262, where contacting comprises forming a lipid nanoparticle.

264. A method of preparing a pharmaceutical composition comprising contacting an antibody to the composition of any one of embodiments 1-214 or the vector of any one of embodiments 216-223.

265. The method of embodiment 264, where contacting comprises forming a lipid nanoparticle.

**Claims**

1. A pharmaceutical composition comprising a recombinant polynucleic acid, wherein the recombinant polynucleic acid comprises a sequence encoding a chimeric fusion protein (CFP), the CFP comprising:

   (i) an extracellular domain comprising an anti-GPC3 binding domain or an anti-HER2 binding domain, and
   (ii) a transmembrane domain operatively linked to the extracellular domain, wherein the transmembrane domain forms a complex with FcRγ when expressed in a cell.

2. The pharmaceutical composition according to claim 1, wherein the anti-GPC3 binding domain or the anti-HER2 binding domain is an scFv or an sdAb domain.

3. The pharmaceutical composition according to claim 1 or claim 2, wherein the anti-GPC3 binding domain or the anti-HER2 binding domain is an scFv.

4. The pharmaceutical composition according to any preceding claim, wherein the extracellular domain comprises an FcRα extracellular domain or a FcRβ extracellular domain.

5. The pharmaceutical composition according to any preceding claim, wherein the transmembrane domain comprises a transmembrane domain from FcRα or FcRβ.

6. The pharmaceutical composition according to any preceding claim, wherein the CFP further comprises an intracellular domain, optionally wherein the intracellular domain comprises an intracellular domain from FcRα or FcRβ.

7. The pharmaceutical composition according to any preceding claim, wherein

   (i) the anti-GPC3 binding domain binds to GPC3 with an affinity of <250nM; or
   (ii) the anti-HER2 binding domain binds to HER2 with an affinity of <250nM.

8. The pharmaceutical composition according to any preceding claim, wherein the CFP further comprises a signal peptide sequence;

   optionally wherein the signal peptide sequence is a GMCSF signal peptide sequence;
   optionally wherein the GMCSF signal peptide sequence has the sequence according to SEQ ID NO: 17.

9. The pharmaceutical composition according to any preceding claim, wherein the recombinant polynucleic acid is an RNA;

   optionally wherein the RNA is an mRNA;
   optionally wherein the mRNA is a synthetic mRNA or an in vitro transcribed mRNA.

10. The pharmaceutical composition of any preceding claim, wherein the pharmaceutical composition further comprises a pro-inflammatory polypeptide; optionally, wherein the pro-inflammatory polypeptide is a chemokine or a cytokine.

11. The pharmaceutical composition according to any preceding claim, wherein the CFP is expressed on myeloid cells.

12. The pharmaceutical composition according to any preceding claim, wherein the recombinant polynucleic acid is encapsulated by a lipid nanoparticle (LNP).

13. The pharmaceutical composition according to any preceding claim, for use as a medicament to treat a disease or condition.

14. The pharmaceutical composition for use according to claim 13, for use in treating cancer in a human subject in need thereof.

15. The pharmaceutical composition for use according to claim 14, wherein the cancer is an ovarian cancer, a breast cancer, a liver cancer, a lung cancer, or a renal cancer.

FIG. 1A

**FIG. 1B**

Extracellular binding domain (eg anti-CD5 scFV, FAB, VHH, receptors, etc)

Transmembrane domain

Signaling domain 1

Signaling domain 2

**FIG. 2A**

EP 4 678 184 A2

Extracellular binding domain (eg anti-CD5 scFV, FAB, VHH, receptors, etc)

Transmembrane domain

Signaling domain 1

Signaling domain 2

Signaling domain 3

Signaling domain n

FIG. 2B

Phagocytosis domain

P13K recruitment

Pro-inflammation

**FIG. 2D**

Anti-CD5 Targeting

Fcγ
Phagocytosis and Activation

PI3K (a,b,d)
Phagocytosis, Survival in TME
& Inflammatory Activation
(IL6, IL8, MIP1, TNF, SDF)

**FIG. 2C**

**Single Target**

**Multi-Target**

**FIG. 3**

Scavenger receptor fusion protein (SFP)

Scavenger Receptor (SR)

| Signal peptide | Antigen-specific scFv | ECD | TM | ICD |
|---|---|---|---|---|

**FIG. 4A**

**FIG. 4B**

**FIG. 4C**

**FIG. 4D**

**FIG. 4E**

## M1-CAR

**FIG. 5A**

**FIG. 5B**

**FIG. 5C**

**FIG. 5D**

**FIG. 5E**

**FIG. 5F**

**FIG. 5G**

Integrin-CAR

**FIG. 6A**

**FIG. 6B**

**FIG. 6C**

**FIG. 6D**

FIG. 6F

FIG. 6E

**FIG. 7**

FIG. 8

CD5-CD8h-
CD8tm-FcR

CD5-CD8h-
CD8tm-no ICD

CD5-CD28h-
CD28tm-FcR-PI3K

CD5-CD8h-
CD68tm-FcR-PI3K

CD5-CD8tm-
FcR-PI3K

CD5-CD28tm-
FcR-PI3K

CD5-CD68tm-
FcR-PI3K

**FIG. 8 (continued)**

**FIG. 9**

FIG. 10A

FIG. 10B

**FIG. 10C**

FIG. 10D

FIG. 10D (continued)

FIG. 10E

**FIG. 10F**

**FIG. 10G**

**FIG. 10H**

139

**FIG. 11A**

**FIG. 11B**

EP 4 678 184 A2

FIG. 11C

**FIG. 12A**

Mock    CD5-FcR-PI3K

pHRodo

H9-pHrodo

Far-Red    Far-Red

**FIG. 12B**

% of phagocytosis

**FIG. 12C**

FIG. 12D

CD40-FcR

FcR-PI3K

FIG. 12D (continued)

FIG. 13

**FIG. 14**

**FIG. 15A**

FIG. 15B

Vehicle

Regimen 1
(1 x 10$^6$ cells)
1 infusion

Regimen 2
(6 x 10$^6$ cells)
6 infusions

**FIG. 15C**

**FIG. 15D**

FIG. 16A

95% Transfection Efficiency

CD5+
95.2

SSC

1.0M
800K
600K
400K
200K
0

$10^0$  $10^2$  $10^4$  $10^6$

CD5-CFP

**FIG. 16B**

FIG. 16C

**FIG. 16D**

**FIG. 17B**

**FIG. 17A**

FIG. 17C

Extracellular binding domain

Transmembrane domain

Signaling domain 1-FcR

Signaling domain TNFR1 or TNFR2

**FIG. 18A**

FIG. 18B

FIG. 18C

EP 4 678 184 A2

FIG. 18C (continued)

Extracellular binding domain

Transmembrane domain

Signaling domain 1-FcR

Signaling domain CD40, PI3K recruitment domain, TNFR2

**FIG. 19A**

IL4, IL10, TGFβ

M2 polarized ATAK monocytes

Antigen-coated plate

Incubation with M2 condition

Measure cytokine secretion

**FIG. 19B**

EP 4 678 184 A2

FIG. 19C

FIG. 19C (continued)

FIG. 19C (continued)

FIG. 20A

**FIG. 20B**

**FIG. 20C**

**FIG. 20D**

EP 4 678 184 A2

**FIG. 20E**

**FIG. 21A**

**FIG. 21B**

EP 4 678 184 A2

FIG. 21C

**FIG. 21C (continued)**

FIG. 22A

FIG. 22B

**FIG. 22C**

**FIG. 23**

EP 4 678 184 A2

**Tumour Size**

FIG. 24

**FIG. 25**

EP 4 678 184 A2

**FIG. 26A**

EP 4 678 184 A2

FIG. 26D

FIG. 26C

FIG. 26B

FIG. 27A

EP 4 678 184 A2

**FIG. 27B**

**FIG. 27C**

**FIG. 27D**

**FIG. 27E**

FIG. 28A

**FIG. 28B**

Vector control
SiRPa_M1

Fold increase

IL6  IL12  IL23  TNF  CXCL9  CXCL9  CXCL10  CXCL11

**FIG. 28C**

Vector control
SiRPa_M1

Fold increase

MHCII  CD80  CD86  iNOS

FIG. 28E

FIG. 28D

**FIG. 29A**

FIG. 29D

FIG. 29C

FIG. 29B

## CARP

| Signal peptide | Antigen-specific scFv | ECD | TM | ICD | T2A | Signal peptide | Sialidase |

**FIG. 30A**

Sialidase

**FIG. 30B**

**FIG. 30C**

FIG. 30D

NFkB activation leads to Sialidase secretion

Sialidase

FIG. 30E

| Signal peptide | Sialidase | ARE sequence |
|---|---|---|

ARE sequence inserted at end of mRNA sequence.

**FIG. 30F**

Metabolic switch

Sialidase

**FIG. 30G**

EP 4 678 184 A2

FcRα-CAR

| Signal peptide | Antigen-specific scFv | ECD | TM | ICD |

FcR alpha chain

Cancer cell

Cancer antigen

scFv

FcR alpha chain

Endogenous
FcR gamma chain

Phagocytosis

Macrophage

**FIG. 31A**

**FIG. 31B**

**FIG. 31C**

**FIG. 31D**

FIG. 32A

EP 4 678 184 A2

**FIG. 32B**

**FIG. 32C**

**FIG. 32D**

EP 4 678 184 A2

# Caspase-CAR

| Signal peptide | Antigen-specific scFv | CD8-hinge | CD8 -TM | ITAM-containing signaling domain | T2A | SH2 | Caspase cleavage sequence | Procaspase1/4/5 |
|---|---|---|---|---|---|---|---|---|

**FIG. 33A**

**FIG. 33B**

**FIG. 33C**

EP 4 678 184 A2

**FIG. 33D**

**FIG. 33E**

EP 4 678 184 A2

FIG. 34A

## Inflammatory metabolic switch construct

| Signal peptide | Protein of interest |
|---|---|

| Proteins of interest such as Interleukin 12, Type 2 interferon, Type 1 interferon, proinflammatory mediators, soluble factors, granules, lytic proteins, Nitric oxide, etc etc – anything that triggers anti-tumor activity (anti-PD1 antibody, etc), FMLP ligand to attract neutrophils | ARE sequence that bind to ADK, ALDH18A1, ALDH6A1, ALDOA, ASS1, CCBL2, CS, DUT, ENO1,FASN, FDPS, GOT2,HADHB, HK2, HSD17B10, MDH2, NME1, NQO1, PKM2, PPP1CC, SUCLG1, TP11, GAPDH, LDH etc |

**FIG. 34B**

## T2A/P2A

(i)

| Signal peptide | Antigen-specific binder | ICD | T2A P2A | Inflammatory Gene | ARE |
|---|---|---|---|---|---|

## Two Promoter

(ii)

| P1 | Antigen-specific binder | ICD | ARE | Inflammatory Gene | P2 |
|---|---|---|---|---|---|

bi-directional Bicistronic vector

(iii)

| P1 | Antigen-specific binder | ICD | P2 | Inflammatory Gene | ARE |
|---|---|---|---|---|---|

Unidirectional Bicistronic Vector

## IRES

(iv)

| Signal peptide | Antigen-specifi binder | ICD | IRES | Inflammatory Gene | ARE |
|---|---|---|---|---|---|

**FIG. 34C**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 62841190
- US 62841183
- US 82738120
- US 82730220
- WO 9301161 A
- US 5571894 A
- US 5869046 A
- US 6248516 B
- US 5587458 A
- EP 404097 A
- WO 9311161 A

- WO 9425591 A
- US 20030130496 A
- US 5641870 A
- US 20170204422 A
- US 5766903 A
- US 5580859 A
- US 5773244 A
- US 6210931 B
- WO 1992001813 A
- US 2012060293 A

### Non-patent literature cited in the description

- **WAHL, G. M** ; **S. L. BERGER**. *Methods Enzymol.*, 1987, vol. 152, 399
- **KIMMEL, A. R.** *Methods Enzymol.*, 1987, vol. 152, 507
- **BENTON** ; **DAVIS**. *Science*, 1977, vol. 196, 180
- **GRUNSTEIN** ; **HOGNESS**. *Proc. Natl. Acad. Sci*, 1975, vol. 72, 3961
- **AUSUBEL et al.** Current Protocols in Molecular Biology. Wiley Interscience, 2001
- **BERGER** ; **KIMMEL**. Guide to Molecular Cloning Techniques. Academic Press, 1987
- **SAMBROOK et al.** Molecular Cloning: A Laboratory Manual. Cold Spring Harbor Laboratory Press
- **RUSSELL, D.G.** *Immunol. Rev*, 2011, vol. 240, 252-268
- **CALDERWOOD DA**. Integrin Activation. *Journal of Cell Science*, 2004, vol. 117, 657-666
- **HUDSON et al.** *Nat. Med*, 2003, vol. 9, 129-134
- **PLUCKTHIIN**. *The Pharmacology of Monoclonal Antibodies*, 1994, vol. 113, 269-315
- **HOLLINGER et al.** *Proc. Natl. Acad. Sci. USA*, 1993, vol. 90, 6444-6448
- Pluckthun in The Pharmacology of Monoclonal Antibodies. Springer-Verlag, 1994, vol. 113, 269-315
- **GUILINGER JP** ; **THOMPSON DB** ; **LIU DR**. Fusion of catalytically inactive Cas9 to FokI nuclease improves the specificity of genome modification. *Nat. Biotechnol.*, 2014, vol. 32 (6), 577-82

- **MICHAEL FINNEY** ; **PAUL E. NISSON**. *Ayoub Rashtchian in Current Protocols in Molecular Biology*, 01 November 2001, vol. 56 (1)
- **JAEHONG PARK** ; **JOSHUA LABAER**. Recombinational Cloning. *Current Protocols in Molecular Biology*, 15 May 2006, vol. 74 (1)
- **KWOH et al.** *Transcription-based Amplification System*, 1989
- **GUATELLI et al.** *Self-Sustained Sequence Replication*, 1990
- **KIEVITIS et al.** *Nucleic Acid Sequence Based Amplification*, 1991
- **WALKER et al.** *Strand Displacement Amplification*, 1992
- **HSU et al.** *Nature*, 1979, vol. 280, 339-340
- **HARLAND** ; **MISHER**. *Development*, 1988, vol. 102, 837-852
- **MEMCZAK et al.** *Nature*, 2013, vol. 495, 333-338
- **JECK et al.** *RNA*, 2013, vol. 19, 141-157
- **GETTS et al.** *Trends Immunol*, 2015, vol. 36 (7), 419-427
- **MEDZIHRADSZKY KF** ; **CHALKLEY RJ**. *Mass Spectrom Rev.*, January 2015, vol. 34 (1), 43-63
- **GETTS et al.** *Trends Immunol.*, 2015, vol. 36 (7), 419-427
- **FLYNN et al.** *BioRxiv*, 2019, 787614
- **KOWALSKI et al.** *Mol. Ther.*, 2019, vol. 27 (4), 710-728